# EUROPÄISCHE PATENTSCHRIFT

(11) **EP 3 169 662 B1**
(45) Veröffentlichungstag und Bekanntmachung des Hinweises auf die Patenterteilung: **11.07.2018**
(21) Anmeldenummer: 15736514.9
(22) Anmeldetag: 14.07.2015
(51) Int. Cl.: C07C 255/46, C07C 255/51, C07D 305/08, C07D 213/80, C07D 213/57, C07D 211/60, C07D 207/16, C07C 311/08, C07C 311/14, C07C 311/16, C07C 311/20, C07C 323/03, C07F 5/02, C07F 7/00, C07F 7/18, C07F 7/22

(54) **SUBSTITUIERTE VINYL- UND ALKINYL-CYANOCYCLOALKANOLE SOWIE VINYL- UND ALKINYL-CYANOHETEROCYCLYLALKANOLE ALS WIRKSTOFFE GEGEN ABIOTISCHEN PFLANZENSTRESS**
SUBSTITUTED VINYL- AND ALKINYL-CYANOCYCLOALKANOLS AND VINYL- AND ALKINYL-CYANOHETEROCYCLOALKANOLS AS AGENTS TO COMBAT ABIOTIC PLANT STRESS
VINYL- ET ALKINYL-CYANOCYCLOALCANOLS SUBSTITUÉS AINSI QUE VINYL- ET ALKINYL-CYANOHÉTÉROCYCLOALCANOLS EN TANT QU'AGENTS ACTIFS CONTRE LE STRESS ABIOTIQUE DES PLANTES

(30) Priorität: 18.07.2014 EP 14177722
(43) Veröffentlichungstag der Anmeldung: 24.05.2017
(73) Patentinhaber: Bayer CropScience Aktiengesellschaft, 40789 Monheim am Rhein (DE)
(72) Erfinder: FRACKENPOHL, Jens, 60322 Frankfurt (DE); WILLMS, Lothar, 65719 Hofheim (DE); DITTGEN, Jan, 60316 Frankfurt (DE); RUIZ-SANTAELLA MORENO, Juan Pedro, 51371 Leverkusen (DE); SCHMUTZLER, Dirk, 65795 Hattersheim (DE); HILLS, Martin Jeffrey, 65510 Idstein (DE)
(74) Vertreter: BIP Patents
(86) Internationale Anmeldenummer: PCT/EP2015/066010
(87) Internationale Veröffentlichungsnummer: WO 2016/008862

(56) Entgegenhaltungen:
- WO-A1-2012/139891
- WO-A1-2014/086723
- US-A1- 2010 160 166

## Beschreibung

Die Erfindung betrifft substituierte Vinyl- und Alkinyl-cyanocycloalkanole sowie Vinyl- und Alkinyl-cyanoheterocyclylalkanole, Verfahren zu deren Herstellung und ihre Verwendung zur Steigerung der Stresstoleranz in Pflanzen gegenüber abiotischem Stress, zur Stärkung des Pflanzenwachstums und/oder zur Erhöhung des Pflanzenertrags.

Es ist bekannt, dass bestimmte 5-(1,2-Epoxy-2,6,6-trimethylcyclohexyl)-3-methylpenta-2,4-diensäuren und ihre Derivate Eigenschaften besitzen, die den Pflanzenwuchs beeinflussen (vgl. NL6811769). Der wuchsbeeinflussende Effekt bestimmter 1,2-Epoxyanaloga von Abscisinsäure auf Reissetzlinge wird außerdem in Agr. Biol. Chem. 1969, 33, 1357 und Agr. Biol. Chem. 1970, 34, 1393 beschrieben. Die Verwendung von substituierten 5-Cyclohex-2-en-1-ylpenta-2,4-dienyl- und 5-Cyclohex-2-en-1-ylpent-2-en-4-inyl-olen, -thioethern und aminen als Inhibitoren der Epoxycarotenoiddioxygenase und als Keimungsinhibitoren wird in US2010/0160166 beschrieben. Die Herstellung von bestimmten Abscisinsäurederivaten mit 3-Methylsubstituent in der 2,4-Pentadiensäureeinheit und ihre Verwendung zur Beeinflussung der Keimung und des Pflanzenwuchses wird in US5518995 und EP0371882 beschrieben. Es ist weiter bekannt, dass bestimmte Abscisinsäurederivate mit 3-Methylsubstituent zur Erhöhung der Toleranz von Pflanzen gegenüber niedrigen Temperaturen verwendet werden können (vgl. WO94/15467). Die Erhöhung der Ausbeute an Sojabohnensamen durch Verwendung eines Gemisches aus Abscisinsäure und einem geeigneten Dünger wird in US4581057 beschrieben.

Es ist ebenfalls bekannt, daß 5-(Cyclohex-2-en-1-yl)-3-methylpenta-2,4-diensäurederivate mit ungesättigten Substituenten an Position C6 der 5-Cyclohex-2-en-1-yl-Einheit den Wasserhaushalt und die Keimung von Pflanzen beeinflussen können (vgl. WO97/23441). Weiterhin sind Trifluormethyl-, Alkyl- und Methoxymethyl-Substituenten an Position C6 der 5-Cyclohex-2-en-1-yl-Einheit in 5-(Cyclohex-2-en-1-yl)-3-methylpenta-2,4-diensäuren beschrieben (vgl. Biosci. Biotech. Biochem. 1994, 58, 707; Biosci. Biotech. Biochem. 1995, 59, 699; Phytochem. 1995, 38, 561; Bioorg. Med. Chem. Lett. 1995, 5, 275). Bicyclische Tetralon-basierte 3-Methylpenta-2,4-diensäurederivate sind in WO2005/108345 beschrieben.

Die Herstellung von 2-[(E)-2-(1-Hydroxy-2,6,6-trimethyl-4-oxocyclohex-2-en-1-yl)vinyl]benzoesäure, dem analogen Methylester Methyl-2-[(E)-2-(1-hydroxy-2,6,6-trimethyl-4-oxocyclohex-2-en-1-yl)vinyl]benzoat sowie den entsprechenden Hydroxymethyl- und Aldehydvorstufen und deren Wirkung auf das Keimungsverhalten von Salatkeimen ist in Agric. Biol. Chem. 1986, 50, 1097 beschrieben. 2-[(E)-2-(1-Hydroxy-2,6,6-trimethyl-4-oxocyclohex-2-en-1-yl)vinyl]benzoesäure wird ebenfalls in Biosci. Biotech. Biochem. 1992, 56, 624 beschrieben. WO2014/086723 beschreibt die Verwendung substituierter 1-(Arylethinyl)-, 1-(Heteroarylethinyl)-, 1-(Heterocyclylethinyl)- und 1-(Cyloalkenylethinyl)- bicycloalkanole oder deren Salze zur Steigerung der Stresstoleranz in Pflanzen gegenüber abiotischem Stress und/ oder zur Erhöhung des Pflanzenertrags.

WO2012/139891 offenbart ebenfalls Substituierte Vinyl- und Alkinyl-cyclohexenole als Wirkstoffe gegen abiotischen Pflanzenstress.

Es ist ebenfalls bekannt, daß substituierte 1-(Phenylethinyl)-cyclohexanole mit bestimmten Substituentengruppen in der meta-Position des Phenylrestes als Wirkstoffe in der Augenheilkunde verwendet werden können (vgl. WO2009/005794, WO2009/058216). Weiterhin ist die Herstellung von bestimmten substituierten 1-(Phenylethinyl)-cyclohexanolen beschrieben, z. B. 1-[(4-Methoxyphenyl)ethinyl]-2,2,6-trimethylcyclohexanol und 1-[(3-Isopropyl-4-methoxyphenyl)ethinyl]-2,2,6-trimethylcyclohexanol (vgl. Bioorg. Med. Chem. 2007, 15, 2736), 2-Methyl-1-(phenylethinyl)cyclohexanol (Org. Lett. 2005, 7, 1363), 1-[(3-Methoxyphenyl)ethinyl]-2,6-dimethylcyclohexanol und 1-(Phenylethinyl)-2,6-dimethylcyclo-hexanol (Can. J. Chem. 1973, 51, 3620), Ethyl-2-hydroxy-1,3-dimethyl-2-(phenylethinyl)cyclohexancarboxylat und Ethyl-2-hydroxy-2-[(3-isopropylphenyl)ethinyl]-1,3-dimethylcyclohexancarboxylat (J. Am. Chem. Soc. 1954, 76, 5380; Synlett 2005, 2919). Weiterhin sind nur wenige Cyanocycloalkyl-substituierte Alkenole vorbeschrieben wie beispielsweise 1-[(2E)-1-Hydroxy-1,3-diphenylprop-2-en-1-yl]cyclopentancarbonitril (in Dokl. Bolgarsk. Akad. Nauk 1971, 24, 621) und 1-[(2E)-1-Hydroxy-3-phenylprop-2-en-1-yl]cyclohexancarbonitril (in J. Organomet. Chem. 1973, 57, C36-C38).

Von den erfindungsgemäßen substituierten Vinyl- und Alkinyl-cyanocycloalkanolen sowie Vinyl- und Alkinyl-cyanoheterocyclylalkanolen ist dagegen die Verwendung zur Steigerung der Stresstoleranz in Pflanzen gegenüber abiotischem Stress, zur Stärkung des Pflanzenwachstums und/oder zur Erhöhung des Pflanzenertrags nicht beschrieben.

Es ist bekannt, dass Pflanzen auf natürliche Stressbedingungen, wie beispielsweise Kälte, Hitze, Trockenstress (Stress verursacht durch Trockenheit und/oder Wassermangel), Verwundung, Pathogenbefall (Viren, Bakterien, Pilze, Insekten) etc. aber auch auf Herbizide mit spezifischen oder unspezifischen Abwehrmechanismen reagieren können [Pflanzenbiochemie, S. 393-462 , Spektrum Akademischer Verlag, Heidelberg, Berlin, Oxford, Hans W. Heldt, 1996.; Biochemistry and Molecular Biology of Plants, S. 1102-1203, American Society of Plant Physiologists, Rockville, Maryland, eds. Buchanan, Gruissem, Jones, 2000].

In Pflanzen sind zahlreiche Proteine und die sie codierenden Gene bekannt, die an Abwehrreaktionen gegen abiotischen Stress (z.B. Kälte, Hitze, Trockenheit, Salz, Überflutung) beteiligt sind. Diese gehören teilweise zu Signaltransduktionsketten (z.B. Transkriptionsfaktoren, Kinasen, Phosphatasen) oder bewirken eine physiologische Antwort der Pflanzenzelle (z.B. Ionentransport, Entgiftung reaktiver Sauerstoff-Spezies). Zu den Signalkettengenen der abiotischen Stressreaktion gehören u.a. Transkriptionsfaktoren der Klassen DREB und CBF (Jaglo-Ottosen et al., 1998, Science 280: 104-106). An der Reaktion auf Salzstress sind Phosphatasen vom Typ ATPK und MP2C beteiligt. Ferner wird bei Salzstress häufig die Biosynthese von Osmolyten wie Prolin oder Sucrose aktiviert. Beteiligt sind hier z.B. die Sucrose-Synthase und Prolin-Transporter (Hasegawa et al., 2000, Annu Rev Plant Physiol Plant Mol Biol 51: 463-499). Die Stressabwehr der Pflanzen gegen Kälte und Trockenheit benutzt z.T. die gleichen molekularen Mechanismen. Bekannt ist die Akkumulation von sogenannten Late Embryogenesis Abundant Proteins (LEA-Proteine), zu denen als wichtige Klasse die Dehydrine gehören (Ingram and Bartels, 1996, Annu Rev Plant Physiol Plant Mol Biol 47: 277-403, Close, 1997, Physiol Plant 100: 291-296). Es handelt sich dabei um Chaperone, die Vesikel, Proteine und Membranstrukturen in gestressten Pflanzen stabilisieren (Bray, 1993, Plant Physiol 103: 1035-1040). Außerdem erfolgt häufig eine Induktion von Aldehyd-Deydrogenasen, welche die bei oxidativem Stress entstehenden reaktiven Sauerstoff-Spezies (ROS) entgiften (Kirch et al., 2005, Plant Mol Biol 57: 315-332). Heat Shock Faktoren (HSF) und Heat Shock Proteine (HSP) werden bei Hitzestress aktiviert und spielen hier als Chaperone eine ähnliche Rolle wie die Dehydrine bei Kälte- und Trockenstress (Yu et al., 2005, Mol Cells 19: 328-333).

Eine Reihe von pflanzenendogenen Signalstoffen, die in die Stresstoleranz bzw. die Pathogenabwehr involviert sind, sind bereits bekannt. Zu nennen sind hier beispielsweise Salicylsäure, Benzoesäure, Jasmonsäure oder Ethylen [Biochemistry and Molecular Biology of Plants, S. 850-929, American Society of Plant Physiologists, Rockville, Maryland, eds. Buchanan, Gruissem, Jones, 2000]. Einige dieser Substanzen oder deren stabile synthetische Derivate und abgeleitete Strukturen sind auch bei externer Applikation auf Pflanzen oder Saatgutbeizung wirksam und aktivieren Abwehrreaktionen, die eine erhöhte Stress- bzw. Pathogentoleranz der Pflanze zur Folge haben [Sembdner, and Parthier, 1993, Ann. Rev. Plant Physiol. Plant Mol. Biol. 44: 569-589].

Es ist weiter bekannt, dass chemische Substanzen die Toleranz von Pflanzen gegen abiotischen Stress erhöhen können. Derartige Substanzen werden dabei entweder durch Saatgut-Beizung, durch Blattspritzung oder durch Bodenbehandung appliziert. So wird eine Erhöhung der abiotischen Stresstoleranz von Kulturpflanzen durch Behandlung mit Elicitoren der Systemic Acquired Resistance (SAR) oder Abscisinsäure-Derivaten beschrieben (Schading and Wei, WO2000/28055; Churchill et al., 1998, Plant Growth Regul 25: 35-45). Desweiteren wurden Effekte von Wachstumsregulatoren auf die Stresstoleranz von Kulturpflanzen beschrieben (Morrison and Andrews, 1992, J Plant Growth Regul 11: 113-117, RD-259027). In diesem Zusammenhang ist ebenfalls bekannt, dass ein wachstumsregulierendes Naphthylsulfonamid (4-Brom-N-(pyridin-2-ylmethyl)naphthalin-1-sulfonamid) die Keimung von Pflanzensamen in der gleichen Weise wie Abscisinsäure beeinflusst (Park et al. Science 2009, 324, 1068-1071). Außerdem ist bekannt, dass ein weiteres Naphthylsulfonamid, N-(6-aminohexyl)-5-chlornaphthalin-1-sulfonamid, den Calcium-Spiegel in Pflanzen beeinflusst, die einem Kälteschock ausgesetzt wurden (Cholewa et al. Can. J. Botany 1997, 75, 375-382).

Auch bei Anwendung von Fungiziden, insbesondere aus der Gruppe der Strobilurine oder der Succinat Dehydrogenase Inhibitoren werden ähnliche Effekte beobachtet, die häufig auch mit einer Ertragssteigerung einhergehen (Draber et al., DE3534948, Bartlett et al., 2002, Pest Manag Sci 60: 309). Es ist ebenfalls bekannt, dass das Herbizid Glyphosat in niedriger Dosierung das Wachstum einiger Pflanzenarten stimuliert (Cedergreen, Env. Pollution 2008, 156, 1099).

Bei osmotischem Stress ist eine Schutzwirkung durch Applikation von Osmolyten wie z.B. Glycinbetain oder deren biochemischen Vorstufen, z.B. Cholin-Derivate beobachtet worden (Chen et al., 2000, Plant Cell Environ 23: 609-618, Bergmann et al., DE4103253). Auch die Wirkung von Antioxidantien wie z.B Naphtole und Xanthine zur Erhöhung der abiotischen Stresstoleranz in Pflanzen wurde bereits beschrieben (Bergmann et al., DD277832, Bergmann et al., DD277835). Die molekularen Ursachen der Anti-Stress-Wirkung dieser Substanzen sind jedoch weitgehend unbekannt.

Es ist weiter bekannt, dass die Toleranz von Pflanzen gegenüber abiotischem Stress durch eine Modifikation der Aktivität von endogenen Poly-ADP-ribose Polymerasen (PARP) oder Poly-(ADP-ribose) glycohydrolasen (PARG) erhöht werden kann (de Block et al., The Plant Journal, 2004, 41, 95; Levine et al., FEBS Lett. 1998, 440, 1; WO2000/04173; WO2004/090140).

Somit ist bekannt, dass Pflanzen über mehrere endogene Reaktionsmechanismen verfügen, die eine wirksame Abwehr gegenüber verschiedensten Schadorganismen und/oder natürlichem abiotischem Stress bewirken können.

Da sich die ökologischen und ökonomischen Anforderungen an moderne Pflanzenbehandlungsmittel laufend erhöhen, beispielsweise was Toxizität, Selektivität, Aufwandmenge, Rückstandsbildung und günstige Herstellbarkeit angeht, besteht die ständige Aufgabe, neue Pflanzenbehandlungsmittel zu entwickeln, die zumindest in Teilbereichen Vorteile gegenüber den bekannten aufweisen.

Daher bestand die Aufgabe der vorliegenden Erfindung darin, weitere Verbindungen bereitzustellen, die die Toleranz gegenüber abiotischem Stress in Pflanzen erhöhen, insbesondere eine Stärkung des Pflanzenwachstums bewirken und/oder zur Erhöhung des Pflanzenertrags beitragen.

Gegenstand der vorliegenden Erfindung sind demnach substituierte Vinyl- und Alkinyl-cyanocycloalkanole sowie Vinyl- und Alkinyl-cyanoheterocyclylalkanole der allgemeinen Formel (I) oder deren Salze, 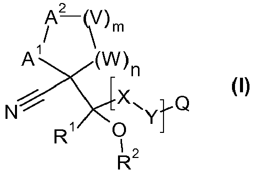 wobei
[X-Y] für die Gruppierungen 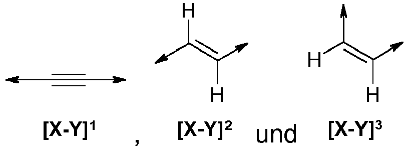 steht,
Q für die carbocyclischen und heterocyclischen Gruppierungen 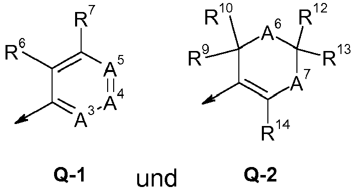 steht, wobei die Gruppierungen R⁶, R⁷, R⁹, R¹⁰, R¹², R¹³, R¹⁴, A³, A⁴, A⁵, A⁶ und A⁷ jeweils die Bedeutung gemäß der nachstehenden Definitionen haben und wobei der Pfeil für eine Bindung zur jeweiligen Gruppierung [X-Y] steht,
R¹ für Alkyl, Aryl, Heteroaryl, Heterocyclyl, Cycloalkyl, Alkenyl, Alkinyl, Alkoxyalkyl, Hydroxyalkyl, Haloalkyl, Haloalkenyl, Haloalkoxyalkyl, Alkylthioalkyl, Arylalkyl, Heterocyclylalkyl, Halocycloalkyl, Cycloalkenyl, Alkoxyalkoxyalkyl, Cycloalkylalkyl, Cycloalkenylalkyl, Haloalkinyl, Alkylsulfinylalkyl, Alkylsulfonylalkyl, Halocycloalkylalkyl Cycloalkylsulfinylalkyl, Cycloalkylsulfonylalkyl, Arylsulfinylalkyl, Arylsulfonylalkyl, Arylthioalkyl, Cycloalkylthioalkyl, Alkoxyhaloalkyl, Haloalkoxyhaloalkyl steht,
R² für Wasserstoff, Alkyl, Alkoxyalkyl, Alkoxyalkoxyalkyl, Alkenyl, Alkinyl, Alkylcarbonyl, Arylcarbonyl, Heteroarylcarbonyl, Cycloalkylcarbonyl, Alkenylcarbonyl, Heterocyclylcarbonyl, Alkoxycarbonyl, Alkenyloxycarbonyl, Aryloxyalkyl, Arylalkoxycarbonyl, Cycloalkoxycarbonyl, Cycloalkylalkoxycarbonyl, Arylalkoxyalkyl, Arylalkyl, Alkoxyalkoxyalkyl, Alkylthioalkyl, Trialkylsilyl, Alkyl(Bis-alkyl)silyl, Alkyl(Bis-aryl)silyl, Aryl(Bis-alkyl)silyl, Cycloalkyl(Bis-alkyl)silyl, Halo(Bis-alkyl)silyl, Trialkylsilylalkoxyalkyl, Trialkylsilylalkyl, Alkinyloxycarbonyl, Cycloalkyl, Cycloalkylalkyl, Aminocarbonyl, Alkylaminocarbonyl, Bis-Alkylaminocarbonyl, Cycloalkylaminocarbonyl, Alkylsulfonyl, Haloalkylsulfonyl, Arylsulfonyl, Heteroarylsulfonyl, Cycloalkylsulfonyl steht,
A¹, A², V, W unabhängig voneinander für eine Gruppe CR³R⁴, eine Gruppe N-R⁵, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht,
R³ und R⁴ unabhängig voneinander für Wasserstoff, Alkyl, Halogen, Cycloalkyl, Alkoxy, Aryl, Heterocyclyl, Heteroaryl, Arylalkyl, Alkylthio, Haloalkyl, Haloalkyloxy, Haloalkylthio, Alkoxyalkyl, Alkylthioalkyl, Heteroarylalkyl, Heterocyclylalkyl, Cycloalkylalkyl, Cycloalkenyl, Alkinyl, Alkenyl, Haloalkenyl, Haloalkinyl, Alkylsulfinyl, Alkylsulfonyl, Cycloalkylsulfinyl, Cycloalkylsulfonyl, Arylsulfinyl, Arylsulfonyl, Alkoxyhaloalkyl, Haloalkoxyhaloalkyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden,
R⁵ für Wasserstoff, Alkyl, Alkenyl, Alkinyl, Alkoxyalkyl, Alkylcarbonyl, Arylcarbonyl, Heteroarylcarbonyl, Cycloalkylcarbonyl, Alkoxycarbonyl, Alkenyloxycarbonyl, Alkoxycarbonylalkyl, Alkylaminocarbonylalkyl, Arylaminocarbonylalkyl, Aryloxyalkyl, Arylalkyl, Heteroarylalkyl, Heterocyclyl, Heterocyclylalkyl, Haloalkyl stehen,
R⁶ und R⁷ unabhängig voneinander für Wasserstoff, Nitro, Amino, Cyano, Thiocyanato, Isothiocyanato, Halogen, Alkyl, Cycloalkyl, Alkenyl, Alkinyl, Aryl, Arylalkyl, Arylalkoxy, Heteroaryl, Alkoxyalkyl, Hydroxyalkyl, Haloalkyl, Halocycloalkyl, Alkoxy, Haloalkoxy, Aryloxy, Heteroaryloxy, Cycloalkyloxy, Hydroxy, Cycloalkylalkoxy, Alkoxycarbonyl, Hydroxycarbonyl, Aminocarbonyl, Alkylaminocarbonyl, Cycloalkylaminocarbonyl, Cyanoalkylaminocarbonyl, Alkenylaminocarbonyl, Alkinylaminocarbonyl, Alkylamino, Alkylthio, Haloalkylthio, Hydrothio, Bisalkylamino, Cycloalkylamino, Alkylcarbonylamino, Cycloalkylcarbonylamino, Formylamino, Haloalkylcarbonylamino, Alkoxycarbonylamino, Alkylaminocarbonylamino, Alkyl(Alkyl)aminocarbonylamino, Alkylsulfonylamino, Cycloalkylsulfonylamino, Arylsulfonylamino, Hetarylsulfonylamino, Sulfonylhaloalkylamino, Aminosulfonyl, Aminoalkylsulfonyl, Aminohaloalkylsulfonyl, Alkylaminosulfonyl, Bisalkylaminosulfonyl, Cycloalkylaminosulfonyl, Haloalkylaminosulfonyl, Arylaminosulfonyl, Arylalkylaminosulfonyl, Alkylsulfonyl, Cycloalkylsulfonyl, Arylsulfonyl, Alkylsulfinyl, Cycloalkylsulfinyl, Arylsulfinyl, N,S-Dialkylsulfonimidoyl, S-Alkylsulfonimidoyl, Alkylsulfonylaminocarbonyl, Cycloalkylsulfonylaminocarbonyl, Cycloalkylaminosulfonyl, Arylalkylcarbonylamino, Cycloalkylalkylcarbonylamino, Heteroarylcarbonylamino, Alkoxyalkylcarbonylamino, Hydroxyalkylcarbonylamino, Trialkylsilyl stehen,
A³, A⁴, A⁵ gleich oder verschieden sind und unabhängig voneinander für N (Stickstoff) oder die Gruppierung C-R⁸ stehen, wobei jedoch in keinem Fall mehr als zwei N-Atome benachbart sind, und wobei R⁸ in der Gruppierung C-R⁸ jeweils gleiche oder verschiedene Bedeutungen gemäß der nachstehenden Definition hat und
A³ und A⁴, wenn beide für eine Gruppe C- R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
A⁴ und A⁵, wenn beide für eine Gruppe C- R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
R⁸ und R¹⁴, unabhängig voneinander jeweils für Wasserstoff, Nitro, Amino, Hydroxy, Hydrothio, Thiocyanato, Isothiocyanato, Halogen, Alkyl, Cycloalkyl, Alkenyl, Alkinyl, Aryl, Arylalkyl, Arylalkoxy, Heteroaryl, Haloalkyl, Halocycloalkyl, Alkoxy, Haloalkoxy, Aryloxy, Heteroaryloxy, Cycloalkyloxy, Cycloalkylalkoxy, Hydroxyalkyl, Alkoxyalkyl, Aryloxyalkyl, Heteroaryloxyalkyl, Alkylamino, Alkylthio, Haloalkylthio, Bisalkylamino, Cycloalkylamino, Alkylcarbonylamino, Cycloalkylcarbonylamino, Formylamino, Haloalkylcarbonylamino, Alkoxycarbonylamino, Alkylaminocarbonylamino, Alkyl(Alkyl)aminocarbonylamino, Alkylsulfonylamino, Cycloalkylsulfonylamino, Arylsulfonylamino, Hetarylsulfonylamino, Sulfonylhaloalkylamino, Aminoalkylsulfonyl, Aminohaloalkylsulfonyl, Alkylaminosulfonyl, Bisalkylaminosulfonyl, Cycloalkylaminosulfonyl, Haloalkylaminosulfonyl, Arylaminosulfonyl, Arylalkylaminosulfonyl, Alkylsulfonyl, Cycloalkylsulfonyl, Arylsulfonyl, Alkylsulfinyl, Cycloalkylsulfinyl, Arylsulfinyl, N,S-Dialkylsulfonimidoyl, S-Alkylsulfonimidoyl, Alkylsulfonylaminocarbonyl, Cycloalkylsulfonylaminocarbonyl, Cycloalkylaminosulfonyl, Arylalkylcarbonylamino, Cycloalkylalkylcarbonylamino, Heteroarylcarbonylamino, Alkoxyalkylcarbonylamino, Hydroxyalkylcarbonylamino, Cyano, Cyanoalkyl, Hydroxycarbonyl, Alkoxycarbonyl, Cycloalkoxycarbonyl, Cycloalkylalkoxycarbonyl, Aryloxycarbonyl, Arylalkoxycarbonyl, Aminocarbonyl, Alkylaminocarbonyl, Bisalkylaminocarbonyl, Alkyl(Alkoxy)aminocarbonyl, Cycloalkylaminocarbonyl, Arylalkylaminocarbonyl, Heteroarylalkylaminocarbonyl, Cyanoalkylaminocarbonyl, Haloalkylaminocarbonyl, Alkinylaminocarbonyl, Alkoxycarbonylaminocarbonyl, Arylalkoxycarbonylaminocarbonyl, Hydroxycarbonylalkyl, Alkoxycarbonylalkyl, Cycloalkoxycarbonylalkyl, Cycloalkylalkoxycarbonylalkyl, Alkylaminocarbonylalkyl, Aminocarbonylalkyl, Bisalkylaminocarbonylalkyl, Cycloalkylaminocarbonylalkyl, Arylalkylaminocarbonylalkyl, Heteroarylalkylaminocarbonylalkyl, Cyanoalkylaminocarbonylalkyl, Haloalkylaminocarbonylalkyl, Alkinylaminocarbonylalkyl, Cycloalkylalkylaminocarbonylalkyl, Alkoxycarbonylaminocarbonylalkyl, Arylalkoxycarbonylaminocarbonylalkyl, Alkoxycarbonylalkylaminocarbonyl, Hydroxycarbonylalkylaminocarbonyl, Aminocarbonylalkylaminocarbonyl, Alkylaminocarbonylalkylaminocarbonyl, Cycloalkylaminocarbonylalkylaminocarbonyl, Cycloalkylalkylaminocarbonyl, Cycloalkylalkylaminocarbonylalkyl, Alkenyloxycarbonyl, Alkenyloxycarbonylalkyl, Alkenylaminocarbonyl, Alkenylaminocarbonylalkyl, Alkylcarbonyl, Cycloalkylcarbonyl, Formyl, Hydroxyiminomethyl, Aminoiminomethyl, Alkoxyiminomethyl, Alkylaminoiminomethyl, Dialkylaminoiminomethyl, Cycloalkoxyiminomethyl, Cycloalkylalkoximinomethyl, Aryloximinomethyl, Arylalkoxyiminomethyl, Arylalkylaminoiminomethyl, Alkenyloxyiminomethyl, Arylaminoiminomethyl, Arylsulfonylaminoiminomethyl, Heteroarylalkyl, Heterocyclylalkyl, Hydroxycarbonylheterocyclyl, Alkoxycarbonylheterocyclyl, Alkenyloxycarbonylheterocyclyl, Alkenylalkoxycarbonylheterocyclyl, Arylalkoxycarbonylheterocyclyl, Cycloalkoxycarbonylheterocyclyl, Cycloalkylalkoxycarbonylheterocyclyl, Aminocarbonylheterocyclyl, Alkylaminocarbonylheterocyclyl, Bis-Alkylaminocarbonylheterocyclyl, Cycloalkylaminocarbonylheterocyclyl, Arylalkylaminocarbonylheterocyclyl, Alkenylaminocarbonylheterocyclyl, Hydroxycarbonylheterocyclylalkyl, Alkoxycarbonylheterocyclylalkyl, Hydroxycarbonylcycloalkylalkyl, Alkoxycarbonylcycloalkylalkyl, Heterocyclyl, Heterocyclyloxy, Heterocyclylamino, Heterocyclylcarbonyl, Heterocyclylcarbonylalkyl, Hydroxycarbonylheterocyclylcarbonyl, Alkoxycarbonylheterocyclylcarbonyl, Alkenyloxycarbonylheterocyclylcarbonyl, Alkenylalkoxycarbonylheterocyclylcarbonyl, Arylalkoxycarbonylheterocyclylcarbonyl, Cycloalkoxycarbonylheterocyclylcarbonyl, Cycloalkylalkoxycarbonylheterocyclylcarbonyl, Aminocarbonylheterocyclylcarbonyl, Alkylaminocarbonylheterocyclylcarbonyl, Bis-Alkylaminocarbonylheterocyclylcarbonyl, Cycloalkylaminocarbonylheterocyclylcarbonyl, Arylalkylaminocarbonylheterocyclylcarbonyl, Alkenylaminocarbonylheterocyclylcarbonyl stehen,
R⁹ und R¹⁰ unabhängig voneinander für Wasserstoff, Halogen, Alkyl, Alkoxy, Haloalkyl, Haloalkoxy, Aryl, Heteroaryl, Arylalkyl stehen oder gemeinsam mit dem Atom, an das sie gebunden sind, eine Carbonylgruppe bilden,
A⁶, A⁷ gleich oder verschieden sind und unabhängig voneinander für N-R¹¹, Sauerstoff, Schwefel oder die Gruppierung CR¹⁵R¹⁶ stehen, wobei jedoch in keinem Fall mehr als ein Sauerstoffatom im Heterocyclus enthalten ist, und wobei R¹¹, R¹⁵, R¹⁶ in den Gruppierungen N-R¹¹ und CR¹⁵R¹⁶ jeweils gleiche oder verschiedene Bedeutungen gemäß der nachstehenden Definition haben,
R¹¹ für Wasserstoff, Alkyl, Alkenyl, Alkinyl, Alkoxyalkyl, Alkylcarbonyl, Arylcarbonyl, Heteroarylcarbonyl, Cycloalkylcarbonyl, Alkoxycarbonyl, Alkenyloxycarbonyl, Alkoxycarbonylalkyl, Alkylaminocarbonylalkyl, Arylaminocarbonylalkyl, Aryloxyalkyl, Arylalkyl, Heteroarylalkyl, Heterocyclyl, Heterocyclylalkyl steht,
R¹² und R¹³ unabhängig voneinander für Wasserstoff, Halogen, Alkyl, Alkoxy, Haloalkyl, Haloalkoxy, Aryl, Heteroaryl, Arylalkyl, Heterocyclyl, Heteroarylalkyl, Heterocyclylalkyl stehen oder gemeinsam mit dem Atom, an das sie gebunden sind, eine Carbonylgruppe bilden und
R¹⁵ und R¹⁶ unabhängig voneinander für Wasserstoff, Alkyl, Alkenyl, Alkinyl, Alkoxy, Alkoxyalkyl, Alkylcarbonyl, Arylcarbonyl, Heteroarylcarbonyl, Hydroxycarbonyl, Alkoxycarbonyl, Heterocyclyl, Haloalkyl stehen,
mit Ausnahme von 1-[(2E)-1-Hydroxy-1,3-diphenylprop-2-en-1-yl]cyclopentan-carbonitril.

Die Verbindungen der Formel (I) können Salze bilden. Salzbildung kann durch Einwirkung einer Base auf solche Verbindungen der Formel (I) erfolgen, die ein acides Wasserstoffatom tragen, z.B. im Falle dass R¹ eine COOH-Gruppe oder eine Sulfonamid-Gruppe -NHSO₂- enthält. Geeignete Basen sind beispielsweise organische Amine , wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R''']⁺, worin R bis R''' jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Im Folgenden werden die erfindungsgemäßen und/oder erfindungsgemäß verwendeten Verbindungen der allgemeinen Formel (I) und ihre Salze auch kurz als "Verbindungen der allgemeinen Formel (I)" bezeichnet.

Bevorzugt sind Verbindungen der allgemeinen Formel (I), worin
[X-Y] für die Gruppierungen 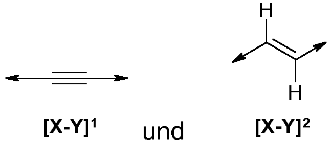 steht,
Q für die carbocyclischen und heterocyclischen Gruppierungen 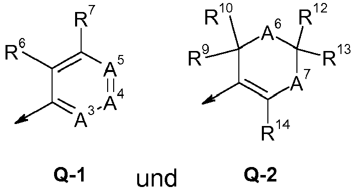 steht, wobei die Gruppierungen R⁶, R⁷, R⁹, R¹⁰, R¹², R¹³, R¹⁴, A³, A⁴, A⁵, A⁶ und A⁷ jeweils die Bedeutung gemäß der nachstehenden Definitionen haben und wobei der Pfeil für eine Bindung zur jeweiligen Gruppierung [X-Y] steht,
R¹ für (C₁-C₈)-Alkyl, Aryl, Heteroaryl, Heterocyclyl, (C₃-C₁₀)-Cycloalkyl, (C₂-C₈)-Alkenyl, (C₂-C₈)-Alkinyl, (C₁-C₈)-Alkoxy-(C₁-C₈)-alkyl, Hydroxy-(C₁-C₈)-alkyl, (C₁-C₈)-Haloalkyl, (C₂-C₈)-Haloalkenyl, (C₁-C₈)-Haloalkoxy-(C₁-C₈)-alkyl, (C₁-C₈)-Alkylthio-(C₁-C₈)-alkyl, Aryl-(C₁-C₈)-alkyl, Heterocyclyl-(C₁-C₈)-alkyl, (C₃-C₈)-Halocycloalkyl, (C₄-C₃)-Cycloalkenyl, (C₁-C₈)-Alkoxy-(C₁-C₈)-alkoxy-(C₁-C₈)-alkyl, (C₃-C₈)-Cycloalkyl-(C₁-C₈)-alkyl, (C₄-C₈)-Cycloalkenyl-(C₁-C₈)-alkyl, (C₂-C₈)-Haloalkinyl, (C₃-C₈)-Alkylsulfinyl-(C₃-C₈)-alkyl, (C₁-C₈)-Alkylsulfonyl-(C₁-C₈)-alkyl, (C₃-C₈)-Halocycloalkyl-(C₁-C₈)-alkyl, (C₃-C₈)-Cycloalkylsulfinyl-(C₁-C₈)-alkyl, (C₃-C₈)-Cycloalkylsulfonyl-(C₁-C₈)-alkyl, Arylsulfinyl-(C₁-C₈)-alkyl, Arylsulfonyl-(C₁-C₈)-alkyl, Arylthio-(C₁-C₈)-alkyl, (C₃-C₈)-Cycloalkylthio-(C₁-C₈)-alkyl, (C₁-C₈)-Alkoxy-(C₁-C₈)-haloalkyl, (C₁-C₈)-Haloalkoxy-(C₁-C₈)-haloalkyl steht,
R² für Wasserstoff, (C₁-C₈)-Alkyl, (C₁-C₈)-Alkoxy-(C₁-C₈)-alkyl, (C₁-C₈)-Alkoxy-(C₁-C₈)-alkoxy-(C₁-C₈)-alkyl, (C₂-C₈)-Alkenyl, (C₂-C₈)-Alkinyl, (C₁-C₈)-Alkylcarbonyl, Arylcarbonyl, Heteroarylcarbonyl, (C₃-C₈)-Cycloalkylcarbonyl, (C₂-C₈)-Alkenylcarbonyl, Heterocyclylcarbonyl, (C₁-C₈)-Alkoxycarbonyl, (C₂-C₈)-Alkenyloxycarbonyl, Aryloxy-(C₁-C₈)-alkyl, Aryl-(C₁-C₈)-alkoxycarbonyl, (C₃-C₈)-Cycloalkoxycarbonyl, (C₃-C₃)-Cycloalkyl-(C₁-C₈)-alkoxycarbonyl, Aryl-(C₁-C₈)-alkoxyalkyl, Aryl-(C₁-C₈)-alkyl, (C₁-C₈)-Alkoxy-(C₁-C₈)-alkoxy-(C₁-C₈)-alkyl, (C₁-C₈)-Alkylthio-(C₁-C₈)-alkyl, Tris[(C₁-C₈)-alkyl]silyl, (C₁-C₈)-Alkyl{Bis-[(C₁-C₈)-alkyl]}silyl, (C₁-C₈)-Alkyl(Bis-aryl)silyl, Aryl{Bis-[(C₁-C₈)-alkyl]}silyl, Cycloalkyl{Bis-[(C₁-C₈)-alkyl]}silyl, Halo{Bis-[(C₁-C₈)-alkyl]}silyl, Tris-[(C₁-C₈)-alkyl]silyl-(C₁-C₈)-alkoxy-(C₁-C₈)-alkyl, Tris[(C₁-C₈)-alkyl]silyl-(C₁-C₈)-alkyl, (C₂-C₈)-Alkinyloxycarbonyl, (C₃-C₈)-Cycloalkyl, (C₃-C₈)-Cycloalkyl-(C₁-C₈)-alkyl, Aminocarbonyl, (C₁-C₈)-Alkylaminocarbonyl, Bis-[(C₁-C₈)-Alkyl]aminocarbonyl, (C₃-C₈)-Cycloalkylaminocarbonyl, (C₁-C₈)-Alkylsulfonyl, (C₁-C₈)-Haloalkylsulfonyl, Arylsulfonyl, Heteroarylsulfonyl, (C₃-C₃)-Cycloalkylsulfonyl steht,
A¹, A², V, W unabhängig voneinander für eine Gruppe CR³R⁴, eine Gruppe N-R⁵, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht,
R³ und R⁴ unabhängig voneinander für Wasserstoff, (C₁-C₈)-Alkyl, Halogen, (C₃-C₈)-Cycloalkyl, (C₁-C₈)-Alkoxy, Aryl, Heterocyclyl, Heteroaryl, Aryl-(C₁-C₈)-alkyl, (C₁-C₈)-Alkylthio, (C₁-C₈)-Haloalkyl, (C₁-C₈)-Haloalkyloxy, (C₁-C₈)-Haloalkylthio, (C₁-C₈)-Alkoxy-(C₁-C₈)-alkyl, (C₁-C₈)-Alkylthio-(C₁-C₈)-alkyl, Heteroaryl-(C₁-C₈)-alkyl, Heterocyclyl-(C₁-C₈)-alkyl, (C₃-C₈)-Cycloalkyl-(C₁-C₈)-alkyl, (C₄-C₈)-Cycloalkenyl, (C₂-C₈)-Alkinyl, (C₂-C₈)-Alkenyl, (C₂-C₈)-Haloalkenyl, (C₂-C₈)-Haloalkinyl, (C₁-C₈)-Alkylsulfinyl, (C₁-C₈)-Alkylsulfonyl, (C₃-C₈)-Cycloalkylsulfinyl, (C₃-C₃)-Cycloalkylsulfonyl, Arylsulfinyl, Arylsulfonyl, (C₁-C₈)-Alkoxy-(C₁-C₈)-haloalkyl, (C₁-C₈)-Haloalkoxy-(C₁-C₈)-haloalkyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden,
R⁵ für Wasserstoff, (C₁-C₈)-Alkyl, (C₂-C₈)-Alkenyl, (C₂-C₈)-Alkinyl, (C₁-C₈)-Alkoxy-(C₁-C₈)-alkyl, (C₁-C₈)-Alkylcarbonyl, Arylcarbonyl, Heteroarylcarbonyl, (C₃-C₈)-Cycloalkylcarbonyl, (C₁-C₈)-Alkoxycarbonyl, (C₂-C₈)-Alkenyloxycarbonyl, (C₁-C₈)-Alkoxycarbonyl-(C₁-C₈)-alkyl, (C₁-C₈)-Alkylaminocarbonyl-(C₁-C₈)-alkyl, Arylaminocarbonyl-(C₁-C₈)-alkyl, Aryloxy-(C₁-C₈)-alkyl, Aryl-(C₁-C₈)-alkyl, Heteroaryl-(C₁-C₈)-alkyl, Heterocyclyl, Heterocyclyl-(C₁-C₈)-alkyl, (C₁-C₈)-Haloalkyl stehen,
R⁶ und R⁷ unabhängig voneinander für Wasserstoff, Nitro, Amino, Cyano, Thiocyanato, Isothiocyanato, Halogen, (C₁-C₈)-Alkyl, (C₃-C₃)-Cycloalkyl, (C₂-C₈)-Alkenyl, (C₂-C₈)-Alkinyl, Aryl, Aryl-(C₁-C₈)-alkyl, Aryl-(C₁-C₈)-alkoxy, Heteroaryl, (C₁-C₈)-Alkoxy-(C₁-C₈)-alkyl, Hydroxy-(C₁-C₈)-alkyl, (C₁-C₈)-Haloalkyl, (C₃-C₈)-Halocycloalkyl, (C₁-C₈)-Alkoxy, (C₁-C₈)-Haloalkoxy, Aryloxy, Heteroaryloxy, (C₃-C₃)-Cycloalkyloxy, Hydroxy, (C₃-C₈)-Cycloalkyl-(C₁-C₈)-alkoxy, (C₁-C₈)-Alkoxycarbonyl, Hydroxycarbonyl, Aminocarbonyl, (C₁-C₈)-Alkylaminocarbonyl, (C₃-C₈)-Cycloalkylaminocarbonyl, Cyano-(C₁-C₈)-alkylaminocarbonyl, (C₂-C₈)-Alkenylaminocarbonyl, (C₂-C₈)-Alkinylaminocarbonyl, (C₁-C₈)-Alkylamino, (C₁-C₈)-Alkylthio, (C₁-C₈)-Haloalkylthio, Hydrothio, Bis-[(C₁-C₈)-alkyl]amino, (C₃-C₃)-Cycloalkylamino, (C₁-C₈)-Alkylcarbonylamino, (C₃-C₈)-Cycloalkylcarbonylamino, Formylamino, (C₁-C₈)-Haloalkylcarbonylamino, (C₁-C₈)-Alkoxycarbonylamino, (C₁-C₈)-Alkylaminocarbonylamino, (C₁-C₈)-Alkyl-[(C₁-C₈)-Alkyl]aminocarbonylamino, (C₁-C₈)-Alkylsulfonylamino, (C₃-C₈)-Cycloalkylsulfonylamino, Arylsulfonylamino, Hetarylsulfonylamino, Sulfonyl-(C₁-C₈)-haloalkylamino, Aminosulfonyl, Amino-(C₁-C₈)-alkylsulfonyl, Amino-(C₁-C₈)-haloalkylsulfonyl, (C₁-C₈)-Alkylaminosulfonyl, Bis[(C₁-C₈)-alkyl]aminosulfonyl, (C₃-C₈)-Cycloalkylaminosulfonyl, (C₁-C₈)-Haloalkylaminosulfonyl, Arylaminosulfonyl, Aryl-(C₁-C₈)-alkylaminosulfonyl, (C₁-C₈)-Alkylsulfonyl, (C₃-C₃)-Cycloalkylsulfonyl, Arylsulfonyl, (C₁-C₈)-Alkylsulfinyl, (C₃-C₈)-Cycloalkylsulfinyl, Arylsulfinyl, N,S-Bis[(C₁-C₈)-alkyl]sulfonimidoyl, S-(C₁-C₈)-Alkylsulfonimidoyl, (C₁-C₈)-Alkylsulfonylaminocarbonyl, (C₃-C₈)-Cycloalkylsulfonylaminocarbonyl, (C₃-C₈)-Cycloalkylaminosulfonyl, Aryl-(C₁-C₈)-alkylcarbonylamino, (C₃-C₃)-Cycloalkyl-(C₁-C₈)-alkylcarbonylamino, Heteroarylcarbonylamino, (C₁-C₈)-Alkoxy-(C₁-C₈)-alkylcarbonylamino, Hydroxy-(C₁-C₈)-alkylcarbonylamino, Tris[(C₁-C₈)-alkyl]silyl stehen,
A³, A⁴, A⁵ gleich oder verschieden sind und unabhängig voneinander für N (Stickstoff) oder die Gruppierung C-R⁸ stehen, wobei jedoch in keinem Fall mehr als zwei N-Atome benachbart sind, und wobei R⁸ in der Gruppierung C-R⁸ jeweils gleiche oder verschiedene Bedeutungen gemäß der nachstehenden Definition hat und
A³ und A⁴, wenn beide für eine Gruppe C- R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
A⁴ und A⁵, wenn beide für eine Gruppe C- R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
R⁸ und R¹⁴ unabhängig voneinander jeweils für Wasserstoff, Nitro, Amino, Hydroxy, Hydrothio, Thiocyanato, Isothiocyanato, Halogen, (C₁-C₈)-Alkyl, (C₃-C₈)-Cycloalkyl, (C₂-C₈)-Alkenyl, (C₂-C₈)-Alkinyl, Aryl, Aryl-(C₁-C₈)-alkyl, Aryl-(C₁-C₈)-alkoxy, Heteroaryl, (C₁-C₈)-Haloalkyl, (C₃-C₈)-Halocycloalkyl, (C₁-C₈)-Alkoxy, (C₁-C₈)-Haloalkoxy, Aryloxy, Heteroaryloxy, (C₃-C₈)-Cycloalkyloxy, (C₃-C₈)-Cycloalkyl-(C₁-C₈)-alkoxy, Hydroxy-(C₁-C₈)-alkyl, (C₁-C₈)-Alkoxy-(C₁-C₈)-alkyl, Aryloxy-(C₁-C₈)-alkyl, Heteroaryloxy-(C₁-C₈)-alkyl, (C₁-C₈)-Alkylamino, (C₁-C₈)-Alkylthio, (C₁-C₈)-Haloalkylthio, Bis[(C₁-C₈)-alkyl]amino, (C₃-C₈)-Cycloalkylamino, (C₁-C₈)-Alkylcarbonylamino, (C₃-C₈)-Cycloalkylcarbonylamino, Formylamino, (C₁-C₈)-Haloalkylcarbonylamino, (C₁-C₈)-Alkoxycarbonylamino, (C₁-C₈)-Alkylaminocarbonylamino, (C₁-C₈)-Alkyl[(C₁-C₈)-Alkyl]aminocarbonylamino, (C₁-C₈)-Alkylsulfonylamino, (C₃-C₈)-Cycloalkylsulfonylamino, Arylsulfonylamino, Hetarylsulfonylamino, Sulfonyl-(C₁-C₈)-haloalkylamino, Amino-(C₁-C₈)-alkylsulfonyl, Amino-(C₁-C₈)-haloalkylsulfonyl, (C₁-C₈)-Alkylaminosulfonyl, Bis[(C₁-C₈)-alkyl]aminosulfonyl, (C₃-C₈)-Cycloalkylaminosulfonyl, (C₁-C₈)-Haloalkylaminosulfonyl, Arylaminosulfonyl, Aryl-(C₁-C₈)-alkylaminosulfonyl, (C₁-C₈)-Alkylsulfonyl, (C₃-C₃)-Cycloalkylsulfonyl, Arylsulfonyl, (C₁-C₈)-Alkylsulfinyl, (C₃-C₈)-Cycloalkylsulfinyl, Arylsulfinyl, N,S-Bis[(C₁-C₈)-alkyl]sulfonimidoyl, S-(C₁-C₈)-Alkylsulfonimidoyl, (C₁-C₈)-Alkylsulfonylaminocarbonyl, (C₃-C₈)-Cycloalkylsulfonylaminocarbonyl, (C₃-C₈)-Cycloalkylaminosulfonyl, Aryl-(C₁-C₈)-alkylcarbonylamino, (C₃-C₈)-Cycloalkyl-(C₁-C₈)-alkylcarbonylamino, Heteroarylcarbonylamino, (C₁-C₈)-Alkoxy-(C₁-C₈)-alkylcarbonylamino, Hydroxy-(C₁-C₈)-alkylcarbonylamino, Cyano, Cyano-(C₁-C₈)-alkyl, Hydroxycarbonyl, (C₁-C₈)-Alkoxycarbonyl, (C₃-C₈)-Cycloalkoxycarbonyl, (C₃-C₈)-Cycloalkyl-(C₁-C₈)-alkoxycarbonyl, Aryloxycarbonyl, Aryl-(C₁-C₈)-alkoxycarbonyl, Aminocarbonyl, (C₁-C₈)-Alkylaminocarbonyl, Bis[(C₁-C₈)-alkyl]aminocarbonyl, (C₁-C₈)-Alkyl[(C₁-C₈)-Alkoxy]aminocarbonyl, (C₃-C₈)-Cycloalkylaminocarbonyl, Aryl-(C₁-C₈)-alkylaminocarbonyl, Heteroaryl-(C₁-C₈)-alkylaminocarbonyl, Cyano-(C₁-C₈)-alkylaminocarbonyl, (C₁-C₈)-Haloalkylaminocarbonyl, (C₂-C₈)-Alkinylaminocarbonyl, (C₁-C₈)-Alkoxycarbonylaminocarbonyl, Aryl-(C₁-C₈)-alkoxycarbonylaminocarbonyl, Hydroxycarbonyl-(C₁-C₈)-alkyl, (C₁-C₈)-Alkoxycarbonyl-(C₁-C₈)-alkyl, (C₃-C₈)-Cycloalkoxycarbonyl-(C₁-C₈)-alkyl, (C₃-C₈)-Cycloalkyl-(C₁-C₈)-alkoxycarbonyl-(C₁-C₈)-alkyl, (C₁-C₈)-Alkylaminocarbonyl-(C₁-C₈)-alkyl, Aminocarbonyl-(C₁-C₈)-alkyl, Bis[(C₁-C₈)-alkyl]aminocarbonyl-(C₁-C₈)-alkyl, (C₃-C₈)-Cycloalkylaminocarbonyl-(C₁-C₈)-alkyl, Aryl-(C₁-C₈)-alkylaminocarbonyl-(C₁-C₈)-alkyl, Heteroaryl-(C₁-C₈)-alkylaminocarbonyl-(C₁-C₈)-alkyl, Cyano-(C₁-C₈)-alkylaminocarbonyl-(C₁-C₈)-alkyl, (C₁-C₈)-Haloalkylaminocarbonyl-(C₁-C₈)-alkyl, (C₂-C₈)-Alkinylaminocarbonyl-(C₁-C₈)-alkyl, (C₃-C₈)-Cycloalkyl-(C₁-C₈)-alkylaminocarbonyl-(C₁-C₈)-alkyl, (C₁-C₈)-Alkoxycarbonylaminocarbonyl-(C₁-C₈)-alkyl, Aryl-(C₁-C₈)-alkoxycarbonylaminocarbonyl-(C₁-C₈)-alkyl, (C₁-C₈)-Alkoxycarbonyl-(C₁-C₈)-alkylaminocarbonyl, Hydroxycarbonyl-(C₁-C₈)-alkylaminocarbonyl, Aminocarbonyl-(C₁-C₈)-alkylaminocarbonyl, (C₁-C₈)-Alkylaminocarbonyl-(C₁-C₈)-alkylaminocarbonyl, (C₃-C₈)-Cycloalkylaminocarbonyl-(C₁-C₈)-alkylaminocarbonyl, (C₃-C₈)-Cycloalkyl-(C₁-C₈)-alkylaminocarbonyl, (C₃-C₈)-Cycloalkyl-(C₁-C₈)-alkylaminocarbonyl-(C₁-C₈)-alkyl, (C₂-C₈)-Alkenyloxycarbonyl, (C₂-C₈)-Alkenyloxycarbonyl-(C₁-C₈)-alkyl, (C₂-C₈)-Alkenylaminocarbonyl, (C₂-C₈)-Alkenylaminocarbonyl-(C₁-C₈)-alkyl, (C₁-C₈)-Alkylcarbonyl, (C₃-C₈)-Cycloalkylcarbonyl, Formyl, Hydroxyiminomethyl, Aminoiminomethyl, (C₁-C₈)-Alkoxyiminomethyl, (C₁-C₈)-Alkylaminoiminomethyl, Bis[(C₁-C₈)-alkyl]aminoiminomethyl, (C₃-C₈)-Cycloalkoxyiminomethyl, (C₃-C₈)-Cycloalkyl-(C₁-C₈)-alkoximinomethyl, Aryloximinomethyl, Aryl-(C₁-C₈)-alkoxyiminomethyl, Aryl-(C₁-C₈)-alkylaminoiminomethyl, (C₂-C₈)-Alkenyloxyiminomethyl, Arylaminoiminomethyl, Arylsulfonylaminoiminomethyl, Heteroaryl-(C₁-C₈)-alkyl, Heterocyclyl-(C₁-C₈)-alkyl, Hydroxycarbonylheterocyclyl, (C₁-C₈)-Alkoxycarbonylheterocyclyl, (C₂-C₈)-Alkenyloxycarbonylheterocyclyl, (C₂-C₈)-Alkenyl-(C₁-C₈)-alkoxycarbonylheterocyclyl, Aryl-(C₁-C₈)-alkoxycarbonylheterocyclyl, (C₃-C₈)-Cycloalkoxycarbonylheterocyclyl, (C₃-C₈)-Cycloalkyl-(C₁-C₈)-alkoxycarbonylheterocyclyl, Aminocarbonylheterocyclyl, (C₁-C₈)-Alkylaminocarbonylheterocyclyl, Bis-[(C₁-C₈)-Alkyl]aminocarbonylheterocyclyl, (C₃-C₈)-Cycloalkylaminocarbonylheterocyclyl, Aryl-(C₁-C₈)-alkylaminocarbonylheterocyclyl, (C₂-C₈)-Alkenylaminocarbonylheterocyclyl, Hydroxycarbonylheterocyclyl-(C₁-C₈)-alkyl, (C₁-C₈)-Alkoxycarbonylheterocyclyl-(C₁-C₈)-alkyl, Hydroxycarbonyl-(C₃-C₈)-cycloalkyl-(C₁-C₈)-alkyl, (C₁-C₈)-Alkoxycarbonyl-(C₃-C₈)-cycloalkyl-(C₁-C₈)-alkyl, Heterocyclyl, Heterocyclyloxy, Heterocyclylamino, Heterocyclylcarbonyl, Heterocyclylcarbonyl-(C₁-C₈)-alkyl, Hydroxycarbonylheterocyclylcarbonyl, (C₁-C₈)-Alkoxycarbonylheterocyclylcarbonyl, (C₂-C₈)-Alkenyloxycarbonylheterocyclylcarbonyl, (C₂-C₈)-Alkenyl-(C₁-C₈)-alkoxycarbonylheterocyclylcarbonyl, Aryl-(C₁-C₈)-alkoxycarbonylheterocyclylcarbonyl, (C₃-C₈)-Cycloalkoxycarbonylheterocyclylcarbonyl, (C₃-C₈)-Cycloalkyl-(C₁-C₈)-alkoxycarbonylheterocyclylcarbonyl, Aminocarbonylheterocyclylcarbonyl, (C₁-C₈)-Alkylaminocarbonylheterocyclylcarbonyl, Bis-[(C₁-C₈)-Alkyl]aminocarbonylheterocyclylcarbonyl, (C₃-C₈)-Cycloalkylaminocarbonylheterocyclylcarbonyl, Aryl-(C₁-C₈)-alkylaminocarbonylheterocyclylcarbonyl, (C₂-C₈)-Alkenylaminocarbonylheterocyclylcarbonyl stehen,
R⁹ und R¹⁰ unabhängig voneinander für Wasserstoff, Halogen, (C₁-C₈)-Alkyl, (C₁-C₈)-Alkoxy, (C₁-C₈)-Haloalkyl, (C₁-C₈)-Haloalkoxy, Aryl, Heteroaryl, Aryl-(C₁-C₈)-alkyl stehen oder gemeinsam mit dem Atom, an das sie gebunden sind, eine Carbonylgruppe bilden,
A⁶, A⁷ gleich oder verschieden sind und unabhängig voneinander für N-R¹¹, Sauerstoff, Schwefel oder die Gruppierung CR¹⁵R¹⁶ stehen, wobei jedoch in keinem Fall mehr als ein Sauerstoffatom im Heterocyclus enthalten ist, und wobei R¹¹, R¹⁵, R¹⁶ in den Gruppierungen N-R¹¹ und CR¹⁵R¹⁶ jeweils gleiche oder verschiedene Bedeutungen gemäß der nachstehenden Definition haben,
R¹¹ für Wasserstoff, (C₁-C₈)-Alkyl, (C₂-C₈)-Alkenyl, (C₂-C₈)-Alkinyl, (C₁-C₈)-Alkoxy-(C₁-C₈)-alkyl, (C₁-C₈)-Alkylcarbonyl, Arylcarbonyl, Heteroarylcarbonyl, (C₃-C₈)-Cycloalkylcarbonyl, (C₁-C₈)-Alkoxycarbonyl, (C₂-C₈)-Alkenyloxycarbonyl, (C₁-C₈)-Alkoxycarbonyl-(C₁-C₈)-alkyl, (C₁-C₈)-Alkylaminocarbonyl-(C₁-C₈)-alkyl, Arylaminocarbonyl-(C₁-C₈)-alkyl, Aryloxy-(C₁-C₈)-alkyl, Aryl-(C₁-C₈)-alkyl, Heteroaryl-(C₁-C₈)-alkyl, Heterocyclyl, Heterocyclyl-(C₁-C₈)-alkyl steht,
R¹² und R¹³ unabhängig voneinander für Wasserstoff, Halogen, (C₁-C₈)-Alkyl, (C₁-C₈)-Alkoxy, (C₁-C₈)-Haloalkyl, (C₁-C₈)-Haloalkoxy, Aryl, Heteroaryl, Aryl-(C₁-C₈)-alkyl, Heterocyclyl, Heteroaryl-(C₁-C₈)-alkyl, Heterocyclyl-(C₁-C₈)-alkyl stehen oder gemeinsam mit dem Atom, an das sie gebunden sind, eine Carbonylgruppe bilden und
R¹⁵ und R¹⁶ unabhängig voneinander für Wasserstoff, (C₁-C₈)-Alkyl, (C₂-C₈)-Alkenyl, (C₂-C₈)-Alkinyl, (C₁-C₈)-Alkoxy, (C₁-C₈)-Alkoxy-(C₁-C₈)-alkyl, (C₁-C₈)-Alkylcarbonyl, Arylcarbonyl, Heteroarylcarbonyl, Hydroxycarbonyl, (C₁-C₈)-Alkoxycarbonyl, Heterocyclyl, (C₁-C₈)-Haloalkyl stehen,
mit Ausnahme von 1-[(2E)-1-Hydroxy-1,3-diphenylprop-2-en-1-yl]cyclopentancarbonitril.

Besonders bevorzugt sind Verbindungen der allgemeinen Formel (I), worin
[X-Y] für die Gruppierungen 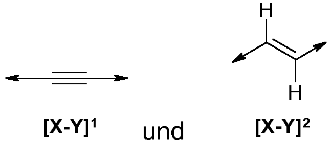 steht,
Q für die carbocyclischen und heterocyclischen Gruppierungen 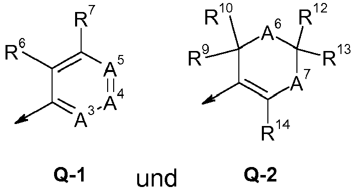 steht, wobei die Gruppierungen R⁶, R⁷, R⁹, R¹⁰, R¹², R¹³, R¹⁴, A³, A⁴, A⁵, A⁶ und A⁷ jeweils die Bedeutung gemäß der nachstehenden Definitionen haben und wobei der Pfeil für eine Bindung zur jeweiligen Gruppierung [X-Y] steht,
R¹ für (C₁-C₇)-Alkyl, Aryl, Heteroaryl, Heterocyclyl, (C₃-C₁₀)-Cycloalkyl, (C₂-C₇)-Alkenyl, (C₂-C₇)-Alkinyl, (C₁-C₇)-Alkoxy-(C₁-C₇)-alkyl, Hydroxy-(C₁-C₇)-alkyl, (C₁-C₇)-Haloalkyl, (C₂-C₇)-Haloalkenyl, (C₁-C₇)-Haloalkoxy-(C₁-C₇)-alkyl, (C₁-C₇)-Alkylthio-(C₁-C₇)-alkyl, Aryl-(C₁-C₇)-alkyl, Heterocyclyl-(C₁-C₇)-alkyl, (C₃-C₇)-Halocycloalkyl, (C₄-C₇)-Cycloalkenyl, (C₁-C₇)-Alkoxy-(C₁-C₇)-alkoxy-(C₁-C₇)-alkyl, (C₁-C₇)-Alkoxy-(C₁-C₇)-haloalkyl, (C₁-C₇)-Haloalkoxy-(C₁-C₇)-haloalkyl, (C₃-C₈)-Cycloalkyl-(C₁-C₇)-alkyl steht,
R² für Wasserstoff, (C₁-C₇)-Alkyl, (C₁-C₇)-Alkoxy-(C₁-C₇)-alkyl, (C₁-C₇)-Alkoxy-(C₁-C₇)-alkoxy-(C₁-C₇)-alkyl, (C₂-C₇)-Alkenyl, (C₂-C₇)-Alkinyl, (C₁-C₇)-Alkylcarbonyl, Arylcarbonyl, Heteroarylcarbonyl, (C₃-C₇)-Cycloalkylcarbonyl, (C₂-C₇)-Alkenylcarbonyl, Heterocyclylcarbonyl, (C₁-C₇)-Alkoxycarbonyl, (C₂-C₇)-Alkenyloxycarbonyl, Aryloxy-(C₁-C₇)-alkyl, Aryl-(C₁-C₇)-alkoxycarbonyl, (C₃-C₇)-Cycloalkoxycarbonyl, (C₃-C₇)-Cycloalkyl-(C₁-C₇)-alkoxycarbonyl, Aryl-(C₁-C₇)-alkoxy-(C₁-C₇)-alkyl, Aryl-(C₁-C₇)-alkyl, (C₁-C₇)-Alkoxy-(C₁-C₇)-alkoxy-(C₁-C₇)-alkyl, (C₁-C₇)-Alkylthio-(C₁-C₇)-alkyl, Tris[(C₁-C₇)-alkyl]silyl, (C₁-C₇)-Alkyl-Bis-[(C₁-C₇)-alkyl]silyl, (C₁-C₇)-Alkyl-Bis(aryl)silyl, Aryl-Bis[(C₁-C₇)-alkyl]silyl, (C₃-C₇)-Cycloalkyl-Bis[(C₁-C₇)-alkyl]silyl, Halo-Bis[(C₁-C₇)-alkyl]silyl, Tris[(C₁-C₇)-alkyl]silyl-(C₁-C₇)-alkoxy-(C₁-C₇)-alkyl, Tris[(C₁-C₇)-alkyl]silyl-(C₁-C₇)-alkyl steht,
A¹, A², V, W unabhängig voneinander für eine Gruppe CR³R⁴, eine Gruppe N-R⁵, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht,
R³ und R⁴ unabhängig voneinander für Wasserstoff, (C₁-C₇)-Alkyl, Halogen, (C₃-C₇)-Cycloalkyl, (C₁-C₇)-Alkoxy, Aryl, Heterocyclyl, Heteroaryl, Aryl-(C₁-C₇)-alkyl, (C₁-C₇)-Alkylthio, (C₁-C₇)-Haloalkyl, (C₁-C₇)-Haloalkyloxy, (C₁-C₇)-Haloalkylthio, (C₁-C₇)-Alkoxy-(C₁-C₇)-alkyl, (C₁-C₇)-Alkylthio-(C₁-C₇)-alkyl, Heteroaryl-(C₁-C₇)-alkyl, Heterocyclyl-(C₁-C₇)-alkyl, (C₃-C₇)-Cycloalkyl-(C₁-C₇)-alkyl, (C₄-C₇)-Cycloalkenyl, (C₂-C₇)-Alkinyl, (C₂-C₇)-Alkenyl, (C₂-C₇)-Haloalkenyl, (C₂-C₇)-Haloalkinyl, (C₁-C₇)-Alkylsulfinyl, (C₁-C₇)-Alkylsulfonyl, (C₃-C₇)-Cycloalkylsulfinyl, (C₃-C₇)-Cycloalkylsulfonyl, Arylsulfinyl, Arylsulfonyl, (C₁-C₇)-Alkoxy-(C₁-C₇)-haloalkyl, (C₁-C₇)-Haloalkoxy-(C₁-C₇)-haloalkyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden,
R⁵ für Wasserstoff, (C₁-C₇)-Alkyl, (C₂-C₇)-Alkenyl, (C₂-C₇)-Alkinyl, (C₁-C₇)-Alkoxy-(C₁-C₇)-alkyl, (C₁-C₇)-Alkylcarbonyl, Arylcarbonyl, Heteroarylcarbonyl, (C₃-C₇)-Cycloalkylcarbonyl, (C₁-C₇)-Alkoxycarbonyl, (C₂-C₇)-Alkenyloxycarbonyl, (C₁-C₇)-Alkoxycarbonyl-(C₁-C₇)-alkyl, (C₁-C₇)-Alkylaminocarbonyl-(C₁-C₇)-alkyl, Arylaminocarbonyl-(C₁-C₇)-alkyl, Aryloxy-(C₁-C₇)-alkyl, Aryl-(C₁-C₇)-alkyl, Heteroaryl-(C₁-C₇)-alkyl, Heterocyclyl, Heterocyclyl-(C₁-C₇)-alkyl, stehen,
R⁶ und R⁷ unabhängig voneinander für Wasserstoff, Nitro, Amino, Cyano, Thiocyanato, Isothiocyanato, Halogen, (C₁-C₇)-Alkyl, (C₃-C₇)-Cycloalkyl, (C₂-C₇)-Alkenyl, (C₂-C₇)-Alkinyl, Aryl, Aryl-(C₁-C₇)-alkyl, Aryl-(C₁-C₇)-alkoxy, Heteroaryl, (C₁-C₇)-Alkoxy-(C₁-C₇)-alkyl, Hydroxy-(C₁-C₇)-alkyl, (C₁-C₇)-Haloalkyl, (C₃-C₇)-Halocycloalkyl, (C₁-C₇)-Alkoxy, (C₁-C₇)-Haloalkoxy, Aryloxy, Heteroaryloxy, (C₃-C₇)-Cycloalkyloxy, Hydroxy, (C₃-C₇)-Cycloalkyl-(C₁-C₇)-alkoxy, (C₁-C₇)-Alkoxycarbonyl, Hydroxycarbonyl, Aminocarbonyl, (C₁-C₇)-Alkylaminocarbonyl, (C₃-C₇)-Cycloalkylaminocarbonyl, Cyano-(C₁-C₇)-alkylaminocarbonyl, (C₂-C₇)-Alkenylaminocarbonyl, (C₂-C₇)-Alkinylaminocarbonyl, (C₁-C₇)-Alkylamino, (C₁-C₇)-Alkylthio, (C₁-C₇)-Haloalkylthio, Hydrothio, Bis-[(C₁-C₇)-alkyl]amino, (C₃-C₇)-Cycloalkylamino, (C₁-C₇)-Alkylcarbonylamino, (C₃-C₇)-Cycloalkylcarbonylamino, Formylamino, (C₁-C₇)-Haloalkylcarbonylamino, (C₁-C₇)-Alkoxycarbonylamino, (C₁-C₇)-Alkylaminocarbonylamino, (C₁-C₇)-Alkyl-[(C₁-C₇)-Alkyl]aminocarbonylamino, (C₁-C₇)-Alkylsulfonylamino, (C₃-C₇)-Cycloalkylsulfonylamino, Arylsulfonylamino, Hetarylsulfonylamino, Sulfonyl-(C₁-C₇)-haloalkylamino, Aminosulfonyl, Amino-(C₁-C₇)-alkylsulfonyl, Amino-(C₁-C₇)-haloalkylsulfonyl, (C₁-C₇)-Alkylaminosulfonyl, Bis[(C₁-C₇)-alkyl]aminosulfonyl, (C₃-C₇)-Cycloalkylaminosulfonyl, (C₁-C₇)-Haloalkylaminosulfonyl, Arylaminosulfonyl, Aryl-(C₁-C₇)-alkylaminosulfonyl, (C₁-C₇)-Alkylsulfonyl, (C₃-C₇)-Cycloalkylsulfonyl, Arylsulfonyl, (C₁-C₇)-Alkylsulfinyl, (C₃-C₇)-Cycloalkylsulfinyl, Arylsulfinyl, N,S-Bis[(C₁-C₇)-alkyl]sulfonimidoyl, S-(C₁-C₇)-Alkylsulfonimidoyl, (C₁-C₇)-Alkylsulfonylaminocarbonyl, (C₃-C₇)-Cycloalkylsulfonylaminocarbonyl, (C₃-C₇)-Cycloalkylaminosulfonyl, Aryl-(C₁-C₇)-alkylcarbonylamino, (C₃-C₇)-Cycloalkyl-(C₁-C₇)-alkylcarbonylamino, Heteroarylcarbonylamino, (C₁-C₇)-Alkoxy-(C₁-C₇)-alkylcarbonylamino, Hydroxy-(C₁-C₇)-alkylcarbonylamino, Tris[(C₁-C₇)-alkyl]silyl stehen,
A³, A⁴, A⁵ gleich oder verschieden sind und unabhängig voneinander für N (Stickstoff) oder die Gruppierung C-R⁸ stehen, wobei jedoch in keinem Fall mehr als zwei N-Atome benachbart sind, und wobei R⁸ in der Gruppierung C-R⁸ jeweils gleiche oder verschiedene Bedeutungen gemäß der nachstehenden Definition hat und
A³ und A⁴, wenn beide für eine Gruppe C- R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
A⁴ und A⁵, wenn beide für eine Gruppe C- R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
R⁸ und R¹⁴ unabhängig voneinander jeweils für Wasserstoff, Nitro, Amino, Hydroxy, Hydrothio, Thiocyanato, Isothiocyanato, Halogen, (C₁-C₇)-Alkyl, (C₃-C₇)-Cycloalkyl, (C₂-C₇)-Alkenyl, (C₂-C₇)-Alkinyl, Aryl, Aryl-(C₁-C₇)-alkyl, Aryl-(C₁-C₇)-alkoxy, Heteroaryl, (C₁-C₇)-Haloalkyl, (C₃-C₇)-Halocycloalkyl, (C₁-C₇)-Alkoxy, (C₁-C₇)-Haloalkoxy, Aryloxy, Heteroaryloxy, (C₃-C₇)-Cycloalkyloxy, (C₃-C₇)-Cycloalkyl-(C₁-C₇)-alkoxy, Hydroxy-(C₁-C₇)-alkyl, (C₁-C₇)-Alkoxy-(C₁-C₇)-alkyl, Aryloxy-(C₁-C₇)-alkyl, Heteroaryloxy-(C₁-C₇)-alkyl, (C₁-C₇)-Alkylamino, (C₁-C₇)-Alkylthio, (C₁-C₇)-Haloalkylthio, Bis[(C₁-C₇)-alkyl]amino, (C₃-C₇)-Cycloalkylamino, (C₁-C₇)-Alkylcarbonylamino, (C₃-C₇)-Cycloalkylcarbonylamino, Formylamino, (C₁-C₇)-Haloalkylcarbonylamino, (C₁-C₇)-Alkoxycarbonylamino, (C₁-C₇)-Alkylaminocarbonylamino, (C₁-C₇)-Alkyl[(C₁-C₇)-Alkyl]aminocarbonylamino, (C₁-C₇)-Alkylsulfonylamino, (C₃-C₇)-Cycloalkylsulfonylamino, Arylsulfonylamino, Hetarylsulfonylamino, Sulfonyl-(C₁-C₇)-haloalkylamino, Amino-(C₁-C₇)-alkylsulfonyl, Amino-(C₁-C₇)-haloalkylsulfonyl, (C₁-C₇)-Alkylaminosulfonyl, Bis[(C₁-C₇)-alkyl]aminosulfonyl, (C₃-C₇)-Cycloalkylaminosulfonyl, (C₁-C₇)-Haloalkylaminosulfonyl, Arylaminosulfonyl, Aryl-(C₁-C₇)-alkylaminosulfonyl, (C₁-C₇)-Alkylsulfonyl, (C₃-C₇)-Cycloalkylsulfonyl, Arylsulfonyl, (C₁-C₇)-Alkylsulfinyl, (C₃-C₇)-Cycloalkylsulfinyl, Arylsulfinyl, N,S-Bis[(C₁-C₇)-alkyl]sulfonimidoyl, S-(C₁-C₇)-Alkylsulfonimidoyl, (C₁-C₇)-Alkylsulfonylaminocarbonyl, (C₃-C₇)-Cycloalkylsulfonylaminocarbonyl, (C₃-C₇)-Cycloalkylaminosulfonyl, Aryl-(C₁-C₇)-alkylcarbonylamino, (C₃-C₇)-Cycloalkyl-(C₁-C₇)-alkylcarbonylamino, Heteroarylcarbonylamino, (C₁-C₇)-Alkoxy-(C₁-C₇)-alkylcarbonylamino, Hydroxy-(C₁-C₇)-alkylcarbonylamino, Cyano, Cyano-(C₁-C₇)-alkyl, Hydroxycarbonyl, (C₁-C₇)-Alkoxycarbonyl, (C₃-C₇)-Cycloalkoxycarbonyl, (C₃-C₇)-Cycloalkyl-(C₁-C₇)-alkoxycarbonyl, Aryloxycarbonyl, Aryl-(C₁-C₇)-alkoxycarbonyl, Aminocarbonyl, (C₁-C₇)-Alkylaminocarbonyl, Bis[(C₁-C₇)-alkyl]aminocarbonyl, (C₁-C₇)-Alkyl[(C₁-C₇)-Alkoxy]aminocarbonyl, (C₃-C₇)-Cycloalkylaminocarbonyl, Aryl-(C₁-C₇)-alkylaminocarbonyl, Heteroaryl-(C₁-C₇)-alkylaminocarbonyl, Cyano-(C₁-C₇)-alkylaminocarbonyl, (C₁-C₇)-Haloalkylaminocarbonyl, (C₂-C₇)-Alkinylaminocarbonyl, (C₁-C₇)-Alkoxycarbonylaminocarbonyl, Aryl-(C₁-C₇)-alkoxycarbonylaminocarbonyl, Hydroxycarbonyl-(C₁-C₇)-alkyl, (C₁-C₇)-Alkoxycarbonyl-(C₁-C₇)-alkyl, (C₃-C₇)-Cycloalkoxycarbonyl-(C₁-C₇)-alkyl, (C₃-C₇)-Cycloalkyl-(C₁-C₇)-alkoxycarbonyl-(C₁-C₇)-alkyl, (C₁-C₇)-Alkylaminocarbonyl-(C₁-C₇)-alkyl, Aminocarbonyl-(C₁-C₇)-alkyl, Bis[(C₁-C₇)-alkyl]aminocarbonyl-(C₁-C₇)-alkyl, (C₃-C₇)-Cycloalkylaminocarbonyl-(C₁-C₇)-alkyl, Aryl-(C₁-C₇)-alkylaminocarbonyl-(C₁-C₇)-alkyl, Heteroaryl-(C₁-C₇)-alkylaminocarbonyl-(C₁-C₇)-alkyl, Cyano-(C₁-C₇)-alkylaminocarbonyl-(C₁-C₇)-alkyl, (C₁-C₇)-Haloalkylaminocarbonyl-(C₁-C₇)-alkyl, (C₂-C₇)-Alkinylaminocarbonyl-(C₁-C₇)-alkyl, (C₃-C₇)-Cycloalkyl-(C₁-C₇)-alkylaminocarbonyl-(C₁-C₇)-alkyl, (C₁-C₇)-Alkoxycarbonylaminocarbonyl-(C₁-C₇)-alkyl, Aryl-(C₁-C₇)-alkoxycarbonylaminocarbonyl-(C₁-C₇)-alkyl, (C₁-C₇)-Alkoxycarbonyl-(C₁-C₇)-alkylaminocarbonyl, Hydroxycarbonyl-(C₁-C₇)-alkylaminocarbonyl, Aminocarbonyl-(C₁-C₇)-alkylaminocarbonyl, (C₁-C₇)-Alkylaminocarbonyl-(C₁-C₇)-alkylaminocarbonyl, (C₃-C₇)-Cycloalkylaminocarbonyl-(C₁-C₇)-alkylaminocarbonyl, (C₃-C₇)-Cycloalkyl-(C₁-C₇)-alkylaminocarbonyl, (C₃-C₇)-Cycloalkyl-(C₁-C₇)-alkylaminocarbonyl-(C₁-C₇)-alkyl, (C₂-C₇)-Alkenyloxycarbonyl, (C₂-C₇)-Alkenyloxycarbonyl-(C₁-C₇)-alkyl, (C₂-C₇)-Alkenylaminocarbonyl, (C₂-C₇)-Alkenylaminocarbonyl-(C₁-C₇)-alkyl, (C₁-C₇)-Alkylcarbonyl, (C₃-C₇)-Cycloalkylcarbonyl, Formyl, Hydroxyiminomethyl, Aminoiminomethyl, (C₁-C₇)-Alkoxyiminomethyl, (C₁-C₇)-Alkylaminoiminomethyl, Bis[(C₁-C₇)-alkyl]aminoiminomethyl, (C₃-C₇)-Cycloalkoxyiminomethyl, (C₃-C₇)-Cycloalkyl-(C₁-C₇)-alkoximinomethyl, Aryloximinomethyl, Aryl-(C₁-C₇)-alkoxyiminomethyl, Aryl-(C₁-C₇)-alkylaminoiminomethyl, (C₂-C₇)-Alkenyloxyiminomethyl, Arylaminoiminomethyl, Arylsulfonylaminoiminomethyl, Heteroaryl-(C₁-C₇)-alkyl, Heterocyclyl-(C₁-C₇)-alkyl, Hydroxycarbonylheterocyclyl, (C₁-C₇)-Alkoxycarbonylheterocyclyl, (C₂-C₇)-Alkenyloxycarbonylheterocyclyl, (C₂-C₇)-Alkenyl-(C₁-C₇)-alkoxycarbonylheterocyclyl, Aryl-(C₁-C₇)-alkoxycarbonylheterocyclyl, (C₃-C₇)-Cycloalkoxycarbonylheterocyclyl, (C₃-C₇)-Cycloalkyl-(C₁-C₇)-alkoxycarbonylheterocyclyl, Aminocarbonylheterocyclyl, (C₁-C₇)-Alkylaminocarbonylheterocyclyl, Bis-[(C₁-C₇)-Alkyl]aminocarbonylheterocyclyl, (C₃-C₇)-Cycloalkylaminocarbonylheterocyclyl, Aryl-(C₁-C₇)-alkylaminocarbonylheterocyclyl, (C₂-C₇)-Alkenylaminocarbonylheterocyclyl, Hydroxycarbonylheterocyclyl-(C₁-C₇)-alkyl, (C₁-C₇)-Alkoxycarbonylheterocyclyl-(C₁-C₇)-alkyl, Hydroxycarbonyl-(C₃-C₇)-cycloalkyl-(C₁-C₇)-alkyl, (C₁-C₇)-Alkoxycarbonyl-(C₃-C₇)-cycloalkyl-(C₁-C₇)-alkyl, Heterocyclyl, Heterocyclyloxy, Heterocyclylamino, Heterocyclylcarbonyl, Heterocyclylcarbonyl-(C₁-C₇)-alkyl, Hydroxycarbonylheterocyclylcarbonyl, (C₁-C₇)-Alkoxycarbonylheterocyclylcarbonyl, (C₂-C₇)-Alkenyloxycarbonylheterocyclylcarbonyl, (C₂-C₇)-Alkenyl-(C₁-C₇)-alkoxycarbonylheterocyclylcarbonyl, Aryl-(C₁-C₇)-alkoxycarbonylheterocyclylcarbonyl, (C₃-C₇)-Cycloalkoxycarbonylheterocyclylcarbonyl, (C₃-C₇)-Cycloalkyl-(C₁-C₇)-alkoxycarbonylheterocyclylcarbonyl, Aminocarbonylheterocyclylcarbonyl, (C₁-C₇)-Alkylaminocarbonylheterocyclylcarbonyl, Bis-[(C₁-C₇)-Alkyl]aminocarbonylheterocyclylcarbonyl, (C₃-C₇)-Cycloalkylaminocarbonylheterocyclylcarbonyl, Aryl-(C₁-C₇)-alkylaminocarbonylheterocyclylcarbonyl, (C₂-C₇)-Alkenylaminocarbonylheterocyclylcarbonyl stehen,
R⁹ und R¹⁰ unabhängig voneinander für Wasserstoff, Halogen, (C₁-C₇)-Alkyl, (C₁-C₇)-Alkoxy, (C₁-C₇)-Haloalkyl, (C₁-C₇)-Haloalkoxy, Aryl, Heteroaryl, Aryl-(C₁-C₇)-alkyl stehen oder gemeinsam mit dem Atom, an das sie gebunden sind, eine Carbonylgruppe bilden,
A⁶, A⁷ gleich oder verschieden sind und unabhängig voneinander für N-R¹¹, Sauerstoff, Schwefel oder die Gruppierung CR¹⁵R¹⁶ stehen, wobei jedoch in keinem Fall mehr als ein Sauerstoffatom im Heterocyclus enthalten ist, und wobei R¹¹, R¹⁵, R¹⁶ in den Gruppierungen N-R¹¹ und CR¹⁵R¹⁶ jeweils gleiche oder verschiedene Bedeutungen gemäß der nachstehenden Definition haben,
R¹¹ für Wasserstoff, (C₁-C₇)-Alkyl, (C₂-C₇)-Alkenyl, (C₂-C₇)-Alkinyl, (C₁-C₇)-Alkoxy-(C₁-C₇)-alkyl, (C₁-C₇)-Alkylcarbonyl, Arylcarbonyl, Heteroarylcarbonyl, (C₃-C₇)-Cycloalkylcarbonyl, (C₁-C₇)-Alkoxycarbonyl, (C₂-C₇)-Alkenyloxycarbonyl, (C₁-C₇)-Alkoxycarbonyl-(C₁-C₇)-alkyl, (C₁-C₇)-Alkylaminocarbonyl-(C₁-C₇)-alkyl, Arylaminocarbonyl-(C₁-C₇)-alkyl, Aryloxy-(C₁-C₇)-alkyl, Aryl-(C₁-C₇)-alkyl, Heteroaryl-(C₁-C₇)-alkyl, Heterocyclyl, Heterocyclyl-(C₁-C₇)-alkyl steht,
R¹² und R¹³ unabhängig voneinander für Wasserstoff, Halogen, (C₁-C₇)-Alkyl, (C₁-C₇)-Alkoxy, (C₁-C₇)-Haloalkyl, (C₁-C₇)-Haloalkoxy, Aryl, Heteroaryl, Aryl-(C₁-C₇)-alkyl, Heterocyclyl, Heteroaryl-(C₁-C₇)-alkyl, Heterocyclyl-(C₁-C₇)-alkyl stehen oder gemeinsam mit dem Atom, an das sie gebunden sind, eine Carbonylgruppe bilden und
R¹⁵ und R¹⁶ unabhängig voneinander für Wasserstoff, (C₁-C₇)-Alkyl, (C₂-C₇)-Alkenyl, (C₂-C₇)-Alkinyl, (C₁-C₇)-Alkoxy, (C₁-C₇)-Alkoxy-(C₁-C₇)-alkyl, (C₁-C₇)-Alkylcarbonyl, Arylcarbonyl, Heteroarylcarbonyl, Hydroxycarbonyl, (C₁-C₇)-Alkoxycarbonyl, Heterocyclyl, (C₁-C₇)-Haloalkyl stehen,
mit Ausnahme von 1-[(2E)-1-Hydroxy-1,3-diphenylprop-2-en-1-yl]cyclopentancarbonitril.

Ganz besonders bevorzugt sind Verbindungen der allgemeinen Formel (I), worin
[X-Y] für die Gruppierungen 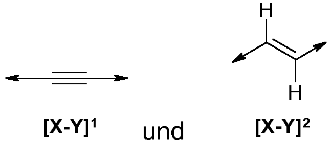 steht,
Q für die carbocyclischen und heterocyclischen Gruppierungen 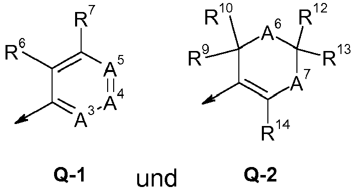 steht, wobei die Gruppierungen R⁶, R⁷, R⁹, R¹⁰, R¹², R¹³, R¹⁴, A³, A⁴, A⁵, A⁶ und A⁷ jeweils die Bedeutung gemäß der nachstehenden Definitionen haben und wobei der Pfeil für eine Bindung zur jeweiligen Gruppierung [X-Y] steht,
R¹ für Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, Cyclopropylmethyl, Cyclobutylmethyl, Cyclopentylmethyl, Cyclohexylmethyl, gegebenenfalls substituiertes Phenyl, Heteroaryl, Heterocyclyl, Cyclopropyl, Cyclobutyl, Cyclopentyl, Cyclohexyl, , Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Allylcyclopropyl, 1-Vinylcyclobutyl, 1-Vinylcyclopropyl, 1-Ethylcyclopropyl, 1-Methylcyclohexyl, 2-Methylcyclohexyl, 3-Methylcyclohexyl, 1-Methoxycyclohexyl, 2-Methoxycyclohexyl, 3-Methoxycyclohexyl, Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl,Methoxymethyl, Ethoxymethyl, Ethoxyethyl, Methoxyethyl, Methoxy-n-propyl, Ethoxy-n-propyl, Methoxybutyl, Methoxy-iso-Propyl, iso-Propoxymethyl, iso-Propoxyethyl, Hydroxymethyl, Hydroxyethyl, Hydroxypropyl, Hydroxybutyl, Trifluormethyl, Pentafluorethyl, 1,1,2,2-Tetrafluorethyl, Heptafluorpropyl, Nonafluorbutyl, Chlordifluormethyl, Bromdifluormethyl, Dichlorfluormethyl, Difluormethyl, 2,2-Difluorethyl, 2,2,2-Trifluorethyl, Difluor-tert.-butyl, Trifluormethoxymethyl, Trifluormethoxyethyl, Trifluormethoxy-n-propyl, Difluormethoxymethyl, Difluormethoxyethyl, Difluormethoxy-n-propyl, 2,2-Difluorethoxymethyl, 2,2-Difluorethoxyethyl, 2,2-Difluorethoxy-n-propyl, 2,2,2-Trifluorethoxymethyl, 2,2,2-Trifluorethoxyethyl, 2,2,2-Trifluorethoxy-n-propyl, Methylthiomethyl, Methylthioethyl, Ethylthioethyl, Methylthio-n-propyl, Ethylthio-n-propyl, Aryl-(C₁-C₆)-alkyl, Heterocyclyl-(C₁-C₆)-alkyl, (C₃-C₆)-Halocycloalkyl, 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, Methoxymethoxymethyl, Methoxyethoxymethyl, Methoxyethoxyethyl, Methoxymethoxyethyl, Ethoxy-n-propoxymethyl, Ethoxy-n-propoxyethyl, Ethoxyethoxymethyl, Ethoxyethoxyethyl steht,
R² für Wasserstoff, tert.-Butyldimethylsilyl, Trimethylsilyl, Triethylsilyl, Tri-(iso-Propyl)silyl, Tri-(n-Propyl)silyl, Dimethyl(phenyl)silyl, tert.-Butyldiphenylsilyl, Diethylisopropylsilyl, Isopropyldimethylsilyl, tert.-Hexyldimethylsilyl, 2-(Trimethylsilyl)ethoxymethyl, 2-(Trimethylsilyl)ethyl, Methyl, Ethyl, n-Propyl, iso-Propyl, n-Butyl, iso-Butyl, n-Pentyl, iso-Pentyl, Methoxymethyl, Ethoxymethyl, Ethoxyethyl, Methoxyethyl, Methoxy-n-propyl, Ethoxy-n-propyl, Methoxybutyl, Methoxy-iso-Propyl, iso-Propoxymethyl, iso-Propoxyethyl, Methoxymethoxymethyl, Methoxyethoxymethyl, Methoxyethoxyethyl, Methoxymethoxyethyl, Ethoxy-n-propoxymethyl, Ethoxy-n-propoxyethyl, Ethoxyethoxymethyl, Ethoxyethoxyethyl, Allyl, Methylcarbonyl, Ethylcarbonyl, n-Propylcarbonyl, iso-Propylcarbonyl, n-Butylcarbonyl, n-Pentylcarbonyl, tert-Butylcarbonyl, iso-Butylcarbonyl, iso-Pentylcarbonyl, neo-Pentylcarbonyl, n-Hexylcarbonyl, Arylcarbonyl, Heteroarylcarbonyl, Cyclopropylcarbonyl, Cyclobutylcarbonyl, Cyclopentylcarbonyl, Cyclohexylcarbonyl, (C₂-C₆)-Alkenylcarbonyl, Heterocyclylcarbonyl, Methoxycarbonyl, Ethoxycarbonyl, n-Propoxycarbonyl, n-Butyloxycarbonyl, iso-Propoxycarbonyl, iso-Butyloxycarbonyl, tert-Butyloxycarbonyl, Allyloxycarbonyl, Phenyloxycarbonyl, p-Cl-Phenyloxycarbonyl, Benzyloxycarbonyl, p-Cl-Benzyloxycarbonyl, p-Methoxybenzyloxycarbonyl, p-Methylbenzyloxycarbonyl, Cyclopropyloxycarbonyl, Cyclobutyloxycarbonyl, Cyclopentyloxycarbonyl, Cyclohexyloxycarbonyl, Benzyl, p-Cl-Benzyl, p-F-Benzyl, p-Methoxybenzyl, p-Methylbenzyl, Methylthiomethyl, Methylthioethyl, Ethylthioethyl, Methylthio-n-propyl, Ethylthio-n-propyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1 steht,
n für 0, 1 steht,
R³ und R⁴ unabhängig voneinander für Wasserstoff, Methyl, Ethyl, n-Propyl, iso-Propyl, n-Butyl, iso-Butyl, tert.-Butyl, n-Hexyl, Fluor, Chlor, Brom, lod, Cyclopropyl, Cyclobutyl, Cyclopentyl, Cyclohexyl, Cyclopropyl-cyclopropyl, Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Bicyclo[2.2.2]octan-2-yl, Adamantan-1-yl und Adamantan-2-yl, Methoxy, Ethoxy, n-Propoxy, iso-Propoxy, n-Butyloxy, tert.-Butyloxy, iso-Butyloxy, n-Pentyloxy, Aryl, Heterocyclyl, Heteroaryl, Benzyl, p-Cl-Benzyl, p-F-Benzyl, p-Methoxybenzyl, p-Methylbenzyl, Methylthio, Ethylthio, n-Propylthio, n-Butylthio, n-Pentylthio, iso-Propylthio, iso-Butylthio, tert-Butylthio, n-Pentylthio, Trifluormethyl, Pentafluorethyl, 1,1,2,2-Tetrafluorethyl, Heptafluorpropyl, Nonafluorbutyl, Chlordifluormethyl, Bromdifluormethyl, Dichlorfluormethyl, Bromfluormethyl, 1-Fluorethyl, 2-Fluorethyl, Fluormethyl, Difluormethyl, 2,2-Difluorethyl, 2,2,2-Trifluorethyl, 2,2-Dichlor-2-fluororethyl, 2-Chlor-2,2-difluorethyl, Difluor-tert.-butyl, Trifluormethoxymethyl, Trifluormethoxyethyl, Trifluormethoxy-n-propyl, Difluormethoxymethyl, Difluormethoxyethyl, Difluormethoxy-n-propyl, 2,2-Difluorethoxymethyl, 2,2-Difluorethoxyethyl, 2,2-Difluorethoxy-n-propyl, 2,2,2-Trifluorethoxymethyl, 2,2,2-Trifluorethoxyethyl, 2,2,2-Trifluorethoxy-n-propyl, , Vinyl, Prop-1-en-1-yl, But-1-en-1-yl, Allyl, 1-Methyl-prop-2-en-1-yl, 2-Methyl-prop-2-en-1-yl, But-2-en-1-yl, 1-Methyl-but-3-en-1-yl und 1-Methyl-but-2-en-1-yl, 2-Methyl-prop-1-en-1-yl, But-3-en-1-yl, Pentenyl, 2-Methylpentenyl, Hexenyl, Ethinyl, Propargyl, 1-Methyl-prop-2-in-1-yl, 2-Butinyl, 2-Pentinyl, 2-Hexinyl, But-2-in-1-yl, But-3-in-1-yl, 1-Methyl-but-3-in-1-yl, Trifluormethoxy, Difluormethoxy, 2,2-Difluorethoxy, 2,2,2-Trifluorethoxy, Trifluormethylthio, Methoxymethyl, Ethoxymethyl, Ethoxyethyl, Methoxyethyl, Methoxy-n-propyl, Ethoxy-n-propyl, Methoxybutyl, Methoxy-iso-Propyl, iso-Propoxymethyl, iso-Propoxyethyl, Methylthiomethyl, Heteroaryl-(C₁-C₆)-alkyl, Heterocyclyl-(C₁-C₆)-alkyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden,
R⁶ und R⁷ unabhängig voneinander für Wasserstoff, Nitro, Amino, Cyano, Thiocyanato, Isothiocyanato, Fluor, Chlor, Brom, lod, (C₁-C₆)-Alkyl, (C₃-C₆)-Cycloalkyl, (C₂-C₆)-Alkenyl, (C₂-C₆)-Alkinyl, gegebenenfalls substituiertes Phenyl, Aryl-(C₁-C₆)-alkyl, Aryl-(C₁-C₆)-alkoxy, Heteroaryl, (C₁-C₆)-Alkoxy-(C₁-C₆)-alkyl, Hydroxy-(C₁-C₆)-alkyl, (C₁-C₆)-Haloalkyl, (C₃-C₆)-Halocycloalkyl, (C₁-C₆)-Alkoxy, (C₁-C₆)-Haloalkoxy, Aryloxy, Heteroaryloxy, (C₃-C₆)-Cycloalkyloxy, Hydroxy, (C₃-C₆)-Cycloalkyl-(C₁-C₆)-alkoxy, (C₁-C₆)-Alkoxycarbonyl, Hydroxycarbonyl, Aminocarbonyl, (C₁-C₆)-Alkylaminocarbonyl, (C₃-C₆)-Cycloalkylaminocarbonyl, Cyano-(C₁-C₆)-alkylaminocarbonyl, (C₂-C₆)-Alkenylaminocarbonyl, (C₂-C₆)-Alkinylaminocarbonyl, (C₁-C₆)-Alkylamino, (C₁-C₆)-Alkylthio, (C₁-C₆)-Haloalkylthio, Hydrothio, Bis-[(C₁-C₆)-alkyl]amino, (C₃-C₆)-Cycloalkylamino, (C₁-C₆)-Alkylcarbonylamino, (C₃-C₆)-Cycloalkylcarbonylamino, Formylamino, (C₁-C₆)-Haloalkylcarbonylamino, (C₁-C₆)-Alkoxycarbonylamino, (C₁-C₆)-Alkylaminocarbonylamino, (C₁-C₆)-Alkyl-[(C₁-C₆)-Alkyl]aminocarbonylamino, (C₁-C₆)-Alkylsulfonylamino, (C₃-C₆)-Cycloalkylsulfonylamino, Arylsulfonylamino, Hetarylsulfonylamino, Sulfonyl-(C₁-C₆)-haloalkylamino, Aminosulfonyl, Amino-(C₁-C₆)-alkylsulfonyl, Amino-(C₁-C₆)-haloalkylsulfonyl, (C₁-C₆)-Alkylaminosulfonyl, Bis[(C₁-C₆)-alkyl]aminosulfonyl, (C₃-C₆)-Cycloalkylaminosulfonyl, (C₁-C₆)-Haloalkylaminosulfonyl, Arylaminosulfonyl, Aryl-(C₁-C₆)-alkylaminosulfonyl, (C₁-C₆)-Alkylsulfonyl, (C₃-C₆)-Cycloalkylsulfonyl, Arylsulfonyl, (C₁-C₆)-Alkylsulfinyl, (C₃-C₆)-Cycloalkylsulfinyl, Arylsulfinyl, N,S-Bis[(C₁-C₆)-alkyl]sulfonimidoyl, S-(C₁-C₆)-Alkylsulfonimidoyl, (C₁-C₆)-Alkylsulfonylaminocarbonyl, (C₃-C₆)-Cycloalkylsulfonylaminocarbonyl, (C₃-C₆)-Cycloalkylaminosulfonyl, Aryl-(C₁-C₆)-alkylcarbonylamino, (C₃-C₆)-Cycloalkyl-(C₁-C₆)-alkylcarbonylamino, Heteroarylcarbonylamino, (C₁-C₆)-Alkoxy-(C₁-C₆)-alkylcarbonylamino, Hydroxy-(C₁-C₆)-alkylcarbonylamino, Tris[(C₁-C₆)-alkyl]silyl stehen,
A³, A⁴, A⁵ gleich oder verschieden sind und unabhängig voneinander für N (Stickstoff) oder die Gruppierung C-R⁸ stehen, wobei jedoch in keinem Fall mehr als zwei N-Atome benachbart sind, und wobei R⁸ in der Gruppierung C-R⁸ jeweils gleiche oder verschiedene Bedeutungen gemäß der nachstehenden Definition hat und
A³ und A⁴, wenn beide für eine Gruppe C-R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
A⁴ und A⁵, wenn beide für eine Gruppe C- R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
R⁸ und R¹⁴ unabhängig voneinander jeweils für Wasserstoff, Nitro, Amino, Hydroxy, Hydrothio, Thiocyanato, Isothiocyanato, Fluor, Chlor, Brom, lod, (C₁-C₆)-Alkyl, (C₃-C₆)-Cycloalkyl, (C₂-C₆)-Alkenyl, (C₂-C₆)-Alkinyl, gegebenenfalls substituiertes Phenyl, Aryl-(C₁-C₆)-alkyl, Aryl-(C₁-C₆)-alkoxy, Heteroaryl, (C₁-C₆)-Haloalkyl, (C₃-C₆)-Halocycloalkyl, (C₁-C₆)-Alkoxy, (C₁-C₆)-Haloalkoxy, Aryloxy, Heteroaryloxy, (C₃-C₆)-Cycloalkyloxy, (C₃-C₆)-Cycloalkyl-(C₁-C₆)-alkoxy, Hydroxy-(C₁-C₆)-alkyl, (C₁-C₆)-Alkoxy-(C₁-C₆)-alkyl, Aryloxy-(C₁-C₆)-alkyl, Heteroaryloxy-(C₁-C₆)-alkyl, (C₁-C₆)-Alkylamino, (C₁-C₆)-Alkylthio, (C₁-C₆)-Haloalkylthio, Bis[(C₁-C₆)-alkyl]amino, (C₃-C₆)-Cycloalkylamino, (C₁-C₆)-Alkylcarbonylamino, (C₃-C₆)-Cycloalkylcarbonylamino, Formylamino, (C₁-C₆)-Haloalkylcarbonylamino, (C₁-C₆)-Alkoxycarbonylamino, (C₁-C₆)-Alkylaminocarbonylamino, (C₁-C₆)-Alkyl[(C₁-C₆)-Alkyl]aminocarbonylamino, (C₁-C₆)-Alkylsulfonylamino, (C₃-C₆)-Cycloalkylsulfonylamino, Arylsulfonylamino, Hetarylsulfonylamino, Sulfonyl-(C₁-C₆)-haloalkylamino, Amino-(C₁-C₆)-alkylsulfonyl, Amino-(C₁-C₆)-haloalkylsulfonyl, (C₁-C₆)-Alkylaminosulfonyl, Bis[(C₁-C₆)-alkyl]aminosulfonyl, (C₃-C₆)-Cycloalkylaminosulfonyl, (C₁-C₆)-Haloalkylaminosulfonyl, Arylaminosulfonyl, Aryl-(C₁-C₆)-alkylaminosulfonyl, (C₁-C₆)-Alkylsulfonyl, (C₃-C₆)-Cycloalkylsulfonyl, Arylsulfonyl, (C₁-C₆)-Alkylsulfinyl, (C₃-C₆)-Cycloalkylsulfinyl, Arylsulfinyl, N,S-Bis[(C₁-C₆)-alkyl]sulfonimidoyl, S-(C₁-C₆)-Alkylsulfonimidoyl, (C₁-C₆)-Alkylsulfonylaminocarbonyl, (C₃-C₆)-Cycloalkylsulfonylaminocarbonyl, (C₃-C₆)-Cycloalkylaminosulfonyl, Aryl-(C₁-C₆)-alkylcarbonylamino, (C₃-C₆)-Cycloalkyl-(C₁-C₆)-alkylcarbonylamino, Heteroarylcarbonylamino, (C₁-C₆)-Alkoxy-(C₁-C₆)-alkylcarbonylamino, Hydroxy-(C₁-C₆)-alkylcarbonylamino, Cyano, Cyano-(C₁-C₆)-alkyl, Hydroxycarbonyl, (C₁-C₆)-Alkoxycarbonyl, (C₃-C₆)-Cycloalkoxycarbonyl, (C₃-C₆)-Cycloalkyl-(C₁-C₆)-alkoxycarbonyl, Aryloxycarbonyl, Aryl-(C₁-C₆)-alkoxycarbonyl, Aminocarbonyl, (C₁-C₆)-Alkylaminocarbonyl, Bis[(C₁-C₆)-alkyl]aminocarbonyl, (C₁-C₆)-Alkyl[(C₁-C₆)-Alkoxy]aminocarbonyl, (C₃-C₆)-Cycloalkylaminocarbonyl, Aryl-(C₁-C₆)-alkylaminocarbonyl, Heteroaryl-(C₁-C₆)-alkylaminocarbonyl, Cyano-(C₁-C₆)-alkylaminocarbonyl, (C₁-C₆)-Haloalkylaminocarbonyl, (C₂-C₆)-Alkinylaminocarbonyl, (C₁-C₆)-Alkoxycarbonylaminocarbonyl, Aryl-(C₁-C₆)-alkoxycarbonylaminocarbonyl, Hydroxycarbonyl-(C₁-C₆)-alkyl, (C₁-C₆)-Alkoxycarbonyl-(C₁-C₆)-alkyl, (C₃-C₆)-Cycloalkoxycarbonyl-(C₁-C₆)-alkyl, (C₃-C₆)-Cycloalkyl-(C₁-C₆)-alkoxycarbonyl-(C₁-C₆)-alkyl, (C₁-C₆)-Alkylaminocarbonyl-(C₁-C₆)-alkyl, Aminocarbonyl-(C₁-C₆)-alkyl, Bis[(C₁-C₆)-alkyl]aminocarbonyl-(C₁-C₆)-alkyl, (C₃-C₆)-Cycloalkylaminocarbonyl-(C₁-C₆)-alkyl, Aryl-(C₁-C₆)-alkylaminocarbonyl-(C₁-C₆)-alkyl, Heteroaryl-(C₁-C₆)-alkylaminocarbonyl-(C₁-C₆)-alkyl, Cyano-(C₁-C₆)-alkylaminocarbonyl-(C₁-C₆)-alkyl, (C₁-C₆)-Haloalkylaminocarbonyl-(C₁-C₆)-alkyl, (C₂-C₆)-Alkinylaminocarbonyl-(C₁-C₆)-alkyl, (C₃-C₆)-Cycloalkyl-(C₁-C₆)-alkylaminocarbonyl-(C₁-C₆)-alkyl, (C₁-C₆)-Alkoxycarbonylaminocarbonyl-(C₁-C₆)-alkyl, Aryl-(C₁-C₆)-alkoxycarbonylaminocarbonyl-(C₁-C₆)-alkyl, (C₁-C₆)-Alkoxycarbonyl-(C₁-C₆)-alkylaminocarbonyl, Hydroxycarbonyl-(C₁-C₆)-alkylaminocarbonyl, Aminocarbonyl-(C₁-C₆)-alkylaminocarbonyl, (C₁-C₆)-Alkylaminocarbonyl-(C₁-C₆)-alkylaminocarbonyl, (C₃-C₆)-Cycloalkylaminocarbonyl-(C₁-C₆)-alkylaminocarbonyl, (C₃-C₆)-Cycloalkyl-(C₁-C₆)-alkylaminocarbonyl, (C₃-C₆)-Cycloalkyl-(C₁-C₆)-alkylaminocarbonyl-(C₁-C₆)-alkyl, (C₂-C₆)-Alkenyloxycarbonyl, (C₂-C₆)-Alkenyloxycarbonyl-(C₁-C₆)-alkyl, (C₂-C₆)-Alkenylaminocarbonyl, (C₂-C₆)-Alkenylaminocarbonyl-(C₁-C₆)-alkyl, (C₁-C₆)-Alkylcarbonyl, (C₃-C₆)-Cycloalkylcarbonyl, Formyl, Hydroxyiminomethyl, Aminoiminomethyl, (C₁-C₆)-Alkoxyiminomethyl, (C₁-C₆)-Alkylaminoiminomethyl, Bis[(C₁-C₆)-alkyl]aminoiminomethyl, (C₃-C₆)-Cycloalkoxyiminomethyl, (C₃-C₆)-Cycloalkyl-(C₁-C₆)-alkoximinomethyl, Aryloximinomethyl, Aryl-(C₁-C₆)-alkoxyiminomethyl, Aryl-(C₁-C₆)-alkylaminoiminomethyl, (C₂-C₆)-Alkenyloxyiminomethyl, Arylaminoiminomethyl, Arylsulfonylaminoiminomethyl, Heteroaryl-(C₁-C₆)-alkyl, Heterocyclyl-(C₁-C₆)-alkyl, Hydroxycarbonylheterocyclyl, (C₁-C₆)-Alkoxycarbonylheterocyclyl, (C₂-C₆)-Alkenyloxycarbonylheterocyclyl, (C₂-C₆)-Alkenyl-(C₁-C₆)-alkoxycarbonylheterocyclyl, Aryl-(C₁-C₆)-alkoxycarbonylheterocyclyl, (C₃-C₆)-Cycloalkoxycarbonylheterocyclyl, (C₃-C₆)-Cycloalkyl-(C₁-C₆)-alkoxycarbonylheterocyclyl, Aminocarbonylheterocyclyl, (C₁-C₆)-Alkylaminocarbonylheterocyclyl, Bis-[(C₁-C₆)-Alkyl]aminocarbonylheterocyclyl, (C₃-C₆)-Cycloalkylaminocarbonylheterocyclyl, Aryl-(C₁-C₆)-alkylaminocarbonylheterocyclyl, (C₂-C₆)-Alkenylaminocarbonylheterocyclyl, Hydroxycarbonylheterocyclyl-(C₁-C₆)-alkyl, (C₁-C₆)-Alkoxycarbonylheterocyclyl-(C₁-C₆)-alkyl, Hydroxycarbonyl-(C₃-C₆)-cycloalkyl-(C₁-C₆)-alkyl, (C₁-C₆)-Alkoxycarbonyl-(C₃-C₆)-cycloalkyl-(C₁-C₆)-alkyl, Heterocyclyl, Heterocyclyloxy, Heterocyclylamino, Heterocyclylcarbonyl, Heterocyclylcarbonyl-(C₁-C₆)-alkyl, Hydroxycarbonylheterocyclylcarbonyl, (C₁-C₆)-Alkoxycarbonylheterocyclylcarbonyl, (C₂-C₆)-Alkenyloxycarbonylheterocyclylcarbonyl, (C₂-C₆)-Alkenyl-(C₁-C₆)-alkoxycarbonylheterocyclylcarbonyl, Aryl-(C₁-C₆)-alkoxycarbonylheterocyclylcarbonyl, (C₃-C₆)-Cycloalkoxycarbonylheterocyclylcarbonyl, (C₃-C₆)-Cycloalkyl-(C₁-C₆)-alkoxycarbonylheterocyclylcarbonyl, Aminocarbonylheterocyclylcarbonyl, (C₁-C₆)-Alkylaminocarbonylheterocyclylcarbonyl, Bis-[(C₁-C₆)-Alkyl]aminocarbonylheterocyclylcarbonyl, (C₃-C₆)-Cycloalkylaminocarbonylheterocyclylcarbonyl, Aryl-(C₁-C₆)-alkylaminocarbonylheterocyclylcarbonyl, (C₂-C₆)-Alkenylaminocarbonylheterocyclylcarbonyl stehen,
R⁹ und R¹⁰ unabhängig voneinander für Wasserstoff, Fluor, Chlor, Brom, lod, (C₁-C₆)-Alkyl, (C₁-C₆)-Alkoxy, (C₁-C₆)-Haloalkyl, (C₁-C₆)-Haloalkoxy, gegebenenfalls substituiertes Phenyl, Heteroaryl, Aryl-(C₁-C₆)-alkyl stehen oder gemeinsam mit dem Atom, an das sie gebunden sind, eine Carbonylgruppe bilden,
A⁶, A⁷ gleich oder verschieden sind und unabhängig voneinander für N-R¹¹, Sauerstoff, Schwefel oder die Gruppierung CR¹⁵R¹⁶ stehen, wobei jedoch in keinem Fall mehr als ein Sauerstoffatom im Heterocyclus enthalten ist, und wobei R¹¹, R¹⁵, R¹⁶ in den Gruppierungen N-R¹¹ und CR¹⁵R¹⁶ jeweils gleiche oder verschiedene Bedeutungen gemäß der nachstehenden Definition haben,
R¹¹ für Wasserstoff, (C₁-C₆)-Alkyl, (C₂-C₆)-Alkenyl, (C₂-C₆)-Alkinyl, (C₁-C₆)-Alkoxy-(C₁-C₆)-alkyl, (C₁-C₆)-Alkylcarbonyl, Arylcarbonyl, Heteroarylcarbonyl, (C₃-C₆)-Cycloalkylcarbonyl, (C₁-C₆)-Alkoxycarbonyl, (C₂-C₆)-Alkenyloxycarbonyl, (C₁-C₆)-Alkoxycarbonyl-(C₁-C₆)-alkyl, (C₁-C₆)-Alkylaminocarbonyl-(C₁-C₆)-alkyl, Arylaminocarbonyl-(C₁-C₆)-alkyl, Aryloxy-(C₁-C₆)-alkyl, Aryl-(C₁-C₆)-alkyl, Heteroaryl-(C₁-C₆)-alkyl, Heterocyclyl, Heterocyclyl-(C₁-C₆)-alkyl, steht,
R¹² und R¹³ unabhängig voneinander für Wasserstoff, Fluor, Chlor, Brom, lod, (C₁-C₆)-Alkyl, (C₁-C₆)-Alkoxy, (C₁-C₆)-Haloalkyl, (C₁-C₆)-Haloalkoxy, gegebenenfalls substituiertes Phenyl, Heteroaryl, Aryl-(C₁-C₆)-alkyl, Heterocyclyl, Heteroaryl-(C₁-C₆)-alkyl, Heterocyclyl-(C₁-C₆)-alkyl stehen oder gemeinsam mit dem Atom, an das sie gebunden sind, eine Carbonylgruppe bilden und
R¹⁵ und R¹⁶ unabhängig voneinander für Wasserstoff, (C₁-C₇)-Alkyl, (C₂-C₇)-Alkenyl, (C₂-C₇)-Alkinyl, (C₁-C₇)-Alkoxy, (C₁-C₇)-Alkoxy-(C₁-C₇)-alkyl, (C₁-C₇)-Alkylcarbonyl, Arylcarbonyl, Heteroarylcarbonyl, Hydroxycarbonyl, (C₁-C₇)-Alkoxycarbonyl, Heterocyclyl, (C₁-C₇)-Haloalkyl stehen,
mit Ausnahme von 1-[(2E)-1-Hydroxy-1,3-diphenylprop-2-en-1-yl]cyclopentan-carbonitril.

Im Speziellen bevorzugt sind Verbindungen der allgemeinen Formel (I), worin
[X-Y] für die Gruppierungen 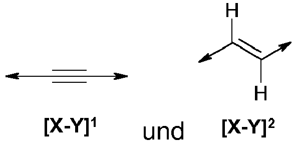 steht,
R¹ für Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropylmethyl, Cyclobutylmethyl, Cyclopentylmethyl, Cyclohexylmethyl, gegebenenfalls substituiertes Phenyl, Heteroaryl, Heterocyclyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Methylcyclohexyl, 2-Methylcyclohexyl, 3-Methylcyclohexyl, 1-Methoxycyclohexyl, 2-Methoxycyclohexyl, 3-Methoxycyclohexyl, Aryl-(C₁-C₅)-alkyl, Heterocyclyl-(C₁-C₅)-alkyl steht,
R² für Wasserstoff, tert.-Butyldimethylsilyl, Trimethylsilyl, Triethylsilyl, Tri-(iso-Propyl)silyl, Tri-(n-Propyl)silyl, Dimethyl(phenyl)silyl, tert.-Butyldiphenylsilyl, Diethylisopropylsilyl, Isopropyldimethylsilyl, tert.-Hexyldimethylsilyl, 2-(Trimethylsilyl)ethoxymethyl, 2-(Trimethylsilyl)ethyl, Methyl, Ethyl, Allyl, Methylcarbonyl, Ethylcarbonyl, n-Propylcarbonyl, iso-Propylcarbonyl, n-Butylcarbonyl, tert-Butylcarbonyl, iso-Butylcarbonyl, iso-Pentylcarbonyl, neo-Pentylcarbonyl, Arylcarbonyl, Heteroarylcarbonyl, Cyclopropylcarbonyl, Cyclobutylcarbonyl, Cyclopentylcarbonyl, Cyclohexylcarbonyl, Heterocyclylcarbonyl, Methoxycarbonyl, Ethoxycarbonyl, n-Propoxycarbonyl, n-Butyloxycarbonyl, iso-Propoxycarbonyl, iso-Butyloxycarbonyl, tert-Butyloxycarbonyl, Allyloxycarbonyl, Benzyl, p-CI-Benzyl, p-F-Benzyl, p-Methoxybenzyl, p-Methylbenzyl, Methylthiomethyl, Methylthioethyl, Ethylthioethyl, Methylthio-n-propyl, Ethylthio-n-propyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1 steht,
n für 0, 1 steht,
R³ und R⁴ unabhängig voneinander für Wasserstoff, Methyl, Ethyl, n-Propyl, iso-Propyl, n-Butyl, iso-Butyl, tert.-Butyl, Fluor, Chlor, Brom, lod, Cyclopropyl, Cyclobutyl, Cyclopentyl, Cyclohexyl, Methoxy, Ethoxy, n-Propoxy, iso-Propoxy, n-Butyloxy, tert.-Butyloxy, iso-Butyloxy, n-Pentyloxy, gegebenenfalls substituiertes Phenyl, Heterocyclyl, Heteroaryl, Benzyl, p-Cl-Benzyl, p-F-Benzyl, p-Methoxybenzyl, p-Methylbenzyl, Methylthio, Ethylthio, n-Propylthio, n-Butylthio, n-Pentylthio, iso-Propylthio, iso-Butylthio, Trifluormethyl, Pentafluorethyl, 1,1,2,2-Tetrafluorethyl, Heptafluorpropyl, Nonafluorbutyl, Chlordifluormethyl, Bromdifluormethyl, Dichlorfluormethyl, Bromfluormethyl, 1-Fluorethyl, 2-Fluorethyl, Fluormethyl, Difluormethyl, 2,2-Difluorethyl, 2,2,2-Trifluorethyl, 2,2-Dichlor-2-fluororethyl, 2-Chlor-2,2-difluorethyl, Difluor-tert.-butyl, Trifluormethoxymethyl, Trifluormethoxyethyl, Trifluormethoxy-n-propyl, Difluormethoxymethyl, Difluormethoxyethyl, Difluormethoxy-n-propyl, 2,2-Difluorethoxymethyl, 2,2-Difluorethoxyethyl, 2,2-Difluorethoxy-n-propyl, 2,2,2-Trifluorethoxymethyl, 2,2,2-Trifluorethoxyethyl, 2,2,2-Trifluorethoxy-n-propyl, Vinyl, Prop-1-en-1-yl, But-1-en-1-yl, Allyl, 1-Methyl-prop-2-en-1-yl, 2-Methyl-prop-2-en-1-yl, But-2-en-1-yl, 1-Methyl-but-3-en-1-yl und 1-Methyl-but-2-en-1-yl, Ethinyl, Propargyl, 1-Methyl-prop-2-in-1-yl, 2-Butinyl, 2-Pentinyl, Trifluormethoxy, Difluormethoxy, 2,2-Difluorethoxy, 2,2,2-Trifluorethoxy, Trifluormethylthio, Methoxymethyl, Ethoxymethyl, Ethoxyethyl, Methoxyethyl, Methoxy-n-propyl, Ethoxy-n-propyl, Methoxybutyl, Methoxy-iso-Propyl, iso-Propoxymethyl, iso-Propoxyethyl, Methylthiomethyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden
und Q für eine der in der folgenden Tabelle beschriebenen Gruppierungen Q-1.1 bis Q-2.24 steht

| | | | | | |
|---|---|---|---|---|---|
| 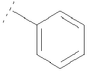 | 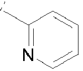 | 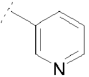 | 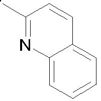 | 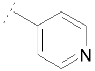 | 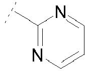 |
| Q-1.1 | Q-1.2 | Q-1.3 | Q-1.4 | Q-1.5 | Q-1.6 |

| | | | | | |
|---|---|---|---|---|---|
| 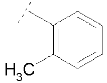 | 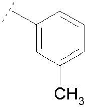 | 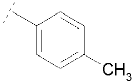 | 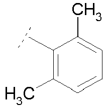 | 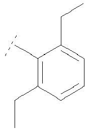 | 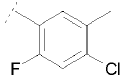 |
| Q-1.7 | Q-1.8 | Q-1.9 | Q-1.10 | Q-1.11 | Q-1.12 |

| | | | | | |
|---|---|---|---|---|---|
| 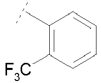 | 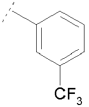 | 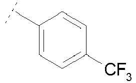 | 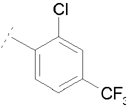 | 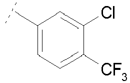 | 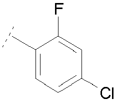 |
| Q-1.13 | Q-1.14 | Q-1.15 | Q-1.16 | Q-1.17 | Q-1.18 |

| | | | | | |
|---|---|---|---|---|---|
| 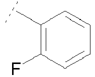 | 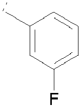 | 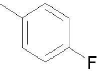 | 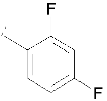 | 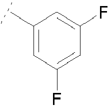 | 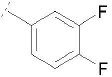 |
| Q-1.19 | Q-1.20 | Q-1.21 | Q-1.22 | Q-1.23 | Q-1.24 |

| | | | | | |
|---|---|---|---|---|---|
| 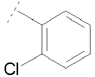 | 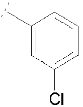 | 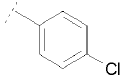 | 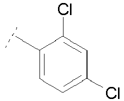 | 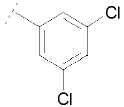 | 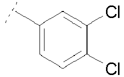 |
| Q-1.25 | Q-1.26 | Q-1.27 | Q-1.28 | Q-1.29 | Q-1.30 |

| | | | | | |
|---|---|---|---|---|---|
| 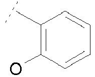 | 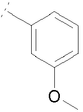 | 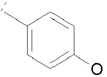 | 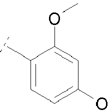 | 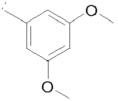 | 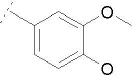 |
| Q-1.31 | Q-1.32 | Q-1.33 | Q-1.34 | Q-1.35 | Q-1.36 |

| | | | | | |
|---|---|---|---|---|---|
| 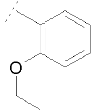 | 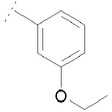 | 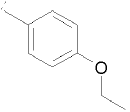 | 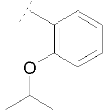 | 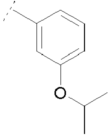 | 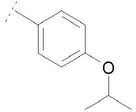 |
| Q-1.37 | Q-1.38 | Q-1.39 | Q-1.40 | Q-1.41 | Q-1.42 |

| | | | | | |
|---|---|---|---|---|---|
| 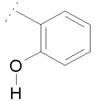 | 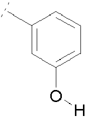 | 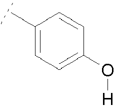 | 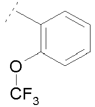 | 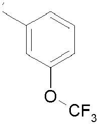 | 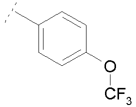 |
| Q-1.43 | Q-1.44 | Q-1.45 | Q-1.46 | Q-1.47 | Q-1.48 |

| | | | | | |
|---|---|---|---|---|---|
| 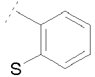 | 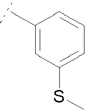 | 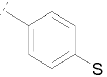 | 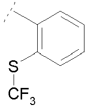 | 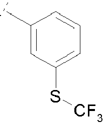 | 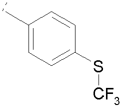 |
| Q-1.49 | Q-1.50 | Q-1.51 | Q-1.52 | Q-1.53 | Q-1.54 |

| | | | | | |
|---|---|---|---|---|---|
| 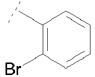 | 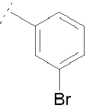 | 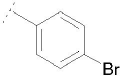 | 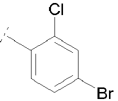 | 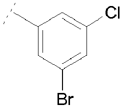 | 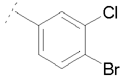 |
| Q-1.55 | Q-1.56 | Q-1.57 | Q-1.58 | Q-1.59 | Q-1.60 |

| | | | | | |
|---|---|---|---|---|---|
| 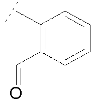 | 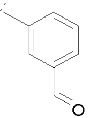 | 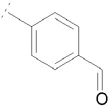 | 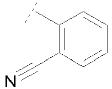 | 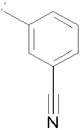 | 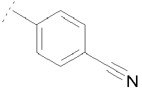 |
| Q-1.61 | Q-1.62 | Q-1.63 | Q-1.64 | Q-1.65 | Q-1.66 |

| | | | | | |
|---|---|---|---|---|---|
| 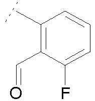 | 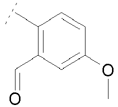 | 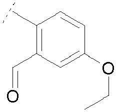 | 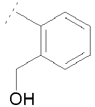 | 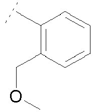 | 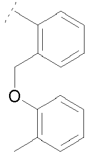 |
| Q-1.67 | Q-1.68 | Q-1.69 | Q-1.70 | Q-1.71 | Q-1.72 |

| | | | | | |
|---|---|---|---|---|---|
| 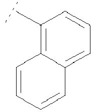 | 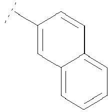 | 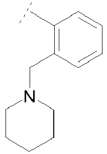 | 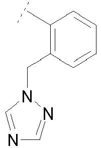 | 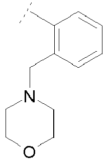 | 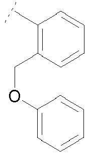 |
| Q-1.73 | Q-1.74 | Q-1.75 | Q-1.76 | Q-1.77 | Q-1.78 |

| | | | | | |
|---|---|---|---|---|---|
| 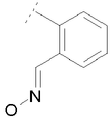 | 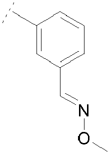 | 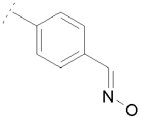 | 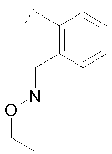 | 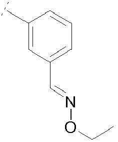 | 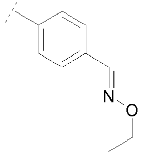 |
| Q-1.79 | Q-1.80 | Q-1.81 | Q-1.82 | Q-1.83 | Q-1.84 |

| | | | | | |
|---|---|---|---|---|---|
| 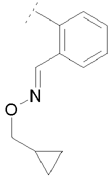 | 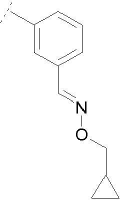 | 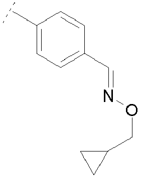 | 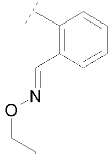 | 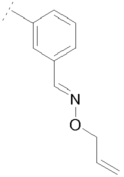 | 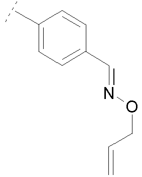 |
| Q-1.85 | Q-1.86 | Q-1.87 | Q-1.88 | Q-1.89 | Q-1.90 |

| | | | | | |
|---|---|---|---|---|---|
| 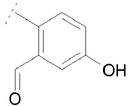 | 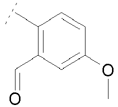 | 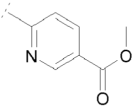 | 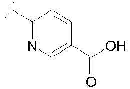 | 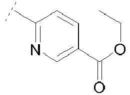 | 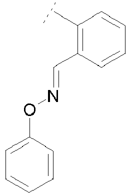 |
| Q-1.91 | Q-1.92 | Q-1.93 | Q-1.94 | Q-1.95 | Q-1.96 |

| | | | | | |
|---|---|---|---|---|---|
| 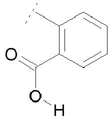 | 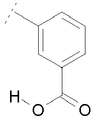 | 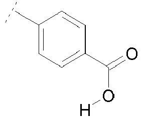 | 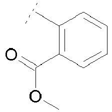 | 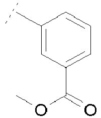 | 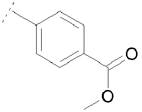 |
| Q-1.97 | Q-1.98 | Q-1.99 | Q-1.100 | Q-1.101 | Q-1.102 |

| | | | | | |
|---|---|---|---|---|---|
| 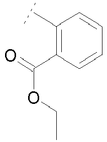 | 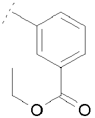 | 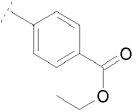 | 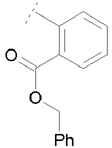 | 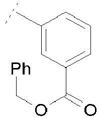 | 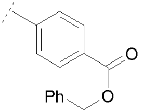 |
| Q-1.103 | Q-1.104 | Q-1.105 | Q-1.106 | Q-1.107 | Q-1.108 |

| | | | | | |
|---|---|---|---|---|---|
| 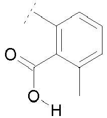 | 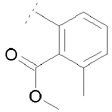 | 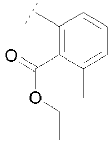 | 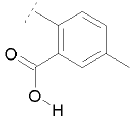 | 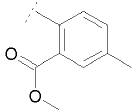 | 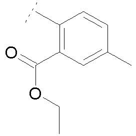 |
| Q-1.109 | Q-1.110 | Q-1.111 | Q-1.112 | Q-1.113 | Q-1.114 |

| | | | | | |
|---|---|---|---|---|---|
| 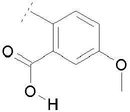 | 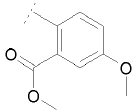 | 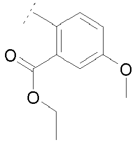 | 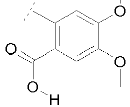 | 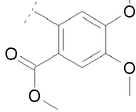 | 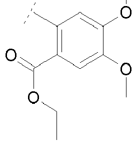 |
| Q-1.115 | Q-1.116 | Q-1.117 | Q-1.118 | Q-1.119 | Q-1.120 |

| | | | | | |
|---|---|---|---|---|---|
| 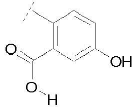 | 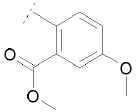 | 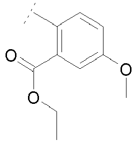 | 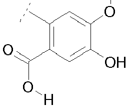 | 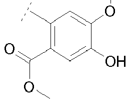 | 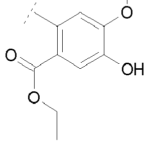 |
| Q-1.121 | Q-1.122 | Q-1.123 | Q-1.124 | Q-1.125 | Q-1.126 |

| | | | | | |
|---|---|---|---|---|---|
| 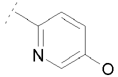 | 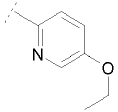 | 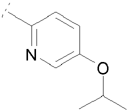 | 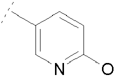 | 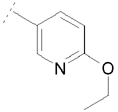 | 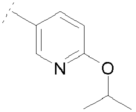 |
| Q-1.127 | Q-1.128 | Q-1.129 | Q-1.130 | Q-1.131 | Q-1.132 |

| | | | | | |
|---|---|---|---|---|---|
| 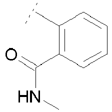 | 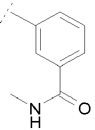 | 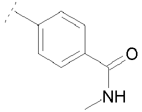 | 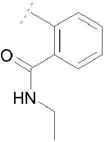 | 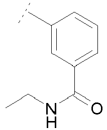 | 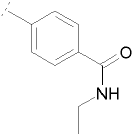 |
| Q-1.133 | Q-1.134 | Q-1.135 | Q-1.136 | Q-1.137 | Q-1.138 |

| | | | | | |
|---|---|---|---|---|---|
| 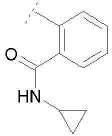 | 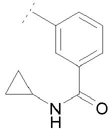 | 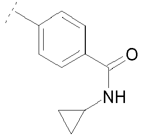 | 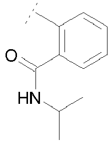 | 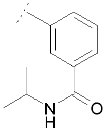 | 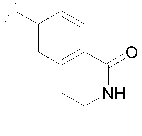 |
| Q-1.139 | Q-1.140 | Q-1.141 | Q-1.142 | Q-1.143 | Q-1.144 |

| | | | | | |
|---|---|---|---|---|---|
| 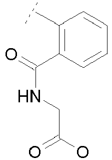 | 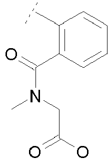 | 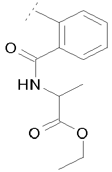 | 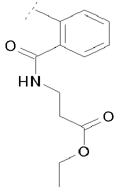 | 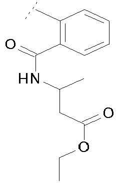 | 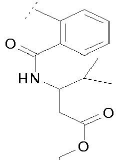 |
| Q-1.145 | Q-1.146 | Q-1.147 | Q-1.148 | Q-1.149 | Q-1.150 |

| | | | | | |
|---|---|---|---|---|---|
| 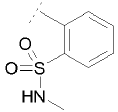 | 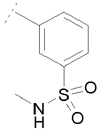 | 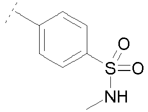 | 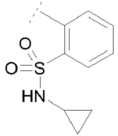 | 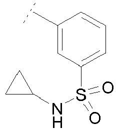 | 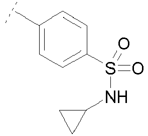 |
| Q-1.151 | Q-1.152 | Q-1.153 | Q-1.154 | Q-1.155 | Q-1.156 |

| | | | | | |
|---|---|---|---|---|---|
| 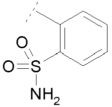 | 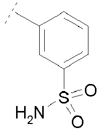 | 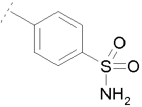 | 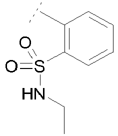 | 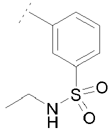 | 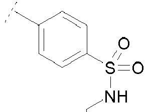 |
| Q-1.157 | Q-1.158 | Q-1.159 | Q-1.160 | Q-1.161 | Q-1.162 |

| | | | | | |
|---|---|---|---|---|---|
| 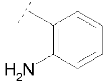 | 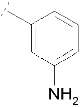 | 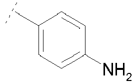 | 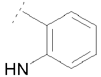 | 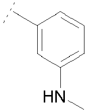 | 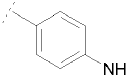 |
| Q-1.163 | Q-1.164 | Q-1.165 | Q-1.166 | Q-1.167 | Q-1.168 |

| | | | | | |
|---|---|---|---|---|---|
| 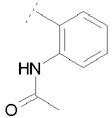 | 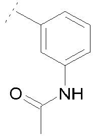 | 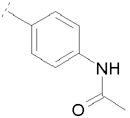 | 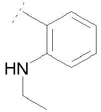 | 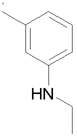 | 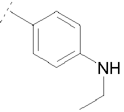 |
| Q-1.169 | Q-1.170 | Q-1.171 | Q-1.172 | Q-1.173 | Q-1.174 |

| | | | | | |
|---|---|---|---|---|---|
| 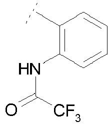 | 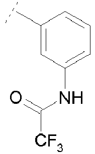 | 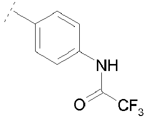 | 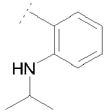 | 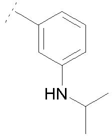 | 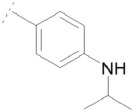 |
| Q-1.175 | Q-1.176 | Q-1.177 | Q-1.178 | Q-1.179 | Q-1.180 |

| | | | | | |
|---|---|---|---|---|---|
| 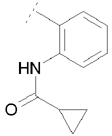 | 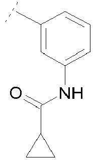 | 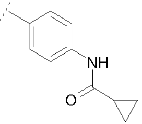 | 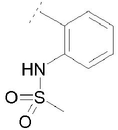 | 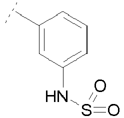 | 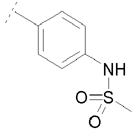 |
| Q-1.181 | Q-1.182 | Q-1.183 | Q-1.184 | Q-1.185 | Q-1.186 |

| | | | | | |
|---|---|---|---|---|---|
| 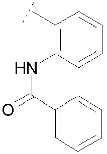 | 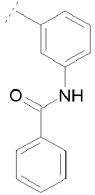 | 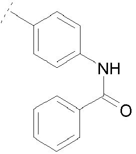 | 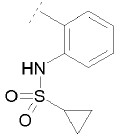 | 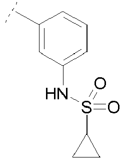 | 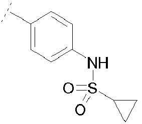 |
| Q-1.187 | Q-1.188 | Q-1.189 | Q-1.190 | Q-1.191 | Q-1.192 |

| | | | | | |
|---|---|---|---|---|---|
| 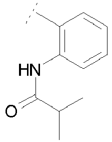 | 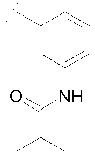 | 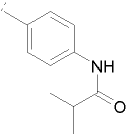 | 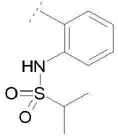 | 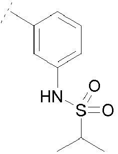 | 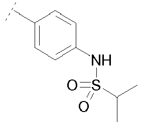 |
| Q-1.193 | Q-1.194 | Q-1.195 | Q-1.196 | Q-1.197 | Q-1.198 |

| | | | | | |
|---|---|---|---|---|---|
| 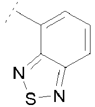 | 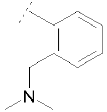 | 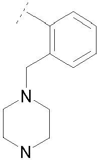 | 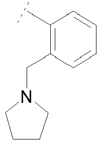 | 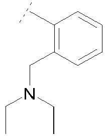 | 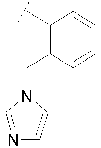 |
| Q-1.199 | Q-1.200 | Q-1.201 | Q-1.202 | Q-1.203 | Q-1.204 |

| | | | | | |
|---|---|---|---|---|---|
| 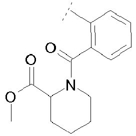 | 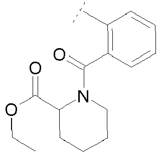 | 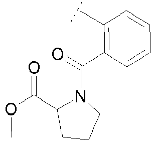 | 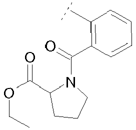 | 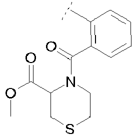 | 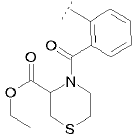 |
| Q-1.205 | Q-1.206 | Q-1.207 | Q-1.208 | Q-1.209 | Q-1.210 |

| | | | | | |
|---|---|---|---|---|---|
| 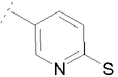 | 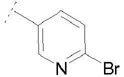 | 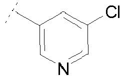 | 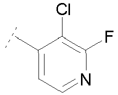 | 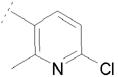 | 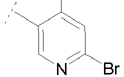 |
| Q-1.211 | Q-1.212 | Q-1.213 | Q-1.214 | Q-1.215 | Q-1.216 |

| | | | | | |
|---|---|---|---|---|---|
| 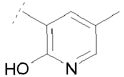 | 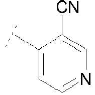 | 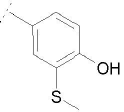 | 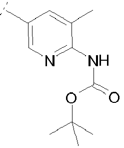 | 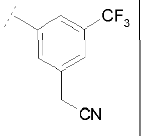 | 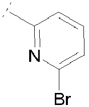 |
| Q-1.217 | Q-1.218 | Q-1.219 | Q-1.220 | Q-1.221 | Q-1.222 |

| | | | | | |
|---|---|---|---|---|---|
| 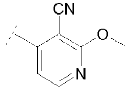 | 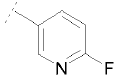 | 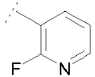 | 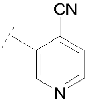 | 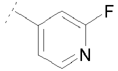 | 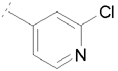 |
| Q-1.223 | Q-1.224 | Q-1.225 | Q-1.226 | Q-1.227 | Q-1.228 |

| | | | | | |
|---|---|---|---|---|---|
| 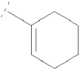 | 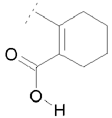 | 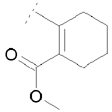 | 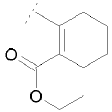 | 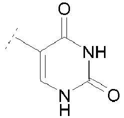 | 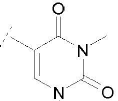 |
| Q-2.1 | Q-2.2 | Q-2.3 | Q-2.4 | Q-2.5 | Q-2.6 |

| | | | | | |
|---|---|---|---|---|---|
| 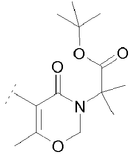 | 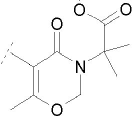 | 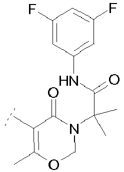 | 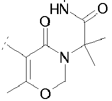 | 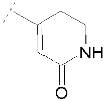 | 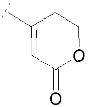 |
| Q-2.7 | Q-2.8 | Q-2.9 | Q-2.10 | Q-2.11 | Q-2.12 |

| | | | | | |
|---|---|---|---|---|---|
| 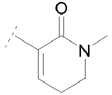 | 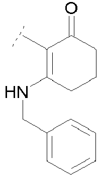 | 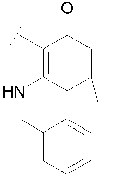 | 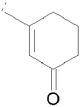 | 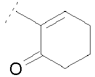 | 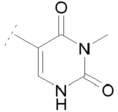 |
| Q-2.13 | Q-2.14 | Q-2.15 | Q-2.16 | Q-2.17 | Q-2.18 |

| | | | | | |
|---|---|---|---|---|---|
| 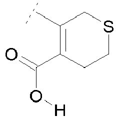 | 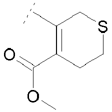 | 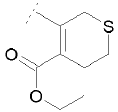 | 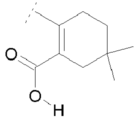 | 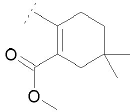 | 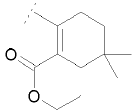 |
| Q-2.19 | Q-2.20 | Q-2.21 | Q-2.22 | Q-2.23 | Q-2.24 |

mit Ausnahme von 1-[(2E)-1-Hydroxy-1,3-diphenylprop-2-en-1-yl]cyclopentan-carbonitril.

Im ganz Speziellen bevorzugt sind Verbindungen der allgemeinen Formel (I), worin
[X-Y] für die Gruppierungen 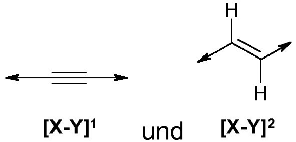 steht,
R¹ für Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropylmethyl, Cyclobutylmethyl, Cyclopentylmethyl, Cyclohexylmethyl, gegebenenfalls substituiertes Phenyl, Heteroaryl, Heterocyclyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Methylcyclohexyl, 2-Methylcyclohexyl, 3-Methylcyclohexyl, 1-Methoxycyclohexyl, 2-Methoxycyclohexyl, 3-Methoxycyclohexyl, Aryl-(C₁-C₅)-alkyl, Heterocyclyl-(C₁-C₅)-alkyl steht,
R² für Wasserstoff, tert.-Butyldimethylsilyl, Trimethylsilyl, Triethylsilyl, Tri-(iso-Propyl)silyl, Tri-(n-Propyl)silyl, Dimethyl(phenyl)silyl, tert.-Butyldiphenylsilyl, Diethylisopropylsilyl, Isopropyldimethylsilyl, tert.-Hexyldimethylsilyl, 2-(Trimethylsilyl)ethoxymethyl, 2-(Trimethylsilyl)ethyl, Methyl, Ethyl, Allyl, Methylcarbonyl, Ethylcarbonyl, n-Propylcarbonyl, iso-Propylcarbonyl, n-Butylcarbonyl, tert-Butylcarbonyl, iso-Butylcarbonyl, iso-Pentylcarbonyl, neo-Pentylcarbonyl, Arylcarbonyl, Heteroarylcarbonyl, Cyclopropylcarbonyl, Cyclobutylcarbonyl, Cyclopentylcarbonyl, Cyclohexylcarbonyl, Heterocyclylcarbonyl, Methoxycarbonyl, Ethoxycarbonyl, n-Propoxycarbonyl, n-Butyloxycarbonyl, iso-Propoxycarbonyl, iso-Butyloxycarbonyl, tert-Butyloxycarbonyl, Allyloxycarbonyl, Benzyl, p-CI-Benzyl, p-F-Benzyl, p-Methoxybenzyl, p-Methylbenzyl, Methylthiomethyl, Methylthioethyl, Ethylthioethyl, Methylthio-n-propyl, Ethylthio-n-propyl steht,
A¹, V, W unabhängig voneinander für eine Gruppe CR³R⁴, stehen,
A² für eine Gruppe CR³R⁴ oder Sauerstoff steht,
m für 0 steht,
n für 0, 1 steht,
R³ und R⁴ unabhängig voneinander für Wasserstoff, Methyl, Ethyl, n-Propyl, iso-Propyl, n-Butyl, iso-Butyl, tert.-Butyl, Fluor, Chlor, Brom, lod, Cyclopropyl, Cyclobutyl, Cyclopentyl, Cyclohexyl, Methoxy, gegebenenfalls substituiertes Phenyl, Heterocyclyl, Heteroaryl, Methylthio, Trifluormethyl, Difluormethyl, Vinyl, Prop-1-en-1-yl, But-1-en-1-yl, Allyl, Trifluormethoxy, Difluormethoxy, 2,2-Difluorethoxy, 2,2,2-Trifluorethoxy, Trifluormethylthio, Methoxymethyl, Ethoxymethyl, Ethoxyethyl, Methoxyethyl, Methylthiomethyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden,
und Q für eine der in der oben stehenden Tabelle beschriebenen Gruppierungen Q-1.1 bis Q-2.24 steht

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte.Diese Restedefinitionen können untereinander, also auch zwischen den angegebenen bevorzugten Bereichen beliebig kombiniert werden.

Ebenfalls noch nicht bekannt und somit weiterer Teil der Erfindung sind Verbindungen der Formel (II) oder deren Salze, 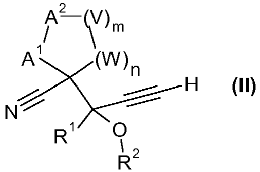 die als Zwischenprodukte zur Herstellung der erfindungsgemäßen Verbindungen der allgemeinen Formel (I) dienen,
wobei
R¹ für Wasserstoff, (C₁-C₈)-Alkyl, Aryl, Heteroaryl, Heterocyclyl, (C₃-C₈)-Cycloalkyl, (C₂-C₈)-Alkenyl, (C₂-C₈)-Alkinyl, (C₂-C₈)-Alkinyl-(C₁-C₈)-alkyl, (C₁-C₈)-Alkoxy-(C₁-C₈)-alkyl, Hydroxy-(C₁-C₈)-alkyl, (C₁-C₈)-Haloalkyl, (C₂-C₈)-Haloalkenyl, (C₁-C₈)-Haloalkoxy-(C₁-C₈)-alkyl, (C₁-C₈)-Alkylthio-(C₁-C₈)-alkyl, Aryl-(C₁-C₈)-alkyl, Heterocyclyl-(C₁-C₈)-alkyl, (C₃-C₈)-Halocycloalkyl, (C₄-C₈)-Cycloalkenyl, (C₁-C₈)-Alkoxy-(C₁-C₈)-alkoxy-(C₁-C₈)-alkyl, (C₁-C₈)-Alkoxy-(C₁-C₈)-haloalkyl, (C₁-C₈)-Haloalkoxy-(C₁-C₈)-haloalkyl, (C₃-C₈)-Cycloalkyl-(C₁-C₈)-alkyl steht,
R² für Wasserstoff, (C₁-C₈)-Alkyl, (C₁-C₈)-Alkoxy-(C₁-C₈)-alkyl, (C₁-C₈)-Alkoxy-(C₁-C₈)-alkoxy-(C₁-C₈)-alkyl, (C₂-C₈)-Alkenyl, (C₂-C₈)-Alkinyl, (C₁-C₈)-Alkylcarbonyl, Arylcarbonyl, Heteroarylcarbonyl, (C₃-C₈)-Cycloalkylcarbonyl, (C₂-C₈)-Alkenylcarbonyl, Heterocyclylcarbonyl, (C₁-C₈)-Alkoxycarbonyl, (C₂-C₈)-Alkenyloxycarbonyl, Aryloxy-(C₁-C₈)-alkyl, Aryl-(C₁-C₈)-alkoxycarbonyl, (C₃-C₈)-Cycloalkoxycarbonyl, (C₃-C₈)-Cycloalkyl-(C₁-C₈)-alkoxycarbonyl, Aryl-(C₁-C₈)-alkoxy-(C₁-C₈)-alkyl, Aryl-(C₁-C₈)-alkyl, (C₁-C₈)-Alkoxy-(C₁-C₈)-alkoxy-(C₁-C₈)-alkyl, (C₁-C₈)-Alkylthio-(C₁-C₈)-alkyl, Tris[(C₁-C₈)-alkyl]silyl, (C₁-C₈)-Alkyl-Bis-[(C₁-C₈)-alkyl]silyl, (C₁-C₈)-Alkyl-Bis(aryl)silyl, Aryl-Bis[(C₁-C₈)-alkyl]silyl, (C₃-C₈)-Cycloalkyl-Bis[(C₁-C₈)-alkyl]silyl, Halo-Bis[(C₁-C₈)-alkyl]silyl, Tris[(C₁-C₈)-alkyl]silyl-(C₁-C₈)-alkoxy-(C₁-C₈)-alkyl, Tris[(C₁-C₈)-alkyl]silyl-(C₁-C₈)-alkyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden seind und wobei die Sauerstoff- oder Schwefelatome jeweils nicht benachbart sind,
m für 0, 1, 2 steht,
n für 0, 1, 2 steht und R³ und R⁴ unabhängig voneinander für Wasserstoff, (C₁-C₈)-Alkyl, Halogen, (C₃-C₈)-Cycloalkyl, (C₁-C₈)-Alkoxy, Aryl, Heterocyclyl, Heteroaryl, Aryl-(C₁-C₈)-alkyl, (C₁-C₈)-Alkylthio, (C₁-C₈)-Haloalkyl, (C₁-C₈)-Haloalkyloxy, (C₁-C₈)-Haloalkylthio, (C₁-C₈)-Alkoxy-(C₁-C₈)-alkyl, (C₁-C₈)-Alkylthio-(C₁-C₈)-alkyl, Heteroaryl-(C₁-C₈)-alkyl, Heterocyclyl-(C₁-C₈)-alkyl, (C₁-C₈)-Alkoxy-(C₁-C₈)-haloalkyl, (C₁-C₈)-Haloalkoxy-(C₁-C₈)-haloalkyl, (C₂-C₈)-Alkenyl, (C₂-C₈)-Alkinyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden.

Bevorzugt sind Verbindungen der Formel (II), wobei
R¹ für Wasserstoff, (C₁-C₆)-Alkyl, Aryl, Heteroaryl, Heterocyclyl, (C₃-C₆)-Cycloalkyl, (C₂-C₆)-Alkenyl, (C₂-C₆)-Alkinyl, (C₂-C₆)-Alkinyl-(C₁-C₆)-alkyl, (C₁-C₆)-Alkoxy-(C₁-C₆)-alkyl, Hydroxy-(C₁-C₆)-alkyl, (C₁-C₆)-Haloalkyl, (C₂-C₆)-Haloalkenyl, (C₁-C₆)-Haloalkoxy-(C₁-C₆)-alkyl, (C₁-C₆)-Alkylthio-(C₁-C₆)-alkyl, Aryl-(C₁-C₆)-alkyl, Heterocyclyl-(C₁-C₆)-alkyl, (C₃-C₆)-Halocycloalkyl, (C₄-C₆)-Cycloalkenyl, (C₁-C₆)-Alkoxy-(C₁-C₆)-alkoxy-(C₁-C₆)-alkyl, (C₁-C₆)-Alkoxy-(C₁-C₆)-haloalkyl, (C₁-C₆)-Haloalkoxy-(C₁-C₆)-haloalkyl, (C₃-C₆)-Cycloalkyl-(C₁-C₆)-alkyl steht,
R² für Wasserstoff, (C₁-C₆)-Alkyl, (C₁-C₆)-Alkoxy-(C₁-C₆)-alkyl, (C₁-C₆)-Alkoxy-(C₁-C₆)-alkoxy-(C₁-C₆)-alkyl, (C₂-C₆)-Alkenyl, (C₂-C₆)-Alkinyl, (C₁-C₆)-Alkylcarbonyl, Arylcarbonyl, Heteroarylcarbonyl, (C₃-C₆)-Cycloalkylcarbonyl, (C₂-C₆)-Alkenylcarbonyl, Heterocyclylcarbonyl, (C₁-C₆)-Alkoxycarbonyl, (C₂-C₆)-Alkenyloxycarbonyl, Aryloxy-(C₁-C₆)-alkyl, Aryl-(C₁-C₆)-alkoxycarbonyl, (C₃-C₆)-Cycloalkoxycarbonyl, (C₃-C₆)-Cycloalkyl-(C₁-C₆)-alkoxycarbonyl, Aryl-(C₁-C₆)-alkoxy-(C₁-C₆)-alkyl, Aryl-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, Tris[(C₁-C₆)-alkyl]silyl, (C₁-C₆)-Alkyl-Bis-[(C₁-C₆)-alkyl]silyl, (C₁-C₆)-Alkyl-Bis(aryl)silyl, Aryl-Bis[(C₁-C₆)-alkyl]silyl, (C₃-C₆)-Cycloalkyl-Bis[(C₁-C₆)-alkyl]silyl, Halo-Bis[(C₁-C₆)-alkyl]silyl, Tris[(C₁-C₆)-alkyl]silyl-(C₁-C₆)-alkoxy-(C₁-C₆)-alkyl, Tris[(C₁-C₆)-alkyl]silyl-(C₁-C₆)-alkyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht und R³ und R⁴ unabhängig voneinander für Wasserstoff, (C₁-C₆)-Alkyl, Halogen, (C₃-C₆)-Cycloalkyl, (C₁-C₆)-Alkoxy, Aryl, Heterocyclyl, Heteroaryl, Aryl-(C₁-C₆)-alkyl, (C₁-C₆)-Alkylthio, (C₁-C₆)-Haloalkyl, (C₁-C₆)-Haloalkyloxy, (C₁-C₆)-Haloalkylthio, (C₁-C₆)-Alkoxy-(C₁-C₆)-alkyl, (C₁-C₆)-Alkylthio-(C₁-C₆)-alkyl, Heteroaryl-(C₁-C₆)-alkyl, Heterocyclyl-(C₁-C₆)-alkyl, (C₁-C₆)-Alkoxy-(C₁-C₆)-haloalkyl, (C₁-C₆)-Haloalkoxy-(C₁-C₆)-haloalkyl, (C₂-C₆)-Alkenyl, (C₂-C₆)-Alkinyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden.

Besonders bevorzugt sind Verbindungen der Formel (II), worin
R¹ für Wasserstoff, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropylmethyl, Cyclobutylmethyl, Cyclopentylmethyl, Cyclohexylmethyl, gegebenenfalls substituiertes Phenyl, Heteroaryl, Heterocyclyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Methylcyclohexyl, 2-Methylcyclohexyl, 3-Methylcyclohexyl, 1-Methoxycyclohexyl, 2-Methoxycyclohexyl, 3-Methoxycyclohexyl, Aryl-(C₁-C₅)-alkyl, Heterocyclyl-(C₁-C₅)-alkyl steht,
R² für Wasserstoff, tert.-Butyldimethylsilyl, Trimethylsilyl, Triethylsilyl, Tri-(iso-Propyl)silyl, Tri-(n-Propyl)silyl, Dimethyl(phenyl)silyl, tert.-Butyldiphenylsilyl, Diethylisopropylsilyl, Isopropyldimethylsilyl, tert.-Hexyldimethylsilyl, 2-(Trimethylsilyl)ethoxymethyl, 2-(Trimethylsilyl)ethyl, Methyl, Ethyl, Allyl, Methylcarbonyl, Ethylcarbonyl, n-Propylcarbonyl, iso-Propylcarbonyl, n-Butylcarbonyl, tert-Butylcarbonyl, iso-Butylcarbonyl, iso-Pentylcarbonyl, neo-Pentylcarbonyl, Arylcarbonyl, Heteroarylcarbonyl, Cyclopropylcarbonyl, Cyclobutylcarbonyl, Cyclopentylcarbonyl, Cyclohexylcarbonyl, Heterocyclylcarbonyl, Methoxycarbonyl, Ethoxycarbonyl, n-Propoxycarbonyl, n-Butyloxycarbonyl, iso-Propoxycarbonyl, iso-Butyloxycarbonyl, tert-Butyloxycarbonyl, Allyloxycarbonyl, Benzyl, p-CI-Benzyl, p-F-Benzyl, p-Methoxybenzyl, p-Methylbenzyl, Methylthiomethyl, Methylthioethyl, Ethylthioethyl, Methylthio-n-propyl, Ethylthio-n-propyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1 steht,
n für 0, 1 steht und R³ und R⁴ unabhängig voneinander für Wasserstoff, Methyl, Ethyl, n-Propyl, iso-Propyl, n-Butyl, iso-Butyl, tert.-Butyl, Fluor, Chlor, Brom, lod, Cyclopropyl, Cyclobutyl, Cyclopentyl, Cyclohexyl, Methoxy, Ethoxy, n-Propoxy, iso-Propoxy, n-Butyloxy, tert.-Butyloxy, iso-Butyloxy, n-Pentyloxy, gegebenenfalls substituiertes Phenyl, Heterocyclyl, Heteroaryl, Benzyl, p-Cl-Benzyl, p-F-Benzyl, p-Methoxybenzyl, p-Methylbenzyl, Methylthio, Ethylthio, n-Propylthio, n-Butylthio, n-Pentylthio, iso-Propylthio, iso-Butylthio, Trifluormethyl, Pentafluorethyl, 1,1,2,2-Tetrafluorethyl, Heptafluorpropyl, Nonafluorbutyl, Chlordifluormethyl, Bromdifluormethyl, Dichlorfluormethyl, Bromfluormethyl, 1-Fluorethyl, 2-Fluorethyl, Fluormethyl, Difluormethyl, 2,2-Difluorethyl, 2,2,2-Trifluorethyl, 2,2-Dichlor-2-fluororethyl, 2-Chlor-2,2-difluorethyl, Difluor-tert.-butyl, Trifluormethoxymethyl, Trifluormethoxyethyl, Trifluormethoxy-n-propyl, Difluormethoxymethyl, Difluormethoxyethyl, Difluormethoxy-n-propyl, 2,2-Difluorethoxymethyl, 2,2-Difluorethoxyethyl, 2,2-Difluorethoxy-n-propyl, 2,2,2-Trifluorethoxymethyl, 2,2,2-Trifluorethoxyethyl, 2,2,2-Trifluorethoxy-n-propyl, Vinyl, Prop-1-en-1-yl, But-1-en-1-yl, Allyl, 1-Methyl-prop-2-en-1-yl, 2-Methyl-prop-2-en-1-yl, But-2-en-1-yl, 1-Methyl-but-3-en-1-yl und 1-Methyl-but-2-en-1-yl, Ethinyl, Propargyl, 1-Methyl-prop-2-in-1-yl, 2-Butinyl, 2-Pentinyl, Trifluormethoxy, Difluormethoxy, 2,2-Difluorethoxy, 2,2,2-Trifluorethoxy, Trifluormethylthio, Methoxymethyl, Ethoxymethyl, Ethoxyethyl, Methoxyethyl, Methoxy-n-propyl, Ethoxy-n-propyl, Methoxybutyl, Methoxy-iso-Propyl, iso-Propoxymethyl, iso-Propoxyethyl, Methylthiomethyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden.

Ganz besonders bevorzugt sind Verbindungen der Formel (II), worin
R¹ für Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropylmethyl, Cyclobutylmethyl, Cyclopentylmethyl, Cyclohexylmethyl, gegebenenfalls substituiertes Phenyl, Heteroaryl, Heterocyclyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Methylcyclohexyl, 2-Methylcyclohexyl, 3-Methylcyclohexyl, 1-Methoxycyclohexyl, 2-Methoxycyclohexyl, 3-Methoxycyclohexyl, Aryl-(C₁-C₅)-alkyl, Heterocyclyl-(C₁-C₅)-alkyl steht,
R² für Wasserstoff, tert.-Butyldimethylsilyl, Trimethylsilyl, Triethylsilyl, Tri-(iso-Propyl)silyl, Tri-(n-Propyl)silyl, Dimethyl(phenyl)silyl, tert.-Butyldiphenylsilyl, Diethylisopropylsilyl, Isopropyldimethylsilyl, tert.-Hexyldimethylsilyl, 2-(Trimethylsilyl)ethoxymethyl, 2-(Trimethylsilyl)ethyl, Methyl, Ethyl, Allyl, Methylcarbonyl, Ethylcarbonyl, n-Propylcarbonyl, iso-Propylcarbonyl, n-Butylcarbonyl, tert-Butylcarbonyl, iso-Butylcarbonyl, iso-Pentylcarbonyl, neo-Pentylcarbonyl, Arylcarbonyl, Heteroarylcarbonyl, Cyclopropylcarbonyl, Cyclobutylcarbonyl, Cyclopentylcarbonyl, Cyclohexylcarbonyl, Heterocyclylcarbonyl, Methoxycarbonyl, Ethoxycarbonyl, n-Propoxycarbonyl, n-Butyloxycarbonyl, iso-Propoxycarbonyl, iso-Butyloxycarbonyl, tert-Butyloxycarbonyl, Allyloxycarbonyl, Benzyl, p-CI-Benzyl, p-F-Benzyl, p-Methoxybenzyl, p-Methylbenzyl, Methylthiomethyl, Methylthioethyl, Ethylthioethyl, Methylthio-n-propyl, Ethylthio-n-propyl steht,
A¹, V, W unabhängig voneinander für eine Gruppe CR³R⁴, stehen,
A² für eine Gruppe CR³R⁴ oder Sauerstoff steht,
m für 0 steht,
n für 0, 1 steht und R³ und R⁴ unabhängig voneinander für Wasserstoff, Methyl, Ethyl, n-Propyl, iso-Propyl, n-Butyl, iso-Butyl, tert.-Butyl, Fluor, Chlor, Brom, lod, Cyclopropyl, Cyclobutyl, Cyclopentyl, Cyclohexyl, Methoxy, gegebenenfalls substituiertes Phenyl, Heterocyclyl, Heteroaryl, Methylthio, Trifluormethyl, Difluormethyl, Vinyl, Prop-1-en-1-yl, But-1-en-1-yl, Allyl, Trifluormethoxy, Difluormethoxy, 2,2-Difluorethoxy, 2,2,2-Trifluorethoxy, Trifluormethylthio, Methoxymethyl, Ethoxymethyl, Ethoxyethyl, Methoxyethyl, Methylthiomethyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden.

Weiterhin noch nicht bekannt und somit weiterer Teil der Erfindung sind Verbindungen der Formel (III) oder deren Salze, 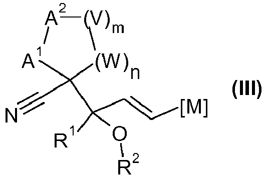 die als Zwischenprodukte zur Herstellung der erfindungsgemäßen Verbindungen der allgemeinen Formel (I) dienen,
wobei
R¹ für Wasserstoff, (C₁-C₈)-Alkyl, Aryl, Heteroaryl, Heterocyclyl, (C₃-C₈)-Cycloalkyl, (C₂-C₈)-Alkenyl, (C₂-C₈)-Alkinyl, (C₂-C₈)-Alkinyl-(C₁-C₈)-alkyl, (C₁-C₈)-Alkoxy-(C₁-C₈)-alkyl, Hydroxy-(C₁-C₈)-alkyl, (C₁-C₈)-Haloalkyl, (C₂-C₈)-Haloalkenyl, (C₁-C₈)-Haloalkoxy-(C₁-C₈)-alkyl, (C₁-C₈)-Alkylthio-(C₁-C₈)-alkyl, Aryl-(C₁-C₈)-alkyl, Heterocyclyl-(C₁-C₈)-alkyl, (C₃-C₈)-Halocycloalkyl, (C₄-C₈)-Cycloalkenyl, (C₁-C₈)-Alkoxy-(C₁-C₈)-alkoxy-(C₁-C₈)-alkyl, (C₁-C₈)-Alkoxy-(C₁-C₈)-haloalkyl, (C₁-C₈)-Haloalkoxy-(C₁-C₈)-haloalkyl steht,
R² für Wasserstoff, (C₁-C₈)-Alkyl, (C₁-C₈)-Alkoxy-(C₁-C₈)-alkyl, (C₁-C₈)-Alkoxy-(C₁-C₈)-alkoxy-(C₁-C₈)-alkyl, (C₂-C₈)-Alkenyl, (C₂-C₈)-Alkinyl, (C₁-C₈)-Alkylcarbonyl, Arylcarbonyl, Heteroarylcarbonyl, (C₃-C₈)-Cycloalkylcarbonyl, (C₂-C₈)-Alkenylcarbonyl, Heterocyclylcarbonyl, (C₁-C₈)-Alkoxycarbonyl, (C₂-C₈)-Alkenyloxycarbonyl, Aryloxy-(C₁-C₈)-alkyl, Aryl-(C₁-C₈)-alkoxycarbonyl, (C₃-C₈)-Cycloalkoxycarbonyl, (C₃-C₈)-Cycloalkyl-(C₁-C₈)-alkoxycarbonyl, Aryl-(C₁-C₈)-alkoxy-(C₁-C₈)-alkyl, Aryl-(C₁-C₈)-alkyl, (C₁-C₈)-Alkoxy-(C₁-C₈)-alkoxy-(C₁-C₈)-alkyl, (C₁-C₈)-Alkylthio-(C₁-C₈)-alkyl, Tris[(C₁-C₈)-alkyl]silyl, (C₁-C₈)-Alkyl-Bis-[(C₁-C₈)-alkyl]silyl, (C₁-C₈)-Alkyl-Bis(aryl)silyl, Aryl-Bis[(C₁-C₈)-alkyl]silyl, (C₃-C₈)-Cycloalkyl-Bis[(C₁-C₈)-alkyl]silyl, Halo-Bis[(C₁-C₈)-alkyl]silyl, Tris[(C₁-C₈)-alkyl]silyl-(C₁-C₈)-alkoxy-(C₁-C₈)-alkyl, Tris[(C₁-C₈)-alkyl]silyl-(C₁-C₈)-alkyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht,
R³ und R⁴ unabhängig voneinander für Wasserstoff, (C₁-C₈)-Alkyl, Halogen, (C₃-C₈)-Cycloalkyl, (C₁-C₈)-Alkoxy, Aryl, Heterocyclyl, Heteroaryl, Aryl-(C₁-C₈)-alkyl, (C₁-C₈)-Alkylthio, (C₁-C₈)-Haloalkyl, (C₁-C₈)-Haloalkyloxy, (C₁-C₈)-Haloalkylthio, (C₁-C₈)-Alkoxy-(C₁-C₈)-alkyl, (C₁-C₈)-Alkylthio-(C₁-C₈)-alkyl, Heteroaryl-(C₁-C₈)-alkyl, Heterocyclyl-(C₁-C₈)-alkyl, (C₁-C₈)-Alkoxy-(C₁-C₈)-haloalkyl, (C₁-C₈)-Haloalkoxy-(C₁-C₈)-haloalkyl, (C₂-C₈)-Alkenyl, (C₂-C₈)-Alkinyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden und
[M] für Tris-[(C₁-C₆)-Alkyl]stannyl, Tris-[(C₃-C₈)-Cycloalkyl]stannyl, Tris-[(C₁-C₆)-Alkyl]germanyl, Tris-[(C₃-C₈)-Cycloalkyl]germanyl, Bis-(Cyclopentadienyl)zirconyl, Bis-(1,2,3,4,5-Pentamethylcyclopentadienyl)zirconyl, Bis-(Cyclopentadienyl)hafnyl, Bis-(1,2,3,4,5-Pentamethylcyclopentadienyl)hafnyl, Bis-(Hydroxy)boryl, Bis-[(C₁-C₆)-Alkoxy]-boryl, (C₁-C₆)-Alkyl-1,3,2-dioxaborolan-2-yl, Bis-[(C₁-C₆)-Alkyl]-1,3,2-dioxaborolan-2-yl, Tetrakis-[(C₁-C₆)-Alkyl]-1,3,2-dioxaborolan-2-yl, 1,3,2-Dioxaborinan-2-yl, Bis-[(C₁-C₆)-Alkyl]-1,3,2-Dioxaborinan-2-yl, (C₁-C₆)-Alkyl-1,3,2-Dioxaborinan-2-yl, Tris-[(C₁-C₆)-Alkyl]-1,3,2-Dioxaborinan-2-yl, 2,6,7-Trioxa-1-Boranuidabicyclo[2.2.2]octanyl, (C₁-C₆)-Alkyl-2,6,7-Trioxa-1-Boranuidabicyclo[2.2.2]octanyl, Tris-[(C₁-C₆)-Alkyl]plumbanyl, Tris-[(C₃-C₈)-Cycloalkyl]plumbanyl, Tris-[(C₁-C₆)-Alkylcarbonyloxy]plumbanyl, Tris-Arylplumbanyl, Bis-[(C₁-C₆)-Alkylcarbonyloxy]-arylplumbanyl, Bis-[(C₁-C₆)-Alkyl]-alanyl, Bis-[(C₁-C₆)-Cycloalkyl]-alanyl, Dichloralanyl, Chlormagnesyl, Brommagnesyl, Chlorzinkyl, Chlorhydrargyl, Bromhydrargyl, (C₁-C₆)-Alkylhydrargyl, (C₃-C₆)-Cycloalkylhydrargyl, Tris-[(C₁-C₆)-alkyl]silyl, (C₁-C₆)-Alkyl-[bis-(C₁-C₆)-alkyl]silyl, (C₁-C₆)-Alkyl-bis-(aryl)silyl, Aryl-bis-[(C₁-C₆)-alkyl)]silyl, (C₃-C₇)-Cycloalkyl-bis-[(C₁-C₆)-alkyl]silyl steht.

Bevorzugt sind Verbindungen der Formel (III), wobei
R¹ für Wasserstoff, (C₁-C₆)-Alkyl, Aryl, Heteroaryl, Heterocyclyl, (C₃-C₆)-Cycloalkyl, (C₂-C₆)-Alkenyl, (C₂-C₆)-Alkinyl, (C₂-C₆)-Alkinyl-(C₁-C₆)-alkyl, (C₁-C₆)-Alkoxy-(C₁-C₆)-alkyl, Hydroxy-(C₁-C₆)-alkyl, (C₁-C₆)-Haloalkyl, (C₂-C₆)-Haloalkenyl, (C₁-C₆)-Haloalkoxy-(C₁-C₆)-alkyl, (C₁-C₆)-Alkylthio-(C₁-C₆)-alkyl, Aryl-(C₁-C₆)-alkyl, Heterocyclyl-(C₁-C₆)-alkyl, (C₃-C₆)-Halocycloalkyl, (C₄-C₆)-Cycloalkenyl, (C₁-C₆)-Alkoxy-(C₁-C₆)-alkoxy-(C₁-C₆)-alkyl, (C₁-C₈)-Alkoxy-(C₁-C₈)-haloalkyl, (C₁-C₈)-Haloalkoxy-(C₁-C₈)-haloalkyl steht,
R² für Wasserstoff, (C₁-C₆)-Alkyl, (C₁-C₆)-Alkoxy-(C₁-C₆)-alkyl, (C₁-C₆)-Alkoxy-(C₁-C₆)-alkoxy-(C₁-C₆)-alkyl, (C₂-C₆)-Alkenyl, (C₂-C₆)-Alkinyl, (C₁-C₆)-Alkylcarbonyl, Arylcarbonyl, Heteroarylcarbonyl, (C₃-C₆)-Cycloalkylcarbonyl, (C₂-C₆)-Alkenylcarbonyl, Heterocyclylcarbonyl, (C₁-C₆)-Alkoxycarbonyl, (C₂-C₆)-Alkenyloxycarbonyl, Aryloxy-(C₁-C₆)-alkyl, Aryl-(C₁-C₆)-alkoxycarbonyl, (C₃-C₆)-Cycloalkoxycarbonyl, (C₃-C₆)-Cycloalkyl-(C₁-C₆)-alkoxycarbonyl, Aryl-(C₁-C₆)-alkoxy-(C₁-C₆)-alkyl, Aryl-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, Tris[(C₁-C₆)-alkyl]silyl, (C₁-C₆)-Alkyl-Bis-[(C₁-C₆)-alkyl]silyl, (C₁-C₆)-Alkyl-Bis(aryl)silyl, Aryl-Bis[(C₁-C₆)-alkyl]silyl, (C₃-C₆)-Cycloalkyl-Bis[(C₁-C₆)-alkyl]silyl, Halo-Bis[(C₁-C₆)-alkyl]silyl, Tris[(C₁-C₆)-alkyl]silyl-(C₁-C₆)-alkoxy-(C₁-C₆)-alkyl, Tris[(C₁-C₆)-alkyl]silyl-(C₁-C₆)-alkyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht,
R³ und R⁴ unabhängig voneinander für Wasserstoff, (C₁-C₆)-Alkyl, Halogen, (C₃-C₆)-Cycloalkyl, (C₁-C₆)-Alkoxy, Aryl, Heterocyclyl, Heteroaryl, Aryl-(C₁-C₆)-alkyl, (C₁-C₆)-Alkylthio, (C₁-C₆)-Haloalkyl, (C₁-C₆)-Haloalkyloxy, (C₁-C₆)-Haloalkylthio, (C₁-C₆)-Alkoxy-(C₁-C₆)-alkyl, (C₁-C₆)-Alkylthio-(C₁-C₆)-alkyl, Heteroaryl-(C₁-C₆)-alkyl, Heterocyclyl-(C₁-C₆)-alkyl, (C₁-C₆)-Alkoxy-(C₁-C₆)-haloalkyl, (C₁-C₆)-Haloalkoxy-(C₁-C₆)-haloalkyl, (C₂-C₆)-Alkenyl, (C₂-C₆)-Alkinyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden und
[M] für Tris-[(C₁-C₆)-Alkyl]stannyl, Tris-[(C₃-C₆)-Cycloalkyl]stannyl, Tris-[(C₁-C₆)-Alkyl]germanyl, Tris-[(C₃-C₆)-Cycloalkyl]germanyl, Bis-(Cyclopentadienyl)zirconyl, Bis-(1,2,3,4,5-Pentamethylcyclopentadienyl)zirconyl, Bis-(Cyclopentadienyl)hafnyl, Bis-(1,2,3,4,5-Pentamethylcyclopentadienyl)hafnyl, Bis-(Hydroxy)boryl, Bis-[(C₁-C₆)-Alkoxy]-boryl, (C₁-C₆)-Alkyl-1,3,2-dioxaborolan-2-yl, Bis-[(C₁-C₆)-Alkyl]-1,3,2-dioxaborolan-2-yl, Tetrakis-[(C₁-C₆)-Alkyl]-1,3,2-dioxaborolan-2-yl, 1,3,2-Dioxaborinan-2-yl, Bis-[(C₁-C₆)-Alkyl]-1,3,2-Dioxaborinan-2-yl, (C₁-C₆)-Alkyl-1,3,2-Dioxaborinan-2-yl, Tris-[(C₁-C₆)-Alkyl]-1,3,2-Dioxaborinan-2-yl, 2,6,7-Trioxa-1-Boranuidabicyclo[2.2.2]octanyl, (C₁-C₆)-Alkyl-2,6,7-Trioxa-1-Boranuidabicyclo[2.2.2]octanyl, Tris-[(C₁-C₆)-Alkyl]plumbanyl, Tris-[(C₃-C₆)-Cycloalkyl]plumbanyl, Tris-[(C₁-C₆)-Alkylcarbonyloxy]plumbanyl, Tris-Arylplumbanyl, Bis-[(C₁-C₆)-Alkylcarbonyloxy]-arylplumbanyl, Bis-[(C₁-C₆)-Alkyl]-alanyl, Bis-[(C₁-C₆)-Cycloalkyl]-alanyl, Dichloralanyl, Chlormagnesyl, Brommagnesyl, Chlorzinkyl, Chlorhydrargyl, Bromhydrargyl, (C₁-C₆)-Alkylhydrargyl, (C₃-C₆)-Cycloalkylhydrargyl, Tris-[(C₁-C₆)-alkyl]silyl, (C₁-C₆)-Alkyl-[bis-(C₁-C₆)-alkyl]silyl, (C₁-C₆)-Alkyl-bis-(aryl)silyl, Aryl-bis-[(C₁-C₆)-alkyl)]silyl, (C₃-C₇)-Cycloalkyl-bis-[(C₁-C₆)-alkyl]silyl steht.

Besonders bevorzugt sind Verbindungen der Formel (III), wobei
R¹ für Wasserstoff, Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropylmethyl, Cyclobutylmethyl, Cyclopentylmethyl, Cyclohexylmethyl, gegebenenfalls substituiertes Phenyl, Heteroaryl, Heterocyclyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Methylcyclohexyl, 2-Methylcyclohexyl, 3-Methylcyclohexyl, 1-Methoxycyclohexyl, 2-Methoxycyclohexyl, 3-Methoxycyclohexyl, Aryl-(C₁-C₅)-alkyl, Heterocyclyl-(C₁-C₅)-alkyl steht,
R² für Wasserstoff, tert.-Butyldimethylsilyl, Trimethylsilyl, Triethylsilyl, Tri-(iso-Propyl)silyl, Tri-(n-Propyl)silyl, Dimethyl(phenyl)silyl, tert.-Butyldiphenylsilyl, Diethylisopropylsilyl, Isopropyldimethylsilyl, tert.-Hexyldimethylsilyl, 2-(Trimethylsilyl)ethoxymethyl, 2-(Trimethylsilyl)ethyl, Methyl, Ethyl, Allyl, Methylcarbonyl, Ethylcarbonyl, n-Propylcarbonyl, iso-Propylcarbonyl, n-Butylcarbonyl, tert-Butylcarbonyl, iso-Butylcarbonyl, iso-Pentylcarbonyl, neo-Pentylcarbonyl, Arylcarbonyl, Heteroarylcarbonyl, Cyclopropylcarbonyl, Cyclobutylcarbonyl, Cyclopentylcarbonyl, Cyclohexylcarbonyl, Heterocyclylcarbonyl, Methoxycarbonyl, Ethoxycarbonyl, n-Propoxycarbonyl, n-Butyloxycarbonyl, iso-Propoxycarbonyl, iso-Butyloxycarbonyl, tert-Butyloxycarbonyl, Allyloxycarbonyl, Benzyl, p-CI-Benzyl, p-F-Benzyl, p-Methoxybenzyl, p-Methylbenzyl, Methylthiomethyl, Methylthioethyl, Ethylthioethyl, Methylthio-n-propyl, Ethylthio-n-propyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1 steht,
n für 0, 1 steht,
R³ und R⁴ unabhängig voneinander für Wasserstoff, Methyl, Ethyl, n-Propyl, iso-Propyl, n-Butyl, iso-Butyl, tert.-Butyl, Fluor, Chlor, Brom, lod, Cyclopropyl, Cyclobutyl, Cyclopentyl, Cyclohexyl, Methoxy, Ethoxy, n-Propoxy, iso-Propoxy, n-Butyloxy, tert.-Butyloxy, iso-Butyloxy, n-Pentyloxy, gegebenenfalls substituiertes Phenyl, Heterocyclyl, Heteroaryl, Benzyl, p-Cl-Benzyl, p-F-Benzyl, p-Methoxybenzyl, p-Methylbenzyl, Methylthio, Ethylthio, n-Propylthio, n-Butylthio, n-Pentylthio, iso-Propylthio, iso-Butylthio, Trifluormethyl, Pentafluorethyl, 1,1,2,2-Tetrafluorethyl, Heptafluorpropyl, Nonafluorbutyl, Chlordifluormethyl, Bromdifluormethyl, Dichlorfluormethyl, Bromfluormethyl, 1-Fluorethyl, 2-Fluorethyl, Fluormethyl, Difluormethyl, 2,2-Difluorethyl, 2,2,2-Trifluorethyl, 2,2-Dichlor-2-fluororethyl, 2-Chlor-2,2-difluorethyl, Difluor-tert.-butyl, Trifluormethoxymethyl, Trifluormethoxyethyl, Trifluormethoxy-n-propyl, Difluormethoxymethyl, Difluormethoxyethyl, Difluormethoxy-n-propyl, 2,2-Difluorethoxymethyl, 2,2-Difluorethoxyethyl, 2,2-Difluorethoxy-n-propyl, 2,2,2-Trifluorethoxymethyl, 2,2,2-Trifluorethoxyethyl, 2,2,2-Trifluorethoxy-n-propyl, Vinyl, Prop-1-en-1-yl, But-1-en-1-yl, Allyl, 1-Methyl-prop-2-en-1-yl, 2-Methyl-prop-2-en-1-yl, But-2-en-1-yl, 1-Methyl-but-3-en-1-yl und 1-Methyl-but-2-en-1-yl, Ethinyl, Propargyl, 1-Methyl-prop-2-in-1-yl, 2-Butinyl, 2-Pentinyl, Trifluormethoxy, Difluormethoxy, 2,2-Difluorethoxy, 2,2,2-Trifluorethoxy, Trifluormethylthio, Methoxymethyl, Ethoxymethyl, Ethoxyethyl, Methoxyethyl, Methoxy-n-propyl, Ethoxy-n-propyl, Methoxybutyl, Methoxy-iso-Propyl, iso-Propoxymethyl, iso-Propoxyethyl, Methylthiomethyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden und
[M] für Trimethylstannyl, Triethylstannyl, Tris-(n-propyl)stannyl, Tris-(iso-propyl)stannyl, Tris-(n-butyl)stannyl, Tris-(sec-butyl)stannyl, Tris(tert-Butyl)stannyl, Tris-(n-pentyl)stannyl, Tris-(n-hexyl)stannyl, für Trimethylgermanyl, Triethylgermanyl, Tris-(n-propyl)germanyl, Tris-(iso-propyl)germanyl, Tris-(n-butyl)germanyl, Tris-(sec-butyl)germanyl, Tris(tert-Butyl)germanyl, Tris-(n-pentyl)germanyl, Tris-(n-hexyl)germanyl, tris-(cyclohexyl)stannyl, Tris-(cyclohexyl)germanyl, Bis-(Cyclopentadienyl)zirconyl, Bis-(1,2,3,4,5-Pentamethylcyclopentadienyl)zirconyl, Bis-(Cyclopentadienyl)hafnyl, Bis-(1,2,3,4,5-Pentamethylcyclopentadienyl)hafnyl, Bis-(Hydroxy)boryl, Bis-(methoxy)boryl, Bis-(ethoxy)boryl, Bis-(n-propyloxy)boryl, Bis-(iso-propyloxy)boryl, Bis-(n-butyloxy)boryl, Bis-(sec-butyloxy)boryl, Bis-(tert-butyloxy)boryl, Bis-(n-pentyloxy)boryl, Bis-(iso-pentyloxy)boryl, Bis-(neo-pentyloxy)boryl, Bis-(n-hexyloxy)boryl, (C₁-C₆)-Alkyl-1,3,2-dioxaborolan-2-yl, Bis-[(C₁-C₆)-Alkyl]-1,3,2-dioxaborolan-2-yl, Tetrakis-[(C₁-C₆)-Alkyl]-1,3,2-dioxaborolan-2-yl, 1,3,2-Dioxaborinan-2-yl, Bis-[(C₁-C₆)-Alkyl]-1,3,2-Dioxaborinan-2-yl, (C₁-C₆)-Alkyl-1,3,2-Dioxaborinan-2-yl, Tris-[(C₁-C₆)-Alkyl]-1,3,2-Dioxaborinan-2-yl, 2,6,7-Trioxa-1-Boranuidabicyclo[2.2.2]octanyl, (C₁-C₆)-Alkyl-2,6,7-Trioxa-1-Boranuidabicyclo[2.2.2]octanyl, Tris-[(C₁-C₆)-Alkyl]plumbanyl, Tris-(cycloalkyl)plumbanyl, Tris-[(C₁-C₆)-Alkylcarbonyloxy]plumbanyl, Tris-Arylplumbanyl, Bis-[(C₁-C₆)-Alkylcarbonyloxy]-arylplumbanyl, Bis-[(C₁-C₆)-Alkyl]-alanyl, Bis-[(C₁-C₆)-Cycloalkyl]-alanyl, Dichloralanyl, Chlormagnesyl, Brommagnesyl, Chlorzinkyl, Chlorhydrargyl, Bromhydrargyl, (C₁-C₆)-Alkylhydrargyl, (C₃-C₆)-Cycloalkylhydrargyl, Tris-[(C₁-C₆)-alkyl]silyl, (C₁-C₆)-Alkyl-[bis-(C₁-C₆)-alkyl]silyl, (C₁-C₆)-Alkyl-bis-(aryl)silyl, Aryl-bis-[(C₁-C₆)-alkyl)]silyl, (C₃-C₇)-Cycloalkyl-bis-[(C₁-C₆)-alkyl]silyl steht.

Ganz besonders bevorzugt sind Verbindungen der Formel (III), wobei
R¹ für Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Cyclopropylmethyl, Cyclobutylmethyl, Cyclopentylmethyl, Cyclohexylmethyl, gegebenenfalls substituiertes Phenyl, Heteroaryl, Heterocyclyl, Cyclopropyl, Cyclobutyl, Cyclopentyl, Cyclohexyl, Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl, Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl, Adamantan-2-yl, 1-Methylcyclopropyl, 2-Methylcyclopropyl, 2,2-Dimethylcyclopropyl, 2,3-Dimethylcyclopropyl, 1,1'-Bi(cyclopropyl)-1-yl, 1,1'-Bi(cyclopropyl)-2-yl, 2'-Methyl-1,1'-bi(cyclopropyl)-2-yl, 1-Cyanopropyl, 2-Cyanopropyl, 1-Methylcyclobutyl, 2-Methylcyclobutyl, 3-Methylcyclobutyl, 1-Cyanocyclobutyl, 2-Cyanocyclobutyl, 3-Cyanocyclobutyl, 1-Methylcyclohexyl, 2-Methylcyclohexyl, 3-Methylcyclohexyl, 1-Methoxycyclohexyl, 2-Methoxycyclohexyl, 3-Methoxycyclohexyl, Aryl-(C₁-C₅)-alkyl, Heterocyclyl-(C₁-C₅)-alkyl steht,
R² für Wasserstoff, tert.-Butyldimethylsilyl, Trimethylsilyl, Triethylsilyl, Tri-(iso-Propyl)silyl, Tri-(n-Propyl)silyl, Dimethyl(phenyl)silyl, tert.-Butyldiphenylsilyl, Diethylisopropylsilyl, Isopropyldimethylsilyl, tert.-Hexyldimethylsilyl, 2-(Trimethylsilyl)ethoxymethyl, 2-(Trimethylsilyl)ethyl, Methyl, Ethyl, Allyl, Methylcarbonyl, Ethylcarbonyl, n-Propylcarbonyl, iso-Propylcarbonyl, n-Butylcarbonyl, tert-Butylcarbonyl, iso-Butylcarbonyl, iso-Pentylcarbonyl, neo-Pentylcarbonyl, Arylcarbonyl, Heteroarylcarbonyl, Cyclopropylcarbonyl, Cyclobutylcarbonyl, Cyclopentylcarbonyl, Cyclohexylcarbonyl, Heterocyclylcarbonyl, Methoxycarbonyl, Ethoxycarbonyl, n-Propoxycarbonyl, n-Butyloxycarbonyl, iso-Propoxycarbonyl, iso-Butyloxycarbonyl, tert-Butyloxycarbonyl, Allyloxycarbonyl, Benzyl, p-CI-Benzyl, p-F-Benzyl, p-Methoxybenzyl, p-Methylbenzyl, Methylthiomethyl, Methylthioethyl, Ethylthioethyl, Methylthio-n-propyl, Ethylthio-n-propyl steht,
A¹, V, W unabhängig voneinander für eine Gruppe CR³R⁴, stehen,
A² für eine Gruppe CR³R⁴ oder Sauerstoff steht,
m für 0 steht,
n für 0, 1 steht,
R³ und R⁴ unabhängig voneinander für Wasserstoff, Methyl, Ethyl, n-Propyl, iso-Propyl, n-Butyl, iso-Butyl, tert.-Butyl, Fluor, Chlor, Brom, lod, Cyclopropyl, Cyclobutyl, Cyclopentyl, Cyclohexyl, Methoxy, gegebenenfalls substituiertes Phenyl, Heterocyclyl, Heteroaryl, Methylthio, Trifluormethyl, Difluormethyl, Vinyl, Prop-1-en-1-yl, But-1-en-1-yl, Allyl, Trifluormethoxy, Difluormethoxy, 2,2-Difluorethoxy, 2,2,2-Trifluorethoxy, Trifluormethylthio, Methoxymethyl, Ethoxymethyl, Ethoxyethyl, Methoxyethyl, Methylthiomethyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden und
[M] für Trimethylstannyl, Triethylstannyl, Tris-(n-propyl)stannyl, Tris-(iso-propyl)stannyl, Tris-(n-butyl)stannyl, Tris-(sec-butyl)stannyl, Tris(tert-Butyl)stannyl, Tris-(n-pentyl)stannyl, Tris-(n-hexyl)stannyl, für Trimethylgermanyl, Triethylgermanyl, Tris-(n-propyl)germanyl, Tris-(iso-propyl)germanyl, Tris-(n-butyl)germanyl, Tris-(sec-butyl)germanyl, Tris(tert-Butyl)germanyl, Tris-(n-pentyl)germanyl, Tris-(n-hexyl)germanyl, tris-(cyclohexyl)stannyl, Tris-(cyclohexyl)germanyl, Bis-(Cyclopentadienyl)zirconyl, Bis-(1,2,3,4,5-Pentamethylcyclopentadienyl)zirconyl, Bis-(Cyclopentadienyl)hafnyl, Bis-(1,2,3,4,5-Pentamethylcyclopentadienyl)hafnyl, Bis-(Hydroxy)boryl, Bis-(methoxy)boryl, Bis-(ethoxy)boryl, Bis-(n-propyloxy)boryl, Bis-(iso-propyloxy)boryl, Bis-(n-butyloxy)boryl, Bis-(sec-butyloxy)boryl, Bis-(tert-butyloxy)boryl, Bis-(n-pentyloxy)boryl, Bis-(iso-pentyloxy)boryl, Bis-(neo-pentyloxy)boryl, Bis-(n-hexyloxy)boryl, (C₁-C₆)-Alkyl-1,3,2-dioxaborolan-2-yl, Bis-[(C₁-C₆)-Alkyl]-1,3,2-dioxaborolan-2-yl, Tetrakis-[(C₁-C₆)-Alkyl]-1,3,2-dioxaborolan-2-yl, 1,3,2-Dioxaborinan-2-yl, Bis-[(C₁-C₆)-Alkyl]-1,3,2-Dioxaborinan-2-yl, (C₁-C₆)-Alkyl-1,3,2-Dioxaborinan-2-yl, Tris-[(C₁-C₆)-Alkyl]-1,3,2-Dioxaborinan-2-yl, 2,6,7-Trioxa-1-Boranuidabicyclo[2.2.2]octanyl, (C₁-C₆)-Alkyl-2,6,7-Trioxa-1-Boranuidabicyclo[2.2.2]octanyl, Tris-[(C₁-C₆)-Alkyl]plumbanyl, Tris-(cycloalkyl)plumbanyl, Tris-[(C₁-C₆)-Alkylcarbonyloxy]plumbanyl, Tris-Arylplumbanyl, Bis-[(C₁-C₆)-Alkylcarbonyloxy]-arylplumbanyl, Bis-[(C₁-C₆)-Alkyl]-alanyl, Bis-[(C₁-C₆)-Cycloalkyl]-alanyl, Dichloralanyl, Chlormagnesyl, Brommagnesyl, Chlorzinkyl, Chlorhydrargyl, Bromhydrargyl, (C₁-C₆)-Alkylhydrargyl, (C₃-C₆)-Cycloalkylhydrargyl, Tris-[(C₁-C₆)-alkyl]silyl, (C₁-C₆)-Alkyl-[bis-(C₁-C₆)-alkyl]silyl, (C₁-C₆)-Alkyl-bis-(aryl)silyl, Aryl-bis-[(C₁-C₆)-alkyl)]silyl, (C₃-C₇)-Cycloalkyl-bis-[(C₁-C₆)-alkyl]silyl steht.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:
Erfindungsgemäß steht "Arylsulfonyl" für gegebenenfalls substituiertes Phenylsulfonyl oder gegebenenfalls substituiertes polycyclisches Arylsulfonyl, hier insbesondere gegebenenfalls substituiertes Naphthyl-sulfonyl, beispielsweise substituiert durch Fluor, Chlor, Brom, lod, Cyano, Nitro, Alkyl-, Haloalkyl-, Haloalkoxy-, Amino-, Alkylamino-, Alkylcarbonylamino-, Dialkylamino- oder Alkoxy-gruppen.

Erfindungsgemäß steht "Cycloalkylsulfonyl" - in Alleinstellung oder als Bestandteil einer chemischen Gruppe - für gegebenenfalls substituiertes Cycloalkylsulfonyl, vorzugsweise mit 3 bis 6 Kohlenstoffatomen wie beispielsweise Cyclopropylsulfonyl, Cyclobutylsulfonyl, Cyclopentylsulfonyl oder Cyclohexylsulfonyl.

Erfindungsgemäß steht "Alkylsulfonyl" - in Alleinstellung oder als Bestandteil einer chemischen Gruppe - für geradkettiges oder verzweigtes Alkylsulfonyl, vorzugsweise mit 1 bis 8, oder mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, sec-Butylsulfonyl und tert-Butylsulfonyl.

Erfindungsgemäß steht "Heteroarylsulfonyl" für gegebenenfalls substituiertes Pyridylsulfonyl, Pyrimidinylsulfonyl, Pyrazinylsulfonyl oder gegebenenfalls substituiertes polycyclisches Heteroarylsulfonyl, hier insbesondere gegebenenfalls substituiertes Chinolinylsulfonyl, beispielsweise substituiert durch Fluor, Chlor, Brom, lod, Cyano, Nitro, Alkyl-, Haloalkyl-, Haloalkoxy-, Amino-, Alkylamino-, Alkylcarbonylamino-, Dialkylamino- oder Alkoxygruppen.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 8, oder mit 1 bis 6 Kohlenstoffatomen, wie beispielsweise Methylthio, Ethylthio, n-Propylthio, Isopropylthio, n-Butylthio, Isobutylthio, sec-Butylthio und tert-Butylthio. Alkenylthio bedeutet ein über ein Schwefelatom gebundenen Alkenylrest, Alkinylthio bedeutet ein über ein Schwefelatom gebundenen Alkinylrest, Cycloalkylthio bedeutet ein über ein Schwefelatom gebundenen Cycloalkylrest und Cycloalkenylthio bedeutet ein über ein Schwefelatom gebundenen Cycloalkenylrest.

"Alkoxy" bedeutet ein über ein Sauerstoffatom gebundenen Alkylrest, Alkenyloxy bedeutet ein über ein Sauerstoffatom gebundenen Alkenylrest, Alkinyloxy bedeutet ein über ein Sauerstoffatom gebundenen Alkinylrest, Cycloalkyloxy bedeutet ein über ein Sauerstoffatom gebundenen Cycloalkylrest und Cycloalkenyloxy bedeutet ein über ein Sauerstoffatom gebundenen Cycloalkenylrest.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P) der gesättigt, ungesättigt, teilgesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfaßt, wie beispielsweise 8-Aza-bicyclo[3.2.1]octanyl, 8-Aza-bicyclo[2.2.2]octanyl oder 1-Aza-bicyclo[2.2.1]heptyl. Im Falle von gegebenenfalls substituiertem Heterocyclyl werden auch spirocyclische Systeme umfaßt, wie beispielsweise 1-Oxa-5-aza-spiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und, S 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1 - oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetrahydropyridin-1 - oder 2- oder 3- oder 4- oder 5- oder 6-yl; 1,2,3,4-Tetrahydropyridin-1 - oder 2- oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2- oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2- oder 3- oder 4-yl; 3,4,5,6-Tetrahydro-2H-azepin-2- oder 3- oder 4- oder 5- oder 6- oder 7-yl; 4,5-Dihydro-1H-azepin-1- oder 2- oder 3- oder 4-yl; 2,5-Dihydro-1H-azepin-1- oder -2- oder 3- oder 4- oder 5- oder 6- oder 7-yl; 2,7-Dihydro-1H-azepin-1- oder -2- oder 3- oder 4-yl; 2,3-Dihydro-1H-azepin-1- oder -2- oder 3- oder 4- oder 5- oder 6- oder 7-yl; 3,4-Dihydro-2H-azepin-2- oder 3- oder 4- oder 5- oder 6- oder 7-yl; 3,6-Dihydro-2H-azepin-2- oder 3- oder 4- oder 5- oder 6- oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5- oder 6- oder 7-yl; 1H-Azepin-1- oder -2- oder 3- oder 4- oder 5- oder 6- oder 7-yl; 2H-Azepin-2- oder 3- oder 4- oder 5- oder 6- oder 7-yl; 3H-Azepin-2- oder 3- oder 4- oder 5- oder 6- oder 7-yl; 4H-Azepin-2- oder 3- oder 4- oder 5- oder 6- oder 7-yl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2- oder 3- oder 4-Oxanyl (= 2- oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3- oder 4- oder 5- oder 6-yl; 4H-Pyran-2- oder 3- oder 4-yl, 2- oder 3- oder 4-Oxepanyl; 2,3,4,5-Tetrahydrooxepin-2- oder 3- oder 4- oder 5- oder 6- oder 7-yl; 2,3,4,7-Tetrahydrooxepin-2- oder 3- oder 4- oder 5- oder 6- oder 7-yl; 2,3,6,7-Tetrahydrooxepin-2- oder 3- oder 4-yl; 2,3-Dihydrooxepin-2- oder 3- oder 4- oder 5- oder 6- oder 7-yl; 4,5-Dihydrooxepin-2- oder 3- oder 4-yl; 2,5-Dihydrooxepin-2- oder 3- oder 4- oder 5- oder 6- oder 7-yl; Oxepin-2- oder 3- oder 4- oder 5- oder 6- oder 7-yl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3- oder 4- oder 5- oder 6-yl; 4H-Thiopyran-2- oder 3- oder 4-yl. Bevorzugte 3-Ring und 4-Ring-Heterocyclen sind beispielsweise 1- oder 2-Aziridinyl, Oxiranyl, Thiiranyl, 1- oder 2- oder 3-Azetidinyl, 2- oder 3-Oxetanyl, 2- oder 3-Thietanyl, 1,3-Dioxetan-2-yl. Weitere Beispiele für "Heterocyclyl" sind ein partiell oder vollständig hydrierter heterocyclischer Rest mit zwei Heteroatomen aus der Gruppe N, O und S, wie beispielsweise 1- oder 2- oder 3- oder 4-Pyrazolidinyl; 4,5-Dihydro-3H-pyrazol- 3- oder 4- oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2- oder 4- oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3- oder 4- oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5- oder 6-yl; 1,2,3,4-Tetrahydropyrazin-1- oder 2- oder 3- oder 4- oder 5- oder 6-yl; 1,2-Dihydropyrazin-1- oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2- oder 4-yl; 1,3-Dioxan-2- oder 4- oder 5-yl; 4H-1,3-Dioxin-2- oder 4- oder 5- oder 6-yl; 1,4-Dioxan-2- oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro-1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2- oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2- oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3- oder 4- oder 5- oder 6-yl; 6H-1,2-Oxazin-3- oder 4- oder 5- oder 6-yl; 4H-1,2-Oxazin-3- oder 4- oder 5- oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2- oder 4- oder 5- oder 6-yl; Morpholin-2- oder 3- oder 4-yl; 3,4-Dihydro-2H-1,4-oxazin-2- oder 3- oder 4- oder 5- oder 6-yl; 3,6-Dihydro-2H-1,4-oxazin-2- oder 3- oder 5- oder 6-yl; 2H-1,4-oxazin-2- oder 3- oder 5- oder 6-yl; 4H-1,4-oxazin-2- oder 3-yl; 1,2-Oxazepan-2- oder 3- oder 4- oder 5- oder 6- oder 7-yl; 2,3,4,5-Tetrahydro-1,2-oxazepin-2- oder 3- oder 4- oder 5- oder 6- oder 7-yl; 2,3,4,7-Tetrahydro-1,2-oxazepin-2- oder 3- oder 4- oder 5- oder 6- oder 7-yl; 2,3,6,7-Tetrahydro-1,2-oxazepin-2- oder 3- oder 4- oder 5- oder 6- oder 7-yl; 2,5,6,7-Tetrahydro-1,2-oxazepin-2- oder 3- oder 4- oder 5- oder 6- oder 7-yl; 4,5,6,7-Tetrahydro-1,2-oxazepin-3- oder 4- oder 5- oder 6- oder 7-yl; 2,3-Dihydro-1,2-oxazepin-2- oder 3- oder 4- oder 5- oder 6- oder 7-yl; 2,5-Dihydro-1,2-oxazepin-2- oder 3- oder 4- oder 5- oder 6- oder 7-yl; 2,7-Dihydro-1,2-oxazepin-2- oder 3- oder 4- oder 5- oder 6- oder 7-yl; 4,5-Dihydro-1,2-oxazepin-3- oder 4- oder 5- oder 6- oder 7-yl; 4,7-Dihydro-1,2-oxazepin-3- oder 4- oder 5- oder 6- oder 7-yl; 6,7-Dihydro-1,2-oxazepin-3- oder 4- oder 5- oder 6- oder 7-yl; 1,2-Oxazepin-3-oder 4- oder 5- oder 6- oder 7-yl; 1,3-Oxazepan-2- oder 3- oder 4- oder 5- oder 6- oder 7-yl; 2,3,4,5-Tetrahydro-1,3-oxazepin-2- oder 3- oder 4- oder 5- oder 6- oder 7-yl; 2,3,4,7-Tetrahydro-1,3-oxazepin-2- oder 3- oder 4- oder 5- oder 6- oder 7-yl; 2,3,6,7-Tetrahydro-1,3-oxazepin-2- oder 3- oder 4- oder 5- oder 6- oder 7-yl; 2,5,6,7-Tetrahydro-1,3-oxazepin-2- oder 4- oder 5- oder 6- oder 7-yl; 4,5,6,7-Tetrahydro-1,3-oxazepin-2- oder 4- oder 5- oder 6- oder 7-yl; 2,3-Dihydro-1,3-oxazepin-2- oder 3- oder 4- oder 5- oder 6- oder 7-yl; 2,5-Dihydro-1,3-oxazepin-2- oder 4- oder 5- oder 6- oder 7-yl; 2,7-Dihydro-1,3-oxazepin-2- oder 4- oder 5- oder 6- oder 7-yl; 4,5-Dihydro-1,3-oxazepin-2- oder 4- oder 5- oder 6- oder 7-yl; 4,7-Dihydro-1,3-oxazepin-2- oder 4- oder 5- oder 6- oder 7-yl; 6,7-Dihydro-1,3-oxazepin-2- oder 4- oder 5- oder 6- oder 7-yl; 1,3-Oxazepin-2- oder 4- oder 5- oder 6- oder 7-yl; 1,4-Oxazepan-2- oder 3- oder 5- oder 6- oder 7-yl; 2,3,4,5-Tetrahydro-1,4-oxazepin-2- oder 3- oder 4- oder 5- oder 6- oder 7-yl; 2,3,4,7-Tetrahydro-1,4-oxazepin-2- oder 3- oder 4- oder 5- oder 6- oder 7-yl; 2,3,6,7-Tetrahydro-1,4-oxazepin-2- oder 3- oder 5- oder 6- oder 7-yl; 2,5,6,7-Tetrahydro-1,4-oxazepin-2- oder 3- oder 5- oder 6- oder 7-yl; 4,5,6,7-Tetrahydro-1,4-oxazepin-2- oder 3- oder 4- oder 5- oder 6- oder 7-yl; 2,3-Dihydro-1,4-oxazepin-2- oder 3- oder 5- oder 6- oder 7-yl; 2,5-Dihydro-1,4-oxazepin-2- oder 3- oder 5- oder 6- oder 7-yl; 2,7-Dihydro-1,4-oxazepin-2- oder 3- oder 5- oder 6- oder 7-yl; 4,5-Dihydro-1,4-oxazepin-2- oder 3- oder 4- oder 5- oder 6- oder 7-yl; 4,7-Dihydro-1,4-oxazepin-2- oder 3- oder 4- oder 5- oder 6- oder 7-yl; 6,7-Dihydro-1,4-oxazepin-2- oder 3- oder 5- oder 6- oder 7-yl; 1,4-Oxazepin-2- oder 3- oder 5- oder 6- oder 7-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5-yl; 4,5-Dihydroisothiazol-3- oder 4- oder 5-yl; 1,3-Thiazolidin-2- oder 3- oder 4- oder 5-yl; 2,3-Dihydro-1,3-thiazol-2- oder 3- oder 4- oder 5-yl; 2,5-Dihydro-1,3-thiazol-2- oder 4- oder 5-yl; 4,5-Dihydro-1,3-thiazol-2- oder 4- oder 5-yl; 1,3-Thiazinan-2- oder 3- oder 4- oder 5- oder 6-yl; 3,4-Dihydro-2H-1,3-thiazin-2- oder 3- oder 4- oder 5- oder 6-yl; 3,6-Dihydro-2H-1,3-thiazin-2- oder 3- oder 4- oder 5- oder 6-yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5- oder 6-yl; 6H-1,3-Thiazin-2- oder 4- oder 5- oder 6-yl; 4H-1,3-Thiazin-2- oder 4- oder 5- oder 6-yl. Weitere Beispiele für "Heterocyclyl" sind ein partiell oder vollständig hydrierter heterocyclischer Rest mit 3 Heteroatomen aus der Gruppe N, O und S, wie beispielsweise 1,4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl; 1,4,2-Dioxazepan-2- oder 3- oder 5- oder 6- oder 7-yl; 6,7-Dihydro-5H-1,4,2-Dioxazepin-3- oder 5- oder 6- oder 7-yl; 2,3-Dihydro-7H-1,4,2-Dioxazepin-2- oder 3- oder 5- oder 6- oder 7-yl; 2,3-Dihydro-5H-1,4,2-Dioxazepin-2- oder 3- oder 5- oder 6- oder 7-yl; 5H-1,4,2-Dioxazepin-3- oder 5- oder 6- oder 7-yl; 7H-1,4,2-Dioxazepin-3- oder 5- oder 6- oder 7-yl. Strukturbeispiele für gegebenenfalls weiter substituierte Heterocyclen sind auch im Folgenden aufgeführt:

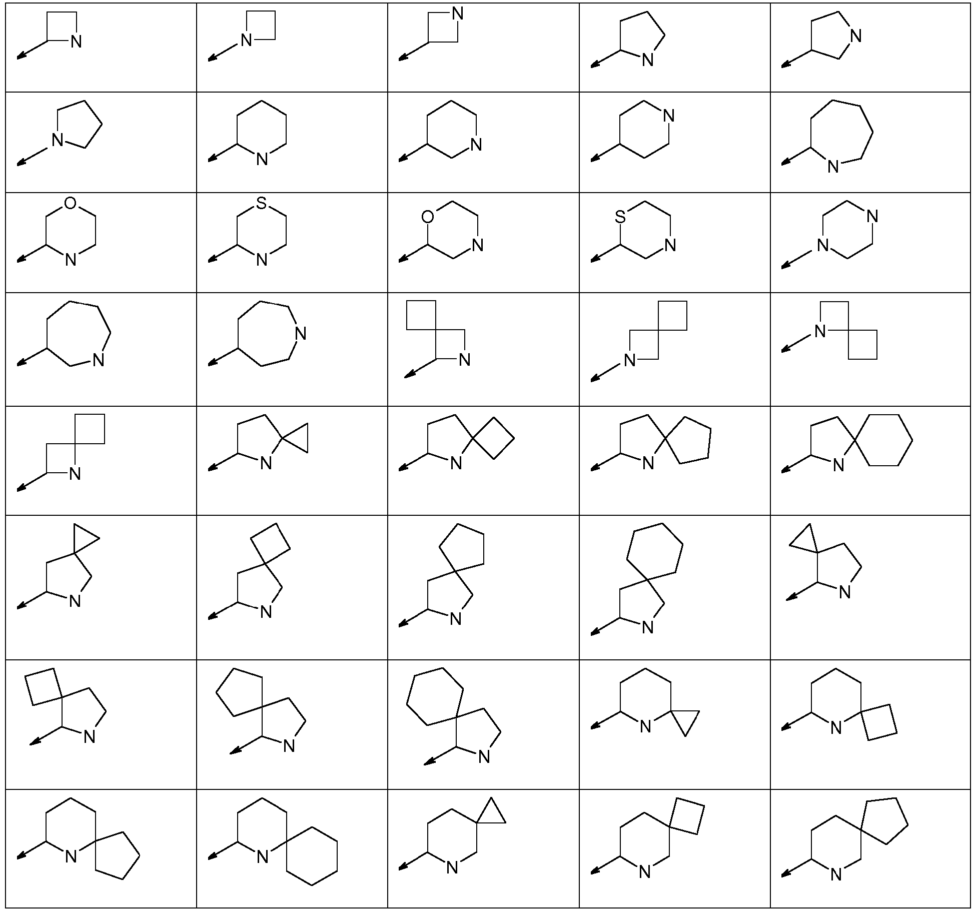

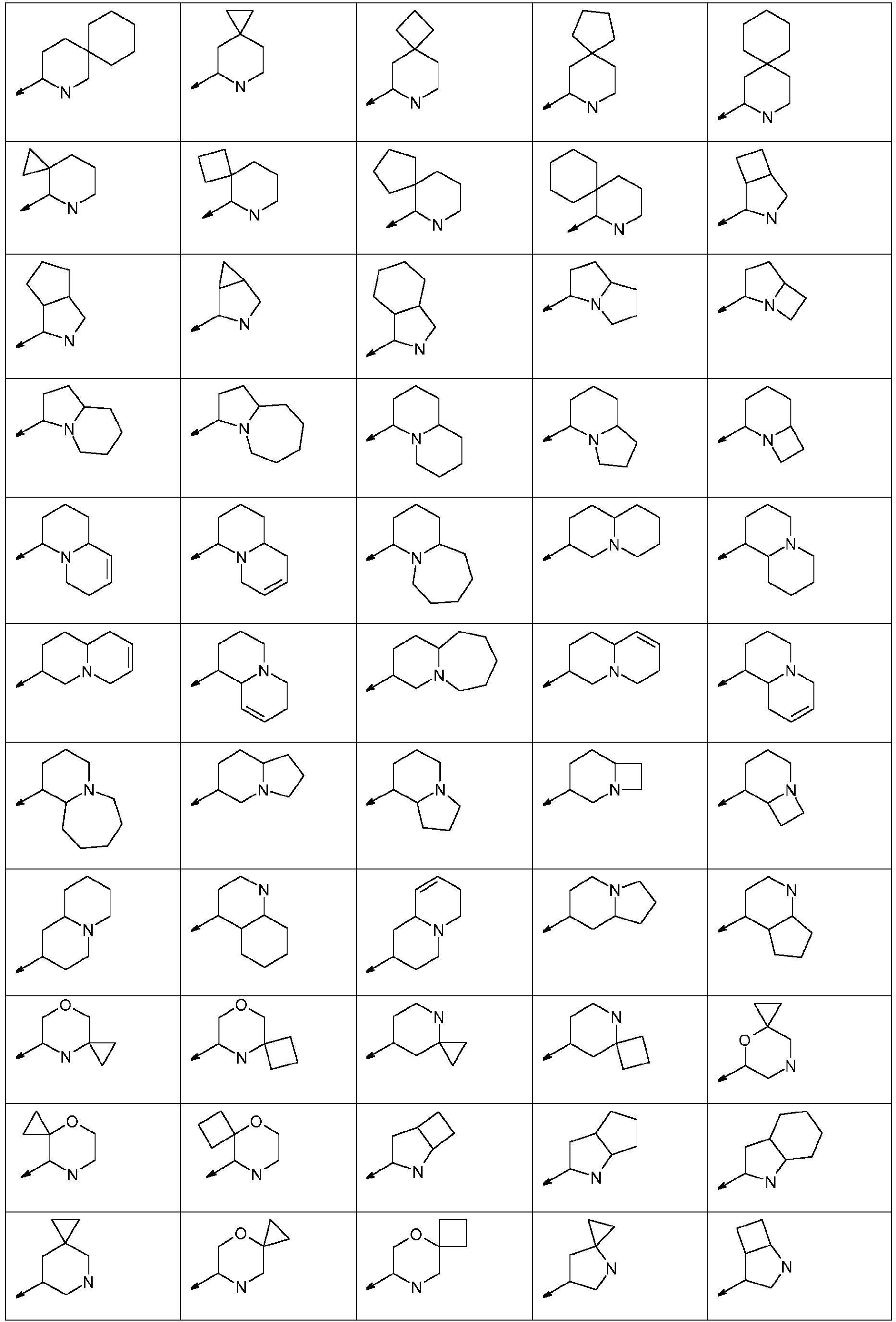

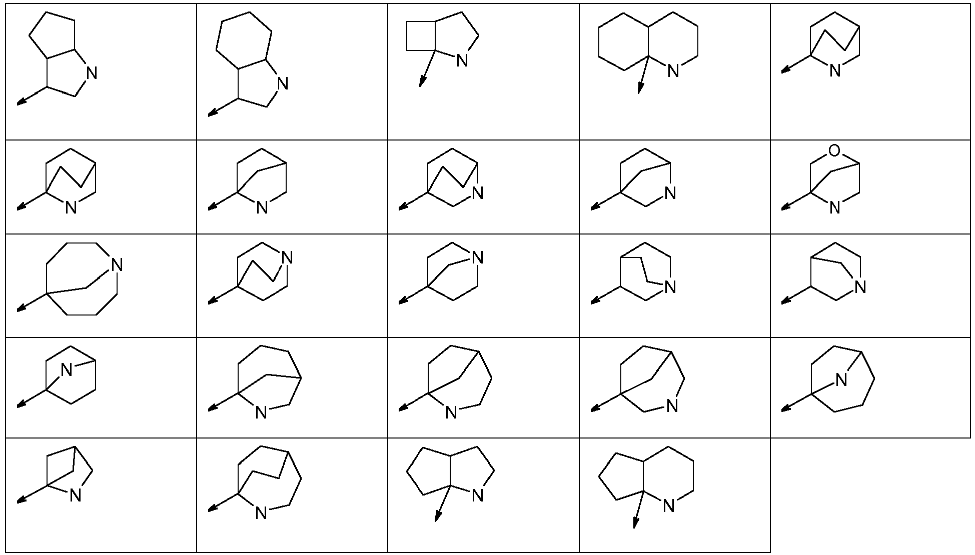

Die oben aufgeführten Heterocyclen sind bevorzugt beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Tris-alkylsilylalkinyl, Nitro, Amino, Cyano, Haloalkoxy, Haloalkylthio, Alkylthio, Hydrothio, Hydroxyalkyl, Oxo, Heteroarylalkoxy, Arylalkoxy, Heterocyclylalkoxy, Heterocyclylalkylthio, Heterocyclyloxy, Heterocyclylthio, Heteroaryloxy, Bis-alkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bisalkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Handelt es sich um einen teilweise oder vollständig gesättigten StickstoffHeterocyclus, so kann dieser sowohl über Kohlenstoff als auch über den Stickstoff mit dem Rest des Moleküls verknüpft sein.

Als Substituenten für einen substituierten heterocyclischen Rest kommen die weiter unten genannten Substituenten in Frage, zusätzlich auch Oxo und Thioxo. Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen N(O), S(O) (auch kurz SO) und S(O)2 (auch kurz SO2) im heterocyclischen Ring. Im Fall von -N(O)- und -S(O)-Gruppen sind jeweils beide Enantiomere umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1 oder 2 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, Oxepinyl, Thiepinyl, 1,2,4-Triazolonyl und 1,2,4-Diazepinyl, 2H-1,2,3,4-Tetrazol-5-yl, 1H-1,2,3,4-Tetrazol-5-yl, 1,2,3,4-Oxatriazol-5-yl, 1,2,3,4-Thiatriazol-5-yl, 1,2,3,5-Oxatriazol-4-yl, 1,2,3,5-Thiatriazol-4-yl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinoline (z. B. Chinolin-2-yl, Chinolin-3-yl, Chinolin-4-yl, Chinolin-5-yl, Chinolin-6-yl, Chinolin-7-yl, Chinolin-8-yl); Isochinoline (z. B. Isochinolin-1-yl, Isochinolin-3-yl, Isochinolin-4-yl, Isochinolin-5-yl, Isochinolin-6-yl, Isochinolin-7-yl, Isochinolin-8-yl); Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7-Naphthyridin; Phthalazin; Pyridopyrazine; Pyridopyrimidine; Pyridopyridazine; Pteridine; Pyrimidopyrimidine. Beispiele für Heteroaryl sind auch 5- oder 6-gliedrige benzokondensierte Ringe aus der Gruppe 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 2H-Indazol-3-yl, 2H-Indazol-4-yl, 2H-Indazol-5-yl, 2H-Indazol-6-yl, 2H-Indazol-7-yl, 2H-Isoindol-2-yl, 2H-Isoindol-1-yl, 2H-Isoindol-3-yl, 2H-Isoindol-4-yl, 2H-Isoindol-5-yl, 2H-Isoindol-6-yl; 2H-Isoindol-7-yl, 1H-Benzimidazol-1-yl, 1H-Benzimidazol-2-yl, 1H-Benzimidazol-4-yl, 1H-Benzimidazol-5-yl, 1H-Benzimidazol-6-yl, 1H-Benzimidazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, 1,3-Benzthiazol-2-yl, 1,3-Benzthiazol-4-yl, 1,3-Benzthiazol-5-yl, 1,3-Benzthiazol-6-yl, 1,3-Benzthiazol-7-yl, 1,2-Benzisoxazol-3-yl, 1,2-Benzisoxazol-4-yl, 1,2-Benzisoxazol-5-yl, 1,2-Benzisoxazol-6-yl, 1,2-Benzisoxazol-7-yl, 1,2-Benzisothiazol-3-yl, 1,2-Benzisothiazol-4-yl, 1,2-Benzisothiazol-5-yl, 1,2-Benzisothiazol-6-yl, 1,2-Benzisothiazol-7-yl.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder lodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist und im letzteren Falle als "substituiertes Alkyl" bezeichnet wird. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod.

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. CH₂CH₂Cl, CH₂CH₂Br, CHClCH₃, CH₂Cl, CH₂F; Perhaloalkyl wie z. B. CCl₃, CClF₂, CFCl₂,CF₂CClF₂, CF₂CClFCF₃; Polyhaloalkyl wie z. B. CH₂CHFCl, CF₂CClFH, CF₂CBrFH, CH₂CF₃; Der Begriff Perhaloalkyl umfasst dabei auch den Begriff Perfluoralkyl.

Teilfluoriertes Alkyl bedeutet einen geradkettigen oder verzweigten, gesättigten Kohlenwasserstoff, der einfach oder mehrfach durch Fluor substituiert ist, wobei sich die entsprechenden Fluoratome als Substituenten an einem oder mehreren verschiedenen Kohlenstoffatomen der geradkettigen oder verzweigten Kohlenwasserstoffkette befinden können, wie z. B. CHFCH₃, CH₂CH₂F, CH₂CH₂CF₃, CHF₂, CH₂F, CHFCF₂CF₃

Teilfluoriertes Haloalkyl bedeutet einen geradkettigen oder verzweigten, gesättigten Kohlenwasserstoff, der durch verschiedenene Halogenatomen mit mindestens einem Fluoratom substituiert ist, wobei alle anderen gegebenenfalls vorhandenen Halogenatome ausgewählt sind aus der Gruppe Fluor, Chlor oder Brom, Iod. Die entsprechenden Halogenatome können sich dabei als Substituenten an einem oder mehreren verschiedenen Kohlenstoffatomen der geradkettigen oder verzweigten Kohlenwasserstoffkette befinden. Teilfluoriertes Haloalkyl schließt auch die vollständige Substitution der geradkettigen oder verzweigten Kette durch Halogen unter Beteiligung von mindestens einem Fluoratom ein.

Haloalkoxy ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Der hier beispielhaft genannte Ausdruck "(C₁-C₄)-Alkyl" bedeutet eine Kurzschreibweise für geradkettiges oder verzweigtes Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)-Alkyl", umfassen entsprechend auch geradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.

Der Begriff "Alkenyl" schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl. Alkenyl bedeutet z.B. Vinyl, welches ggf. durch weitere Alkylreste substituiert sein kann, z.B. Prop-1-en-1-yl, But-1-en-1-yl, Allyl, 1-Methyl-prop-2-en-1-yl, 2-Methyl-prop-2-en-1-yl, But-2-en-1-yl, 1-Methyl-but-3-en-1-yl und 1-Methyl-but-2-en-1-yl, 2-Methyl-prop-1-en-1-yl, 1-Methylprop-1-en-1-yl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl, Pentenyl, 2-Methylpentenyl oder Hexenyl.

Der Begriff "Alkinyl" schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl. (C₂-C₆)-Alkinyl bedeutet beispielsweise Ethinyl, Propargyl, 1-Methyl-prop-2-in-1-yl, 2-Butinyl, 2-Pentinyl oder 2-Hexinyl, vorzugsweise Propargyl, But-2-in-1-yl, But-3-in-1-yl oder 1-Methyl-but-3-in-1-yl.

Der Begriff "Cycloalkyl" bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Bicyclo[2.2.2]octan-2-yl, Adamantan-1-yl und Adamantan-2-yl. Der Ausdruck "(C₃-C₇)-Cycloalkyl" bedeutet eine Kurzschreibweise für Cycloalkyl mit drei bis 7 Kohlenstoffatomen entsprechend der Bereichsangabe für C-Atome.

Im Falle von substituiertem Cycloalkyl werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

"Cycloalkenyl" bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form (C₁-C₁₀)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H_{5.} Cycloalkyliden bedeutet ein carbocyclischer Rest, der über eine Zweifachbindung gebunden ist.

Der Begriff "Stannyl" steht für einen weiter substituierten Rest, der ein Zinn-Atom enthält; "Germanyl" steht analog für einen weiter substituierten Rest, der ein Germanium-Atom enthält. "Zirconyl" steht für einen weiter substituierten Rest, der ein Zirconium-Atom enthält. "Hafnyl" steht für einen weiter substituierten Rest, der ein Hafnium-Atom enthält. "Boryl", "Borolanyl" und "Borinanyl" steht für weiter substituierte und gegebenenfalls cyclische Gruppen, die jeweils ein Bor-Atom enthalten. "Plumbanyl" steht für einen weiter substituierten Rest, der ein Blei-Atom enthält. "Hydrargyl" steht für einen weiter substituierten Rest, der ein Quecksilber-Atom enthält. "Alanyl" steht für einen weiter substituierten Rest, der ein Aluminium-Atom enthält. "Magnesyl" steht für einen weiter substituierten Rest, der ein Magnesium-Atom enthält. "Zinkyl" steht für einen weiter substituierten Rest, der ein Zink-Atom enthält.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst, jedoch nicht mit ihrer spezifischen Stereoform angegeben sind, sowie deren Gemische.

Synthese von substituierten Vinyl- und Alkinyl-cyanocycloalkanolen sowie Vinyl- und Alkinyl-cyanoheterocyclylalkanolen.

Die erfindungsgemäßen substituierten Vinyl- und Alkinyl-cyanocycloalkanole sowie Vinyl- und Alkinyl-cyanoheterocyclylalkanole der allgemeinen Formel (I) können ausgehend von bekannten Verfahren hergestellt werden. Der bekannte und strukturverwandte pflanzliche Naturstoff Abscisinsäure kann auf verschiedenen Syntheserouten erhalten werden (vgl. Hanson et al. J. Chem. Res. (S), 2003, 426; Constantino et al. J. Org. Chem. 1986, 51, 253; Constantino et al. 1989, 54, 681; Marsh et al. Org. Biomol. Chem. 2006, 4, 4186; WO94/15467). Einige der darin beschriebenen Verfahren zur Synthese des Abscisinsäuregrundgerüstes wurden optimiert und durch alternative Syntheseschritte ersetzt. Die eingesetzten und untersuchten Syntheserouten gehen dabei von kommerziell erhältlichen oder leicht herstellbaren Ketonen und Alkinsäurederivaten aus. Als erstes Schlüsselintermediat für die Synthese der erfindungsgemäßen Verbindungen der allgemeinen Formel (I) wird ein gegebenenfalls weiter substituiertes 1-(2-Hydroxybut-3-in-2-yl)cycloalkylcarbonitril der allgemeinen Formel (II) hergestellt.

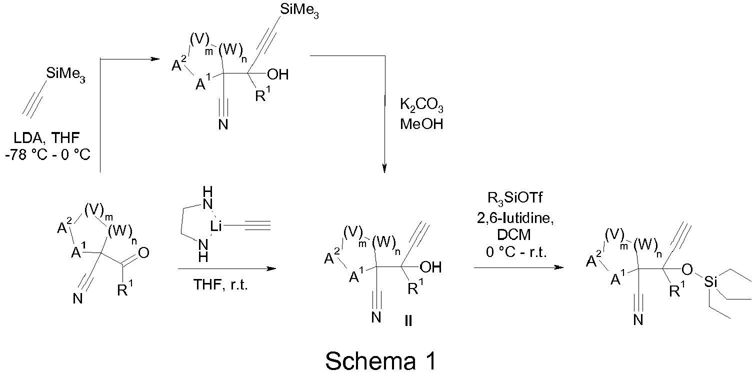

Dies erfolgt durch Umsetzung eines entsprechenden Ketons mit einem Lithiumacetylid-Ethylendiaminkomplex in einem geeigneten polar-aprotischen Lösungsmittel (z. B. Tetrahydrofuran) oder in zwei Schritten durch Umsetzung mit Trimethylsilylacetylen und LDA (Lithiumdiisopropylamid) in einem Temperaturbereich von -78 °C bis 0 °C in einem geeigneten polar-aprotischen Lösungsmittel (z. B. Tetrahydrofuran) und nachfolgende Abspaltung der Trimethylsilylgruppe mit Hilfe eines geeigneten Trialkylammoniumfluorids (z. B. Tetrabutylammoniumfluorid) in einem polar-aprotischen Lösungsmittel oder mit einer geeigneten Carbonatbase (z. B. Kaliumcarbonat) in einem polar-protischen Lösungsmittel (z. B. Methanol) (vgl. J. Chem. Res. (S) 2003, 426) zu einem entsprechend substituierten erfindungsgemäßen 1-(2-Hydroxybut-3-in-2-yl)cycloalkylcarbonitril der allgemeinen Formel II (Schema 1). A¹, A², V, W, m, n und R¹ haben im oben angegebenen Schema 1 die zuvor definierten Bedeutungen. Für R² steht in Schema 1 beispielhaft ein Wasserstoffatom oder eine Triethylsilylgruppe. Die Herstellung der für die Umsetzungen in Schema 1 und 2 verwendeten Ketonedukte erfolgt über literaturbeschriebene Synthesewege (vgl. J. Org. Chem. 1992, 57, 436; Zh. Org. Khim. 1992, 28, 256).

Ausgehend von den obenen beschrieben und substituierten 1-(2-Hydroxybut-3-in-2-yl)cycloalkylcarbonitrilen können die erfindungsgemäßen substituierten Alkinyl-cyanocycloalkanole I(a)-I(b) durch Übergangsmetall-katalysierte Kupplung mit geeigneten substituierten Aryl-, Cycloalkenyl-, Heterocyclyl- oder Heteroarylhalogeniden (vgl. J. Chem. Res. (S), 2003, 426; J. Chem. Soc., Perkin Trans. 1 2001, 47; Adv. Synth. Catal. 2005, 347, 872; Synlett 2010, 150; Org. Lett. 2008, 10, 1569; Helv. Chim. Acta 2009, 92, 826, Can. J. Chem. 1993, 71, 983) unter Verwendung eines geeigneten Übergangsmetallkatalysatorsystems (z. B. Bis(Triphenylphosphin)palladiumdichlorid, Palladium(II)acetat zusammen mit Triphenylphosphin oder Bis-(Cycloacta-1,5-dienyl)Iridiumchlorid in Kombination mit einem bidentaten Liganden, z.B. 2,2'-Bis(diphenylphosphino)-1,1'-binaphthyl oder 1,4-bis-(diphenylphosphino)butan) und eines geeigneten Kupfer(I)halogenids (z. B. Kupfer(I)iodid) in einem geeigneten Lösungsmittelgemisch aus einem Amin und einem polar aprotischen Lösungsmittel (z. B. Diisopropylamin und Toluol oder Triethylamin und Tetrahydrofuran) hergestellt werden (Schema 2). A¹, A², V, W, m, n, R¹, R², A³, A⁴, A⁵, A⁶, A⁷, R⁶, R⁷, R⁹, R¹⁰, R¹², R¹³ und R¹⁴ haben im folgenden Schema 2 die zuvor definierten Bedeutungen.

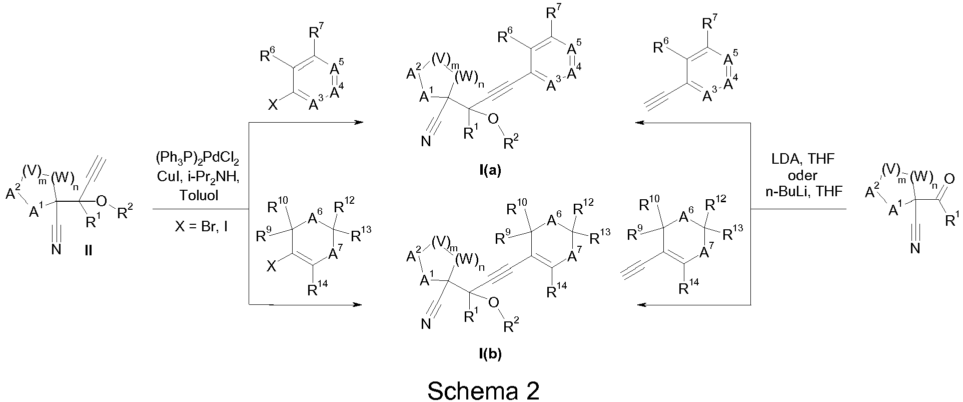

Alternativ können die erfindungsgemäßen substituierten Alkinyl-cyanocycloalkanole I(a)-I(b) auch durch Reaktion eines geeigneten weiter substituierten Ketons mit entsprechenden substituierten Aryl-, Cycloalkenyl-, Heteroaryl- oder Heterocyclylalkinen unter Verwendung einer geeigneten Base (z. B. Lithiumdiisopropylamid oder n-Butyllithium, das verkürzt auch als n-BuLi bezeichnet wird) in einem geeigneten polar-aprotischen Lösungsmittel, z. B. Tetrahydrofuran (THF) hergestellt werden (Schema 2).

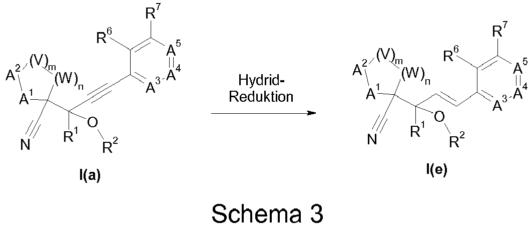

Die erfindungsgemäßen substituierten (E)-konfigurierten Alkenyl-cyanocycloalkanole I(e) können durch Reduktion der Alkingruppe der entsprechenden erfindungsgemäßen Alkinyl-cyanocycloalkanole I(a) unter Verwendung geeigneter Aluminiumhydridreagenzien (z. B. Natrium-bis-(2-methoxyethoxy)-aluminiumhydrid oder Lithiumaluminiumhydrid) in einem geeigneten polar-aprotischen Lösungsmittel (z. B. Tetrahydrofuran) (vgl. Org. Biomol. Chem. 2006, 4, 4186; Bioorg. Med. Chem. 2004, 12, 363-370; Tetrahedron 2003, 59, 9091-9100; Org. Biomol. Chem. 2006, 4, 1400-1412; Synthesis 1977, 561; Tetrahedron Letters 1992, 33, 3477 und Tetrahedron Letters 1974, 1593), unter Einsatz von Borhydridreagenzien (z. B. Natriumborhydrid) in einem geeigneten polar-protischen Lösungsmittel (z. B. Methanol) (vgl. Org. Lett. 2004, 6, 1785), unter Verwendung von Lithium gelöst in einem Gemisch aus Ethylamin und tert.-Butanol (z. B. Helvetica Chimica Acta 1986, 69, 368) oder unter Nutzung eines geeigneten Trialkoxysilans in Gegenwart eines geeigneten Übergangsmetallkatalysators (z. B. Tris-(acetonitril)ruthenium-1,2,3,4,5-pentamethylcyclopentadienylhexafluorophosphat oder Tris-(acetonitril)ruthenium-cyclopentadienylhexafluorophosphat; vgl. J. Am. Chem. Soc. 2002, 124, 7622; J. Am. Chem. Soc. 2005, 127, 17645) hergestellt werden (Schema 3). Eine weitere Variante zur Reduktion der Alkingruppe stellt die Umsetzung des betreffenden Alkins mit Zink in konz. Essigsäure oder mit Zink und einem passenden Ammoniumsalz in einem geeigneten polar-aprotischen Lösungsmittel (z. B. Dichlormethan) dar (vgl. WO2006/027243). In Abhängigkeit von den Reaktionsbedingungen können bei den Hydrierungen der Dreifachbindung als weitere Reaktionsprodukte auch die entsprechenden erfindungsgemäßen (Z)-konfigurierten Analoga erhalten werden. A¹, A², V, W, m, n, R¹, R², A³, A⁴, A⁵, R⁶ und R⁷ haben im oben stehenden Schema 3 die zuvor definierten Bedeutungen. Einen alternativen Zugang zu den erfindungsgemäßen substituierten (E)-konfigurierten Alkenyl-cyanocycloalkanolen I(e) bietet die Metall- oder Halbmetallhydrid-vermittelte Überführung der oben beschriebenen substituierten 1-(2-Hydroxybut-3-in-2-yl)cycloalkylcarbonitrile der allgemeinen Formel II in einem geeigneten polar-aprotischen Lösungsmittel (z. B. Tetrahydrofuran oder Dichlormethan) in entsprechende substituierte 1-[(3E)-2-Hydroxy-4-[M]-but-3-en-2-yl]cycloalkylcarbonitrile der allgemeinen Formel III (vgl. Org. Lett. 2002, 4, 703; Angew. Int. Ed. 2006, 45, 2916), wobei [M] der vorgenannten Definition entspricht (z. B. [M] = Tri-n-butylstannyl oder Bis-cyclopentadienylchlorzirconyl) (vgl. auch Org. Lett. 2010, 12, 1056; Org. Lett 2005, 7, 5191, J. Am. Chem. Soc. 2010, 132, 10961; Tetrahedron 1994, 50, 5189;Angew. Chem. Int. Ed. 2000, 39, 1930). Die so erhaltenen substituierten 1-[(3E)-2-Hydroxy-4-[M]-but-3-en-2-yl]cycloalkylcarbonitrile können durch Kupplung mit einem entsprechenden substituierten Aryl- oder Hetarylhalogenid in einem geeigneten Lösungsmittel (z. B. Tetrahydrofuran oder N,N-Dimethylformamid) unter Verwendung von geeigneten Übergangsmetallkatalysatoren (z. B. Bis-(triphenylphosphin)palladiumdicyanid, Tetrakis-(triphenylphosphin)palladium oder Bis-(triphenylphosphin)palladiumdichlorid) zu den erfindungsgemäßen substituierten, (E)-konfigurierten Alkenyl-cyanocycloalkanolen I(e) umgesetzt werden (Schema 4).

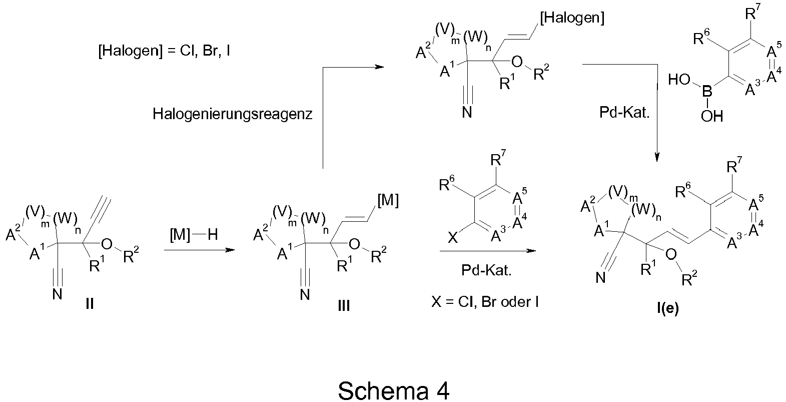

Die betreffenden substituierten 1-[(3E)-2-Hydroxy-4-[M]-but-3-en-2-yl]cycloalkylcarbonitrile können mit einem geeigneten Halogenierungsmittel (z. B. N-Bromsuccinimid, N-Iodsuccinimid oder Iod) in einem geeigneten polar-aprotischen Lösungsmittel (z. B. Dichlormethan) in die entsprechenden erfindungsgemäßen substituierten 1-[(3E)-2-Hydroxy-4-[Halogen]-but-3-en-2-yl]cycloalkylcarbonitrile überführt werden, die dann durch Kupplung mit einer entsprechend substituierten Aryl- oder Hetarylboronsäure in einem geeigneten Lösungsmittelgemisch (z. B. Dioxan, Wasser und ges. Natriumhydrogencarbonatlösung, Tetrahydrofuran und wässrige Kaliumcarbonatlösung oder Toluol und wäßrige Natriumcarbonatlösung) unter Verwendung von geeigneten Übergangsmetallkatalysatoren (z. B. Tetrakis-(triphenylphosphin)palladium, Tris-(cyclohexyl)phosphin, [1,1'-Bis(diphenylphosphino)ferrocen]palladiumdichlorid, Tris-(dibenzylidenaceton)dipalladium(0)) zu den erfindungsgemäßen substituierten, (E)-konfigurierten Alkenyl-cyanocycloalkanolen I(e) umgesetzt werden (Schema 4). A¹, A², V, W, m, n, R¹, R², A³, A⁴, A⁵, R⁶ und R⁷ haben im oben stehenden Schema 4 die zuvor definierten Bedeutungen. Die entsprechenden erfindungsgemäßen Alkenyl-cyanocycloalkanole I(f) können in analogen Umsetzungen ausgehend von substituierten 1-[(3E)-2-Hydroxy-4-[M]-but-3-en-2-yl]cycloalkylcarbonitrilen und 1-[(3E)-2-Hydroxy-4-[Halogen]-but-3-en-2-yl]cycloalkylcarbonitrilen hergestellt werden (Schema 5).

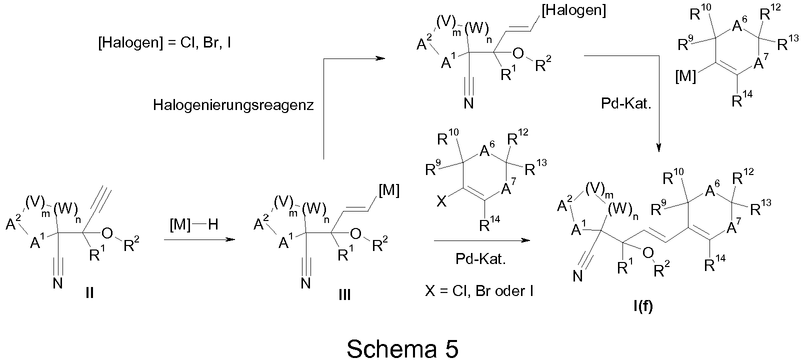

A¹, A², V, W, m, n, R¹, R², A⁶, A⁷R⁹, R¹⁰, R¹², R¹³, R¹⁴ haben im oben stehenden Schema 5 die zuvor definierten Bedeutungen.
Die Reduktion von erfindungsgemäßen substituierten Alkinyl-cyanocycloalkanolen I(a) zu den erfindungsgemäßen substituierten (Z)-konfigurierten Alkenyl-cyanocycloalkanolen I(i)) läßt sich in Gegenwart eines Übergangsmetallkatalysators wie zum Beispiel Lindlars Katalysator mit Wasserstoff in einem geeigneten polar-aprotischen Lösungsmittel (wie z. B. n-Butanol) durchführen (vgl. Tetrahedron 1987, 43, 4107; Tetrahedron 1983, 39, 2315; J. Org. Synth. 1983, 48, 4436 und J. Am. Chem. Soc. 1984, 106, 2735) (Schema 6). A¹, A², V, W, m, n, R¹, R², A³, A⁴, A⁵, R⁶ und R⁷ haben im nachfolgenden Schema 6 die zuvor definierten Bedeutungen.

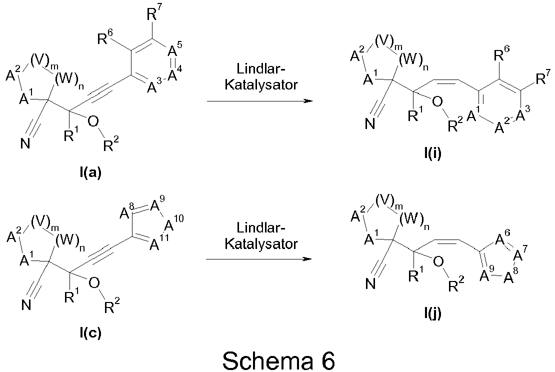

Die Herstellung von erfindungsgemäßen Alkinyl- und Alkenylcyanocycloalkanolen I(f), die durch gegebenenfalls weiter substituierte Cycloalkenylgruppen substituiert sind, ist durch Umsetzung von substituierten 1-(2-Hydroxybut-3-in-2-yl)cycloalkylcarbonitrilen II oder substituierten 1-[(3E)-2-Hydroxy-4-[M]-but-3-en-2-yl]cycloalkylcarbonitrilen III mit einem entsprechenden substituierten Vinyltrifluormethansulfonat in einem geeigneten Lösungsmittel (z. B. Tetrahydrofuran oder N,N-Dimethylformamid) unter Verwendung von geeigneten Übergangsmetallkatalysatoren (z. B. Bis-(triphenylphosphin)palladiumdicyanid, Tetrakis(triphenylphosphin)palladium oder Bis-(triphenylphosphin)palladiumdichlorid) möglich (Schema 7). A¹, A², V, W, m, n, R¹, R², A⁶, A⁷, R⁹, R¹⁰, R¹², R¹³ und R¹⁴ haben im nachfolgenden Schema 7 die zuvor definierten Bedeutungen.

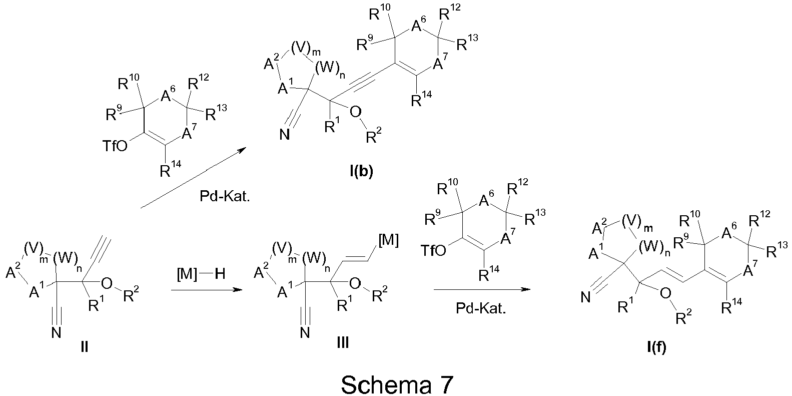

Ausgewählte detaillierte Synthesebeispiele für die erfindungsgemäßen Verbindungen der allgemeinen Formel (I) sind im Folgenden aufgeführt. Die angegebenen Beispielnummern entsprechen den in den nachstehenden Tabellen 1 bis 5 genannten Numerierungen. Die ¹H-NMR-, ¹³C-NMR- und ¹⁹F-NMR-spektroskopischen Daten, die für die in den nachfolgenden Abschnitten beschriebenen chemischen Beispiele angegeben sind, (400 MHz bei ¹H-NMR und 150 MHz bei ¹³C-NMR und 375 MHz bei ¹⁹F-NMR, Lösungsmittel CDCl₃, CD₃OD oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Die verwendeten Abkürzungen für chemische Gruppen haben die nachfolgenden Bedeutungen: Me = CH₃, Et = CH₂CH₃, t-Hex = C(CH₃)₂CH(CH₃)₂, t-Bu = C(CH₃)₃, n-Bu = unverzweigtes Butyl, n-Pr = unverzweigtes Propyl, c-Hex = Cyclohexyl. Bei Diastereomerengemischen werden entweder die jeweils signifikanten Signale beider Diastereomere oder das charakteristische Signal des Hauptdiastereomers angegeben.

### Synthesebeispiele:

### No. 1.1-130: Methyl-2-[3-(1-cyancyclopropyl)-3-hydroxy-4-methylpent-1-in-1-yl]benzoat

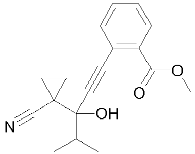

1-Isobutyrylcyclopropancarbonitril (4.00 g, 29.16 mmol) wurde in einem Rundkolben unter Argon in abs. Tetrahydrofuran (120 ml) gelöst und tropfenweise zu einer auf 0 °C eingekühlten Lösung eines Lithiumacetylid-Ethylendiaminkomplexes (4.11 g, 37.91 mmol, 85% Gehalt) in abs. Tetrahydrofuran (80 ml) gegeben. Die Reaktionslösung wurde nach erfolgter Zugabe 4 h lang bei Raumtemperatur gerührt, anschließend mit Wasser versetzt und unter vermindertem Druck eingeengt. Der verbleibende Rückstand wurde mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(3-Hydroxy-4-methylpent-1-yn-3-yl)cyclopropancarbonitril (2.59 g, 54 % der Theorie) als farbloser Feststoff isoliert. ¹H-NMR (400 MHz, CDCl₃ δ, ppm) 2.48 (s, 1H), 2.41 (sept, 1H), 2.19 (br. s, 1H, OH), 1.34-1.28 (m, 3H), 1.21 (m, 1H), 1.12 (d, 3H), 1.09 (d, 3H). Kupfer(I)iodid (117 mg, 0.61 mmol) und Bis(triphenylphosphin)palladium(II)chlorid (323 mg, 0.46 mmol) wurden unter Argon in einem ausgeheizten Rundkolben vorgelegt und mit abs. Toluol (6 ml) sowie Methyl-2-lodbenzoat (803 mg, 3.06 mmol) versetzt. Nach 10 Minuten Rühren bei Raumtemperatur erfolgte die tropfenweise Zugabe einer Lösung von 1-(3-Hydroxy-4-methylpent-1-yn-3-yl)cyclopropancarbonitril (500 mg, 3.06 mmol) in abs. Toluol (9 ml) und von Diisopropylamin (0.86 ml, 6.13 mmol). Das resultierende Reaktionsgemisch wurde 3 h lang bei Raumtemperatur gerührt und danach mit Wasser versetzt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromatographische Reinigung des erhaltenen Rohproduktes (unter Verwendung eines Essigester/Heptan-Gradienten) wurde Methyl-2-[3-(1-cyancyclopropyl)-3-hydroxy-4-methylpent-1-in-1-yl]benzoat (370 mg, 39 % der Theorie) in Form eines farblosen Öles isoliert. ¹H-NMR (400 MHz, CDCl₃ δ, ppm) 7.97 (d, 1H), 7.51 (m, 1H), 7.48 (m, 1H), 7.40 (dd, 1H), 3.91 (s, 3H), 2.51 (sept, 1H), 1.50 (m, 2H), 1.36 (m, 1H), 1.24 (m, 1H), 1.20 (d, 3H), 1.17 (d, 3H).

### No. I.1-127: 2-[3-(1-Cyancyclopropyl)-3-hydroxy-4-methylpent-1-in-1-yl]benzoesäure

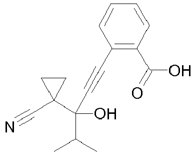

Methyl-2-[3-(1-cyancyclopropyl)-3-hydroxy-4-methylpent-1-in-1-yl]benzoat (100 mg, 0.34 mmol) wurde in Tetrahydrofuran (15 ml) gelöst und mit Wasser (3 ml) sowie fein gepulvertem Natriumhydroxid (13 mg, 0.34 mmol) versetzt. Das resultierende Reaktionsgemisch wurde 4 h lang bei Raumtemperatur gerührt und danach mit Wasser versetzt sowie mit verd. Salzsäure auf pH1 eingestellt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromatographische Reinigung des erhaltenen Rohproduktes (unter Verwendung eines Essigester/Heptan-Gradienten) wurde 2-[3-(1-Cyancyclopropyl)-3-hydroxy-4-methylpent-1-in-1-yl]benzoesäure (90 mg, 88 % der Theorie) in Form eines farblosen Feststoffes isoliert. ¹H-NMR (400 MHz, CDCl₃ δ, ppm) 8.09 (d, 1H), 7.60 (d, 1H), 7.56 (m, 2H), 7.46 (m, 1H), 2.52 (sept, 1H), 1.49 (m, 2H), 1.38 (m, 1H), 1.23 (m, 1H), 1.21 (d, 3H), 1.18 (s, 3H).

### No. I.1-532: 2-[3-(1-Cyanocyclopropyl)-4-ethyl-3-hydroxyhex-1-yn-1-yl]benzonitril

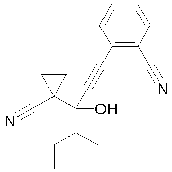

Kupfer(I)iodid (5 mg, 0.03 mmol) und Bis(triphenylphosphin)palladium(II)chlorid (13 mg, 0.02 mmol) wurden unter Argon in einem ausgeheizten Rundkolben vorgelegt und mit abs. Toluol (3 ml) sowie 2-lodbenzonitril (144 mg, 0.63 mmol) versetzt. Nach 10 Minuten Rühren bei Raumtemperatur erfolgte die tropfenweise Zugabe einer Lösung von 1-(4-Ethyl 3-hydroxyhex-1-yn-3-yl)cyclopropancarbonitril (120 mg, 0.63 mmol) in abs. Toluol (2 ml) und von Diisopropylamin (0.26 ml, 1.89 mmol). Das resultierende Reaktionsgemisch wurde 3 h lang bei Raumtemperatur gerührt und danach mit Wasser versetzt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromatographische Reinigung des erhaltenen Rohproduktes (unter Verwendung eines Essigester/Heptan-Gradienten) wurde 2-[3-(1-Cyanocyclopropyl)-4-ethyl-3-hydroxyhex-1-yn-1-yl]benzonitril (133 mg, 73 % der Theorie) in Form eines zähflüssigen Öles isoliert. ¹H-NMR (400 MHz, CDCl₃ δ, ppm) 7.72 (m, 1H), 7.60 (m, 1H), 7.52-7.43 (m, 2H), 2.28 (br. s, 1H, OH), 2.06 (m, 1H), 1.91 (m, 1H), 1.79 (m, 1H), 1.58 (m, 2H), 1.49 (m, 1H), 1.41 (m, 2H), 1.27 (m, 1H), 1.09 (m, 6H).

### No. I.1-538: Methyl-2-[3-(1-cyanocyclopropyl)-4-ethyl-3-hydroxyhex-1-in-1-yl]benzoat

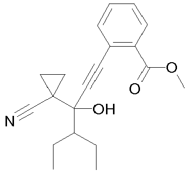

Kupfer(I)iodid (5 mg, 0.03 mmol) und Bis(triphenylphosphin)palladium(II)chlorid (13 mg, 0.02 mmol) wurden unter Argon in einem ausgeheizten Rundkolben vorgelegt und mit abs. Toluol (3 ml) sowie Methyl-2-lodbenzoat (164 mg, 0.63 mmol) versetzt. Nach 10 Minuten Rühren bei Raumtemperatur erfolgte die tropfenweise Zugabe einer Lösung von 1-(4-Ethyl 3-hydroxyhex-1-yn-3-yl)cyclopropancarbonitril (120 mg, 0.63 mmol) in abs. Toluol (2 ml) und von Diisopropylamin (0.26 ml, 1.89 mmol). Das resultierende Reaktionsgemisch wurde 3 h lang bei Raumtemperatur gerührt und danach mit Wasser versetzt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromatographische Reinigung des erhaltenen Rohproduktes (unter Verwendung eines Essigester/Heptan-Gradienten) wurde Methyl-2-[3-(1-cyanocyclopropyl)-4-ethyl-3-hydroxyhex-1-in-1-yl]benzoat (136 mg, 66 % der Theorie) in Form eines zähflüssigen Öles isoliert. ¹H-NMR (400 MHz, CDCl₃ δ, ppm) 7.96 (d, 1H), 7.48 (m, 2H), 7.39 (m, 1H), 3.91 (s, 3H), 2.55 (br. s, 1H, OH), 2.03 (m, 1H), 1.93 (m, 1H), 1.84 (m, 1H), 1.58 (m, 2H), 1.53 (m, 2H), 1.39 (m, 1H), 1.25 (m, 1H), 1.09 (m, 6H).

### No. I.1-539: Methyl-3-[3-(1-cyanocyclopropyl)-4-ethyl-3-hydroxyhex-1-in-1-yl]benzoat

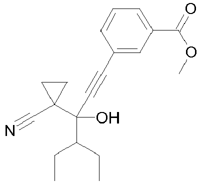

Kupfer(I)iodid (5 mg, 0.03 mmol) und Bis(triphenylphosphin)palladium(II)chlorid (13 mg, 0.02 mmol) wurden unter Argon in einem ausgeheizten Rundkolben vorgelegt und mit abs. Toluol (3 ml) sowie Methyl-3-lodbenzoat (164 mg, 0.63 mmol) versetzt. Nach 10 Minuten Rühren bei Raumtemperatur erfolgte die tropfenweise Zugabe einer Lösung von 1-(4-Ethyl 3-hydroxyhex-1-yn-3-yl)cyclopropancarbonitril (120 mg, 0.63 mmol) in abs. Toluol (2 ml) und von Diisopropylamin (0.26 ml, 1.89 mmol). Das resultierende Reaktionsgemisch wurde 3 h lang bei Raumtemperatur gerührt und danach mit Wasser versetzt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromatographische Reinigung des erhaltenen Rohproduktes (unter Verwendung eines Essigester/Heptan-Gradienten) wurde Methyl-3-[3-(1-cyanocyclopropyl)-4-ethyl-3-hydroxyhex-1-in-1-yl]benzoat (166 mg, 81 % der Theorie) in Form eines zähflüssigen Öles isoliert. ¹H-NMR (400 MHz, CDCl₃ δ, ppm) 8.03-8.00 (m, 2H), 7.56 (m, 1H), 7.42 (m, 1H), 3.93 (s, 3H), 2.26 (br. s, 1H, OH), 2.04 (m, 1H), 1.93-1.88 (m, 1H), 1.84-1.79 (m, 1H), 1.59-1.55 (m, 1H), 1.52-1.47 (m, 1H), 1.43-1.39 (m, 2H), 1.30-1.27 (m, 2H), 1.09 (m, 6H).

### No. I.1-578: Methyl-2-[3-(1-cyanocyclopropyl)-3-cyclopentyl-3-hydroxyprop-1-in-1-yl]benzoat

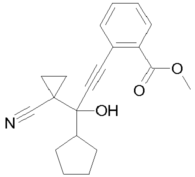

Kupfer(I)iodid (4 mg, 0.02 mmol) und Bis(triphenylphosphin)palladium(II)chlorid (11 mg, 0.02 mmol) wurden unter Argon in einem ausgeheizten Rundkolben vorgelegt und mit abs. Toluol (4 ml) sowie Methyl-3-lodbenzoat (138 mg, 0.53 mmol) versetzt. Nach 10 Minuten Rühren bei Raumtemperatur erfolgte die tropfenweise Zugabe einer Lösung von 1-(1-Cyclopentyl-1-hydroxyprop-2-in-1-yl)cyclopropancarbonitril (100 mg, 0.53 mmol) in abs. Toluol (2 ml) und von Diisopropylamin (0.22 ml, 1.59 mmol). Das resultierende Reaktionsgemisch wurde 4 h lang bei Raumtemperatur gerührt und danach mit Wasser versetzt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromatographische Reinigung des erhaltenen Rohproduktes (unter Verwendung eines Essigester/Heptan-Gradienten) wurde Methyl-2-[3-(1-cyancyclopropyl)-3-cyclopentyl-3-hydroxyprop-1-in-1-yl]benzoat (131 mg, 77 % der Theorie) in Form eines zähflüssigen Öles isoliert. ¹H-NMR (400 MHz, CDCl₃ δ, ppm) 7.96 (m, 1H), 7.51-7.44 (m, 2H), 7.40 (m, 1H), 3.91 (s, 3H), 2.78-2.72 (m, 1H), 2.53 (br. s, 1H, OH), 2.01-1.94 (m, 2H), 1.81-1.63 (m, 6H), 1.55-1.47 (m, 2H), 1.33-1.28 (m, 1H), 1.25-1.22 (m, 1H).

### No. I.3-26: Methyl-2-[3-(1-cyancyclobutyl)-3-hydroxy-4-methylpent-1-in-1-yl]benzoat

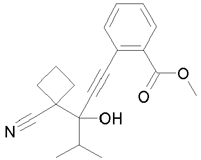

Kupfer(I)iodid (5 mg, 0.03 mmol) und Bis(triphenylphosphin)palladium(II)chlorid (14 mg, 0.02 mmol) wurden unter Argon in einem ausgeheizten Rundkolben vorgelegt und mit abs. Toluol (3 ml) sowie Methyl-2-iodbenzoat (177 mg, 0.68 mmol) versetzt. Nach 10 Minuten Rühren bei Raumtemperatur erfolgte die tropfenweise Zugabe einer Lösung von 1-(3-hydroxy-4-methylpent-1-yn-3-yl)cyclobutancarbonitril (120 mg, 0.68 mmol) in abs. Toluol (3 ml) und von Diisopropylamin (0.29 ml, 2.03 mmol). Das resultierende Reaktionsgemisch wurde 4 h lang bei Raumtemperatur gerührt und danach mit Wasser versetzt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromatographische Reinigung des erhaltenen Rohproduktes (unter Verwendung eines Essigester/Heptan-Gradienten) wurde Methyl-2-[3-(1-cyancyclobutyl)-3-hydroxy-4-methylpent-1-in-1-yl]benzoat (96 mg, 46 % der Theorie) in Form eines farblosen Öles isoliert. ¹H-NMR (400 MHz, CDCl₃ δ, ppm) 7.99 (d, 1H), 7.58 (d, 1H), 7.50 (m, 1H), 7.44 (m, 1H), 3.92 (s, 3H), 2.88-2.77 (m, 2H), 2.55 (br. s, 1H, OH), 2.44-2.27 (m, 4H), 1.96 (m, 1H), 1.16 (d, 3H), 1.08 (d, 3H).

### No. I.3-47: Methyl-2-[3-(1-cyanocyclobutyl)-3-hydroxy-3-phenylprop-1-in-1-yl]benzoat

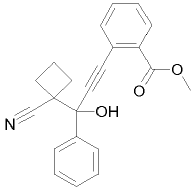

Kupfer(I)iodid (4 mg, 0.02 mmol) und Bis(triphenylphosphin)palladium(II)chlorid (10 mg, 0.01 mmol) wurden unter Argon in einem ausgeheizten Rundkolben vorgelegt und mit abs. Toluol (3 ml) sowie Methyl-2-iodbenzoat (124 mg, 0.47 mmol) versetzt. Nach 10 Minuten Rühren bei Raumtemperatur erfolgte die tropfenweise Zugabe einer Lösung von 1-(1-Hydroxy-1-phenylprop-2-in-1-yl)cyclobutancarbonitril (100 mg, 0.47 mmol) in abs. Toluol (2 ml) und von Diisopropylamin (0.29 ml, 2.03 mmol). Das resultierende Reaktionsgemisch wurde 4 h lang bei Raumtemperatur gerührt und danach mit Wasser versetzt. Die wässrige Phase wurde mehrfach mit Dichlormethan extrahiert. Die vereinigten organischen Phasen wurden über Magnesiumsulfat getrocknet, filtriert und unter vermindertem Druck eingeengt. Durch abschließende säulenchromatographische Reinigung des erhaltenen Rohproduktes (unter Verwendung eines Essigester/Heptan-Gradienten) wurde Methyl-2-[3-(1-cyanocyclobutyl)-3-hydroxy-3-phenylprop-1-in-1-yl]benzoat (105 mg, 64 % der Theorie) in Form eines farblosen Öles isoliert. ¹H-NMR (400 MHz, CDCl₃ δ, ppm) 8.00 (m, 1H), 7.82 (m, 2H), 7.63 (m, 1H), 7.52 (m, 1H), 7.45-7.37 (m, 4H), 3.91 (s, 3H), 3.10 (br. s, 1H, OH), 2.93-2.80 (m, 2H), 2.35-2.29 (m, 2H), 2.25-2.17 (m, 1H), 1.97-1.90 (m, 1H).

### No. II.1-2: 1-(3-hydroxy-4-methylpent-1-yn-3-yl)cyclopropancarbonitril

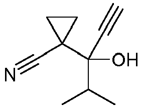

1-Isobutyrylcyclopropancarbonitril (4.00 g, 29.16 mmol) wurde in einem Rundkolben unter Argon in abs. Tetrahydrofuran (120 ml) gelöst und tropfenweise zu einer auf 0 °C eingekühlten Lösung eines Lithiumacetylid-Ethylendiaminkomplexes (4.11 g, 37.91 mmol, 85% Gehalt) in abs. Tetrahydrofuran (80 ml) gegeben. Die Reaktionslösung wurde nach erfolgter Zugabe 4 h lang bei Raumtemperatur gerührt, anschließend mit Wasser versetzt und unter vermindertem Druck eingeengt. Der verbleibende Rückstand wurde mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(3-hydroxy-4-methylpent-1-yn-3-yl)cyclopropancarbonitril (2.59 g, 54 % der Theorie) als farbloser Feststoff isoliert. ¹H-NMR (400 MHz, CDCl₃ δ, ppm) 2.48 (s, 1H), 2.41 (sept, 1H), 2.19 (br. s, 1H, OH), 1.34-1.28 (m, 3H), 1.21 (m, 1H), 1.12 (d, 3H), 1.09 (d, 3H).

### No. II.1-3: 1-(1-Hydroxy-1-phenylprop-2-in-1-yl)cyclopropancarbonitril

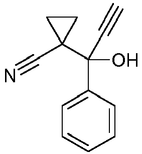

1-Benzoylcyclopropancarbonitril (4.00 g, 23.37 mmol) wurde in einem Rundkolben unter Argon in abs. Tetrahydrofuran (160 ml) gelöst und tropfenweise zu einer auf 0 °C eingekühlten Lösung eines Lithiumacetylid-Ethylendiaminkomplexes (3.29 g, 37.37 mmol, 85% Gehalt) in abs. Tetrahydrofuran (80 ml) gegeben. Die Reaktionslösung wurde nach erfolgter Zugabe 4 h lang bei Raumtemperatur gerührt, anschließend mit Wasser versetzt und unter vermindertem Druck eingeengt. Der verbleibende Rückstand wurde mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(1-Hydroxy-1-phenylprop-2-in-1-yl)cyclopropancarbonitril (3.16 g, 69 % der Theorie) als farbloser Feststoff isoliert. ¹H-NMR (400 MHz, CDCl₃ δ, ppm) 7.70 (m, 2H), 7.41 (m, 2H), 7.29 (m, 1H), 2.78 (s, 1H), 2.62 (br. s, 1H, OH), 1.51 (m, 1H), 1.39 (m, 1H), 1.28 (m, 2H); ¹³C-NMR (150 MHz, CDCl₃ δ, ppm) 140.1, 129.0, 128.5, 127.2, 125.8, 122.3, 120.9, 86.1, 76.8, 72.0, 22.8, 13.2, 12.7.

### No. II.1-7: 1-[1-(4-Fluorphenyl)-1-hydroxyprop-2-in-1-yl]cyclopropancarbonitril

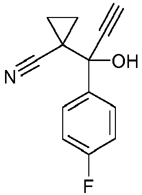

1-(4-Fluorbenzoyl)cyclopropancarbonitril (7.00 g, 37.00 mmol) wurde in einem Rundkolben unter Argon in abs. Tetrahydrofuran (40 ml) gelöst und tropfenweise zu einer auf 0 °C eingekühlten Lösung eines Lithiumacetylid-Ethylendiaminkomplexes (4.92 g, 48.10 mmol, 90% Gehalt) in abs. Tetrahydrofuran (20 ml) gegeben. Die Reaktionslösung wurde nach erfolgter Zugabe 4 h lang bei Raumtemperatur gerührt, anschließend mit Wasser versetzt und unter vermindertem Druck eingeengt. Der verbleibende Rückstand wurde mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-[1-(4-Fluorphenyl)-1-hydroxyprop-2-in-1-yl]cyclopropancarbonitril (0.84 g, 11 % der Theorie) als farbloser wachsartiger Feststoff isoliert. ¹H-NMR (400 MHz, CDCl₃ δ, ppm) 7.69 (m, 2H), 7.11 (m, 2H), 2.79 (s, 1H), 2.62 (br. s, 1H, OH), 1.51 (m, 1H), 1.39 (m, 1H), 1.28 (m, 2H).

### No. II.1-81: 1-(1-Cyclopentyl-1-hydroxyprop-2-in-1-yl)cyclopropancarbonitril

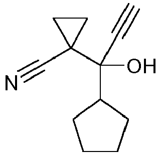

1-(Cyclopentylcarbonyl)cyclopropancarbonitril (9,00 g, 55,14 mmol) wurde in einem Rundkolben unter Argon in abs. Tetrahydrofuran (50 ml) gelöst und tropfenweise zu einer auf 0 °C eingekühlten Lösung eines Lithiumacetylid-Ethylendiaminkomplexes (6,59 g, 71,68 mmol) in abs. Tetrahydrofuran (40 ml) gegeben. Die Reaktionslösung wurde nach erfolgter Zugabe 2 h lang bei Raumtemperatur gerührt, anschließend mit Wasser versetzt und unter vermindertem Druck eingeengt. Der verbleibende Rückstand wurde mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(1-Cyclopentyl-1-hydroxyprop-2-in-1-yl)cyclopropancarbonitril (2.55 g, 25 % der Theorie) als farbloser Feststoff isoliert. ¹H-NMR (400 MHz, CDCl₃ δ, ppm) 2.65 (m, 1H), 2.47 (s, 1H), 2.18 (br. s, 1H, OH), 1.97-1.85 (m, 2H), 1.73-1.59 (m, 4H), 1.45-1.39 (m, 1H), 1.37-1.33 (m, 2H), 1.30-1.26 (m, 2H), 1.22-1.19 (m, 1H).

### No. II.1-84: 1-(4-Ethyl 3-hydroxyhex-1-yn-3-yl)cyclopropancarbonitril

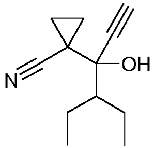

1-(2-ethylbutanoyl)cyclopropancarbonitril (14.00 g, 84.73 mmol) wurde in einem Rundkolben unter Argon in abs. Tetrahydrofuran (50 ml) gelöst und tropfenweise zu einer auf 0 °C eingekühlten Lösung eines Lithiumacetylid-Ethylendiaminkomplexes (10.14 g, 110.15 mmol, 85% Gehalt) in abs. Tetrahydrofuran (40 ml) gegeben. Die Reaktionslösung wurde nach erfolgter Zugabe 3 h lang bei Raumtemperatur gerührt, anschließend mit Wasser versetzt und unter vermindertem Druck eingeengt. Der verbleibende Rückstand wurde mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(4-Ethyl 3-hydroxyhex-1-yn-3-yl)cyclopropancarbonitril (6.15 g, 38 % der Theorie) als farbloser Feststoff isoliert. ¹H-NMR (400 MHz, CDCl₃ δ, ppm) 2.31 (s, 1H), 2.02 (br. s, 1H, OH), 1.77 (m, 1H), 1.67 (m, 1H), 1.58 (m, 1H), 1.33 (m, 1H), 1.25 (m, 1H), 1.19 (m, 2H), 1.08 (m, 2H), 0.89 (m, 6H).

### No. II.2-2: 1-(3-Hydroxy-4-methylpent-1-yn-3-yl)cyclobutancarbonitril

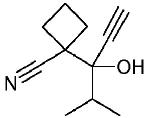

1-Isobutyrylcyclobutancarbonitril (9.00 g, 60.0 mmol) wurde in einem Rundkolben unter Argon in abs. Tetrahydrofuran (50 ml) gelöst und tropfenweise zu einer auf 0 °C eingekühlten Lösung eines Lithiumacetylid-Ethylendiaminkomplexes (7.92 g, 77.0 mmol, 90% Gehalt) in abs. Tetrahydrofuran (20 ml) gegeben. Die Reaktionslösung wurde nach erfolgter Zugabe 2 h lang bei Raumtemperatur gerührt, anschließend mit Wasser versetzt und unter vermindertem Druck eingeengt. Der verbleibende Rückstand wurde mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(3-Hydroxy-4-methylpent-1-yn-3-yl)cyclobutancarbonitril (2.50 g, 24 % der Theorie) als farbloser wachsartiger Feststoff isoliert. ¹H-NMR (400 MHz, CDCl₃ δ, ppm) 2.70 (m, 2H), 2.64 (s, 1H), 2.36 (m, 2H), 2.24 (m, 2H), 2.12 (br. s, 1H, OH), 1.08 (d, 3H), 0.98 (d, 3H).

### No. II.2-3: 1-(1-Hydroxy-1-phenylprop-2-in-1-yl)cyclobutancarbonitril

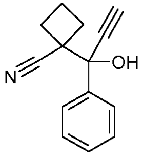

1-Benzoylcyclobutancarbonitril (10.00 g, 53.99 mmol) wurde in einem Rundkolben unter Argon in abs. Tetrahydrofuran (50 ml) gelöst und tropfenweise zu einer auf 0 °C eingekühlten Lösung eines Lithiumacetylid-Ethylendiaminkomplexes (7.18 g, 70.19 mmol, 90% Gehalt) in abs. Tetrahydrofuran (20 ml) gegeben. Die Reaktionslösung wurde nach erfolgter Zugabe 3 h lang bei Raumtemperatur gerührt, anschließend mit Wasser versetzt und unter vermindertem Druck eingeengt. Der verbleibende Rückstand wurde mit Wasser und Dichlormethan versetzt und die wässrige Phase mehrfach mit Dichlormethan extrahiert. Die vereinigten organischen Phasen wurden über Magnesiumsulfat getrocknet, filtriert und unter vermindertem Druck eingeengt. Durch säulenchromatographische Reinigung des erhaltenen Rohproduktes (Gradient Essigester/Heptan) wurde 1-(1-Hydroxy-1-phenylprop-2-in-1-yl)cyclobutancarbonitril (740 mg, 6 % der Theorie) als farbloser wachsartiger Feststoff isoliert. ¹H-NMR (400 MHz, CDCl₃ δ, ppm) 7.69 (m, 2H), 7.42-7.37 (m, 3H), 2.83 (br. s, 1H, OH), 2.80-2.65 (m, 2H), 2.62 (s, 1H), 2.36-2.15 (m, 4H), 1.91-1.84 (m, 2H).

### No. III.1-2: 1-[(1E)-3-Hydroxy-4-methyl-1-(tributylstannyl)pent-1-en-3-yl]cyclopropancarbonitril

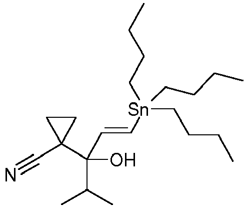

Tetrakis(triphenylphosphin)palladium(0) (198 mg, 0.17 mmol) wurde unter Argon in einem ausgeheizten Rundkolben vorgelegt und mit abs. Tetrahydrofuran (20 ml) sowie 1-(3-hydroxy-4-methylpent-1-yn-3-yl)cyclopropancarbonitril (560 mg, 3.41 mmol) versetzt. Nach 5 Minuten Rühren bei Raumtemperatur erfolgte die Zugabe von Tributylzinnhydrid (1.10 ml, 4.12 mmol). Das resultierende Reaktionsgemisch wurde 1 h lang bei Raumtemperatur gerührt und danach mit Wasser versetzt. Die wässrige Phase wurde mehrfach gründlich mit Dichlormethan extrahiert und die vereinigten organischen Phasen anschließend über Magnesiumsulfat getrocknet, filtriert und unter vermindertem Druck eingeengt. Durch abschließende säulenchromatographische Reinigung des resultierenden Rohproduktes (Essigester/Heptan-Gradient) konnte 1-[(1E)-3-Hydroxy-4-methyl-1-(tributylstannyl)pent-1-en-3-yl]cyclopropancarbonitril (0.38 g, 24 % der Theorie) in Form eines farblosen Öls erhalten werden. ¹H-NMR (400 MHz, CDCl₃ δ, ppm) 6.18 (d, 1H), 6.03 (d, 1H), 2.32 (sept, 1H), 1.53-1.45 (m, 6H), 1.35-1.29 (m, 6H), 1.28 (m, 1H), 1.14 (m, 1H), 1.03 (d, 3H), 0.94-0.88 (m, 18H), 0.72 (m, 2H).

In Analogie zu oben angeführten und in den nachstehenden Tabellen rezitierten Herstellungsbeispielen und unter Berücksichtigung der allgemeinen Angaben zur Herstellung von substituierten Vinyl- und Alkinyl-cyanocycloalkanolen sowie Vinyl- und Alkinyl-cyanoheterocyclylalkanolen sowie deren Analoga der allgemeinen Formel (I) erhält man folgende in den Tabellen 1 bis 8 spezifisch genannten Verbindungen, und wobei Q einem der vorgenannten bevorzugten spezifischen Reste entspricht.

**Tabelle 1:**

| | | | | | |
|---|---|---|---|---|---|
| 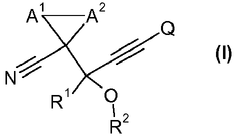 | | | Q entspricht jeweils einer der weiter vorne als besonders bevorzugt definierten Struktur | | |

| No. | R¹ | R² | A¹ | A² | Q |
|---|---|---|---|---|---|
| I.1-1 | CH₃ | H | CH₂ | CH₂ | Q-1.2 |
| I.1-2 | CH₃ | H | CH₂ | CH₂ | Q-1.3 |
| I.1-3 | CH₃ | H | CH₂ | CH₂ | Q-1.7 |
| I.1-4 | CH₃ | H | CH₂ | CH₂ | Q-1.13 |
| I.1-5 | CH₃ | H | CH₂ | CH₂ | Q-1.19 |
| I.1-6 | CH₃ | H | CH₂ | CH₂ | Q-1.25 |
| I.1-7 | CH₃ | H | CH₂ | CH₂ | Q-1.26 |
| I.1-8 | CH₃ | H | CH₂ | CH₂ | Q-1.31 |
| I.1-9 | CH₃ | H | CH₂ | CH₂ | Q-1.32 |
| I.1-10 | CH₃ | H | CH₂ | CH₂ | Q-1.37 |
| I.1-11 | CH₃ | H | CH₂ | CH₂ | Q-1.43 |
| I.1-12 | CH₃ | H | CH₂ | CH₂ | Q-1.44 |
| I.1-13 | CH₃ | H | CH₂ | CH₂ | Q-1.46 |
| I.1-14 | CH₃ | H | CH₂ | CH₂ | Q-1.49 |
| I.1-15 | CH₃ | H | CH₂ | CH₂ | Q-1.52 |
| I.1-16 | CH₃ | H | CH₂ | CH₂ | Q-1.55 |
| I.1-17 | CH₃ | H | CH₂ | CH₂ | Q-1.61 |
| I.1-18 | CH₃ | H | CH₂ | CH₂ | Q-1.62 |
| I.1-19 | CH₃ | H | CH₂ | CH₂ | Q-1.64 |
| I.1-20 | CH₃ | H | CH₂ | CH₂ | Q-1.65 |
| I.1-21 | CH₃ | H | CH₂ | CH₂ | Q-1.79 |
| I.1-22 | CH₃ | H | CH₂ | CH₂ | Q-1.82 |
| I.1-23 | CH₃ | H | CH₂ | CH₂ | Q-1.85 |
| I.1-24 | CH₃ | H | CH₂ | CH₂ | Q-1.91 |
| I.1-25 | CH₃ | H | CH₂ | CH₂ | Q-1.92 |
| I.1-26 | CH₃ | H | CH₂ | CH₂ | Q-1.93 |
| I.1-27 | CH₃ | H | CH₂ | CH₂ | Q-1.97 |
| I.1-28 | CH₃ | H | CH₂ | CH₂ | Q-1.98 |
| I.1-29 | CH₃ | H | CH₂ | CH₂ | Q-1.99 |
| I.1-30 | CH₃ | H | CH₂ | CH₂ | Q-1.100 |
| I.1-31 | CH₃ | H | CH₂ | CH₂ | Q-1.101 |
| I.1-32 | CH₃ | H | CH₂ | CH₂ | Q-1.102 |
| I.1-33 | CH₃ | H | CH₂ | CH₂ | Q-1.103 |
| I.1-34 | CH₃ | H | CH₂ | CH₂ | Q-1.104 |
| I.1-35 | CH₃ | H | CH₂ | CH₂ | Q-1.105 |
| I.1-36 | CH₃ | H | CH₂ | CH₂ | Q-1.106 |
| I.1-37 | CH₃ | H | CH₂ | CH₂ | Q-1.107 |
| I.1-38 | CH₃ | H | CH₂ | CH₂ | Q-1.108 |
| I.1-39 | CH₃ | H | CH₂ | CH₂ | Q-1.109 |
| I.1-41 | CH₃ | H | CH₂ | CH₂ | Q-1.110 |
| I.1-42 | CH₃ | H | CH₂ | CH₂ | Q-1.111 |
| I.1-43 | CH₃ | H | CH₂ | CH₂ | Q-1.112 |
| I.1-44 | CH₃ | H | CH₂ | CH₂ | Q-1.113 |
| I.1-45 | CH₃ | H | CH₂ | CH₂ | Q-1.114 |
| I.1-46 | CH₃ | H | CH₂ | CH₂ | Q-1.115 |
| I.1-47 | CH₃ | H | CH₂ | CH₂ | Q-1.116 |
| I.1-48 | CH₃ | H | CH₂ | CH₂ | Q-1.117 |
| I.1-49 | CH₃ | H | CH₂ | CH₂ | Q-1.118 |
| I.1-50 | CH₃ | H | CH₂ | CH₂ | Q-1.119 |
| I.1-51 | CH₃ | H | CH₂ | CH₂ | Q-1.120 |
| I.1-52 | CH₃ | H | CH₂ | CH₂ | Q-1.121 |
| I.1-53 | CH₃ | H | CH₂ | CH₂ | Q-1.122 |
| I.1-54 | CH₃ | H | CH₂ | CH₂ | Q-1.123 |
| I.1-55 | CH₃ | H | CH₂ | CH₂ | Q-1.133 |
| I.1-56 | CH₃ | H | CH₂ | CH₂ | Q-1.134 |
| I.1-57 | CH₃ | H | CH₂ | CH₂ | Q-1.139 |
| I.1-58 | CH₃ | H | CH₂ | CH₂ | Q-1.142 |
| I.1-59 | CH₃ | H | CH₂ | CH₂ | Q-1.145 |
| I.1-60 | CH₃ | H | CH₂ | CH₂ | Q-1.146 |
| I.1-61 | CH₃ | H | CH₂ | CH₂ | Q-1.147 |
| I.1-62 | CH₃ | H | CH₂ | CH₂ | Q-1.148 |
| I.1-63 | CH₃ | H | CH₂ | CH₂ | Q-1.149 |
| I.1-64 | CH₃ | H | CH₂ | CH₂ | Q-1.150 |
| I.1-65 | CH₃ | H | CH₂ | CH₂ | Q-1.154 |
| I.1-66 | CH₃ | H | CH₂ | CH₂ | Q-1.157 |
| I.1-67 | CH₃ | H | CH₂ | CH₂ | Q-1.163 |
| I.1-68 | CH₃ | H | CH₂ | CH₂ | Q-1.166 |
| I.1-69 | CH₃ | H | CH₂ | CH₂ | Q-1.169 |
| I.1-70 | CH₃ | H | CH₂ | CH₂ | Q-1.175 |
| I.1-71 | CH₃ | H | CH₂ | CH₂ | Q-1.178 |
| I.1-72 | CH₃ | H | CH₂ | CH₂ | Q-1.181 |
| I.1-73 | CH₃ | H | CH₂ | CH₂ | Q-1.184 |
| I.1-74 | CH₃ | H | CH₂ | CH₂ | Q-1.187 |
| I.1-75 | CH₃ | H | CH₂ | CH₂ | Q-1.190 |
| I.1-76 | CH₃ | H | CH₂ | CH₂ | Q-1.193 |
| I.1-77 | CH₃ | H | CH₂ | CH₂ | Q-1.196 |
| I.1-78 | CH₃ | H | CH₂ | CH₂ | Q-1.205 |
| I.1-79 | CH₃ | H | CH₂ | CH₂ | Q-1.206 |
| I.1-80 | CH₃ | H | CH₂ | CH₂ | Q-1.207 |
| I.1-81 | CH₃ | H | CH₂ | CH₂ | Q-1.208 |
| I.1-82 | CH₃ | H | CH₂ | CH₂ | Q-2.1 |
| I.1-83 | CH₃ | H | CH₂ | CH₂ | Q-2.2 |
| I.1-84 | CH₃ | H | CH₂ | CH₂ | Q-2.3 |
| I.1-85 | CH₃ | H | CH₂ | CH₂ | Q-2.4 |
| I.1-86 | CH₃ | H | CH₂ | CH₂ | Q-2.22 |
| I.1-87 | CH₃ | H | CH₂ | CH₂ | Q-2.23 |
| I.1-88 | CH₃ | H | CH₂ | CH₂ | Q-2.24 |
| I.1-101 | i-Pr | H | CH₂ | CH₂ | Q-1.2 |
| I.1-102 | i-Pr | H | CH₂ | CH₂ | Q-1.3 |
| I.1-103 | i-Pr | H | CH₂ | CH₂ | Q-1.7 |
| I.1-104 | i-Pr | H | CH₂ | CH₂ | Q-1.13 |
| I.1-105 | i-Pr | H | CH₂ | CH₂ | Q-1.19 |
| I.1-106 | i-Pr | H | CH₂ | CH₂ | Q-1.25 |
| I.1-107 | i-Pr | H | CH₂ | CH₂ | Q-1.26 |
| I.1-108 | i-Pr | H | CH₂ | CH₂ | Q-1.31 |
| I.1-109 | i-Pr | H | CH₂ | CH₂ | Q-1.32 |
| I.1-110 | i-Pr | H | CH₂ | CH₂ | Q-1.37 |
| I.1-111 | i-Pr | H | CH₂ | CH₂ | Q-1.43 |
| I.1-112 | i-Pr | H | CH₂ | CH₂ | Q-1.44 |
| I.1-113 | i-Pr | H | CH₂ | CH₂ | Q-1.46 |
| I.1-114 | i-Pr | H | CH₂ | CH₂ | Q-1.49 |
| I.1-115 | i-Pr | H | CH₂ | CH₂ | Q-1.52 |
| I.1-116 | i-Pr | H | CH₂ | CH₂ | Q-1.55 |
| I.1-117 | i-Pr | H | CH₂ | CH₂ | Q-1.61 |
| I.1-118 | i-Pr | H | CH₂ | CH₂ | Q-1.62 |
| I.1-119 | i-Pr | H | CH₂ | CH₂ | Q-1.64 |
| I.1-120 | i-Pr | H | CH₂ | CH₂ | Q-1.65 |
| I.1-121 | i-Pr | H | CH₂ | CH₂ | Q-1.79 |
| I.1-122 | i-Pr | H | CH₂ | CH₂ | Q-1.82 |
| I.1-123 | i-Pr | H | CH₂ | CH₂ | Q-1.85 |
| I.1-124 | i-Pr | H | CH₂ | CH₂ | Q-1.91 |
| I.1-125 | i-Pr | H | CH₂ | CH₂ | Q-1.92 |
| I.1-126 | i-Pr | H | CH₂ | CH₂ | Q-1.93 |
| I.1-127 | i-Pr | H | CH₂ | CH₂ | Q-1.97 |
| I.1-128 | i-Pr | H | CH₂ | CH₂ | Q-1.98 |
| I.1-129 | i-Pr | H | CH₂ | CH₂ | Q-1.99 |
| I.1-130 | i-Pr | H | CH₂ | CH₂ | Q-1.100 |
| I.1-131 | i-Pr | H | CH₂ | CH₂ | Q-1.101 |
| I.1-132 | i-Pr | H | CH₂ | CH₂ | Q-1.102 |
| I.1-133 | i-Pr | H | CH₂ | CH₂ | Q-1.103 |
| I.1-134 | i-Pr | H | CH₂ | CH₂ | Q-1.104 |
| I.1-135 | i-Pr | H | CH₂ | CH₂ | Q-1.105 |
| I.1-136 | i-Pr | H | CH₂ | CH₂ | Q-1.106 |
| I.1-137 | i-Pr | H | CH₂ | CH₂ | Q-1.107 |
| I.1-138 | i-Pr | H | CH₂ | CH₂ | Q-1.108 |
| I.1-139 | i-Pr | H | CH₂ | CH₂ | Q-1.109 |
| I.1-141 | i-Pr | H | CH₂ | CH₂ | Q-1.110 |
| I.1-142 | i-Pr | H | CH₂ | CH₂ | Q-1.111 |
| I.1-143 | i-Pr | H | CH₂ | CH₂ | Q-1.112 |
| I.1-144 | i-Pr | H | CH₂ | CH₂ | Q-1.113 |
| I.1-145 | i-Pr | H | CH₂ | CH₂ | Q-1.114 |
| I.1-146 | i-Pr | H | CH₂ | CH₂ | Q-1.115 |
| I.1-147 | i-Pr | H | CH₂ | CH₂ | Q-1.116 |
| I.1-148 | i-Pr | H | CH₂ | CH₂ | Q-1.117 |
| I.1-149 | i-Pr | H | CH₂ | CH₂ | Q-1.118 |
| I.1-150 | i-Pr | H | CH₂ | CH₂ | Q-1.119 |
| I.1-151 | i-Pr | H | CH₂ | CH₂ | Q-1.120 |
| I.1-152 | i-Pr | H | CH₂ | CH₂ | Q-1.121 |
| I.1-153 | i-Pr | H | CH₂ | CH₂ | Q-1.122 |
| I.1-154 | i-Pr | H | CH₂ | CH₂ | Q-1.123 |
| I.1-155 | i-Pr | H | CH₂ | CH₂ | Q-1.133 |
| I.1-156 | i-Pr | H | CH₂ | CH₂ | Q-1.134 |
| I.1-157 | i-Pr | H | CH₂ | CH₂ | Q-1.139 |
| I.1-158 | i-Pr | H | CH₂ | CH₂ | Q-1.142 |
| I.1-159 | i-Pr | H | CH₂ | CH₂ | Q-1.145 |
| I.1-160 | i-Pr | H | CH₂ | CH₂ | Q-1.146 |
| I.1-161 | i-Pr | H | CH₂ | CH₂ | Q-1.147 |
| I.1-162 | i-Pr | H | CH₂ | CH₂ | Q-1.148 |
| I.1-163 | i-Pr | H | CH₂ | CH₂ | Q-1.149 |
| I.1-164 | i-Pr | H | CH₂ | CH₂ | Q-1.150 |
| I.1-165 | i-Pr | H | CH₂ | CH₂ | Q-1.154 |
| I.1-166 | i-Pr | H | CH₂ | CH₂ | Q-1.157 |
| I.1-167 | i-Pr | H | CH₂ | CH₂ | Q-1.163 |
| I.1-168 | i-Pr | H | CH₂ | CH₂ | Q-1.166 |
| I.1-169 | i-Pr | H | CH₂ | CH₂ | Q-1.169 |
| I.1-170 | i-Pr | H | CH₂ | CH₂ | Q-1.175 |
| I.1-171 | i-Pr | H | CH₂ | CH₂ | Q-1.178 |
| I.1-172 | i-Pr | H | CH₂ | CH₂ | Q-1.181 |
| I.1-173 | i-Pr | H | CH₂ | CH₂ | Q-1.184 |
| I.1-174 | i-Pr | H | CH₂ | CH₂ | Q-1.187 |
| I.1-175 | i-Pr | H | CH₂ | CH₂ | Q-1.190 |
| I.1-176 | i-Pr | H | CH₂ | CH₂ | Q-1.193 |
| I.1-177 | i-Pr | H | CH₂ | CH₂ | Q-1.196 |
| I.1-178 | i-Pr | H | CH₂ | CH₂ | Q-1.205 |
| I.1-179 | i-Pr | H | CH₂ | CH₂ | Q-1.206 |
| I.1-180 | i-Pr | H | CH₂ | CH₂ | Q-1.207 |
| I.1-181 | i-Pr | H | CH₂ | CH₂ | Q-1.208 |
| I.1-182 | i-Pr | H | CH₂ | CH₂ | Q-2.1 |
| I.1-183 | i-Pr | H | CH₂ | CH₂ | Q-2.2 |
| I.1-184 | i-Pr | H | CH₂ | CH₂ | Q-2.3 |
| I.1-185 | i-Pr | H | CH₂ | CH₂ | Q-2.4 |
| I.1-186 | i-Pr | H | CH₂ | CH₂ | Q-2.22 |
| I.1-187 | i-Pr | H | CH₂ | CH₂ | Q-2.23 |
| I.1-188 | i-Pr | H | CH₂ | CH₂ | Q-2.24 |
| I.1-201 | Ph | H | CH₂ | CH₂ | Q-1.2 |
| I.1-202 | Ph | H | CH₂ | CH₂ | Q-1.3 |
| I.1-203 | Ph | H | CH₂ | CH₂ | Q-1.7 |
| I.1-204 | Ph | H | CH₂ | CH₂ | Q-1.13 |
| I.1-205 | Ph | H | CH₂ | CH₂ | Q-1.19 |
| I.1-206 | Ph | H | CH₂ | CH₂ | Q-1.25 |
| I.1-207 | Ph | H | CH₂ | CH₂ | Q-1.26 |
| I.1-208 | Ph | H | CH₂ | CH₂ | Q-1.31 |
| I.1-209 | Ph | H | CH₂ | CH₂ | Q-1.32 |
| I.1-210 | Ph | H | CH₂ | CH₂ | Q-1.37 |
| I.1-211 | Ph | H | CH₂ | CH₂ | Q-1.43 |
| I.1-212 | Ph | H | CH₂ | CH₂ | Q-1.44 |
| I.1-213 | Ph | H | CH₂ | CH₂ | Q-1.46 |
| I.1-214 | Ph | H | CH₂ | CH₂ | Q-1.49 |
| I.1-215 | Ph | H | CH₂ | CH₂ | Q-1.52 |
| I.1-216 | Ph | H | CH₂ | CH₂ | Q-1.55 |
| I.1-217 | Ph | H | CH₂ | CH₂ | Q-1.61 |
| I.1-218 | Ph | H | CH₂ | CH₂ | Q-1.62 |
| I.1-219 | Ph | H | CH₂ | CH₂ | Q-1.64 |
| I.1-220 | Ph | H | CH₂ | CH₂ | Q-1.65 |
| I.1-221 | Ph | H | CH₂ | CH₂ | Q-1.79 |
| I.1-222 | Ph | H | CH₂ | CH₂ | Q-1.82 |
| I.1-223 | Ph | H | CH₂ | CH₂ | Q-1.85 |
| I.1-224 | Ph | H | CH₂ | CH₂ | Q-1.91 |
| I.1-225 | Ph | H | CH₂ | CH₂ | Q-1.92 |
| I.1-226 | Ph | H | CH₂ | CH₂ | Q-1.93 |
| I.1-227 | Ph | H | CH₂ | CH₂ | Q-1.97 |
| I.1-228 | Ph | H | CH₂ | CH₂ | Q-1.98 |
| I.1-229 | Ph | H | CH₂ | CH₂ | Q-1.99 |
| I.1-230 | Ph | H | CH₂ | CH₂ | Q-1.100 |
| I.1-231 | Ph | H | CH₂ | CH₂ | Q-1.101 |
| I.1-232 | Ph | H | CH₂ | CH₂ | Q-1.102 |
| I.1-233 | Ph | H | CH₂ | CH₂ | Q-1.103 |
| I.1-234 | Ph | H | CH₂ | CH₂ | Q-1.104 |
| I.1-235 | Ph | H | CH₂ | CH₂ | Q-1.105 |
| I.1-236 | Ph | H | CH₂ | CH₂ | Q-1.106 |
| I.1-237 | Ph | H | CH₂ | CH₂ | Q-1.107 |
| I.1-238 | Ph | H | CH₂ | CH₂ | Q-1.108 |
| I.1-239 | Ph | H | CH₂ | CH₂ | Q-1.109 |
| I.1-241 | Ph | H | CH₂ | CH₂ | Q-1.110 |
| I.1-242 | Ph | H | CH₂ | CH₂ | Q-1.111 |
| I.1-243 | Ph | H | CH₂ | CH₂ | Q-1.112 |
| I.1-244 | Ph | H | CH₂ | CH₂ | Q-1.113 |
| I.1-245 | Ph | H | CH₂ | CH₂ | Q-1.114 |
| I.1-246 | Ph | H | CH₂ | CH₂ | Q-1.115 |
| I.1-247 | Ph | H | CH₂ | CH₂ | Q-1.116 |
| I.1-248 | Ph | H | CH₂ | CH₂ | Q-1.117 |
| I.1-249 | Ph | H | CH₂ | CH₂ | Q-1.118 |
| I.1-250 | Ph | H | CH₂ | CH₂ | Q-1.119 |
| I.1-251 | Ph | H | CH₂ | CH₂ | Q-1.120 |
| I.1-252 | Ph | H | CH₂ | CH₂ | Q-1.121 |
| I.1-253 | Ph | H | CH₂ | CH₂ | Q-1.122 |
| I.1-254 | Ph | H | CH₂ | CH₂ | Q-1.123 |
| I.1-255 | Ph | H | CH₂ | CH₂ | Q-1.133 |
| I.1-256 | Ph | H | CH₂ | CH₂ | Q-1.134 |
| I.1-257 | Ph | H | CH₂ | CH₂ | Q-1.139 |
| I.1-258 | Ph | H | CH₂ | CH₂ | Q-1.142 |
| I.1-259 | Ph | H | CH₂ | CH₂ | Q-1.145 |
| I.1-260 | Ph | H | CH₂ | CH₂ | Q-1.146 |
| I.1-261 | Ph | H | CH₂ | CH₂ | Q-1.147 |
| I.1-262 | Ph | H | CH₂ | CH₂ | Q-1.148 |
| I.1-263 | Ph | H | CH₂ | CH₂ | Q-1.149 |
| I.1-264 | Ph | H | CH₂ | CH₂ | Q-1.150 |
| I.1-265 | Ph | H | CH₂ | CH₂ | Q-1.154 |
| I.1-266 | Ph | H | CH₂ | CH₂ | Q-1.157 |
| I.1-267 | Ph | H | CH₂ | CH₂ | Q-1.163 |
| I.1-268 | Ph | H | CH₂ | CH₂ | Q-1.166 |
| I.1-269 | Ph | H | CH₂ | CH₂ | Q-1.169 |
| I.1-270 | Ph | H | CH₂ | CH₂ | Q-1.175 |
| I.1-271 | Ph | H | CH₂ | CH₂ | Q-1.178 |
| I.1-272 | Ph | H | CH₂ | CH₂ | Q-1.181 |
| I.1-273 | Ph | H | CH₂ | CH₂ | Q-1.184 |
| I.1-274 | Ph | H | CH₂ | CH₂ | Q-1.187 |
| I.1-275 | Ph | H | CH₂ | CH₂ | Q-1.190 |
| I.1-276 | Ph | H | CH₂ | CH₂ | Q-1.193 |
| I.1-277 | Ph | H | CH₂ | CH₂ | Q-1.196 |
| I.1-278 | Ph | H | CH₂ | CH₂ | Q-1.205 |
| I.1-279 | Ph | H | CH₂ | CH₂ | Q-1.206 |
| I.1-280 | Ph | H | CH₂ | CH₂ | Q-1.207 |
| I.1-281 | Ph | H | CH₂ | CH₂ | Q-1.208 |
| I.1-282 | Ph | H | CH₂ | CH₂ | Q-2.1 |
| I.1-283 | Ph | H | CH₂ | CH₂ | Q-2.2 |
| I.1-284 | Ph | H | CH₂ | CH₂ | Q-2.3 |
| I.1-285 | Ph | H | CH₂ | CH₂ | Q-2.4 |
| I.1-286 | Ph | H | CH₂ | CH₂ | Q-2.22 |
| I.1-287 | Ph | H | CH₂ | CH₂ | Q-2.23 |
| I.1-288 | Ph | H | CH₂ | CH₂ | Q-2.24 |
| I.1-301 | CH₃ | CH₃ | CH₂ | CH₂ | Q-1.13 |
| I.1-302 | CH₃ | CH₃ | CH₂ | CH₂ | Q-1.31 |
| I.1-303 | CH₃ | CH₃ | CH₂ | CH₂ | Q-1.61 |
| I.1-304 | CH₃ | CH₃ | CH₂ | CH₂ | Q-1.97 |
| I.1-305 | CH₃ | CH₃ | CH₂ | CH₂ | Q-1.98 |
| I.1-306 | CH₃ | CH₃ | CH₂ | CH₂ | Q-1.100 |
| I.1-307 | CH₃ | CH₃ | CH₂ | CH₂ | Q-1.101 |
| I.1-308 | CH₃ | CH₃ | CH₂ | CH₂ | Q-1.103 |
| I.1-309 | CH₃ | CH₃ | CH₂ | CH₂ | Q-1.104 |
| I.1-310 | CH₃ | CH₃ | CH₂ | CH₂ | Q-1.109 |
| I.1-311 | CH₃ | CH₃ | CH₂ | CH₂ | Q-1.110 |
| I.1-312 | CH₃ | CH₃ | CH₂ | CH₂ | Q-1.111 |
| I.1-313 | CH₃ | CH₃ | CH₂ | CH₂ | Q-1.112 |
| I.1-314 | CH₃ | CH₃ | CH₂ | CH₂ | Q-1.113 |
| I.1-315 | CH₃ | CH₃ | CH₂ | CH₂ | Q-1.114 |
| I.1-321 | CH₃ | C(=O)CH₃ | CH₂ | CH₂ | Q-1.13 |
| I.1-322 | CH₃ | C(=O)CH₃ | CH₂ | CH₂ | Q-1.31 |
| I.1-323 | CH₃ | C(=O)CH₃ | CH₂ | CH₂ | Q-1.61 |
| I.1-324 | CH₃ | C(=O)CH₃ | CH₂ | CH₂ | Q-1.97 |
| I.1-325 | CH₃ | C(=O)CH₃ | CH₂ | CH₂ | Q-1.98 |
| I.1-326 | CH₃ | C(=O)CH₃ | CH₂ | CH₂ | Q-1.100 |
| I.1-327 | CH₃ | C(=O)CH₃ | CH₂ | CH₂ | Q-1.101 |
| I.1-328 | CH₃ | C(=O)CH₃ | CH₂ | CH₂ | Q-1.103 |
| I.1-329 | CH₃ | C(=O)CH₃ | CH₂ | CH₂ | Q-1.104 |
| I.1-330 | CH₃ | C(=O)CH₃ | CH₂ | CH₂ | Q-1.109 |
| I.1-331 | CH₃ | C(=O)CH₃ | CH₂ | CH₂ | Q-1.110 |
| I.1-332 | CH₃ | C(=O)CH₃ | CH₂ | CH₂ | Q-1.111 |
| I.1-333 | CH₃ | C(=O)CH₃ | CH₂ | CH₂ | Q-1.112 |
| I.1-334 | CH₃ | C(=O)CH₃ | CH₂ | CH₂ | Q-1.113 |
| I.1-335 | CH₃ | C(=O)CH₃ | CH₂ | CH₂ | Q-1.114 |
| I.1-341 | CH₃ | SiEt₃ | CH₂ | CH₂ | Q-1.13 |
| I.1-342 | CH₃ | SiEt₃ | CH₂ | CH₂ | Q-1.31 |
| I.1-343 | CH₃ | SiEt₃ | CH₂ | CH₂ | Q-1.61 |
| I.1-344 | CH₃ | SiEt₃ | CH₂ | CH₂ | Q-1.97 |
| I.1-345 | CH₃ | SiEt₃ | CH₂ | CH₂ | Q-1.98 |
| I.1-346 | CH₃ | SiEt₃ | CH₂ | CH₂ | Q-1.100 |
| I.1-347 | CH₃ | SiEt₃ | CH₂ | CH₂ | Q-1.101 |
| I.1-348 | CH₃ | SiEt₃ | CH₂ | CH₂ | Q-1.103 |
| I.1-349 | CH₃ | SiEt₃ | CH₂ | CH₂ | Q-1.104 |
| I.1-350 | CH₃ | SiEt₃ | CH₂ | CH₂ | Q-1.109 |
| I.1-351 | CH₃ | SiEt₃ | CH₂ | CH₂ | Q-1.110 |
| I.1-352 | CH₃ | SiEt₃ | CH₂ | CH₂ | Q-1.111 |
| I.1-353 | CH₃ | SiEt₃ | CH₂ | CH₂ | Q-1.112 |
| I.1-354 | CH₃ | SiEt₃ | CH₂ | CH₂ | Q-1.113 |
| I.1-355 | CH₃ | SiEt₃ | CH₂ | CH₂ | Q-1.114 |
| I.1-361 | Ph | CH₃ | CH₂ | CH₂ | Q-1.13 |
| I.1-362 | Ph | CH₃ | CH₂ | CH₂ | Q-1.31 |
| I.1-363 | Ph | CH₃ | CH₂ | CH₂ | Q-1.61 |
| I.1-364 | Ph | CH₃ | CH₂ | CH₂ | Q-1.97 |
| I.1-365 | Ph | CH₃ | CH₂ | CH₂ | Q-1.98 |
| I.1-366 | Ph | CH₃ | CH₂ | CH₂ | Q-1.100 |
| I.1-367 | Ph | CH₃ | CH₂ | CH₂ | Q-1.101 |
| I.1-368 | Ph | CH₃ | CH₂ | CH₂ | Q-1.103 |
| I.1-369 | Ph | CH₃ | CH₂ | CH₂ | Q-1.104 |
| I.1-370 | Ph | CH₃ | CH₂ | CH₂ | Q-1.109 |
| I.1-371 | Ph | CH₃ | CH₂ | CH₂ | Q-1.110 |
| I.1-372 | Ph | CH₃ | CH₂ | CH₂ | Q-1.111 |
| I.1-373 | Ph | CH₃ | CH₂ | CH₂ | Q-1.112 |
| I.1-374 | Ph | CH₃ | CH₂ | CH₂ | Q-1.113 |
| I.1-375 | Ph | CH₃ | CH₂ | CH₂ | Q-1.114 |
| I.1-381 | Ph | C(=O)CH₃ | CH₂ | CH₂ | Q-1.13 |
| I.1-382 | Ph | C(=O)CH₃ | CH₂ | CH₂ | Q-1.31 |
| I.1-383 | Ph | C(=O)CH₃ | CH₂ | CH₂ | Q-1.61 |
| I.1-384 | Ph | C(=O)CH₃ | CH₂ | CH₂ | Q-1.97 |
| I.1-385 | Ph | C(=O)CH₃ | CH₂ | CH₂ | Q-1.98 |
| I.1-386 | Ph | C(=O)CH₃ | CH₂ | CH₂ | Q-1.100 |
| I.1-387 | Ph | C(=O)CH₃ | CH₂ | CH₂ | Q-1.101 |
| I.1-388 | Ph | C(=O)CH₃ | CH₂ | CH₂ | Q-1.103 |
| I.1-389 | Ph | C(=O)CH₃ | CH₂ | CH₂ | Q-1.104 |
| I.1-390 | Ph | C(=O)CH₃ | CH₂ | CH₂ | Q-1.109 |
| I.1-391 | Ph | C(=O)CH₃ | CH₂ | CH₂ | Q-1.110 |
| I.1-392 | Ph | C(=O)CH₃ | CH₂ | CH₂ | Q-1.111 |
| I.1-393 | Ph | C(=O)CH₃ | CH₂ | CH₂ | Q-1.112 |
| I.1-394 | Ph | C(=O)CH₃ | CH₂ | CH₂ | Q-1.113 |
| I.1-395 | Ph | C(=O)CH₃ | CH₂ | CH₂ | Q-1.114 |
| I.1-401 | Ph | SiEt₃ | CH₂ | CH₂ | Q-1.13 |
| I.1-402 | Ph | SiEt₃ | CH₂ | CH₂ | Q-1.31 |
| I.1-403 | Ph | SiEt₃ | CH₂ | CH₂ | Q-1.61 |
| I.1-404 | Ph | SiEt₃ | CH₂ | CH₂ | Q-1.97 |
| I.1-405 | Ph | SiEt₃ | CH₂ | CH₂ | Q-1.98 |
| I.1-406 | Ph | SiEt₃ | CH₂ | CH₂ | Q-1.100 |
| I.1-407 | Ph | SiEt₃ | CH₂ | CH₂ | Q-1.101 |
| I.1-408 | Ph | SiEt₃ | CH₂ | CH₂ | Q-1.103 |
| I.1-409 | Ph | SiEt₃ | CH₂ | CH₂ | Q-1.104 |
| I.1-410 | Ph | SiEt₃ | CH₂ | CH₂ | Q-1.109 |
| I.1-411 | Ph | SiEt₃ | CH₂ | CH₂ | Q-1.110 |
| I.1-412 | Ph | SiEt₃ | CH₂ | CH₂ | Q-1.111 |
| I.1-413 | Ph | SiEt₃ | CH₂ | CH₂ | Q-1.112 |
| I.1-414 | Ph | SiEt₃ | CH₂ | CH₂ | Q-1.113 |
| I.1-415 | Ph | SiEt₃ | CH₂ | CH₂ | Q-1.114 |
| I.1-421 | i-Pr | CH₃ | CH₂ | CH₂ | Q-1.13 |
| I.1-422 | i-Pr | CH₃ | CH₂ | CH₂ | Q-1.31 |
| I.1-423 | i-Pr | CH₃ | CH₂ | CH₂ | Q-1.61 |
| I.1-424 | i-Pr | CH₃ | CH₂ | CH₂ | Q-1.97 |
| I.1-425 | i-Pr | CH₃ | CH₂ | CH₂ | Q-1.98 |
| I.1-426 | i-Pr | CH₃ | CH₂ | CH₂ | Q-1.100 |
| I.1-427 | i-Pr | CH₃ | CH₂ | CH₂ | Q-1.101 |
| I.1-428 | i-Pr | CH₃ | CH₂ | CH₂ | Q-1.103 |
| I.1-429 | i-Pr | CH₃ | CH₂ | CH₂ | Q-1.104 |
| I.1-430 | i-Pr | CH₃ | CH₂ | CH₂ | Q-1.109 |
| I.1-431 | i-Pr | CH₃ | CH₂ | CH₂ | Q-1.110 |
| I.1-432 | i-Pr | CH₃ | CH₂ | CH₂ | Q-1.111 |
| I.1-433 | i-Pr | CH₃ | CH₂ | CH₂ | Q-1.112 |
| I.1-434 | i-Pr | CH₃ | CH₂ | CH₂ | Q-1.113 |
| I.1-435 | i-Pr | CH₃ | CH₂ | CH₂ | Q-1.114 |
| I.1-441 | i-Pr | C(=O)CH₃ | CH₂ | CH₂ | Q-1.13 |
| I.1-442 | i-Pr | C(=O)CH₃ | CH₂ | CH₂ | Q-1.31 |
| I.1-443 | i-Pr | C(=O)CH₃ | CH₂ | CH₂ | Q-1.61 |
| I.1-444 | i-Pr | C(=O)CH₃ | CH₂ | CH₂ | Q-1.97 |
| I.1-445 | i-Pr | C(=O)CH₃ | CH₂ | CH₂ | Q-1.98 |
| I.1-446 | i-Pr | C(=O)CH₃ | CH₂ | CH₂ | Q-1.100 |
| I.1-447 | i-Pr | C(=O)CH₃ | CH₂ | CH₂ | Q-1.101 |
| I.1-448 | i-Pr | C(=O)CH₃ | CH₂ | CH₂ | Q-1.103 |
| I.1-449 | i-Pr | C(=O)CH₃ | CH₂ | CH₂ | Q-1.104 |
| I.1-450 | i-Pr | C(=O)CH₃ | CH₂ | CH₂ | Q-1.109 |
| I.1-451 | i-Pr | C(=O)CH₃ | CH₂ | CH₂ | Q-1.110 |
| I.1-452 | i-Pr | C(=O)CH₃ | CH₂ | CH₂ | Q-1.111 |
| I.1-453 | i-Pr | C(=O)CH₃ | CH₂ | CH₂ | Q-1.112 |
| I.1-454 | i-Pr | C(=O)CH₃ | CH₂ | CH₂ | Q-1.113 |
| I.1-455 | i-Pr | C(=O)CH₃ | CH₂ | CH₂ | Q-1.114 |
| I.1-461 | i-Pr | SiEt₃ | CH₂ | CH₂ | Q-1.13 |
| I.1-462 | i-Pr | SiEt₃ | CH₂ | CH₂ | Q-1.31 |
| I.1-463 | i-Pr | SiEt₃ | CH₂ | CH₂ | Q-1.61 |
| I.1-464 | i-Pr | SiEt₃ | CH₂ | CH₂ | Q-1.97 |
| I.1-465 | i-Pr | SiEt₃ | CH₂ | CH₂ | Q-1.98 |
| I.1-466 | i-Pr | SiEt₃ | CH₂ | CH₂ | Q-1.100 |
| I.1-467 | i-Pr | SiEt₃ | CH₂ | CH₂ | Q-1.101 |
| I.1-468 | i-Pr | SiEt₃ | CH₂ | CH₂ | Q-1.103 |
| I.1-469 | i-Pr | SiEt₃ | CH₂ | CH₂ | Q-1.104 |
| I.1-470 | i-Pr | SiEt₃ | CH₂ | CH₂ | Q-1.109 |
| I.1-471 | i-Pr | SiEt₃ | CH₂ | CH₂ | Q-1.110 |
| I.1-472 | i-Pr | SiEt₃ | CH₂ | CH₂ | Q-1.111 |
| I.1-473 | i-Pr | SiEt₃ | CH₂ | CH₂ | Q-1.112 |
| I.1-474 | i-Pr | SiEt₃ | CH₂ | CH₂ | Q-1.113 |
| I.1-475 | i-Pr | SiEt₃ | CH₂ | CH₂ | Q-1.114 |
| I.1-481 | i-Pr | CH₃ | CHF | CH₂ | Q-1.97 |
| I.1-482 | i-Pr | CH₃ | CHF | CH₂ | Q-1.100 |
| I.1-483 | i-Pr | CH₃ | CHF | CH₂ | Q-1.113 |
| I.1-484 | Ph | CH₃ | CHF | CH₂ | Q-1.97 |
| I.1-485 | Ph | CH₃ | CHF | CH₂ | Q-1.100 |
| I.1-486 | Ph | CH₃ | CHF | CH₂ | Q-1.113 |
| I.1-487 | CH₃ | CH₃ | CHF | CH₂ | Q-1.97 |
| I.1-488 | CH₃ | CH₃ | CHF | CH₂ | Q-1.100 |
| I.1-489 | CH₃ | CH₃ | CHF | CH₂ | Q-1.113 |
| I.1-490 | i-Pr | CH₃ | CH(CH₃) | CH₂ | Q-1.97 |
| I.1-491 | i-Pr | CH₃ | CH(CH₃) | CH₂ | Q-1.100 |
| I.1-492 | i-Pr | CH₃ | CH(CH₃) | CH₂ | Q-1.113 |
| I.1-493 | Ph | CH₃ | CH(CH₃) | CH₂ | Q-1.97 |
| I.1-494 | Ph | CH₃ | CH(CH₃) | CH₂ | Q-1.100 |
| I.1-495 | Ph | CH₃ | CH(CH₃) | CH₂ | Q-1.113 |
| I.1-496 | CH₃ | CH₃ | CH(CH₃) | CH₂ | Q-1.97 |
| I.1-497 | CH₃ | CH₃ | CH(CH₃) | CH₂ | Q-1.100 |
| I.1-498 | CH₃ | CH₃ | CH(CH₃) | CH₂ | Q-1.113 |
| I.1-499 | i-Pr | CH₃ | CH(i-Pr) | CH₂ | Q-1.97 |
| I.1-500 | i-Pr | CH₃ | CH(i-Pr) | CH₂ | Q-1.100 |
| I.1-501 | c-Pr | H | CH₂ | CH₂ | Q-1.31 |
| I.1-502 | c-Pr | H | CH₂ | CH₂ | Q-1.64 |
| I.1-503 | c-Pr | H | CH₂ | CH₂ | Q-1.65 |
| I.1-504 | c-Pr | H | CH₂ | CH₂ | Q-1.66 |
| I.1-505 | c-Pr | H | CH₂ | CH₂ | Q-1.97 |
| I.1-506 | c-Pr | H | CH₂ | CH₂ | Q-1.98 |
| I.1-507 | c-Pr | H | CH₂ | CH₂ | Q-1.99 |
| I.1-508 | c-Pr | H | CH₂ | CH₂ | Q-1.100 |
| I.1-509 | c-Pr | H | CH₂ | CH₂ | Q-1.101 |
| I.1-510 | c-Pr | H | CH₂ | CH₂ | Q-1.102 |
| I.1-511 | c-Bu | H | CH₂ | CH₂ | Q-1.31 |
| I.1-512 | c-Bu | H | CH₂ | CH₂ | Q-1.64 |
| I.1-513 | c-Bu | H | CH₂ | CH₂ | Q-1.65 |
| I.1-514 | c-Bu | H | CH₂ | CH₂ | Q-1.66 |
| I.1-515 | c-Bu | H | CH₂ | CH₂ | Q-1.97 |
| I.1-516 | c-Bu | H | CH₂ | CH₂ | Q-1.98 |
| I.1-517 | c-Bu | H | CH₂ | CH₂ | Q-1.99 |
| I.1-518 | c-Bu | H | CH₂ | CH₂ | Q-1.100 |
| I.1-519 | c-Bu | H | CH₂ | CH₂ | Q-1.101 |
| I.1-520 | c-Bu | H | CH₂ | CH₂ | Q-1.102 |
| I.1-521 | c-Hex | H | CH₂ | CH₂ | Q-1.31 |
| I.1-522 | c-Hex | H | CH₂ | CH₂ | Q-1.64 |
| I.1-523 | c-Hex | H | CH₂ | CH₂ | Q-1.65 |
| I.1-524 | c-Hex | H | CH₂ | CH₂ | Q-1.66 |
| I.1-525 | c-Hex | H | CH₂ | CH₂ | Q-1.97 |
| I.1-526 | c-Hex | H | CH₂ | CH₂ | Q-1.98 |
| I.1-527 | c-Hex | H | CH₂ | CH₂ | Q-1.99 |
| I.1-528 | c-Hex | H | CH₂ | CH₂ | Q-1.100 |
| I.1-529 | c-Hex | H | CH₂ | CH₂ | Q-1.101 |
| I.1-530 | c-Hex | H | CH₂ | CH₂ | Q-1.102 |
| I.1-531 | 1-Ethylpropyl | H | CH₂ | CH₂ | Q-1.31 |
| I.1-532 | 1-Ethylpropyl | H | CH₂ | CH₂ | Q-1.64 |
| I.1-533 | 1-Ethylpropyl | H | CH₂ | CH₂ | Q-1.65 |
| I.1-534 | 1-Ethylpropyl | H | CH₂ | CH₂ | Q-1.66 |
| I.1-535 | 1-Ethylpropyl | H | CH₂ | CH₂ | Q-1.97 |
| I.1-536 | 1-Ethylpropyl | H | CH₂ | CH₂ | Q-1.98 |
| I.1-537 | 1-Ethylpropyl | H | CH₂ | CH₂ | Q-1.99 |
| I.1-538 | 1-Ethylpropyl | H | CH₂ | CH₂ | Q-1.100 |
| I.1-539 | 1-Ethylpropyl | H | CH₂ | CH₂ | Q-1.101 |
| I.1-540 | 1-Ethylpropyl | H | CH₂ | CH₂ | Q-1.102 |
| I.1-541 | Adamantyl | H | CH₂ | CH₂ | Q-1.31 |
| I.1-542 | Adamantyl | H | CH₂ | CH₂ | Q-1.64 |
| I.1-543 | Adamantyl | H | CH₂ | CH₂ | Q-1.65 |
| I.1-544 | Adamantyl | H | CH₂ | CH₂ | Q-1.66 |
| I.1-545 | Adamantyl | H | CH₂ | CH₂ | Q-1.97 |
| I.1-546 | Adamantyl | H | CH₂ | CH₂ | Q-1.98 |
| I.1-547 | Adamantyl | H | CH₂ | CH₂ | Q-1.99 |
| I.1-548 | Adamantyl | H | CH₂ | CH₂ | Q-1.100 |
| I.1-549 | Adamantyl | H | CH₂ | CH₂ | Q-1.101 |
| I.1-550 | Adamantyl | H | CH₂ | CH₂ | Q-1.102 |
| I.1-551 | 3-F-Ph | H | CH₂ | CH₂ | Q-1.31 |
| I.1-552 | 3-F-Ph | H | CH₂ | CH₂ | Q-1.64 |
| I.1-553 | 3-F-Ph | H | CH₂ | CH₂ | Q-1.65 |
| I.1-554 | 3-F-Ph | H | CH₂ | CH₂ | Q-1.66 |
| I.1-555 | 3-F-Ph | H | CH₂ | CH₂ | Q-1.97 |
| I.1-556 | 3-F-Ph | H | CH₂ | CH₂ | Q-1.98 |
| I.1-557 | 3-F-Ph | H | CH₂ | CH₂ | Q-1.99 |
| I.1-558 | 3-F-Ph | H | CH₂ | CH₂ | Q-1.100 |
| I.1-559 | 3-F-Ph | H | CH₂ | CH₂ | Q-1.101 |
| I.1-560 | 3-F-Ph | H | CH₂ | CH₂ | Q-1.102 |
| I.1-561 | 4-CH₃-Ph | H | CH₂ | CH₂ | Q-1.31 |
| I.1-562 | 4-CH₃-Ph | H | CH₂ | CH₂ | Q-1.64 |
| I.1-563 | 4-CH₃-Ph | H | CH₂ | CH₂ | Q-1.65 |
| I.1-564 | 4-CH₃-Ph | H | CH₂ | CH₂ | Q-1.66 |
| I.1-565 | 4-CH₃-Ph | H | CH₂ | CH₂ | Q-1.97 |
| I.1-566 | 4-CH₃-Ph | H | CH₂ | CH₂ | Q-1.98 |
| I.1-567 | 4-CH₃-Ph | H | CH₂ | CH₂ | Q-1.99 |
| I.1-568 | 4-CH₃-Ph | H | CH₂ | CH₂ | Q-1.100 |
| I.1-569 | 4-CH₃-Ph | H | CH₂ | CH₂ | Q-1.101 |
| I.1-570 | 4-CH₃-Ph | H | CH₂ | CH₂ | Q-1.102 |
| I.1-571 | c-Pent | H | CH₂ | CH₂ | Q-1.31 |
| I.1-572 | c-Pent | H | CH₂ | CH₂ | Q-1.64 |
| I.1-573 | c-Pent | H | CH₂ | CH₂ | Q-1.65 |
| I.1-574 | c-Pent | H | CH₂ | CH₂ | Q-1.66 |
| I.1-575 | c-Pent | H | CH₂ | CH₂ | Q-1.97 |
| I.1-576 | c-Pent | H | CH₂ | CH₂ | Q-1.98 |
| I.1-577 | c-Pent | H | CH₂ | CH₂ | Q-1.99 |
| I.1-578 | c-Pent | H | CH₂ | CH₂ | Q-1.100 |
| I.1-579 | c-Pent | H | CH₂ | CH₂ | Q-1.101 |
| I.1-580 | c-Pent | H | CH₂ | CH₂ | Q-1.102 |
| I.1-581 | 2-F-Ph | H | CH₂ | CH₂ | Q-1.31 |
| I.1-582 | 2-F-Ph | H | CH₂ | CH₂ | Q-1.64 |
| I.1-583 | 2-F-Ph | H | CH₂ | CH₂ | Q-1.65 |
| I.1-584 | 2-F-Ph | H | CH₂ | CH₂ | Q-1.66 |
| I.1-585 | 2-F-Ph | H | CH₂ | CH₂ | Q-1.97 |
| I.1-586 | 2-F-Ph | H | CH₂ | CH₂ | Q-1.98 |
| I.1-587 | 2-F-Ph | H | CH₂ | CH₂ | Q-1.99 |
| I.1-588 | 2-F-Ph | H | CH₂ | CH₂ | Q-1.100 |
| I.1-589 | 2-F-Ph | H | CH₂ | CH₂ | Q-1.101 |
| I.1-590 | 2-F-Ph | H | CH₂ | CH₂ | Q-1.102 |
| I.1-591 | 4-Cl-Ph | H | CH₂ | CH₂ | Q-1.31 |
| I.1-592 | 4-Cl-Ph | H | CH₂ | CH₂ | Q-1.64 |
| I.1-593 | 4-Cl-Ph | H | CH₂ | CH₂ | Q-1.65 |
| I.1-594 | 4-Cl-Ph | H | CH₂ | CH₂ | Q-1.66 |
| I.1-595 | 4-Cl-Ph | H | CH₂ | CH₂ | Q-1.97 |
| I.1-596 | 4-Cl-Ph | H | CH₂ | CH₂ | Q-1.98 |
| I.1-597 | 4-Cl-Ph | H | CH₂ | CH₂ | Q-1.99 |
| I.1-598 | 4-Cl-Ph | H | CH₂ | CH₂ | Q-1.100 |
| I.1-599 | 4-Cl-Ph | H | CH₂ | CH₂ | Q-1.101 |
| I.1-600 | 4-Cl-Ph | H | CH₂ | CH₂ | Q-1.102 |
| I.1-601 | t-Bu | H | CH₂ | CH₂ | Q-1.31 |
| I.1-602 | t-Bu | H | CH₂ | CH₂ | Q-1.64 |
| I.1-603 | t-Bu | H | CH₂ | CH₂ | Q-1.65 |
| I.1-604 | t-Bu | H | CH₂ | CH₂ | Q-1.66 |
| I.1-605 | t-Bu | H | CH₂ | CH₂ | Q-1.97 |
| I.1-606 | t-Bu | H | CH₂ | CH₂ | Q-1.98 |
| I.1-607 | t-Bu | H | CH₂ | CH₂ | Q-1.99 |
| I.1-608 | t-Bu | H | CH₂ | CH₂ | Q-1.100 |
| I.1-609 | t-Bu | H | CH₂ | CH₂ | Q-1.101 |
| I.1-610 | t-Bu | H | CH₂ | CH₂ | Q-1.102 |
| I.1-611 | 4-F-Ph | H | CH₂ | CH₂ | Q-1.31 |
| I.1-612 | 4-F-Ph | H | CH₂ | CH₂ | Q-1.64 |
| I.1-613 | 4-F-Ph | H | CH₂ | CH₂ | Q-1.65 |
| I.1-614 | 4-F-Ph | H | CH₂ | CH₂ | Q-1.66 |
| I.1-615 | 4-F-Ph | H | CH₂ | CH₂ | Q-1.97 |
| I.1-616 | 4-F-Ph | H | CH₂ | CH₂ | Q-1.98 |
| I.1-617 | 4-F-Ph | H | CH₂ | CH₂ | Q-1.99 |
| I.1-618 | 4-F-Ph | H | CH₂ | CH₂ | Q-1.100 |
| I.1-619 | 4-F-Ph | H | CH₂ | CH₂ | Q-1.101 |
| I.1-620 | 4-F-Ph | H | CH₂ | CH₂ | Q-1.102 |
| I.1-621 | 4-F-Ph | H | CH₂ | CH₂ | Q-1.103 |
| I.1-622 | 4-F-Ph | H | CH₂ | CH₂ | Q-1.113 |
| I.1-623 | 4-F-Ph | H | CH₂ | CH₂ | Q-1.13 |
| I.1-624 | 4-F-Ph | H | CH₂ | CH₂ | Q-1.64 |
| I.1-625 | 1-Ethylpropyl | H | CH₂ | CH₂ | Q-1.113 |
| I.1-626 | 1-Ethylpropyl | H | CH₂ | CH₂ | Q-1.7 |
| I.1-627 | 1-Ethylpropyl | H | CH₂ | CH₂ | Q-1.13 |
| I.1-628 | 1-Ethylpropyl | H | CH₂ | CH₂ | Q-1.9 |
| I.1-629 | 1-Ethylpropyl | H | CH₂ | CH₂ | Q-1.103 |

**Tabelle 2:**

| | | | | | |
|---|---|---|---|---|---|
| 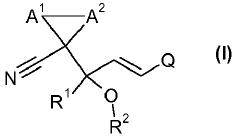 | | | Q entspricht jeweils einer der weiter vorne als besonders bevorzugt definierten Struktur | | |

| No. | R¹ | R² | A¹ | A² | Q |
|---|---|---|---|---|---|
| I.2-1 | CH₃ | H | CH₂ | CH₂ | Q-1.2 |
| I.2-2 | CH₃ | H | CH₂ | CH₂ | Q-1.3 |
| I.2-3 | CH₃ | H | CH₂ | CH₂ | Q-1.7 |
| I.2-4 | CH₃ | H | CH₂ | CH₂ | Q-1.13 |
| I.2-5 | CH₃ | H | CH₂ | CH₂ | Q-1.19 |
| I.2-6 | CH₃ | H | CH₂ | CH₂ | Q-1.25 |
| I.2-7 | CH₃ | H | CH₂ | CH₂ | Q-1.26 |
| I.2-8 | CH₃ | H | CH₂ | CH₂ | Q-1.31 |
| I.2-9 | CH₃ | H | CH₂ | CH₂ | Q-1.32 |
| I.2-10 | CH₃ | H | CH₂ | CH₂ | Q-1.37 |
| I.2-11 | CH₃ | H | CH₂ | CH₂ | Q-1.43 |
| I.2-12 | CH₃ | H | CH₂ | CH₂ | Q-1.44 |
| I.2-13 | CH₃ | H | CH₂ | CH₂ | Q-1.46 |
| I.2-14 | CH₃ | H | CH₂ | CH₂ | Q-1.49 |
| I.2-15 | CH₃ | H | CH₂ | CH₂ | Q-1.52 |
| I.2-16 | CH₃ | H | CH₂ | CH₂ | Q-1.55 |
| I.2-17 | CH₃ | H | CH₂ | CH₂ | Q-1.61 |
| I.2-18 | CH₃ | H | CH₂ | CH₂ | Q-1.62 |
| I.2-19 | CH₃ | H | CH₂ | CH₂ | Q-1.64 |
| I.2-20 | CH₃ | H | CH₂ | CH₂ | Q-1.65 |
| I.2-21 | CH₃ | H | CH₂ | CH₂ | Q-1.79 |
| I.2-22 | CH₃ | H | CH₂ | CH₂ | Q-1.82 |
| I.2-23 | CH₃ | H | CH₂ | CH₂ | Q-1.85 |
| I.2-24 | CH₃ | H | CH₂ | CH₂ | Q-1.91 |
| I.2-25 | CH₃ | H | CH₂ | CH₂ | Q-1.92 |
| I.2-26 | CH₃ | H | CH₂ | CH₂ | Q-1.93 |
| I.2-27 | CH₃ | H | CH₂ | CH₂ | Q-1.97 |
| I.2-28 | CH₃ | H | CH₂ | CH₂ | Q-1.98 |
| I.2-29 | CH₃ | H | CH₂ | CH₂ | Q-1.99 |
| I.2-30 | CH₃ | H | CH₂ | CH₂ | Q-1.100 |
| I.2-31 | CH₃ | H | CH₂ | CH₂ | Q-1.101 |
| I.2-32 | CH₃ | H | CH₂ | CH₂ | Q-1.102 |
| I.2-33 | CH₃ | H | CH₂ | CH₂ | Q-1.103 |
| I.2-34 | CH₃ | H | CH₂ | CH₂ | Q-1.104 |
| I.2-35 | CH₃ | H | CH₂ | CH₂ | Q-1.105 |
| I.2-36 | CH₃ | H | CH₂ | CH₂ | Q-1.106 |
| I.2-37 | CH₃ | H | CH₂ | CH₂ | Q-1.107 |
| I.2-38 | CH₃ | H | CH₂ | CH₂ | Q-1.108 |
| I.2-39 | CH₃ | H | CH₂ | CH₂ | Q-1.109 |
| I.2-41 | CH₃ | H | CH₂ | CH₂ | Q-1.110 |
| I.2-42 | CH₃ | H | CH₂ | CH₂ | Q-1.111 |
| I.2-43 | CH₃ | H | CH₂ | CH₂ | Q-1.112 |
| I.2-44 | CH₃ | H | CH₂ | CH₂ | Q-1.113 |
| I.2-45 | CH₃ | H | CH₂ | CH₂ | Q-1.114 |
| I.2-46 | CH₃ | H | CH₂ | CH₂ | Q-1.115 |
| I.2-47 | CH₃ | H | CH₂ | CH₂ | Q-1.116 |
| I.2-48 | CH₃ | H | CH₂ | CH₂ | Q-1.117 |
| I.2-49 | CH₃ | H | CH₂ | CH₂ | Q-1.118 |
| I.2-50 | CH₃ | H | CH₂ | CH₂ | Q-1.119 |
| I.2-51 | CH₃ | H | CH₂ | CH₂ | Q-1.120 |
| I.2-52 | CH₃ | H | CH₂ | CH₂ | Q-1.121 |
| I.2-53 | CH₃ | H | CH₂ | CH₂ | Q-1.122 |
| I.2-54 | CH₃ | H | CH₂ | CH₂ | Q-1.123 |
| I.2-55 | CH₃ | H | CH₂ | CH₂ | Q-1.133 |
| I.2-56 | CH₃ | H | CH₂ | CH₂ | Q-1.134 |
| I.2-57 | CH₃ | H | CH₂ | CH₂ | Q-1.139 |
| I.2-58 | CH₃ | H | CH₂ | CH₂ | Q-1.142 |
| I.2-59 | CH₃ | H | CH₂ | CH₂ | Q-1.145 |
| I.2-60 | CH₃ | H | CH₂ | CH₂ | Q-1.146 |
| I.2-61 | CH₃ | H | CH₂ | CH₂ | Q-1.147 |
| I.2-62 | CH₃ | H | CH₂ | CH₂ | Q-1.148 |
| I.2-63 | CH₃ | H | CH₂ | CH₂ | Q-1.149 |
| I.2-64 | CH₃ | H | CH₂ | CH₂ | Q-1.150 |
| I.2-65 | CH₃ | H | CH₂ | CH₂ | Q-1.154 |
| I.2-66 | CH₃ | H | CH₂ | CH₂ | Q-1.157 |
| I.2-67 | CH₃ | H | CH₂ | CH₂ | Q-1.163 |
| I.2-68 | CH₃ | H | CH₂ | CH₂ | Q-1.166 |
| I.2-69 | CH₃ | H | CH₂ | CH₂ | Q-1.169 |
| I.2-70 | CH₃ | H | CH₂ | CH₂ | Q-1.175 |
| I.2-71 | CH₃ | H | CH₂ | CH₂ | Q-1.178 |
| I.2-72 | CH₃ | H | CH₂ | CH₂ | Q-1.181 |
| I.2-73 | CH₃ | H | CH₂ | CH₂ | Q-1.184 |
| I.2-74 | CH₃ | H | CH₂ | CH₂ | Q-1.187 |
| I.2-75 | CH₃ | H | CH₂ | CH₂ | Q-1.190 |
| I.2-76 | CH₃ | H | CH₂ | CH₂ | Q-1.193 |
| I.2-77 | CH₃ | H | CH₂ | CH₂ | Q-1.196 |
| I.2-78 | CH₃ | H | CH₂ | CH₂ | Q-1.205 |
| I.2-79 | CH₃ | H | CH₂ | CH₂ | Q-1.206 |
| I.2-80 | CH₃ | H | CH₂ | CH₂ | Q-1.207 |
| I.2-81 | CH₃ | H | CH₂ | CH₂ | Q-1.208 |
| I.2-82 | CH₃ | H | CH₂ | CH₂ | Q-2.1 |
| I.2-83 | CH₃ | H | CH₂ | CH₂ | Q-2.2 |
| I.2-84 | CH₃ | H | CH₂ | CH₂ | Q-2.3 |
| I.2-85 | CH₃ | H | CH₂ | CH₂ | Q-2.4 |
| I.2-86 | CH₃ | H | CH₂ | CH₂ | Q-2.22 |
| I.2-87 | CH₃ | H | CH₂ | CH₂ | Q-2.23 |
| I.2-88 | CH₃ | H | CH₂ | CH₂ | Q-2.24 |
| I.2-101 | i-Pr | H | CH₂ | CH₂ | Q-1.2 |
| I.2-102 | i-Pr | H | CH₂ | CH₂ | Q-1.3 |
| I.2-103 | i-Pr | H | CH₂ | CH₂ | Q-1.7 |
| I.2-104 | i-Pr | H | CH₂ | CH₂ | Q-1.13 |
| I.2-105 | i-Pr | H | CH₂ | CH₂ | Q-1.19 |
| I.2-106 | i-Pr | H | CH₂ | CH₂ | Q-1.25 |
| I.2-107 | i-Pr | H | CH₂ | CH₂ | Q-1.26 |
| I.2-108 | i-Pr | H | CH₂ | CH₂ | Q-1.31 |
| I.2-109 | i-Pr | H | CH₂ | CH₂ | Q-1.32 |
| I.2-110 | i-Pr | H | CH₂ | CH₂ | Q-1.37 |
| I.2-111 | i-Pr | H | CH₂ | CH₂ | Q-1.43 |
| I.2-112 | i-Pr | H | CH₂ | CH₂ | Q-1.44 |
| I.2-113 | i-Pr | H | CH₂ | CH₂ | Q-1.46 |
| I.2-114 | i-Pr | H | CH₂ | CH₂ | Q-1.49 |
| I.2-115 | i-Pr | H | CH₂ | CH₂ | Q-1.52 |
| I.2-116 | i-Pr | H | CH₂ | CH₂ | Q-1.55 |
| I.2-117 | i-Pr | H | CH₂ | CH₂ | Q-1.61 |
| I.2-118 | i-Pr | H | CH₂ | CH₂ | Q-1.62 |
| I.2-119 | i-Pr | H | CH₂ | CH₂ | Q-1.64 |
| I.2-120 | i-Pr | H | CH₂ | CH₂ | Q-1.65 |
| I.2-121 | i-Pr | H | CH₂ | CH₂ | Q-1.79 |
| I.2-122 | i-Pr | H | CH₂ | CH₂ | Q-1.82 |
| I.2-123 | i-Pr | H | CH₂ | CH₂ | Q-1.85 |
| I.2-124 | i-Pr | H | CH₂ | CH₂ | Q-1.91 |
| I.2-125 | i-Pr | H | CH₂ | CH₂ | Q-1.92 |
| I.2-126 | i-Pr | H | CH₂ | CH₂ | Q-1.93 |
| I.2-127 | i-Pr | H | CH₂ | CH₂ | Q-1.97 |
| I.2-128 | i-Pr | H | CH₂ | CH₂ | Q-1.98 |
| I.2-129 | i-Pr | H | CH₂ | CH₂ | Q-1.99 |
| I.2-130 | i-Pr | H | CH₂ | CH₂ | Q-1.100 |
| I.2-131 | i-Pr | H | CH₂ | CH₂ | Q-1.101 |
| I.2-132 | i-Pr | H | CH₂ | CH₂ | Q-1.102 |
| I.2-133 | i-Pr | H | CH₂ | CH₂ | Q-1.103 |
| I.2-134 | i-Pr | H | CH₂ | CH₂ | Q-1.104 |
| I.2-135 | i-Pr | H | CH₂ | CH₂ | Q-1.105 |
| I.2-136 | i-Pr | H | CH₂ | CH₂ | Q-1.106 |
| I.2-137 | i-Pr | H | CH₂ | CH₂ | Q-1.107 |
| I.2-138 | i-Pr | H | CH₂ | CH₂ | Q-1.108 |
| I.2-139 | i-Pr | H | CH₂ | CH₂ | Q-1.109 |
| I.2-141 | i-Pr | H | CH₂ | CH₂ | Q-1.110 |
| I.2-142 | i-Pr | H | CH₂ | CH₂ | Q-1.111 |
| I.2-143 | i-Pr | H | CH₂ | CH₂ | Q-1.112 |
| I.2-144 | i-Pr | H | CH₂ | CH₂ | Q-1.113 |
| I.2-145 | i-Pr | H | CH₂ | CH₂ | Q-1.114 |
| I.2-146 | i-Pr | H | CH₂ | CH₂ | Q-1.115 |
| I.2-147 | i-Pr | H | CH₂ | CH₂ | Q-1.116 |
| I.2-148 | i-Pr | H | CH₂ | CH₂ | Q-1.117 |
| I.2-149 | i-Pr | H | CH₂ | CH₂ | Q-1.118 |
| I.2-150 | i-Pr | H | CH₂ | CH₂ | Q-1.119 |
| I.2-151 | i-Pr | H | CH₂ | CH₂ | Q-1.120 |
| I.2-152 | i-Pr | H | CH₂ | CH₂ | Q-1.121 |
| I.2-153 | i-Pr | H | CH₂ | CH₂ | Q-1.122 |
| I.2-154 | i-Pr | H | CH₂ | CH₂ | Q-1.123 |
| I.2-155 | i-Pr | H | CH₂ | CH₂ | Q-1.133 |
| I.2-156 | i-Pr | H | CH₂ | CH₂ | Q-1.134 |
| I.2-157 | i-Pr | H | CH₂ | CH₂ | Q-1.139 |
| I.2-158 | i-Pr | H | CH₂ | CH₂ | Q-1.142 |
| I.2-159 | i-Pr | H | CH₂ | CH₂ | Q-1.145 |
| I.2-160 | i-Pr | H | CH₂ | CH₂ | Q-1.146 |
| I.2-161 | i-Pr | H | CH₂ | CH₂ | Q-1.147 |
| I.2-162 | i-Pr | H | CH₂ | CH₂ | Q-1.148 |
| I.2-163 | i-Pr | H | CH₂ | CH₂ | Q-1.149 |
| I.2-164 | i-Pr | H | CH₂ | CH₂ | Q-1.150 |
| I.2-165 | i-Pr | H | CH₂ | CH₂ | Q-1.154 |
| I.2-166 | i-Pr | H | CH₂ | CH₂ | Q-1.157 |
| I.2-167 | i-Pr | H | CH₂ | CH₂ | Q-1.163 |
| I.2-168 | i-Pr | H | CH₂ | CH₂ | Q-1.166 |
| I.2-169 | i-Pr | H | CH₂ | CH₂ | Q-1.169 |
| I.2-170 | i-Pr | H | CH₂ | CH₂ | Q-1.175 |
| I.2-171 | i-Pr | H | CH₂ | CH₂ | Q-1.178 |
| I.2-172 | i-Pr | H | CH₂ | CH₂ | Q-1.181 |
| I.2-173 | i-Pr | H | CH₂ | CH₂ | Q-1.184 |
| I.2-174 | i-Pr | H | CH₂ | CH₂ | Q-1.187 |
| I.2-175 | i-Pr | H | CH₂ | CH₂ | Q-1.190 |
| I.2-176 | i-Pr | H | CH₂ | CH₂ | Q-1.193 |
| I.2-177 | i-Pr | H | CH₂ | CH₂ | Q-1.196 |
| I.2-178 | i-Pr | H | CH₂ | CH₂ | Q-1.205 |
| I.2-179 | i-Pr | H | CH₂ | CH₂ | Q-1.206 |
| I.2-180 | i-Pr | H | CH₂ | CH₂ | Q-1.207 |
| I.2-181 | i-Pr | H | CH₂ | CH₂ | Q-1.208 |
| I.2-182 | i-Pr | H | CH₂ | CH₂ | Q-2.1 |
| I.2-183 | i-Pr | H | CH₂ | CH₂ | Q-2.2 |
| I.2-184 | i-Pr | H | CH₂ | CH₂ | Q-2.3 |
| I.2-185 | i-Pr | H | CH₂ | CH₂ | Q-2.4 |
| I.2-186 | i-Pr | H | CH₂ | CH₂ | Q-2.22 |
| I.2-187 | i-Pr | H | CH₂ | CH₂ | Q-2.23 |
| I.2-188 | i-Pr | H | CH₂ | CH₂ | Q-2.24 |
| I.2-201 | Ph | H | CH₂ | CH₂ | Q-1.2 |
| I.2-202 | Ph | H | CH₂ | CH₂ | Q-1.3 |
| I.2-203 | Ph | H | CH₂ | CH₂ | Q-1.7 |
| I.2-204 | Ph | H | CH₂ | CH₂ | Q-1.13 |
| I.2-205 | Ph | H | CH₂ | CH₂ | Q-1.19 |
| I.2-206 | Ph | H | CH₂ | CH₂ | Q-1.25 |
| I.2-207 | Ph | H | CH₂ | CH₂ | Q-1.26 |
| I.2-208 | Ph | H | CH₂ | CH₂ | Q-1.31 |
| I.2-209 | Ph | H | CH₂ | CH₂ | Q-1.32 |
| I.2-210 | Ph | H | CH₂ | CH₂ | Q-1.37 |
| I.2-211 | Ph | H | CH₂ | CH₂ | Q-1.43 |
| I.2-212 | Ph | H | CH₂ | CH₂ | Q-1.44 |
| I.2-213 | Ph | H | CH₂ | CH₂ | Q-1.46 |
| I.2-214 | Ph | H | CH₂ | CH₂ | Q-1.49 |
| I.2-215 | Ph | H | CH₂ | CH₂ | Q-1.52 |
| I.2-216 | Ph | H | CH₂ | CH₂ | Q-1.55 |
| I.2-217 | Ph | H | CH₂ | CH₂ | Q-1.61 |
| I.2-218 | Ph | H | CH₂ | CH₂ | Q-1.62 |
| I.2-219 | Ph | H | CH₂ | CH₂ | Q-1.64 |
| I.2-220 | Ph | H | CH₂ | CH₂ | Q-1.65 |
| I.2-221 | Ph | H | CH₂ | CH₂ | Q-1.79 |
| I.2-222 | Ph | H | CH₂ | CH₂ | Q-1.82 |
| I.2-223 | Ph | H | CH₂ | CH₂ | Q-1.85 |
| I.2-224 | Ph | H | CH₂ | CH₂ | Q-1.91 |
| I.2-225 | Ph | H | CH₂ | CH₂ | Q-1.92 |
| I.2-226 | Ph | H | CH₂ | CH₂ | Q-1.93 |
| I.2-227 | Ph | H | CH₂ | CH₂ | Q-1.97 |
| I.2-228 | Ph | H | CH₂ | CH₂ | Q-1.98 |
| I.2-229 | Ph | H | CH₂ | CH₂ | Q-1.99 |
| I.2-230 | Ph | H | CH₂ | CH₂ | Q-1.100 |
| I.2-231 | Ph | H | CH₂ | CH₂ | Q-1.101 |
| I.2-232 | Ph | H | CH₂ | CH₂ | Q-1.102 |
| I.2-233 | Ph | H | CH₂ | CH₂ | Q-1.103 |
| I.2-234 | Ph | H | CH₂ | CH₂ | Q-1.104 |
| I.2-235 | Ph | H | CH₂ | CH₂ | Q-1.105 |
| I.2-236 | Ph | H | CH₂ | CH₂ | Q-1.106 |
| I.2-237 | Ph | H | CH₂ | CH₂ | Q-1.107 |
| I.2-238 | Ph | H | CH₂ | CH₂ | Q-1.108 |
| I.2-239 | Ph | H | CH₂ | CH₂ | Q-1.109 |
| I.2-241 | Ph | H | CH₂ | CH₂ | Q-1.110 |
| I.2-242 | Ph | H | CH₂ | CH₂ | Q-1.111 |
| I.2-243 | Ph | H | CH₂ | CH₂ | Q-1.112 |
| I.2-244 | Ph | H | CH₂ | CH₂ | Q-1.113 |
| I.2-245 | Ph | H | CH₂ | CH₂ | Q-1.114 |
| I.2-246 | Ph | H | CH₂ | CH₂ | Q-1.115 |
| I.2-247 | Ph | H | CH₂ | CH₂ | Q-1.116 |
| I.2-248 | Ph | H | CH₂ | CH₂ | Q-1.117 |
| I.2-249 | Ph | H | CH₂ | CH₂ | Q-1.118 |
| I.2-250 | Ph | H | CH₂ | CH₂ | Q-1.119 |
| I.2-251 | Ph | H | CH₂ | CH₂ | Q-1.120 |
| I.2-252 | Ph | H | CH₂ | CH₂ | Q-1.121 |
| I.2-253 | Ph | H | CH₂ | CH₂ | Q-1.122 |
| I.2-254 | Ph | H | CH₂ | CH₂ | Q-1.123 |
| I.2-255 | Ph | H | CH₂ | CH₂ | Q-1.133 |
| I.2-256 | Ph | H | CH₂ | CH₂ | Q-1.134 |
| I.2-257 | Ph | H | CH₂ | CH₂ | Q-1.139 |
| I.2-258 | Ph | H | CH₂ | CH₂ | Q-1.142 |
| I.2-259 | Ph | H | CH₂ | CH₂ | Q-1.145 |
| I.2-260 | Ph | H | CH₂ | CH₂ | Q-1.146 |
| I.2-261 | Ph | H | CH₂ | CH₂ | Q-1.147 |
| I.2-262 | Ph | H | CH₂ | CH₂ | Q-1.148 |
| I.2-263 | Ph | H | CH₂ | CH₂ | Q-1.149 |
| I.2-264 | Ph | H | CH₂ | CH₂ | Q-1.150 |
| I.2-265 | Ph | H | CH₂ | CH₂ | Q-1.154 |
| I.2-266 | Ph | H | CH₂ | CH₂ | Q-1.157 |
| I.2-267 | Ph | H | CH₂ | CH₂ | Q-1.163 |
| I.2-268 | Ph | H | CH₂ | CH₂ | Q-1.166 |
| I.2-269 | Ph | H | CH₂ | CH₂ | Q-1.169 |
| I.2-270 | Ph | H | CH₂ | CH₂ | Q-1.175 |
| I.2-271 | Ph | H | CH₂ | CH₂ | Q-1.178 |
| I.2-272 | Ph | H | CH₂ | CH₂ | Q-1.181 |
| I.2-273 | Ph | H | CH₂ | CH₂ | Q-1.184 |
| I.2-274 | Ph | H | CH₂ | CH₂ | Q-1.187 |
| I.2-275 | Ph | H | CH₂ | CH₂ | Q-1.190 |
| I.2-276 | Ph | H | CH₂ | CH₂ | Q-1.193 |
| I.2-277 | Ph | H | CH₂ | CH₂ | Q-1.196 |
| I.2-278 | Ph | H | CH₂ | CH₂ | Q-1.205 |
| I.2-279 | Ph | H | CH₂ | CH₂ | Q-1.206 |
| I.2-280 | Ph | H | CH₂ | CH₂ | Q-1.207 |
| I.2-281 | Ph | H | CH₂ | CH₂ | Q-1.208 |
| I.2-282 | Ph | H | CH₂ | CH₂ | Q-2.1 |
| I.2-283 | Ph | H | CH₂ | CH₂ | Q-2.2 |
| I.2-284 | Ph | H | CH₂ | CH₂ | Q-2.3 |
| I.2-285 | Ph | H | CH₂ | CH₂ | Q-2.4 |
| I.2-286 | Ph | H | CH₂ | CH₂ | Q-2.22 |
| I.2-287 | Ph | H | CH₂ | CH₂ | Q-2.23 |
| I.2-288 | Ph | H | CH₂ | CH₂ | Q-2.24 |
| I.2-301 | CH₃ | CH₃ | CH₂ | CH₂ | Q-1.13 |
| I.2-302 | CH₃ | CH₃ | CH₂ | CH₂ | Q-1.31 |
| I.2-303 | CH₃ | CH₃ | CH₂ | CH₂ | Q-1.61 |
| I.2-304 | CH₃ | CH₃ | CH₂ | CH₂ | Q-1.97 |
| I.2-305 | CH₃ | CH₃ | CH₂ | CH₂ | Q-1.98 |
| I.2-306 | CH₃ | CH₃ | CH₂ | CH₂ | Q-1.100 |
| I.2-307 | CH₃ | CH₃ | CH₂ | CH₂ | Q-1.101 |
| I.2-308 | CH₃ | CH₃ | CH₂ | CH₂ | Q-1.103 |
| I.2-309 | CH₃ | CH₃ | CH₂ | CH₂ | Q-1.104 |
| I.2-310 | CH₃ | CH₃ | CH₂ | CH₂ | Q-1.109 |
| I.2-311 | CH₃ | CH₃ | CH₂ | CH₂ | Q-1.110 |
| I.2-312 | CH₃ | CH₃ | CH₂ | CH₂ | Q-1.111 |
| I.2-313 | CH₃ | CH₃ | CH₂ | CH₂ | Q-1.112 |
| I.2-314 | CH₃ | CH₃ | CH₂ | CH₂ | Q-1.113 |
| I.2-315 | CH₃ | CH₃ | CH₂ | CH₂ | Q-1.114 |
| I.2-321 | CH₃ | C(=O)CH₃ | CH₂ | CH₂ | Q-1.13 |
| I.2-322 | CH₃ | C(=O)CH₃ | CH₂ | CH₂ | Q-1.31 |
| I.2-323 | CH₃ | C(=O)CH₃ | CH₂ | CH₂ | Q-1.61 |
| I.2-324 | CH₃ | C(=O)CH₃ | CH₂ | CH₂ | Q-1.97 |
| I.2-325 | CH₃ | C(=O)CH₃ | CH₂ | CH₂ | Q-1.98 |
| I.2-326 | CH₃ | C(=O)CH₃ | CH₂ | CH₂ | Q-1.100 |
| I.2-327 | CH₃ | C(=O)CH₃ | CH₂ | CH₂ | Q-1.101 |
| I.2-328 | CH₃ | C(=O)CH₃ | CH₂ | CH₂ | Q-1.103 |
| I.2-329 | CH₃ | C(=O)CH₃ | CH₂ | CH₂ | Q-1.104 |
| I.2-330 | CH₃ | C(=O)CH₃ | CH₂ | CH₂ | Q-1.109 |
| I.2-331 | CH₃ | C(=O)CH₃ | CH₂ | CH₂ | Q-1.110 |
| I.2-332 | CH₃ | C(=O)CH₃ | CH₂ | CH₂ | Q-1.111 |
| I.2-333 | CH₃ | C(=O)CH₃ | CH₂ | CH₂ | Q-1.112 |
| I.2-334 | CH₃ | C(=O)CH₃ | CH₂ | CH₂ | Q-1.113 |
| I.2-335 | CH₃ | C(=O)CH₃ | CH₂ | CH₂ | Q-1.114 |
| I.2-341 | CH₃ | SiEt₃ | CH₂ | CH₂ | Q-1.13 |
| I.2-342 | CH₃ | SiEt₃ | CH₂ | CH₂ | Q-1.31 |
| I.2-343 | CH₃ | SiEt₃ | CH₂ | CH₂ | Q-1.61 |
| I.2-344 | CH₃ | SiEt₃ | CH₂ | CH₂ | Q-1.97 |
| I.2-345 | CH₃ | SiEt₃ | CH₂ | CH₂ | Q-1.98 |
| I.2-346 | CH₃ | SiEt₃ | CH₂ | CH₂ | Q-1.100 |
| I.2-347 | CH₃ | SiEt₃ | CH₂ | CH₂ | Q-1.101 |
| I.2-348 | CH₃ | SiEt₃ | CH₂ | CH₂ | Q-1.103 |
| I.2-349 | CH₃ | SiEt₃ | CH₂ | CH₂ | Q-1.104 |
| I.2-350 | CH₃ | SiEt₃ | CH₂ | CH₂ | Q-1.109 |
| I.2-351 | CH₃ | SiEt₃ | CH₂ | CH₂ | Q-1.110 |
| I.2-352 | CH₃ | SiEt₃ | CH₂ | CH₂ | Q-1.111 |
| I.2-353 | CH₃ | SiEt₃ | CH₂ | CH₂ | Q-1.112 |
| I.2-354 | CH₃ | SiEt₃ | CH₂ | CH₂ | Q-1.113 |
| I.2-355 | CH₃ | SiEt₃ | CH₂ | CH₂ | Q-1.114 |
| I.2-361 | Ph | CH₃ | CH₂ | CH₂ | Q-1.13 |
| I.2-362 | Ph | CH₃ | CH₂ | CH₂ | Q-1.31 |
| I.2-363 | Ph | CH₃ | CH₂ | CH₂ | Q-1.61 |
| I.2-364 | Ph | CH₃ | CH₂ | CH₂ | Q-1.97 |
| I.2-365 | Ph | CH₃ | CH₂ | CH₂ | Q-1.98 |
| I.2-366 | Ph | CH₃ | CH₂ | CH₂ | Q-1.100 |
| I.2-367 | Ph | CH₃ | CH₂ | CH₂ | Q-1.101 |
| I.2-368 | Ph | CH₃ | CH₂ | CH₂ | Q-1.103 |
| I.2-369 | Ph | CH₃ | CH₂ | CH₂ | Q-1.104 |
| I.2-370 | Ph | CH₃ | CH₂ | CH₂ | Q-1.109 |
| I.2-371 | Ph | CH₃ | CH₂ | CH₂ | Q-1.110 |
| I.2-372 | Ph | CH₃ | CH₂ | CH₂ | Q-1.111 |
| I.2-373 | Ph | CH₃ | CH₂ | CH₂ | Q-1.112 |
| I.2-374 | Ph | CH₃ | CH₂ | CH₂ | Q-1.113 |
| I.2-375 | Ph | CH₃ | CH₂ | CH₂ | Q-1.114 |
| I.2-381 | Ph | C(=O)CH₃ | CH₂ | CH₂ | Q-1.13 |
| I.2-382 | Ph | C(=O)CH₃ | CH₂ | CH₂ | Q-1.31 |
| I.2-383 | Ph | C(=O)CH₃ | CH₂ | CH₂ | Q-1.61 |
| I.2-384 | Ph | C(=O)CH₃ | CH₂ | CH₂ | Q-1.97 |
| I.2-385 | Ph | C(=O)CH₃ | CH₂ | CH₂ | Q-1.98 |
| I.2-386 | Ph | C(=O)CH₃ | CH₂ | CH₂ | Q-1.100 |
| I.2-387 | Ph | C(=O)CH₃ | CH₂ | CH₂ | Q-1.101 |
| I.2-388 | Ph | C(=O)CH₃ | CH₂ | CH₂ | Q-1.103 |
| I.2-389 | Ph | C(=O)CH₃ | CH₂ | CH₂ | Q-1.104 |
| I.2-390 | Ph | C(=O)CH₃ | CH₂ | CH₂ | Q-1.109 |
| I.2-391 | Ph | C(=O)CH₃ | CH₂ | CH₂ | Q-1.110 |
| I.2-392 | Ph | C(=O)CH₃ | CH₂ | CH₂ | Q-1.111 |
| I.2-393 | Ph | C(=O)CH₃ | CH₂ | CH₂ | Q-1.112 |
| I.2-394 | Ph | C(=O)CH₃ | CH₂ | CH₂ | Q-1.113 |
| I.2-395 | Ph | C(=O)CH₃ | CH₂ | CH₂ | Q-1.114 |
| I.2-401 | Ph | SiEt₃ | CH₂ | CH₂ | Q-1.13 |
| I.2-402 | Ph | SiEt₃ | CH₂ | CH₂ | Q-1.31 |
| I.2-403 | Ph | SiEt₃ | CH₂ | CH₂ | Q-1.61 |
| I.2-404 | Ph | SiEt₃ | CH₂ | CH₂ | Q-1.97 |
| I.2-405 | Ph | SiEt₃ | CH₂ | CH₂ | Q-1.98 |
| I.2-406 | Ph | SiEt₃ | CH₂ | CH₂ | Q-1.100 |
| I.2-407 | Ph | SiEt₃ | CH₂ | CH₂ | Q-1.101 |
| I.2-408 | Ph | SiEt₃ | CH₂ | CH₂ | Q-1.103 |
| I.2-409 | Ph | SiEt₃ | CH₂ | CH₂ | Q-1.104 |
| I.2-410 | Ph | SiEt₃ | CH₂ | CH₂ | Q-1.109 |
| I.2-411 | Ph | SiEt₃ | CH₂ | CH₂ | Q-1.110 |
| I.2-412 | Ph | SiEt₃ | CH₂ | CH₂ | Q-1.111 |
| I.2-413 | Ph | SiEt₃ | CH₂ | CH₂ | Q-1.112 |
| I.2-414 | Ph | SiEt₃ | CH₂ | CH₂ | Q-1.113 |
| I.2-415 | Ph | SiEt₃ | CH₂ | CH₂ | Q-1.114 |
| I.2-421 | i-Pr | CH₃ | CH₂ | CH₂ | Q-1.13 |
| I.2-422 | i-Pr | CH₃ | CH₂ | CH₂ | Q-1.31 |
| I.2-423 | i-Pr | CH₃ | CH₂ | CH₂ | Q-1.61 |
| I.2-424 | i-Pr | CH₃ | CH₂ | CH₂ | Q-1.97 |
| I.2-425 | i-Pr | CH₃ | CH₂ | CH₂ | Q-1.98 |
| I.2-426 | i-Pr | CH₃ | CH₂ | CH₂ | Q-1.100 |
| I.2-427 | i-Pr | CH₃ | CH₂ | CH₂ | Q-1.101 |
| I.2-428 | i-Pr | CH₃ | CH₂ | CH₂ | Q-1.103 |
| I.2-429 | i-Pr | CH₃ | CH₂ | CH₂ | Q-1.104 |
| I.2-430 | i-Pr | CH₃ | CH₂ | CH₂ | Q-1.109 |
| I.2-431 | i-Pr | CH₃ | CH₂ | CH₂ | Q-1.110 |
| I.2-432 | i-Pr | CH₃ | CH₂ | CH₂ | Q-1.111 |
| I.2-433 | i-Pr | CH₃ | CH₂ | CH₂ | Q-1.112 |
| I.2-434 | i-Pr | CH₃ | CH₂ | CH₂ | Q-1.113 |
| I.2-435 | i-Pr | CH₃ | CH₂ | CH₂ | Q-1.114 |
| I.2-441 | i-Pr | C(=O)CH₃ | CH₂ | CH₂ | Q-1.13 |
| I.2-442 | i-Pr | C(=O)CH₃ | CH₂ | CH₂ | Q-1.31 |
| I.2-443 | i-Pr | C(=O)CH₃ | CH₂ | CH₂ | Q-1.61 |
| I.2-444 | i-Pr | C(=O)CH₃ | CH₂ | CH₂ | Q-1.97 |
| I.2-445 | i-Pr | C(=O)CH₃ | CH₂ | CH₂ | Q-1.98 |
| I.2-446 | i-Pr | C(=O)CH₃ | CH₂ | CH₂ | Q-1.100 |
| I.2-447 | i-Pr | C(=O)CH₃ | CH₂ | CH₂ | Q-1.101 |
| I.2-448 | i-Pr | C(=O)CH₃ | CH₂ | CH₂ | Q-1.103 |
| I.2-449 | i-Pr | C(=O)CH₃ | CH₂ | CH₂ | Q-1.104 |
| I.2-450 | i-Pr | C(=O)CH₃ | CH₂ | CH₂ | Q-1.109 |
| I.2-451 | i-Pr | C(=O)CH₃ | CH₂ | CH₂ | Q-1.110 |
| I.2-452 | i-Pr | C(=O)CH₃ | CH₂ | CH₂ | Q-1.111 |
| I.2-453 | i-Pr | C(=O)CH₃ | CH₂ | CH₂ | Q-1.112 |
| I.2-454 | i-Pr | C(=O)CH₃ | CH₂ | CH₂ | Q-1.113 |
| I.2-455 | i-Pr | C(=O)CH₃ | CH₂ | CH₂ | Q-1.114 |
| I.2-461 | i-Pr | SiEt₃ | CH₂ | CH₂ | Q-1.13 |
| I.2-462 | i-Pr | SiEt₃ | CH₂ | CH₂ | Q-1.31 |
| I.2-463 | i-Pr | SiEt₃ | CH₂ | CH₂ | Q-1.61 |
| I.2-464 | i-Pr | SiEt₃ | CH₂ | CH₂ | Q-1.97 |
| I.2-465 | i-Pr | SiEt₃ | CH₂ | CH₂ | Q-1.98 |
| I.2-466 | i-Pr | SiEt₃ | CH₂ | CH₂ | Q-1.100 |
| I.2-467 | i-Pr | SiEt₃ | CH₂ | CH₂ | Q-1.101 |
| I.2-468 | i-Pr | SiEt₃ | CH₂ | CH₂ | Q-1.103 |
| I.2-469 | i-Pr | SiEt₃ | CH₂ | CH₂ | Q-1.104 |
| I.2-470 | i-Pr | SiEt₃ | CH₂ | CH₂ | Q-1.109 |
| I.2-471 | i-Pr | SiEt₃ | CH₂ | CH₂ | Q-1.110 |
| I.2-472 | i-Pr | SiEt₃ | CH₂ | CH₂ | Q-1.111 |
| I.2-473 | i-Pr | SiEt₃ | CH₂ | CH₂ | Q-1.112 |
| I.2-474 | i-Pr | SiEt₃ | CH₂ | CH₂ | Q-1.113 |
| I.2-475 | i-Pr | SiEt₃ | CH₂ | CH₂ | Q-1.114 |
| I.2-481 | i-Pr | CH₃ | CHF | CH₂ | Q-1.97 |
| I.2-482 | i-Pr | CH₃ | CHF | CH₂ | Q-1.100 |
| I.2-483 | i-Pr | CH₃ | CHF | CH₂ | Q-1.113 |
| I.2-484 | Ph | CH₃ | CHF | CH₂ | Q-1.97 |
| I.2-485 | Ph | CH₃ | CHF | CH₂ | Q-1.100 |
| I.2-486 | Ph | CH₃ | CHF | CH₂ | Q-1.113 |
| I.2-487 | CH₃ | CH₃ | CHF | CH₂ | Q-1.97 |
| I.2-488 | CH₃ | CH₃ | CHF | CH₂ | Q-1.100 |
| I.2-489 | CH₃ | CH₃ | CHF | CH₂ | Q-1.113 |
| I.2-490 | i-Pr | CH₃ | CH(CH₃) | CH₂ | Q-1.97 |
| I.2-491 | i-Pr | CH₃ | CH(CH₃) | CH₂ | Q-1.100 |
| I.2-492 | i-Pr | CH₃ | CH(CH₃) | CH₂ | Q-1.113 |
| I.2-493 | Ph | CH₃ | CH(CH₃) | CH₂ | Q-1.97 |
| I.2-494 | Ph | CH₃ | CH(CH₃) | CH₂ | Q-1.100 |
| I.2-495 | Ph | CH₃ | CH(CH₃) | CH₂ | Q-1.113 |
| I.2-496 | CH₃ | CH₃ | CH(CH₃) | CH₂ | Q-1.97 |
| I.2-497 | CH₃ | CH₃ | CH(CH₃) | CH₂ | Q-1.100 |
| I.2-498 | CH₃ | CH₃ | CH(CH₃) | CH₂ | Q-1.113 |
| I.2-499 | i-Pr | CH₃ | CH(i-Pr) | CH₂ | Q-1.97 |
| 1.2-500 | i-Pr | CH₃ | CH(i-Pr) | CH₂ | Q-1.100 |
| I.2-501 | c-Pr | H | CH₂ | CH₂ | Q-1.31 |
| I.2-502 | c-Pr | H | CH₂ | CH₂ | Q-1.64 |
| I.2-503 | c-Pr | H | CH₂ | CH₂ | Q-1.65 |
| I.2-504 | c-Pr | H | CH₂ | CH₂ | Q-1.66 |
| I.2-505 | c-Pr | H | CH₂ | CH₂ | Q-1.97 |
| I.2-506 | c-Pr | H | CH₂ | CH₂ | Q-1.98 |
| I.2-507 | c-Pr | H | CH₂ | CH₂ | Q-1.99 |
| I.2-508 | c-Pr | H | CH₂ | CH₂ | Q-1.100 |
| I.2-509 | c-Pr | H | CH₂ | CH₂ | Q-1.101 |
| I.2-510 | c-Pr | H | CH₂ | CH₂ | Q-1.102 |
| I.2-511 | c-Bu | H | CH₂ | CH₂ | Q-1.31 |
| I.2-512 | c-Bu | H | CH₂ | CH₂ | Q-1.64 |
| I.2-513 | c-Bu | H | CH₂ | CH₂ | Q-1.65 |
| I.2-514 | c-Bu | H | CH₂ | CH₂ | Q-1.66 |
| I.2-515 | c-Bu | H | CH₂ | CH₂ | Q-1.97 |
| I.2-516 | c-Bu | H | CH₂ | CH₂ | Q-1.98 |
| I.2-517 | c-Bu | H | CH₂ | CH₂ | Q-1.99 |
| I.2-518 | c-Bu | H | CH₂ | CH₂ | Q-1.100 |
| I.2-519 | c-Bu | H | CH₂ | CH₂ | Q-1.101 |
| I.2-520 | c-Bu | H | CH₂ | CH₂ | Q-1.102 |
| I.2-521 | c-Hex | H | CH₂ | CH₂ | Q-1.31 |
| I.2-522 | c-Hex | H | CH₂ | CH₂ | Q-1.64 |
| I.2-523 | c-Hex | H | CH₂ | CH₂ | Q-1.65 |
| I.2-524 | c-Hex | H | CH₂ | CH₂ | Q-1.66 |
| I.2-525 | c-Hex | H | CH₂ | CH₂ | Q-1.97 |
| I.2-526 | c-Hex | H | CH₂ | CH₂ | Q-1.98 |
| I.2-527 | c-Hex | H | CH₂ | CH₂ | Q-1.99 |
| I.2-528 | c-Hex | H | CH₂ | CH₂ | Q-1.100 |
| I.2-529 | c-Hex | H | CH₂ | CH₂ | Q-1.101 |
| I.2-530 | c-Hex | H | CH₂ | CH₂ | Q-1.102 |
| I.2-531 | 1-Ethylpropyl | H | CH₂ | CH₂ | Q-1.31 |
| I.2-532 | 1-Ethylpropyl | H | CH₂ | CH₂ | Q-1.64 |
| I.2-533 | 1-Ethylpropyl | H | CH₂ | CH₂ | Q-1.65 |
| I.2-534 | 1-Ethylpropyl | H | CH₂ | CH₂ | Q-1.66 |
| I.2-535 | 1-Ethylpropyl | H | CH₂ | CH₂ | Q-1.97 |
| I.2-536 | 1-Ethylpropyl | H | CH₂ | CH₂ | Q-1.98 |
| I.2-537 | 1-Ethylpropyl | H | CH₂ | CH₂ | Q-1.99 |
| I.2-538 | 1-Ethylpropyl | H | CH₂ | CH₂ | Q-1.100 |
| I.2-539 | 1-Ethylpropyl | H | CH₂ | CH₂ | Q-1.101 |
| I.2-540 | 1-Ethylpropyl | H | CH₂ | CH₂ | Q-1.102 |
| I.2-541 | Adamantyl | H | CH₂ | CH₂ | Q-1.31 |
| I.2-542 | Adamantyl | H | CH₂ | CH₂ | Q-1.64 |
| I.2-543 | Adamantyl | H | CH₂ | CH₂ | Q-1.65 |
| I.2-544 | Adamantyl | H | CH₂ | CH₂ | Q-1.66 |
| I.2-545 | Adamantyl | H | CH₂ | CH₂ | Q-1.97 |
| I.2-546 | Adamantyl | H | CH₂ | CH₂ | Q-1.98 |
| I.2-547 | Adamantyl | H | CH₂ | CH₂ | Q-1.99 |
| I.2-548 | Adamantyl | H | CH₂ | CH₂ | Q-1.100 |
| I.2-549 | Adamantyl | H | CH₂ | CH₂ | Q-1.101 |
| I.2-550 | Adamantyl | H | CH₂ | CH₂ | Q-1.102 |
| I.2-551 | 3-F-Ph | H | CH₂ | CH₂ | Q-1.31 |
| I.2-552 | 3-F-Ph | H | CH₂ | CH₂ | Q-1.64 |
| I.2-553 | 3-F-Ph | H | CH₂ | CH₂ | Q-1.65 |
| I.2-554 | 3-F-Ph | H | CH₂ | CH₂ | Q-1.66 |
| I.2-555 | 3-F-Ph | H | CH₂ | CH₂ | Q-1.97 |
| I.2-556 | 3-F-Ph | H | CH₂ | CH₂ | Q-1.98 |
| I.2-557 | 3-F-Ph | H | CH₂ | CH₂ | Q-1.99 |
| I.2-558 | 3-F-Ph | H | CH₂ | CH₂ | Q-1.100 |
| I.2-559 | 3-F-Ph | H | CH₂ | CH₂ | Q-1.101 |
| I.2-560 | 3-F-Ph | H | CH₂ | CH₂ | Q-1.102 |
| I.2-561 | 4-CH₃-Ph | H | CH₂ | CH₂ | Q-1.31 |
| I.2-562 | 4-CH₃-Ph | H | CH₂ | CH₂ | Q-1.64 |
| I.2-563 | 4-CH₃-Ph | H | CH₂ | CH₂ | Q-1.65 |
| I.2-564 | 4-CH₃-Ph | H | CH₂ | CH₂ | Q-1.66 |
| I.2-565 | 4-CH₃-Ph | H | CH₂ | CH₂ | Q-1.97 |
| I.2-566 | 4-CH₃-Ph | H | CH₂ | CH₂ | Q-1.98 |
| I.2-567 | 4-CH₃-Ph | H | CH₂ | CH₂ | Q-1.99 |
| I.2-568 | 4-CH₃-Ph | H | CH₂ | CH₂ | Q-1.100 |
| I.2-569 | 4-CH₃-Ph | H | CH₂ | CH₂ | Q-1.101 |
| I.2-570 | 4-CH₃-Ph | H | CH₂ | CH₂ | Q-1.102 |
| I.2-571 | c-Pent | H | CH₂ | CH₂ | Q-1.31 |
| I.2-572 | c-Pent | H | CH₂ | CH₂ | Q-1.64 |
| I.2-573 | c-Pent | H | CH₂ | CH₂ | Q-1.65 |
| I.2-574 | c-Pent | H | CH₂ | CH₂ | Q-1.66 |
| I.2-575 | c-Pent | H | CH₂ | CH₂ | Q-1.97 |
| I.2-576 | c-Pent | H | CH₂ | CH₂ | Q-1.98 |
| I.2-577 | c-Pent | H | CH₂ | CH₂ | Q-1.99 |
| I.2-578 | c-Pent | H | CH₂ | CH₂ | Q-1.100 |
| I.2-579 | c-Pent | H | CH₂ | CH₂ | Q-1.101 |
| I.2-580 | c-Pent | H | CH₂ | CH₂ | Q-1.102 |
| I.2-581 | 2-F-Ph | H | CH₂ | CH₂ | Q-1.31 |
| I.2-582 | 2-F-Ph | H | CH₂ | CH₂ | Q-1.64 |
| I.2-583 | 2-F-Ph | H | CH₂ | CH₂ | Q-1.65 |
| I.2-584 | 2-F-Ph | H | CH₂ | CH₂ | Q-1.66 |
| I.2-585 | 2-F-Ph | H | CH₂ | CH₂ | Q-1.97 |
| I.2-586 | 2-F-Ph | H | CH₂ | CH₂ | Q-1.98 |
| I.2-587 | 2-F-Ph | H | CH₂ | CH₂ | Q-1.99 |
| I.2-588 | 2-F-Ph | H | CH₂ | CH₂ | Q-1.100 |
| I.2-589 | 2-F-Ph | H | CH₂ | CH₂ | Q-1.101 |
| I.2-590 | 2-F-Ph | H | CH₂ | CH₂ | Q-1.102 |
| I.2-591 | 4-Cl-Ph | H | CH₂ | CH₂ | Q-1.31 |
| I.2-592 | 4-ClPh | H | CH₂ | CH₂ | Q-1.64 |
| I.2-593 | 4-Cl-Ph | H | CH₂ | CH₂ | Q-1.65 |
| I.2-594 | 4-Cl-Ph | H | CH₂ | CH₂ | Q-1.66 |
| I.2-595 | 4-Cl-Ph | H | CH₂ | CH₂ | Q-1.97 |
| I.2-596 | 4-Cl-Ph | H | CH₂ | CH₂ | Q-1.98 |
| I.2-597 | 4-Cl-Ph | H | CH₂ | CH₂ | Q-1.99 |
| I.2-598 | 4-Cl-Ph | H | CH₂ | CH₂ | Q-1.100 |
| I.2-599 | 4-Cl-Ph | H | CH₂ | CH₂ | Q-1.101 |
| I.2-600 | 4-Cl-Ph | H | CH₂ | CH₂ | Q-1.102 |
| I.2-601 | t-Bu | H | CH₂ | CH₂ | Q-1.31 |
| I.2-602 | t-Bu | H | CH₂ | CH₂ | Q-1.64 |
| I.2-603 | t-Bu | H | CH₂ | CH₂ | Q-1.65 |
| I.2-604 | t-Bu | H | CH₂ | CH₂ | Q-1.66 |
| I.2-605 | t-Bu | H | CH₂ | CH₂ | Q-1.97 |
| I.2-606 | t-Bu | H | CH₂ | CH₂ | Q-1.98 |
| I.2-607 | t-Bu | H | CH₂ | CH₂ | Q-1.99 |
| I.2-608 | t-Bu | H | CH₂ | CH₂ | Q-1.100 |
| I.2-609 | t-Bu | H | CH₂ | CH₂ | Q-1.101 |
| I.2-610 | t-Bu | H | CH₂ | CH₂ | Q-1.102 |
| I.2-611 | 1-Ethylpropyl | H | CH₂ | CH₂ | Q-1.7 |
| I.2-612 | 1-Ethylpropyl | H | CH₂ | CH₂ | Q-1.13 |

**Tabelle 3:**

| | | | | | | |
|---|---|---|---|---|---|---|
| 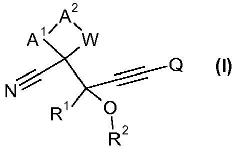 | | | Q entspricht jeweils einer der weiter vorne als besonders bevorzugt definierten Struktur | | | |

| No. | R¹ | R² | A¹ | A² | W | Q |
|---|---|---|---|---|---|---|
| I.3-1 | CH₃ | H | CH₂ | CH₂ | CH₂ | Q-1.31 |
| I.3-2 | CH₃ | H | CH₂ | CH₂ | CH₂ | Q-1.61 |
| I.3-3 | CH₃ | H | CH₂ | CH₂ | CH₂ | Q-1.97 |
| I.3-4 | CH₃ | H | CH₂ | CH₂ | CH₂ | Q-1.98 |
| I.3-5 | CH₃ | H | CH₂ | CH₂ | CH₂ | Q-1.100 |
| I.3-6 | CH₃ | H | CH₂ | CH₂ | CH₂ | Q-1.101 |
| I.3-7 | CH₃ | H | CH₂ | CH₂ | CH₂ | Q-1.103 |
| I.3-8 | CH₃ | H | CH₂ | CH₂ | CH₂ | Q-1.104 |
| I.3-9 | CH₃ | H | CH₂ | CH₂ | CH₂ | Q-1.109 |
| I.3-10 | CH₃ | H | CH₂ | CH₂ | CH₂ | Q-1.110 |
| I.3-11 | CH₃ | H | CH₂ | CH₂ | CH₂ | Q-1.111 |
| I.3-12 | CH₃ | H | CH₂ | CH₂ | CH₂ | Q-1.112 |
| I.3-13 | CH₃ | H | CH₂ | CH₂ | CH₂ | Q-1.113 |
| I.3-14 | CH₃ | H | CH₂ | CH₂ | CH₂ | Q-1.114 |
| I.3-15 | CH₃ | H | CH₂ | CH₂ | CH₂ | Q-2.3 |
| I.3-16 | CH₃ | H | CH₂ | CH₂ | CH₂ | Q-2.4 |
| I.3-22 | i-Pr | H | CH₂ | CH₂ | CH₂ | Q-1.31 |
| I.3-23 | i-Pr | H | CH₂ | CH₂ | CH₂ | Q-1.61 |
| I.3-24 | i-Pr | H | CH₂ | CH₂ | CH₂ | Q-1.97 |
| I.3-25 | i-Pr | H | CH₂ | CH₂ | CH₂ | Q-1.98 |
| I.3-26 | i-Pr | H | CH₂ | CH₂ | CH₂ | Q-1.100 |
| I.3-27 | i-Pr | H | CH₂ | CH₂ | CH₂ | Q-1.101 |
| I.3-28 | i-Pr | H | CH₂ | CH₂ | CH₂ | Q-1.103 |
| I.3-29 | i-Pr | H | CH₂ | CH₂ | CH₂ | Q-1.104 |
| I.3-30 | i-Pr | H | CH₂ | CH₂ | CH₂ | Q-1.109 |
| I.3-31 | i-Pr | H | CH₂ | CH₂ | CH₂ | Q-1.110 |
| I.3-32 | i-Pr | H | CH₂ | CH₂ | CH₂ | Q-1.111 |
| I.3-33 | i-Pr | H | CH₂ | CH₂ | CH₂ | Q-1.112 |
| I.3-34 | i-Pr | H | CH₂ | CH₂ | CH₂ | Q-1.113 |
| I.3-35 | i-Pr | H | CH₂ | CH₂ | CH₂ | Q-1.114 |
| I.3-36 | i-Pr | H | CH₂ | CH₂ | CH₂ | Q-2.3 |
| I.3-37 | i-Pr | H | CH₂ | CH₂ | CH₂ | Q-2.4 |
| I.3-43 | Ph | H | CH₂ | CH₂ | CH₂ | Q-1.31 |
| I.3-44 | Ph | H | CH₂ | CH₂ | CH₂ | Q-1.61 |
| I.3-45 | Ph | H | CH₂ | CH₂ | CH₂ | Q-1.97 |
| I.3-46 | Ph | H | CH₂ | CH₂ | CH₂ | Q-1.98 |
| I.3-47 | Ph | H | CH₂ | CH₂ | CH₂ | Q-1.100 |
| I.3-48 | Ph | H | CH₂ | CH₂ | CH₂ | Q-1.101 |
| I.3-49 | Ph | H | CH₂ | CH₂ | CH₂ | Q-1.103 |
| I.3-50 | Ph | H | CH₂ | CH₂ | CH₂ | Q-1.104 |
| I.3-51 | Ph | H | CH₂ | CH₂ | CH₂ | Q-1.109 |
| I.3-52 | Ph | H | CH₂ | CH₂ | CH₂ | Q-1.110 |
| I.3-53 | Ph | H | CH₂ | CH₂ | CH₂ | Q-1.111 |
| I.3-54 | Ph | H | CH₂ | CH₂ | CH₂ | Q-1.112 |
| I.3-55 | Ph | H | CH₂ | CH₂ | CH₂ | Q-1.113 |
| I.3-56 | Ph | H | CH₂ | CH₂ | CH₂ | Q-1.114 |
| I.3-57 | Ph | H | CH₂ | CH₂ | CH₂ | Q-2.3 |
| I.3-58 | Ph | H | CH₂ | CH₂ | CH₂ | Q-2.4 |
| I.3-64 | CH₃ | H | CH₂ | O | CH₂ | Q-1.31 |
| I.3-65 | CH₃ | H | CH₂ | O | CH₂ | Q-1.61 |
| I.3-66 | CH₃ | H | CH₂ | O | CH₂ | Q-1.97 |
| I.3-67 | CH₃ | H | CH₂ | O | CH₂ | Q-1.98 |
| I.3-68 | CH₃ | H | CH₂ | O | CH₂ | Q-1.100 |
| I.3-69 | CH₃ | H | CH₂ | O | CH₂ | Q-1.101 |
| I.3-70 | CH₃ | H | CH₂ | O | CH₂ | Q-1.103 |
| I.3-71 | CH₃ | H | CH₂ | O | CH₂ | Q-1.104 |
| I.3-72 | CH₃ | H | CH₂ | O | CH₂ | Q-1.109 |
| I.3-73 | CH₃ | H | CH₂ | O | CH₂ | Q-1.110 |
| I.3-74 | CH₃ | H | CH₂ | O | CH₂ | Q-1.111 |
| I.3-75 | CH₃ | H | CH₂ | O | CH₂ | Q-1.112 |
| I.3-76 | CH₃ | H | CH₂ | O | CH₂ | Q-1.113 |
| I.3-77 | CH₃ | H | CH₂ | O | CH₂ | Q-1.114 |
| I.3-78 | CH₃ | H | CH₂ | O | CH₂ | Q-2.3 |
| I.3-79 | CH₃ | H | CH₂ | O | CH₂ | Q-2.4 |
| I.3-85 | i-Pr | H | CH₂ | O | CH₂ | Q-1.31 |
| I.3-86 | i-Pr | H | CH₂ | O | CH₂ | Q-1.61 |
| I.3-87 | i-Pr | H | CH₂ | O | CH₂ | Q-1.97 |
| I.3-88 | i-Pr | H | CH₂ | O | CH₂ | Q-1.98 |
| I.3-89 | i-Pr | H | CH₂ | O | CH₂ | Q-1.100 |
| I.3-90 | i-Pr | H | CH₂ | O | CH₂ | Q-1.101 |
| I.3-91 | i-Pr | H | CH₂ | O | CH₂ | Q-1.103 |
| I.3-92 | i-Pr | H | CH₂ | O | CH₂ | Q-1.104 |
| I.3-93 | i-Pr | H | CH₂ | O | CH₂ | Q-1.109 |
| I.3-94 | i-Pr | H | CH₂ | O | CH₂ | Q-1.110 |
| I.3-95 | i-Pr | H | CH₂ | O | CH₂ | Q-1.111 |
| I.3-96 | i-Pr | H | CH₂ | O | CH₂ | Q-1.112 |
| I.3-97 | i-Pr | H | CH₂ | O | CH₂ | Q-1.113 |
| I.3-98 | i-Pr | H | CH₂ | O | CH₂ | Q-1.114 |
| I.3-99 | i-Pr | H | CH₂ | O | CH₂ | Q-2.3 |
| I.3-100 | i-Pr | H | CH₂ | O | CH₂ | Q-2.4 |
| I.3-106 | Ph | H | CH₂ | O | CH₂ | Q-1.31 |
| I.3-107 | Ph | H | CH₂ | O | CH₂ | Q-1.61 |
| I.3-108 | Ph | H | CH₂ | O | CH₂ | Q-1.97 |
| I.3-109 | Ph | H | CH₂ | O | CH₂ | Q-1.98 |
| I.3-110 | Ph | H | CH₂ | O | CH₂ | Q-1.100 |
| I.3-111 | Ph | H | CH₂ | O | CH₂ | Q-1.101 |
| I.3-112 | Ph | H | CH₂ | O | CH₂ | Q-1.103 |
| I.3-113 | Ph | H | CH₂ | O | CH₂ | Q-1.104 |
| I.3-114 | Ph | H | CH₂ | O | CH₂ | Q-1.109 |
| I.3-115 | Ph | H | CH₂ | O | CH₂ | Q-1.110 |
| I.3-116 | Ph | H | CH₂ | O | CH₂ | Q-1.111 |
| I.3-117 | Ph | H | CH₂ | O | CH₂ | Q-1.112 |
| I.3-118 | Ph | H | CH₂ | O | CH₂ | Q-1.113 |
| I.3-119 | Ph | H | CH₂ | O | CH₂ | Q-1.114 |
| I.3-120 | Ph | H | CH₂ | O | CH₂ | Q-2.3 |
| I.3-121 | Ph | H | CH₂ | O | CH₂ | Q-2.4 |

**Tabelle 4:**

| | | | | | | |
|---|---|---|---|---|---|---|
| 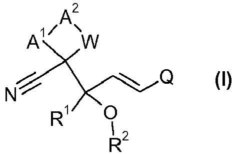 | | | Q entspricht jeweils einer der weiter vorne als besonders bevorzugt definierten Struktur | | | |

| No. | R¹ | R² | A¹ | A² | W | Q |
|---|---|---|---|---|---|---|
| I.4-1 | CH₃ | H | CH₂ | CH₂ | CH₂ | Q-1.31 |
| I.4-2 | CH₃ | H | CH₂ | CH₂ | CH₂ | Q-1.61 |
| I.4-3 | CH₃ | H | CH₂ | CH₂ | CH₂ | Q-1.97 |
| I.4-4 | CH₃ | H | CH₂ | CH₂ | CH₂ | Q-1.98 |
| I.4-5 | CH₃ | H | CH₂ | CH₂ | CH₂ | Q-1.100 |
| I.4-6 | CH₃ | H | CH₂ | CH₂ | CH₂ | Q-1.101 |
| I.4-7 | CH₃ | H | CH₂ | CH₂ | CH₂ | Q-1.103 |
| I.4-8 | CH₃ | H | CH₂ | CH₂ | CH₂ | Q-1.104 |
| I.4-9 | CH₃ | H | CH₂ | CH₂ | CH₂ | Q-1.109 |
| I.4-10 | CH₃ | H | CH₂ | CH₂ | CH₂ | Q-1.110 |
| I.4-11 | CH₃ | H | CH₂ | CH₂ | CH₂ | Q-1.111 |
| I.4-12 | CH₃ | H | CH₂ | CH₂ | CH₂ | Q-1.112 |
| I.4-13 | CH₃ | H | CH₂ | CH₂ | CH₂ | Q-1.113 |
| I.4-14 | CH₃ | H | CH₂ | CH₂ | CH₂ | Q-1.114 |
| I.4-15 | CH₃ | H | CH₂ | CH₂ | CH₂ | Q-2.3 |
| I.4-16 | CH₃ | H | CH₂ | CH₂ | CH₂ | Q-2.4 |
| I.4-22 | i-Pr | H | CH₂ | CH₂ | CH₂ | Q-1.31 |
| I.4-23 | i-Pr | H | CH₂ | CH₂ | CH₂ | Q-1.61 |
| I.4-24 | i-Pr | H | CH₂ | CH₂ | CH₂ | Q-1.97 |
| I.4-25 | i-Pr | H | CH₂ | CH₂ | CH₂ | Q-1.98 |
| I.4-26 | i-Pr | H | CH₂ | CH₂ | CH₂ | Q-1.100 |
| I.4-27 | i-Pr | H | CH₂ | CH₂ | CH₂ | Q-1.101 |
| I.4-28 | i-Pr | H | CH₂ | CH₂ | CH₂ | Q-1.103 |
| I.4-29 | i-Pr | H | CH₂ | CH₂ | CH₂ | Q-1.104 |
| I.4-30 | i-Pr | H | CH₂ | CH₂ | CH₂ | Q-1.109 |
| I.4-31 | i-Pr | H | CH₂ | CH₂ | CH₂ | Q-1.110 |
| I.4-32 | i-Pr | H | CH₂ | CH₂ | CH₂ | Q-1.111 |
| I.4-33 | i-Pr | H | CH₂ | CH₂ | CH₂ | Q-1.112 |
| I.4-34 | i-Pr | H | CH₂ | CH₂ | CH₂ | Q-1.113 |
| I.4-35 | i-Pr | H | CH₂ | CH₂ | CH₂ | Q-1.114 |
| I.4-36 | i-Pr | H | CH₂ | CH₂ | CH₂ | Q-2.3 |
| I.4-37 | i-Pr | H | CH₂ | CH₂ | CH₂ | Q-2.4 |
| I.4-43 | Ph | H | CH₂ | CH₂ | CH₂ | Q-1.31 |
| I.4-44 | Ph | H | CH₂ | CH₂ | CH₂ | Q-1.61 |
| I.4-45 | Ph | H | CH₂ | CH₂ | CH₂ | Q-1.97 |
| I.4-46 | Ph | H | CH₂ | CH₂ | CH₂ | Q-1.98 |
| I.4-47 | Ph | H | CH₂ | CH₂ | CH₂ | Q-1.100 |
| I.4-48 | Ph | H | CH₂ | CH₂ | CH₂ | Q-1.101 |
| I.4-49 | Ph | H | CH₂ | CH₂ | CH₂ | Q-1.103 |
| I.4-50 | Ph | H | CH₂ | CH₂ | CH₂ | Q-1.104 |
| I.4-51 | Ph | H | CH₂ | CH₂ | CH₂ | Q-1.109 |
| I.4-52 | Ph | H | CH₂ | CH₂ | CH₂ | Q-1.110 |
| I.4-53 | Ph | H | CH₂ | CH₂ | CH₂ | Q-1.111 |
| I.4-54 | Ph | H | CH₂ | CH₂ | CH₂ | Q-1.112 |
| I.4-55 | Ph | H | CH₂ | CH₂ | CH₂ | Q-1.113 |
| I.4-56 | Ph | H | CH₂ | CH₂ | CH₂ | Q-1.114 |
| I.4-57 | Ph | H | CH₂ | CH₂ | CH₂ | Q-2.3 |
| I.4-58 | Ph | H | CH₂ | CH₂ | CH₂ | Q-2.4 |
| I.4-64 | CH₃ | H | CH₂ | O | CH₂ | Q-1.31 |
| I.4-65 | CH₃ | H | CH₂ | O | CH₂ | Q-1.61 |
| I.4-66 | CH₃ | H | CH₂ | O | CH₂ | Q-1.97 |
| I.4-67 | CH₃ | H | CH₂ | O | CH₂ | Q-1.98 |
| I.4-68 | CH₃ | H | CH₂ | O | CH₂ | Q-1.100 |
| I.4-69 | CH₃ | H | CH₂ | O | CH₂ | Q-1.101 |
| I.4-70 | CH₃ | H | CH₂ | O | CH₂ | Q-1.103 |
| I.4-71 | CH₃ | H | CH₂ | O | CH₂ | Q-1.104 |
| I.4-72 | CH₃ | H | CH₂ | O | CH₂ | Q-1.109 |
| I.4-73 | CH₃ | H | CH₂ | O | CH₂ | Q-1.110 |
| I.4-74 | CH₃ | H | CH₂ | O | CH₂ | Q-1.111 |
| I.4-75 | CH₃ | H | CH₂ | O | CH₂ | Q-1.112 |
| I.4-76 | CH₃ | H | CH₂ | O | CH₂ | Q-1.113 |
| I.4-77 | CH₃ | H | CH₂ | O | CH₂ | Q-1.114 |
| I.4-78 | CH₃ | H | CH₂ | O | CH₂ | Q-2.3 |
| I.4-79 | CH₃ | H | CH₂ | O | CH₂ | Q-2.4 |
| I.4-85 | i-Pr | H | CH₂ | O | CH₂ | Q-1.31 |
| I.4-86 | i-Pr | H | CH₂ | O | CH₂ | Q-1.61 |
| I.4-87 | i-Pr | H | CH₂ | O | CH₂ | Q-1.97 |
| I.4-88 | i-Pr | H | CH₂ | O | CH₂ | Q-1.98 |
| I.4-89 | i-Pr | H | CH₂ | O | CH₂ | Q-1.100 |
| I.4-90 | i-Pr | H | CH₂ | O | CH₂ | Q-1.101 |
| I.4-91 | i-Pr | H | CH₂ | O | CH₂ | Q-1.103 |
| I.4-92 | i-Pr | H | CH₂ | O | CH₂ | Q-1.104 |
| I.4-93 | i-Pr | H | CH₂ | O | CH₂ | Q-1.109 |
| I.4-94 | i-Pr | H | CH₂ | O | CH₂ | Q-1.110 |
| I.4-95 | i-Pr | H | CH₂ | O | CH₂ | Q-1.111 |
| I.4-96 | i-Pr | H | CH₂ | O | CH₂ | Q-1.112 |
| I.4-97 | i-Pr | H | CH₂ | O | CH₂ | Q-1.113 |
| I.4-98 | i-Pr | H | CH₂ | O | CH₂ | Q-1.114 |
| I.4-99 | i-Pr | H | CH₂ | O | CH₂ | Q-2.3 |
| I.4-100 | i-Pr | H | CH₂ | O | CH₂ | Q-2.4 |
| I.4-106 | Ph | H | CH₂ | O | CH₂ | Q-1.31 |
| I.4-107 | Ph | H | CH₂ | O | CH₂ | Q-1.61 |
| I.4-108 | Ph | H | CH₂ | O | CH₂ | Q-1.97 |
| I.4-109 | Ph | H | CH₂ | O | CH₂ | Q-1.98 |
| I.4-110 | Ph | H | CH₂ | O | CH₂ | Q-1.100 |
| I.4-111 | Ph | H | CH₂ | O | CH₂ | Q-1.101 |
| I.4-112 | Ph | H | CH₂ | O | CH₂ | Q-1.103 |
| I.4-113 | Ph | H | CH₂ | O | CH₂ | Q-1.104 |
| I.4-114 | Ph | H | CH₂ | O | CH₂ | Q-1.109 |
| I.4-115 | Ph | H | CH₂ | O | CH₂ | Q-1.110 |
| I.4-116 | Ph | H | CH₂ | O | CH₂ | Q-1.111 |
| I.4-117 | Ph | H | CH₂ | O | CH₂ | Q-1.112 |
| I.4-118 | Ph | H | CH₂ | O | CH₂ | Q-1.113 |
| I.4-119 | Ph | H | CH₂ | O | CH₂ | Q-1.114 |
| I.4-120 | Ph | H | CH₂ | O | CH₂ | Q-2.3 |
| I.4-121 | Ph | H | CH₂ | O | CH₂ | Q-2.4 |

**Tabelle 5:**

| | | | | |
|---|---|---|---|---|
| 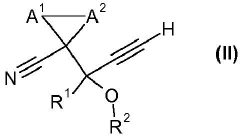 | | | | |

| No. | R¹ | R² | A¹ | A² |
|---|---|---|---|---|
| II.1-1 | CH₃ | H | CH₂ | CH₂ |
| II.1-2 | i-Pr | H | CH₂ | CH₂ |
| II.1-3 | Ph | H | CH₂ | CH₂ |
| II.1-4 | Et | H | CH₂ | CH₂ |
| II.1-5 | c-Pr | H | CH₂ | CH₂ |
| II.1-6 | n-Pr | H | CH₂ | CH₂ |
| II.1-7 | p-F-Ph | H | CH₂ | CH₂ |
| II.1-8 | m-F-Ph | H | CH₂ | CH₂ |
| II.1-9 | p-Cl-Ph | H | CH₂ | CH₂ |
| II.1-10 | m-Cl-Ph | H | CH₂ | CH₂ |
| II.1-11 | p-CH₃-Ph | H | CH₂ | CH₂ |
| II.1-12 | m-CH₃-Ph | H | CH₂ | CH₂ |
| II.1-13 | CH₃ | CH₃ | CH₂ | CH₂ |
| II.1-14 | i-Pr | CH₃ | CH₂ | CH₂ |
| II.1-15 | Ph | CH₃ | CH₂ | CH₂ |
| II.1-16 | Et | CH₃ | CH₂ | CH₂ |
| II.1-17 | c-Pr | CH₃ | CH₂ | CH₂ |
| II.1-18 | n-Pr | CH₃ | CH₂ | CH₂ |
| II.1-19 | p-F-Ph | CH₃ | CH₂ | CH₂ |
| II.1-20 | m-F-Ph | CH₃ | CH₂ | CH₂ |
| II.1-21 | p-Cl-Ph | CH₃ | CH₂ | CH₂ |
| II.1-22 | m-Cl-Ph | CH₃ | CH₂ | CH₂ |
| II.1-23 | p-CH₃-Ph | CH₃ | CH₂ | CH₂ |
| II.1-24 | m-CH₃-Ph | CH₃ | CH₂ | CH₂ |
| II.1-25 | CH₃ | C(=O)CH₃ | CH₂ | CH₂ |
| II.1-26 | i-Pr | C(=O)CH₃ | CH₂ | CH₂ |
| II.1-27 | Ph | C(=O)CH₃ | CH₂ | CH₂ |
| II.1-28 | Et | C(=O)CH₃ | CH₂ | CH₂ |
| II.1-29 | c-Pr | C(=O)CH₃ | CH₂ | CH₂ |
| II.1-30 | n-Pr | C(=O)CH₃ | CH₂ | CH₂ |
| II.1-31 | p-F-Ph | C(=O)CH₃ | CH₂ | CH₂ |
| II.1-32 | m-F-Ph | C(=O)CH₃ | CH₂ | CH₂ |
| II.1-33 | p-Cl-Ph | C(=O)CH₃ | CH₂ | CH₂ |
| II.1-34 | m-Cl-Ph | C(=O)CH₃ | CH₂ | CH₂ |
| II.1-35 | p-CH₃-Ph | C(=O)CH₃ | CH₂ | CH₂ |
| II.1-36 | m-CH₃-Ph | C(=O)CH₃ | CH₂ | CH₂ |
| II.1-37 | CH₃ | SiEt₃ | CH₂ | CH₂ |
| II.1-38 | i-Pr | SiEt₃ | CH₂ | CH₂ |
| II.1-39 | Ph | SiEt₃ | CH₂ | CH₂ |
| II.1-40 | Et | SiEt₃ | CH₂ | CH₂ |
| II.1-41 | c-Pr | SiEt₃ | CH₂ | CH₂ |
| II.1-42 | n-Pr | SiEt₃ | CH₂ | CH₂ |
| II.1-43 | p-F-Ph | SiEt₃ | CH₂ | CH₂ |
| II.1-44 | m-F-Ph | SiEt₃ | CH₂ | CH₂ |
| II.1-45 | p-Cl-Ph | SiEt₃ | CH₂ | CH₂ |
| II.1-46 | m-Cl-Ph | SiEt₃ | CH₂ | CH₂ |
| II.1-47 | p-CH₃-Ph | SiEt₃ | CH₂ | CH₂ |
| II.1-48 | m-CH₃-Ph | SiEt₃ | CH₂ | CH₂ |
| II.1-49 | CH₃ | H | CHF | CH₂ |
| II.1-50 | i-Pr | H | CHF | CH₂ |
| II.1-51 | Ph | H | CHF | CH₂ |
| II.1-52 | CH₃ | H | CH(CH₃) | CH₂ |
| II.1-53 | i-Pr | H | CH(CH₃) | CH₂ |
| II.1-54 | Ph | H | CH(CH₃) | CH₂ |
| II.1-55 | CH₃ | H | CH(i-Pr) | CH₂ |
| II.1-56 | i-Pr | H | CH(i-Pr) | CH₂ |
| II.1-57 | Ph | H | CH(i-Pr) | CH₂ |
| II.1-58 | CH₃ | H | CH(c-Pr) | CH₂ |
| II.1-59 | i-Pr | H | CH(c-Pr) | CH₂ |
| II.1-60 | Ph | H | CH(c-Pr) | CH₂ |
| II.1-61 | CH₃ | H | CF₂ | CH₂ |
| II.1-62 | i-Pr | H | CF₂ | CH₂ |
| II.1-63 | Ph | H | CF₂ | CH₂ |
| II.1-64 | CH₃ | H | CH₂ | CHF |
| II.1-65 | i-Pr | H | CH₂ | CHF |
| II.1-66 | Ph | H | CH₂ | CHF |
| II.1-67 | CH₃ | H | CH₂ | CH(CH₃) |
| II.1-68 | i-Pr | H | CH₂ | CH(CH₃) |
| II.1-69 | Ph | H | CH₂ | CH(CH₃) |
| II.1-70 | CH₃ | H | CH₂ | CH(i-Pr) |
| II.1-71 | i-Pr | H | CH₂ | CH(i-Pr) |
| II.1-72 | Ph | H | CH₂ | CH(i-Pr) |
| II.1-73 | CH₃ | H | CH₂ | CH(c-Pr) |
| II.1-74 | i-Pr | H | CH₂ | CH(c-Pr) |
| II.1-75 | Ph | H | CH₂ | CH(c-Pr) |
| II.1-76 | CH₃ | H | CH₂ | CF₂ |
| II.1-77 | i-Pr | H | CH₂ | CF₂ |
| II.1-78 | Ph | H | CH₂ | CF₂ |
| II.1-79 | 2,4-Cl₂-Ph | H | CH₂ | CH₂ |
| II.1-80 | c-Bu | H | CH₂ | CH₂ |
| II.1-81 | c-Pent | H | CH₂ | CH₂ |
| II.1-82 | c-Hex | H | CH₂ | CH₂ |
| II.1-83 | Adamantyl | H | CH₂ | CH₂ |
| II.1-84 | 1-Ethylpropyl | H | CH₂ | CH₂ |
| II.1-85 | t-Bu | H | CH₂ | CH₂ |
| II.1-86 | o-F-Ph | H | CH₂ | CH₂ |

**Tabelle 6:**

| | | | | | |
|---|---|---|---|---|---|
| 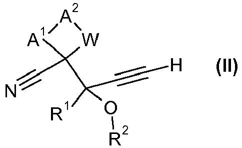 | | | | | |

| No. | R¹ | R² | A¹ | A² | W |
|---|---|---|---|---|---|
| II.2-1 | CH₃ | H | CH₂ | CH₂ | CH₂ |
| II.2-2 | i-Pr | H | CH₂ | CH₂ | CH₂ |
| II.2-3 | Ph | H | CH₂ | CH₂ | CH₂ |
| II.2-4 | n-Pr | H | CH₂ | CH₂ | CH₂ |
| II.2-5 | c-Pr | H | CH₂ | CH₂ | CH₂ |
| II.2-6 | CH₃ | CH₃ | CH₂ | CH₂ | CH₂ |
| II.2-7 | i-Pr | CH₃ | CH₂ | CH₂ | CH₂ |
| II.2-8 | Ph | CH₃ | CH₂ | CH₂ | CH₂ |
| II.2-9 | n-Pr | CH₃ | CH₂ | CH₂ | CH₂ |
| II.2-10 | c-Pr | CH₃ | CH₂ | CH₂ | CH₂ |
| II.2-11 | CH₃ | C(=O)CH₃ | CH₂ | CH₂ | CH₂ |
| II.2-12 | i-Pr | C(=O)CH₃ | CH₂ | CH₂ | CH₂ |
| II.2-13 | Ph | C(=O)CH₃ | CH₂ | CH₂ | CH₂ |
| II.2-14 | n-Pr | C(=O)CH₃ | CH₂ | CH₂ | CH₂ |
| II.2-15 | c-Pr | C(=O)CH₃ | CH₂ | CH₂ | CH₂ |
| II.2-16 | CH₃ | SiEt₃ | CH₂ | CH₂ | CH₂ |
| II.2-17 | i-Pr | SiEt₃ | CH₂ | CH₂ | CH₂ |
| II.2-18 | Ph | SiEt₃ | CH₂ | CH₂ | CH₂ |
| II.2-19 | n-Pr | SiEt₃ | CH₂ | CH₂ | CH₂ |
| II.2-20 | c-Pr | SiEt₃ | CH₂ | CH₂ | CH₂ |
| II.2-21 | CH₃ | H | CH₂ | O | CH₂ |
| II.2-22 | i-Pr | H | CH₂ | O | CH₂ |
| II.2-23 | Ph | H | CH₂ | O | CH₂ |
| II.2-24 | n-Pr | H | CH₂ | O | CH₂ |
| II.2-25 | c-Pr | H | CH₂ | O | CH₂ |
| II.2-26 | CH₃ | CH₃ | CH₂ | O | CH₂ |
| II.2-27 | i-Pr | CH₃ | CH₂ | O | CH₂ |
| II.2-28 | Ph | CH₃ | CH₂ | O | CH₂ |
| II.2-29 | n-Pr | CH₃ | CH₂ | O | CH₂ |
| II.2-30 | c-Pr | CH₃ | CH₂ | O | CH₂ |
| II.2-31 | CH₃ | C(=O)CH₃ | CH₂ | O | CH₂ |
| II.2-32 | i-Pr | C(=O)CH₃ | CH₂ | O | CH₂ |
| II.2-33 | Ph | C(=O)CH₃ | CH₂ | O | CH₂ |
| II.2-34 | n-Pr | C(=O)CH₃ | CH₂ | O | CH₂ |
| II.2-35 | c-Pr | C(=O)CH₃ | CH₂ | O | CH₂ |
| II.2-36 | CH₃ | SiEt₃ | CH₂ | O | CH₂ |
| II.2-37 | i-Pr | SiEt₃ | CH₂ | O | CH₂ |
| II.2-38 | Ph | SiEt₃ | CH₂ | O | CH₂ |
| II.2-39 | n-Pr | SiEt₃ | CH₂ | O | CH₂ |
| II.2-40 | c-Pr | SiEt₃ | CH₂ | O | CH₂ |
| II.2-41 | c-Bu | H | CH₂ | CH₂ | CH₂ |
| II.2-42 | c-Pentyl | H | CH₂ | CH₂ | CH₂ |
| II.2-43 | c-Hexyl | H | CH₂ | CH₂ | CH₂ |
| II.2-44 | p-F-Ph | H | CH₂ | CH₂ | CH₂ |
| II.2-45 | p-Me-Ph | H | CH₂ | CH₂ | CH₂ |
| II.2-46 | p-Cl-Ph | H | CH₂ | CH₂ | CH₂ |
| II.2-47 | m-F-Ph | H | CH₂ | CH₂ | CH₂ |
| II.2-48 | m-Me-Ph | H | CH₂ | CH₂ | CH₂ |
| II.2-49 | m-Cl-Ph | H | CH₂ | CH₂ | CH₂ |
| II.2-50 | Adamantyl | H | CH₂ | CH₂ | CH₂ |
| II.2-51 | t-Bu | H | CH₂ | CH₂ | CH₂ |
| II.2-52 | 1-Ethyl-propyl | H | CH₂ | CH₂ | CH₂ |

**Tabelle 7:**

| | | | | | |
|---|---|---|---|---|---|
| 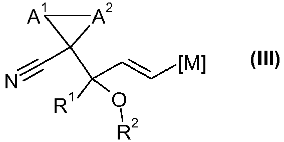 | | | | | |

| No. | R¹ | R² | A¹ | A² | [M] |
|---|---|---|---|---|---|
| III.1-1 | CH₃ | H | CH₂ | CH₂ | Sn(n-Bu)₃ |
| III.1-2 | i-Pr | H | CH₂ | CH₂ | Sn(n-Bu)₃ |
| III.1-3 | Ph | H | CH₂ | CH₂ | Sn(n-Bu)₃ |
| III.1-4 | Et | H | CH₂ | CH₂ | Sn(n-Bu)₃ |
| III.1-5 | c-Pr | H | CH₂ | CH₂ | Sn(n-Bu)₃ |
| III.1-6 | n-Pr | H | CH₂ | CH₂ | Sn(n-Bu)₃ |
| III.1-7 | CH₃ | SiEt₃ | CH₂ | CH₂ | Sn(n-Bu)₃ |
| III.1-8 | i-Pr | SiEt₃ | CH₂ | CH₂ | Sn(n-Bu)₃ |
| III.1-9 | Ph | SiEt₃ | CH₂ | CH₂ | Sn(n-Bu)₃ |
| III.1-10 | c-Pr | SiEt₃ | CH₂ | CH₂ | Sn(n-Bu)₃ |
| III.1-11 | CH₃ | H | CH₂ | CH₂ | Sn(n-Pr)₃ |
| III.1-12 | i-Pr | H | CH₂ | CH₂ | Sn(n-Pr)₃ |
| III.1-13 | Ph | H | CH₂ | CH₂ | Sn(n-Pr)₃ |
| III.1-14 | CH₃ | SiEt₃ | CH₂ | CH₂ | Sn(n-Pr)₃ |
| III.1-15 | i-Pr | SiEt₃ | CH₂ | CH₂ | Sn(n-Pr)₃ |
| III.1-16 | Ph | SiEt₃ | CH₂ | CH₂ | Sn(n-Pr)₃ |
| III.1-17 | CH₃ | H | CH₂ | CH₂ | Sn(c-Hex)₃ |
| III.1-18 | i-Pr | H | CH₂ | CH₂ | Sn(c-Hex)₃ |
| III.1-19 | Ph | H | CH₂ | CH₂ | Sn(c-Hex)₃ |
| III.1-20 | CH₃ | SiEt₃ | CH₂ | CH₂ | Sn(c-Hex)₃ |
| III.1-21 | i-Pr | SiEt₃ | CH₂ | CH₂ | Sn(c-Hex)₃ |
| III.1-22 | Ph | SiEt₃ | CH₂ | CH₂ | Sn(c-Hex)₃ |
| III.1-23 | CH₃ | H | CH₂ | CH₂ | GeEt₃ |
| III.1-24 | i-Pr | H | CH₂ | CH₂ | GeEt₃ |
| III.1-25 | Ph | H | CH₂ | CH₂ | GeEt₃ |
| III.1-26 | CH₃ | SiEt₃ | CH₂ | CH₂ | GeEt₃ |
| III.1-27 | i-Pr | SiEt₃ | CH₂ | CH₂ | GeEt₃ |
| III.1-28 | Ph | SiEt₃ | CH₂ | CH₂ | GeEt₃ |
| III.1-29 | CH₃ | H | CH₂ | CH₂ | Zr(C₅H₅)₂ |
| III.1-30 | i-Pr | H | CH₂ | CH₂ | Zr(C₅H₅)₂ |
| III.1-31 | Ph | H | CH₂ | CH₂ | Zr(C₅H₅)₂ |
| III.1-32 | CH₃ | SiEt₃ | CH₂ | CH₂ | Zr(C₅H₅)₂ |
| III.1-33 | i-Pr | SiEt₃ | CH₂ | CH₂ | Zr(C₅H₅)₂ |
| III.1-34 | Ph | SiEt₃ | CH₂ | CH₂ | Zr(C₅H₅)₂ |
| III.1-35 | CH₃ | H | CH₂ | CH₂ | Hf(C₅H₅)₂ |
| III.1-36 | i-Pr | H | CH₂ | CH₂ | Hf(C₅H₅)₂ |
| III.1-37 | Ph | H | CH₂ | CH₂ | Hf(C₅H₅)₂ |
| III.1-38 | CH₃ | SiEt₃ | CH₂ | CH₂ | Hf(C₅H₅)₂ |
| III.1-39 | i-Pr | SiEt₃ | CH₂ | CH₂ | Hf(C₅H₅)₂ |
| III.1-40 | Ph | SiEt₃ | CH₂ | CH₂ | Hf(C₅H₅)₂ |
| III.1-41 | CH₃ | H | CH₂ | CH₂ | B(OH)₂ |
| III.1-42 | i-Pr | H | CH₂ | CH₂ | B(OH)₂ |
| III.1-43 | Ph | H | CH₂ | CH₂ | B(OH)₂ |
| III.1-44 | CH₃ | SiEt₃ | CH₂ | CH₂ | B(OH)₂ |
| III.1-45 | i-Pr | SiEt₃ | CH₂ | CH₂ | B(OH)₂ |
| III.1-46 | Ph | SiEt₃ | CH₂ | CH₂ | B(OH)₂ |
| III.1-47 | CH₃ | H | CH₂ | CH₂ | B(OMe)2 |
| III.1-48 | i-Pr | H | CH₂ | CH₂ | B(OMe)2 |
| III.1-49 | Ph | H | CH₂ | CH₂ | B(OMe)2 |
| III.1-50 | CH₃ | SiEt₃ | CH₂ | CH₂ | B(OMe)2 |
| III.1-51 | i-Pr | SiEt₃ | CH₂ | CH₂ | B(OMe)2 |
| III.1-52 | Ph | SiEt₃ | CH₂ | CH₂ | B(OMe)2 |
| III.1-53 | CH₃ | H | CH₂ | CH₂ | 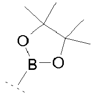 |
| III.1-54 | i-Pr | H | CH₂ | CH₂ | 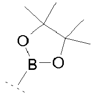 |
| III.1-55 | Ph | H | CH₂ | CH₂ | 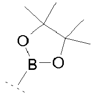 |
| III.1-56 | CH₃ | SiEt₃ | CH₂ | CH₂ | 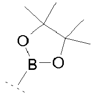 |
| III.1-57 | i-Pr | SiEt₃ | CH₂ | CH₂ | 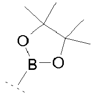 |
| III.1-58 | Ph | SiEt₃ | CH₂ | CH₂ | 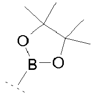 |
| III.1-59 | CH₃ | H | CH₂ | CH₂ | 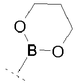 |
| III.1-60 | i-Pr | H | CH₂ | CH₂ | 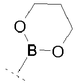 |
| III.1-61 | Ph | H | CH₂ | CH₂ | 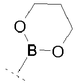 |
| III.1-62 | CH₃ | SiEt₃ | CH₂ | CH₂ | 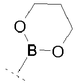 |
| III.1-63 | i-Pr | SiEt₃ | CH₂ | CH₂ | 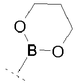 |
| III.1-64 | Ph | SiEt₃ | CH₂ | CH₂ | 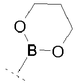 |
| III.1-65 | c-Bu | H | CH₂ | CH₂ | Sn(n-Bu)₃ |
| III.1-66 | c-Hexyl | H | CH₂ | CH₂ | Sn(n-Bu)₃ |
| III.1-67 | 1-Ethylpropyl | H | CH₂ | CH₂ | Sn(n-Bu)₃ |
| III.1-68 | Adamantyl | H | CH₂ | CH₂ | Sn(n-Bu)₃ |
| III.1-69 | p-F-Ph | H | CH₂ | CH₂ | Sn(n-Bu)₃ |
| III.1-70 | m-F-Ph | H | CH₂ | CH₂ | Sn(n-Bu)₃ |
| III.1-71 | p-Cl-Ph | H | CH₂ | CH₂ | Sn(n-Bu)₃ |
| III.1-72 | m-Cl-Ph | H | CH₂ | CH₂ | Sn(n-Bu)₃ |
| III.1-73 | p-CH₃-Ph | H | CH₂ | CH₂ | Sn(n-Bu)₃ |
| III.1-74 | m-CH₃-Ph | H | CH₂ | CH₂ | Sn(n-Bu)₃ |
| III.1-75 | t-Bu | H | CH₂ | CH₂ | Sn(n-Bu)₃ |
| III.1-76 | c-Pentyl | H | CH₂ | CH₂ | Sn(n-Bu)₃ |

**Tabelle 8:**

| | | | | | | |
|---|---|---|---|---|---|---|
| 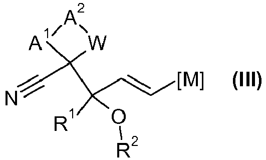 | | | | | | |

| No. | R¹ | R² | A¹ | A² | W | [M] |
|---|---|---|---|---|---|---|
| III.2-1 | CH₃ | H | CH₂ | CH₂ | CH₂ | Sn(n-Bu)₃ |
| III.2-2 | i-Pr | H | CH₂ | CH₂ | CH₂ | Sn(n-Bu)₃ |
| III.2-3 | Ph | H | CH₂ | CH₂ | CH₂ | Sn(n-Bu)₃ |
| III.2-4 | Et | H | CH₂ | CH₂ | CH₂ | Sn(n-Bu)₃ |
| III.2-5 | c-Pr | H | CH₂ | CH₂ | CH₂ | Sn(n-Bu)₃ |
| III.2-6 | n-Pr | H | CH₂ | CH₂ | CH₂ | Sn(n-Bu)₃ |
| III.2-7 | CH₃ | SiEt₃ | CH₂ | CH₂ | CH₂ | Sn(n-Bu)₃ |
| III.2-8 | i-Pr | SiEt₃ | CH₂ | CH₂ | CH₂ | Sn(n-Bu)₃ |
| III.2-9 | Ph | SiEt₃ | CH₂ | CH₂ | CH₂ | Sn(n-Bu)₃ |
| III.2-10 | c-Pr | SiEt₃ | CH₂ | CH₂ | CH₂ | Sn(n-Bu)₃ |
| III.2-11 | CH₃ | H | CH₂ | CH₂ | CH₂ | Sn(n-Pr)₃ |
| III.2-12 | i-Pr | H | CH₂ | CH₂ | CH₂ | Sn(n-Pr)₃ |
| III.2-13 | Ph | H | CH₂ | CH₂ | CH₂ | Sn(n-Pr)₃ |
| III.2-14 | CH₃ | SiEt₃ | CH₂ | CH₂ | CH₂ | Sn(n-Pr)₃ |
| III.2-15 | i-Pr | SiEt₃ | CH₂ | CH₂ | CH₂ | Sn(n-Pr)₃ |
| III.2-16 | Ph | SiEt₃ | CH₂ | CH₂ | CH₂ | Sn(n-Pr)₃ |
| III.2-17 | CH₃ | H | CH₂ | CH₂ | CH₂ | Sn(c-Hex)₃ |
| III.2-18 | i-Pr | H | CH₂ | CH₂ | CH₂ | Sn(c-Hex)₃ |
| III.2-19 | Ph | H | CH₂ | CH₂ | CH₂ | Sn(c-Hex)₃ |
| III.2-20 | CH₃ | SiEt₃ | CH₂ | CH₂ | CH₂ | Sn(c-Hex)₃ |
| III.2-21 | i-Pr | SiEt₃ | CH₂ | CH₂ | CH₂ | Sn(c-Hex)₃ |
| III.2-22 | Ph | SiEt₃ | CH₂ | CH₂ | CH₂ | Sn(c-Hex)₃ |
| III.2-23 | CH₃ | H | CH₂ | CH₂ | CH₂ | GeEt₃ |
| III.2-24 | i-Pr | H | CH₂ | CH₂ | CH₂ | GeEt₃ |
| III.2-25 | Ph | H | CH₂ | CH₂ | CH₂ | GeEt₃ |
| III.2-26 | CH₃ | SiEt₃ | CH₂ | CH₂ | CH₂ | GeEt₃ |
| III.2-27 | i-Pr | SiEt₃ | CH₂ | CH₂ | CH₂ | GeEt₃ |
| III.2-28 | Ph | SiEt₃ | CH₂ | CH₂ | CH₂ | GeEt₃ |
| III.2-29 | CH₃ | H | CH₂ | CH₂ | CH₂ | Zr(C₅H₅)₂ |
| III.2-30 | i-Pr | H | CH₂ | CH₂ | CH₂ | Zr(C₅H₅)₂ |
| III.2-31 | Ph | H | CH₂ | CH₂ | CH₂ | Zr(C₅H₅)₂ |
| III.2-32 | CH₃ | SiEt₃ | CH₂ | CH₂ | CH₂ | Zr(C₅H₅)₂ |
| III.2-33 | i-Pr | SiEt₃ | CH₂ | CH₂ | CH₂ | Zr(C₅H₅)₂ |
| III.2-34 | Ph | SiEt₃ | CH₂ | CH₂ | CH₂ | Zr(C₅H₅)₂ |
| III.2-35 | CH₃ | H | CH₂ | CH₂ | CH₂ | Hf(C₅H₅)₂ |
| III.2-36 | i-Pr | H | CH₂ | CH₂ | CH₂ | Hf(C₅H₅)₂ |
| III.2-37 | Ph | H | CH₂ | CH₂ | CH₂ | Hf(C₅H₅)₂ |
| III.2-38 | CH₃ | SiEt₃ | CH₂ | CH₂ | CH₂ | Hf(C₅H₅)₂ |
| III.2-39 | i-Pr | SiEt₃ | CH₂ | CH₂ | CH₂ | Hf(C₅H₅)₂ |
| III.2-40 | Ph | SiEt₃ | CH₂ | CH₂ | CH₂ | Hf(C₅H₅)₂ |
| III.2-41 | CH₃ | H | CH₂ | CH₂ | CH₂ | B(OH)₂ |
| III.2-42 | i-Pr | H | CH₂ | CH₂ | CH₂ | B(OH)₂ |
| III.2-43 | Ph | H | CH₂ | CH₂ | CH₂ | B(OH)₂ |
| III.2-44 | CH₃ | SiEt₃ | CH₂ | CH₂ | CH₂ | B(OH)₂ |
| III.2-45 | i-Pr | SiEt₃ | CH₂ | CH₂ | CH₂ | B(OH)₂ |
| III.2-46 | Ph | SiEt₃ | CH₂ | CH₂ | CH₂ | B(OH)₂ |
| III.2-47 | CH₃ | H | CH₂ | CH₂ | CH₂ | B(OMe)₂ |
| III.2-48 | i-Pr | H | CH₂ | CH₂ | CH₂ | B(OMe)₂ |
| III.2-49 | Ph | H | CH₂ | CH₂ | CH₂ | B(OMe)₂ |
| III.2-50 | CH₃ | SiEt₃ | CH₂ | CH₂ | CH₂ | B(OMe)₂ |
| III.2-51 | i-Pr | SiEt₃ | CH₂ | CH₂ | CH₂ | B(OMe)₂ |
| III.2-52 | Ph | SiEt₃ | CH₂ | CH₂ | CH₂ | B(OMe)₂ |
| III.2-53 | CH₃ | H | CH₂ | CH₂ | CH₂ | 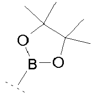 |
| III.2-54 | i-Pr | H | CH₂ | CH₂ | CH₂ | 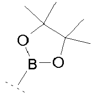 |
| III.2-55 | Ph | H | CH₂ | CH₂ | CH₂ | 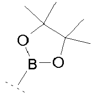 |
| III.2-56 | CH₃ | SiEt₃ | CH₂ | CH₂ | CH₂ | 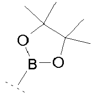 |
| III.2-57 | i-Pr | SiEt₃ | CH₂ | CH₂ | CH₂ | 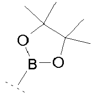 |
| III.2-58 | Ph | SiEt₃ | CH₂ | CH₂ | CH₂ | 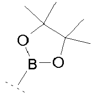 |
| III.2-59 | CH₃ | H | CH₂ | CH₂ | CH₂ | 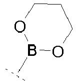 |
| III.2-60 | i-Pr | H | CH₂ | CH₂ | CH₂ | 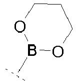 |
| III.2-61 | Ph | H | CH₂ | CH₂ | CH₂ | 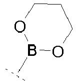 |
| III.2-62 | CH₃ | SiEt₃ | CH₂ | CH₂ | CH₂ | 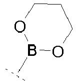 |
| III.2-63 | i-Pr | SiEt₃ | CH₂ | CH₂ | CH₂ | 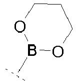 |
| III.2-64 | Ph | SiEt₃ | CH₂ | CH₂ | CH₂ | 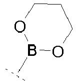 |
| III.2-65 | CH₃ | H | CH₂ | O | CH₂ | Sn(n-Bu)₃ |
| III.2-66 | i-Pr | H | CH₂ | O | CH₂ | Sn(n-Bu)₃ |
| III.2-67 | Ph | H | CH₂ | O | CH₂ | Sn(n-Bu)₃ |
| III.2-68 | CH₃ | SiEt₃ | CH₂ | O | CH₂ | Sn(n-Bu)₃ |
| III.2-69 | i-Pr | SiEt₃ | CH₂ | O | CH₂ | Sn(n-Bu)₃ |
| III.2-70 | Ph | SiEt₃ | CH₂ | O | CH₂ | Sn(n-Bu)₃ |
| III.2-71 | c-Pr | SiEt₃ | CH₂ | O | CH₂ | Sn(n-Bu)₃ |
| III.2-72 | CH₃ | H | CH₂ | O | CH₂ | Sn(n-Pr)₃ |
| III.2-73 | i-Pr | H | CH₂ | O | CH₂ | Sn(n-Pr)₃ |
| III.2-74 | Ph | H | CH₂ | O | CH₂ | Sn(n-Pr)₃ |
| III.2-75 | i-Pr | SiEt₃ | CH₂ | O | CH₂ | Sn(n-Pr)₃ |
| III.2-76 | Ph | SiEt₃ | CH₂ | O | CH₂ | Sn(n-Pr)₃ |
| III.2-77 | CH₃ | H | CH₂ | O | CH₂ | Sn(c-Hex)₃ |
| III.2-78 | i-Pr | H | CH₂ | O | CH₂ | Sn(c-Hex)₃ |
| III.2-79 | Ph | H | CH₂ | O | CH₂ | Sn(c-Hex)₃ |
| III.2-80 | CH₃ | SiEt₃ | CH₂ | O | CH₂ | Sn(c-Hex)₃ |
| III.2-81 | i-Pr | SiEt₃ | CH₂ | O | CH₂ | Sn(c-Hex)₃ |
| III.2-82 | Ph | SiEt₃ | CH₂ | O | CH₂ | Sn(c-Hex)₃ |
| III.2-83 | CH₃ | H | CH₂ | O | CH₂ | GeEt₃ |
| III.2-84 | i-Pr | H | CH₂ | O | CH₂ | GeEt₃ |
| III.2-85 | Ph | H | CH₂ | O | CH₂ | GeEt₃ |
| III.2-86 | CH₃ | SiEt₃ | CH₂ | O | CH₂ | GeEt₃ |
| III.2-87 | i-Pr | SiEt₃ | CH₂ | O | CH₂ | GeEt₃ |
| III.2-88 | Ph | SiEt₃ | CH₂ | O | CH₂ | GeEt₃ |
| III.2-89 | CH₃ | SiEt₃ | CH₂ | O | CH₂ | Zr(C₅H₅)₂ |
| III.2-90 | i-Pr | SiEt₃ | CH₂ | O | CH₂ | Zr(C₅H₅)₂ |
| III.2-91 | Ph | SiEt₃ | CH₂ | O | CH₂ | Zr(C₅H₅)₂ |
| III.2-92 | CH₃ | SiEt₃ | CH₂ | O | CH₂ | Hf(C₅H₅)₂ |
| III.2-93 | i-Pr | SiEt₃ | CH₂ | O | CH₂ | Hf(C₅H₅)₂ |
| III.2-94 | Ph | SiEt₃ | CH₂ | O | CH₂ | Hf(C₅H₅)₂ |
| III.2-95 | CH₃ | H | CH₂ | O | CH₂ | B(OH)₂ |
| III.2-96 | i-Pr | H | CH₂ | O | CH₂ | B(OH)₂ |
| III.2-97 | Ph | H | CH₂ | O | CH₂ | B(OH)₂ |
| III.2-98 | CH₃ | SiEt₃ | CH₂ | O | CH₂ | B(OH)₂ |
| III.2-99 | i-Pr | SiEt₃ | CH₂ | O | CH₂ | B(OH)₂ |
| III.2-100 | Ph | SiEt₃ | CH₂ | O | CH₂ | B(OH)₂ |
| III.2-101 | CH₃ | H | CH₂ | O | CH₂ | B(OMe)2 |
| III.2-102 | i-Pr | H | CH₂ | O | CH₂ | B(OMe)2 |
| III.2-103 | Ph | H | CH₂ | O | CH₂ | B(OMe)2 |
| III.2-104 | CH₃ | SiEt₃ | CH₂ | O | CH₂ | B(OMe)2 |
| III.2-105 | i-Pr | SiEt₃ | CH₂ | O | CH₂ | B(OMe)2 |
| III.2-106 | Ph | SiEt₃ | CH₂ | O | CH₂ | B(OMe)2 |
| III.2-107 | CH₃ | H | CH₂ | O | CH₂ | 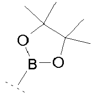 |
| III.2-108 | i-Pr | H | CH₂ | O | CH₂ | 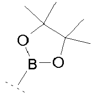 |
| III.2-109 | Ph | H | CH₂ | O | CH₂ | 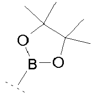 |
| III.2-110 | CH₃ | SiEt₃ | CH₂ | O | CH₂ | 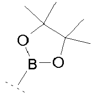 |
| III.2-111 | i-Pr | SiEt₃ | CH₂ | O | CH₂ | 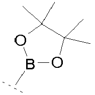 |
| III.2-112 | Ph | SiEt₃ | CH₂ | O | CH₂ | 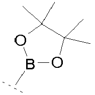 |
| III.2-113 | CH₃ | H | CH₂ | O | CH₂ | 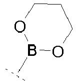 |
| III.2-114 | i-Pr | H | CH₂ | O | CH₂ | 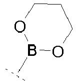 |
| III.2-115 | Ph | H | CH₂ | O | CH₂ | 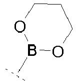 |
| III.2-116 | CH₃ | SiEt₃ | CH₂ | O | CH₂ | 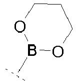 |
| III.2-117 | i-Pr | SiEt₃ | CH₂ | O | CH₂ | 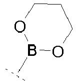 |
| III.2-118 | Ph | SiEt₃ | CH₂ | O | CH₂ | 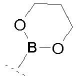 |

### Spektroskopische Daten ausgewählter Tabellenbeispiele:

Beispiel No. 1.1-119:
   ¹H-NMR (400 MHz, CDCl₃ δ, ppm) 7.68 (m, 1H), 7.58 (m, 2H), 7.47 (m, 1H), 2.55 (sept, 1H), 2.45 (br. s, 1H, OH), 1.49 (m, 2H), 1.42 (m, 1H), 1.29 (m, 1H), 1.22 (d, 3H), 1.19 (d, 3H).
Beispiel No. 1.1-133:
   ¹H-NMR (400 MHz, CDCl₃ δ, ppm) 7.96 (m, 1H), 7.52 (m, 1H), 7.47 (m, 1H), 7.39 (m, 1H), 4.38 (q, 2H), 2.62 (br. s, 1H, OH), 2.52 (sept, 1H), 1.50 (m, 2H), 1.38 (t, 3H), 1.34 (m, 1H), 1.23 (m, 1H), 1.21 (d, 3H), 1.17 (d, 3H).
Beispiel No. I.1-144:
   ¹H-NMR (400 MHz, CDCl₃ δ, ppm) 7.76 (d, 1H), 7.41 (d, 1H), 7.29 (dd, 1H), 3.90 (s, 3H), 2.57 (br. s, 1H, OH), 2.50 (sept, 1H), 2.39 (s, 3H), 1.49 (m, 2H), 1.34 (m, 1H), 1.24 (m, 1H), 1.21 (d, 3H), 1.17 (d, 3H).
Beispiel No. I.1-230:
   ¹H-NMR (400 MHz, CDCl₃ δ, ppm) 8.00 (m, 1H), 7.82 (m, 2H), 7.58 (m, 1H), 7.49 (m, 1H), 7.43-7.37 (m, 4H), 3.90 (s, 3H), 3.16 (br. s, 1H, OH), 1.65 (m, 1H), 1.58 (m, 1H), 1.30 (m, 2H).
Beispiel No. I.1-233:
   ¹H-NMR (400 MHz, CDCl₃ δ, ppm) 7.97 (m, 1H), 7.81 (m, 2H), 7.58 (m, 1H), 7.48 (m, 1H), 7.43-7.36 (m, 4H), 4.38 (m, 2H), 3.15 (br. s, 1H, OH), 1.68 (m, 1H), 1.58 (m, 1H), 1.38 (t, 3H), 1.30 (m, 2H).
Beispiel No. I.1-244:
   ¹H-NMR (400 MHz, CDCl₃ δ, ppm) 7.82 (s, 1H), 7.79 (m, 2H), 7.47-7.36 (m, 4H), 7.29 (m, 1H), 3.89 (s, 3H), 3.12 (br. s, 1H, OH), 2.40 (s, 3H), 1.80 (m, 1H), 1.58 (m, 1H), 1.30 (m, 2H).
Beispiel No. I.1-508:
   ¹H-NMR (400 MHz, CDCl₃ δ, ppm) 7.96 (m, 1H), 7.51 (m, 1H), 7.48 (m, 1H), 7.40 (m, 1H), 3.92 (s, 3H), 2.59 (br. s, 1H, OH), 1.53 (m, 1H), 1.41 (m, 1H), 1.28 (m, 3H), 0.85 (m, 1H), 0.80-0.72 (m, 2H), 0.63 (m, 1H).
Beispiel No. I.1-518:
   ¹H-NMR (400 MHz, CDCl₃ δ, ppm) 7.98 (d, 1H), 7.57 (d, 1H), 7.49 (m, 1H), 7.41 (m, 1H), 3.92 (s, 3H), 3.12 (m, 1H), 2.56 (br. s, 1H, OH), 2.32 (m, 1H), 2.20-2.08 (m, 1H), 1.99-1.91 (m, 1H), 1.82 (m, 1H), 1.48 (m, 1H), 1.39 (m, 1H), 1.22-1.18 (m, 2H).
Beispiel No. I.1-528:
   ¹H-NMR (400 MHz, CDCl₃ δ, ppm) 7.96 (d, 1H), 7.52 (d, 1H), 7.48 (m, 1H), 7.41 (m, 1H), 3.91 (s, 3H), 2.58 (br. s, 1H, OH), 2.15 (m, 2H), 2.06 (m, 1H), 1.84 (m, 2H), 1.73 (m, 1H), 1.48 (m, 2H), 1.38-1.31 (m, 4H), 1.24-1.20 (m, 3H).
Beispiel No. I.1-531:
   ¹H-NMR (400 MHz, CDCl₃ δ, ppm) 7.31 (m, 2H), 6.89 (m, 1H), 6.85 (m, 1H), 3.84 (s, 3H), 2.33 (br. s, 1H, OH), 2.04 (m, 1H), 1.92 (m, 1H), 1.80 (m, 1H), 1.61 (m, 1H), 1.50 (m, 3H), 1.39 (m, 1H), 1.23 (m, 1H), 1.09 (m, 6H).
Beispiel No. I.1-533:
   ¹H-NMR (400 MHz, CDCl₃ δ, ppm) 7.66-7.60 (m, 3H), 7.45 (m, 1H), 2.22 (br. s, 1H, OH), 2.03 (m, 1H), 1.93-1.78 (m, 2H), 1.55-1.47 (m, 2H), 1.43-1.37 (m, 2H), 1.35-1.29 (m, 2H).
Beispiel No. I.1-539:
   ¹H-NMR (400 MHz, CDCl₃ δ, ppm) 8.02 (m, 2H), 7.56 (m, 1H), 7.42 (m, 1H), 3.93 (s, 3H), 2.26 (br. s, 1H, OH), 2.03 (m, 1H), 1.91 (m, 1H), 1.82 (m, 1H), 1.58 (m, 2H), 1.50 (m, 1H), 1.41 (m, 2H), 1.29 (m, 1H), 1.10 (m, 6H).
Beispiel No. I.1-548:
   ¹H-NMR (400 MHz, CDCl₃ δ, ppm) 7.97 (m, 1H), 7.56 (m, 1H), 7.48 (m, 1H), 7.40 (m, 1H), 3.92 (s, 3H), 2.54 (br. s, 1H, OH), 2.12-2.04 (m, 8H), 1.98-1.88 (m, 3H), 1.76-1.71 (m, 4H), 1.61 (m, 1H), 1.42 (m, 1H), 1.31-1.23 (m, 2H).
Beispiel No. I.1-558:
   ¹H-NMR (400 MHz, CDCl₃ δ, ppm) 8.01 (m, 1H), 7.63-7.48 (m, 4H), 7.46-7.38 (m, 2H), 7.08 (m, 1H), 3.91 (s, 3H), 3.26 (br. s, 1H, OH), 1.70-1.57 (m, 1H), 1.50-1,35 (m, 2H), 1.17-1.12 (m, 1H).
Beispiel No. I.1-568:
   ¹H-NMR (400 MHz, CDCl₃ δ, ppm) 7.99 (d, 1H), 7.70 (d, 2H), 7.58 (m, 1H), 7.49 (m, 1H), 7.41 (m, 1H), 3.90 (s, 3H), 3.00 (br. s, 1H, OH), 2.38 (s, 3H), 1.66 (m, 1H), 1.55 (m, 1H), 1.32-1.25 (m, 2H).
Beispiel No. I.1-598:
   ¹H-NMR (400 MHz, CDCl₃ δ, ppm) 8.01 (m, 1H), 7.77 (m, 2H), 7.57 (m, 1H), 7.50 (m, 1H), 7.45-7.38 (m, 3H), 3.91 (s, 3H), 3.11 (br. s, 1H, OH), 1.68-1.64 (m, 1H), 1.60 (m, 1H), 1.33-1.28 (m, 2H).
Beispiel No. I.1-608:
   ¹H-NMR (400 MHz, CDCl₃ δ, ppm) 7.97 (m, 1H), 7.55 (m, 1H), 7.49 (m, 1H), 7.40 (m, 1H), 3.92 (s, 3H), 2.57 (br. s, 1H, OH), 1.64-1.60 (m, 1H), 1.47-1.43 (m, 1H), 1.30 (s, 9H), 1.29-1.23 (m, 2H).
Beispiel No. I.1-618:
   ¹H-NMR (400 MHz, CDCl₃ δ, ppm) 8.01 (m, 1H), 7.81 (m, 2H), 7.58 (m, 1H), 7.51 (m, 1H), 7.44 (m, 1H), 7.12 (m, 2H), 3.91 (s, 3H), 3.21 (br. s, 1H, OH), 1.65 (m, 1H), 1.58 (m, 1H), 1.31 (m, 2H).
Beispiel No. I.1-621:
   ¹H-NMR (400 MHz, CDCl₃ δ, ppm) 8.00 (m, 1H), 7.81 (m, 2H), 7.58 (m, 1H), 7.50 (m, 1H), 7.43 (m, 1H), 7.11 (m, 2H), 4.38 (q, 2H), 3.23 (br. s, 1H, OH), 1.67 (m, 1H), 1.58 (m, 1H), 1.38 (t, 3H), 1.30 (m, 2H).
Beispiel No. I.1-622:
   ¹H-NMR (400 MHz, CDCl₃ δ, ppm) 7.81 (m, 3H), 7.47 (d, 1H), 7.31 (m, 1H), 7.12 (m, 2H), 3.90 (s, 3H), 3.17 (br. s, 1H, OH), 2.40 (s, 3H), 1.65 (m, 1H), 1.56 (m, 1H), 1.30 (m, 2H).
Beispiel No. I.1-623:
   ¹H-NMR (400 MHz, CDCl₃ δ, ppm) 7.74 (m, 2H), 7.70 (m, 1H), 7.53 (m, 1H), 7.48 (m, 1H), 7.20 (m, 1H), 7.11 (m, 2H), 2.74 (br. s, 1H, OH), 1.58 (m, 1H), 1.46 (m, 1H), 1.33 (m, 2H).
Beispiel No. I.1-624:
   ¹H-NMR (400 MHz, CDCl₃ δ, ppm) 7.80 (m, 2H), 7.72 (m, 1H), 7.69 (m, 1H), 7.60 (m, 1H), 7.48 (m, 1H), 7.11 (m, 2H), 2.90 (br. s, 1H, OH), 1.63 (m, 1H), 1.46 (m, 1H), 1.38 (m, 2H).
Beispiel No. I.1-625:
   ¹H-NMR (400 MHz, CDCl₃ δ, ppm) 7.76 (d, 1H), 7.38 (d, 1H), 7.28 (m, 1H), 3.90 (s, 3H), 2.52 (br. s, 1H, OH), 2.39 (s, 3H), 2.02 (m, 1H), 1.92 (m, 1H), 1.83 (m, 1H), 1.58 (m, 2H), 1.52 (m, 2H), 1.38 (m, 1H), 1.24 (m, 1H), 1.08 (m, 6H).
Beispiel No. I.1-626:
   ¹H-NMR (400 MHz, CDCl₃ δ, ppm) 7.78 (m, 1H), 7.34 (d, 1H), 7.19 (m, 1H), 7.14 (m, 1H), 2.41 (s, 3H), 2.22 (br. s, 1H, OH), 2.03 (m, 1H), 1.92 (m, 1H), 1.83 (m, 1H), 1.57 (m, 2H), 1.49 (m, 1H), 1.39 (m, 2H), 1.27 (m, 1H), 1.10 (m, 6H).
Beispiel No. I.1-627:
   ¹H-NMR (400 MHz, CDCl₃ δ, ppm) 7.76 (m, 1H), 7.54 (m, 1H), 7.52-7.43 (m, 2H), 2.27 (br. s, 1H, OH), 2.04 (m, 1H), 1.90 (m, 1H), 1.81 (m, 1H), 1.58 (m, 2H), 1.49 (m, 1H), 1.41 (m, 2H), 1.27 (m, 1H), 1.09 (m, 6H).
Beispiel No. I.1-628:
   ¹H-NMR (400 MHz, CDCl₃ δ, ppm) 7.28 (d, 2H), 7.11 (d, 2H), 2.35 (s, 3H), 2.22 (br. s, 1H, OH), 2.02 (m, 1H), 1.90 (m, 1H), 1.81 (m, 1H), 1.58 (m, 2H), 1.49 (m, 1H), 1.39 (m, 2H), 1.24 (m, 1H), 1.09 (m, 6H).
Beispiel No. I.1-629:
   ¹H-NMR (400 MHz, CDCl₃ δ, ppm) 7.93 (m, 1H), 7.52-7.43 (m, 2H), 7.37 (m, 1H), 4.21 (q, 2H), 2.55 (br. s, 1H, OH), 2.02 (m, 1H), 1.95-1.91 (m, 1H), 1.84 (m, 1H), 1.58 (m, 2H), 1.53 (m, 2H), 1.39 (m, 1H), 1.25 (m, 1H), 1.15 (t, 3H), 1.09 (m, 6H).
Beispiel No. II.1-5:
   ¹H-NMR (400 MHz, CDCl₃ δ, ppm) 2.46 (s, 1H), 2.15 (br. s, 1H, OH), 1.57 (m, 1H), 1.47-34 (m, 2H), 1.28-1.23 (m, 2H), 0.75-0.55 (m, 4H).
Beispiel No. II.1-8:
   ¹H-NMR (400 MHz, CDCl₃ δ, ppm) 7.51 (m, 1H), 7.42-7.38 (m, 2H), 7.11-7.07 (m, 1H), 2.79 (s, 1H), 2.73 (br. s, 1H, OH), 1.52 (m, 1H), 1.45-1.37 (m, 1H), 1.32-1.27 (m, 2H).
Beispiel No. II.1-9:
   ¹H-NMR (400 MHz, CDCl₃ δ, ppm) 7.66 (d, 2H), 7.42 (d, 2H), 2.79 (s, 1H), 2.66 (br. s, 1H, OH), 1.50 (m, 1H), 1.40 (m, 1H), 1.32-1.25 (m, 2H).
Beispiel No. II.1-11:
   ¹H-NMR (400 MHz, CDCl₃ δ, ppm) 7.60 (d, 2H), 7.22 (d, 2H), 2.76 (s, 1H), 2.58 (br. s, 1H, OH), 2.38 (s, 3H), 1.50 (m, 1H), 1.38 (m, 1H), 1.28 (m, 2H).
Beispiel No. II.1-80:
   ¹H-NMR (400 MHz, CDCl₃ δ, ppm) 3.04 (m, 1H), 2.77 (s, 1H), 2.21 (m, 1H), 2.09 (br. s, 1H, OH), 2.07 (m, 2H), 1.93 (m, 1H), 1.78 (m, 1H), 1.31 (m, 1H), 1.24-1.15 (m, 4H).
Beispiel No. II.1-82:
   ¹H-NMR (400 MHz, CDCl₃ δ, ppm) 2.48 (m, 1H), 2.18 (br. s, 1H, OH), 2.11-1.94 (m, 3H), 1.86-1.78 (m, 2H), 1.74-1.68 (m, 1H), 1.42-1.23 (m, 5H), 1.21-1.08 (m, 4H).
Beispiel No. II.1-83:
   ¹H-NMR (400 MHz, CDCl₃ δ, ppm) 3.38 (m, 1H), 3.29 (m, 1H), 2.82 (m, 1H), 2.56 (br. s, 1H, OH), 2.11-2.03 (m, 4H), 2.01-1.78 (m, 3H), 1.87 (m, 1H), 1.83 (m, 1H), 1.76-1.65 (m, 4H), 1.48-1.43 (m, 2H), 1.31-1.16 (m, 2H).
Beispiel No. II.1-85:
   ¹H-NMR (400 MHz, CDCl₃ δ, ppm) 2.56 (s, 1H), 2.04 (br. s, 1H, OH), 1.54-1.47 (m, 2H), 1.33-1.25 (m, 2H), 1.24 (s, 9H).
Beispiel No. III.1-3:
   ¹H-NMR (400 MHz, CDCl₃ δ, ppm) 7.71 (m, 2H), 7.57 (m, 1H), 7.39 (m, 2H), 6.57 (d, 1H), 6.18 (d, 1H), 2.08 (br. s, 1H, OH), 1.48 (m, 2H), 1.41-1.36 (m, 12H), 1.31-1.19 (m, 15H), 1.09 (m, 2H).
Beispiel No. III.1-66:
   ¹H-NMR (400 MHz, CDCl₃ δ, ppm) 6.03 (d, 1H), 5.93 (d, 1H), 1.83 (m, 2H), 1.68 (m, 2H), 1.58 (m, 1H), 1.42 (br. s, 1H, OH), 1.41-1.32 (m, 4H), 1.22-1.13 (m, 9H), 1.09 (m, 1H), 1.04-0.99 (m, 2H), 0.95 (m, 1H), 0.88 (m, 2H), 0.82-0.73 (m, 17H), 0.69 (m, 1H).
Beispiel No. III.1-73:
   ¹H-NMR (400 MHz, CDCl₃ δ, ppm) 7.45 (d, 2H), 7.19 (d, 2H), 6.46 (d, 1H), 6.35 (d, 1H), 2.35 (s, 3H), 2.01 (br. s, 1H, OH), 1.52 (m, 2H), 1.37-1.20 (m, 12H), 0.96-0.84 (m, 15H), 0.74 (m, 2H).
Beispiel No. III.1-75:
   ¹H-NMR (400 MHz, CDCl₃ δ, ppm) 6.48 (d, 1H), 6.14 (d, 1H), 1.58 (br. s, 1H, OH), 1.52 (m, 2H), 1.37-1.20 (m, 12H), 1.11 (s, 9H), 0.96-0.84 (m, 15H), 0.74 (m, 2H).

Weiterer Gegenstand der vorliegenden Erfindung ist weiter die Verwendung mindestens einer Verbindung, ausgewählt aus der Gruppe, bestehend aus den erfindingsgemäßen substituierten Vinyl- und Alkinyl-cyanocycloalkanolen sowie Vinyl- und Alkinyl-cyanoheterocyclylalkanolen der allgemeinen Formel (I) inklusive der Verbindung 1-[(2E)-1-Hydroxy-1,3-diphenylprop-2-en-1-yl]cyclopentan-carbonitril, sowie von beliebigen Mischungen dieser erfindungsgemäßen substituierten Vinyl- und Alkinyl-cyanocycloalkanole sowie Vinyl- und Alkinyl-cyanoheterocyclylalkanole der allgemeinen Formel (I) inklusive der Verbindung 1-[(2E)-1-Hydroxy-1,3-diphenylprop-2-en-1-yl]cyclopentan-carbonitril mit weiteren agrochemischen Wirkstoffen, zur Steigerung der Widerstandsfähigkeit von Pflanzen gegenüber abiotischen Stressfaktoren, bevorzugt Trockenstress, sowie zur Stärkung des Pflanzenwachstums und/oder zur Erhöhung des Pflanzenertrags.

Weiterer Gegenstand der vorliegenden Erfindung ist eine Sprühlösung zur Behandlung von Pflanzen, enthaltend eine zur Steigerung der Widerstandsfähigkeit von Pflanzen gegenüber abiotischen Stressfaktoren wirksame Menge von mindestens einer Verbindung, ausgewählt aus der Gruppe, bestehend aus substituierten Vinyl- und Alkinyl-cyanocycloalkanolen sowie Vinyl- und Alkinyl-cyanoheterocyclylalkanolen, der allgemeinen Formel (I) inklusive der Verbindung 1-[(2E)-1-Hydroxy-1,3-diphenylprop-2-en-1-yl]cyclopentan-carbonitril. Zu den dabei relativierbaren abiotischen Streßbedingungen können zum Beispiel Hitze, Dürre, Kälte- und Trockenstress (Stress verursacht durch Trockenheit und/oder Wassermangel), osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen zählen.

In einer Ausführungsform kann beispielsweise vorgesehen sein, dass die erfindungsgemäß vorgesehenen Verbindungen, d. h. die entsprechenden erfindungsgemäßen substituierten Vinyl- und Alkinyl-cyanocycloalkanole sowie Vinyl- und Alkinyl-cyanoheterocyclylalkanole der allgemeinen Formel (I) inklusive der Verbindung 1-[(2E)-1-Hydroxy-1,3-diphenylprop-2-en-1-yl]cyclopentan-carbonitril, durch eine Sprühapplikation auf entsprechende zu behandelnde Pflanzen oder Pflanzenteile aufgebracht werden. Die erfindungsgemäß vorgesehene Verwendung der Verbindungen der allgemeinen Formel (I) inklusive der Verbindung 1-[(2E)-1-Hydroxy-1,3-diphenylprop-2-en-1-yl]cyclopentan-carbonitril oder deren Salze erfolgt vorzugsweise mit einer Dosierung zwischen 0,00005 und 3 kg/ha, besonders bevorzugt zwischen 0,0001 und 2 kg/ha, insbesondere bevorzugt zwischen 0,0005 und 1 kg/ha, im Speziellen bevorzugt zwischen 0,001 und 0,25 kg/ha. Wenn im Rahmen der vorliegenden Erfindung Abscisinsäure gleichzeitig mit substituierten Vinyl- und Alkinyl-cyanocycloalkanolen sowie Vinyl- und Alkinyl-cyanoheterocyclylalkanolen der allgemeinen Formel (I) inklusive der Verbindung 1-[(2E)-1-Hydroxy-1,3-diphenylprop-2-en-1-yl]cyclopentan-carbonitril, beispielsweise in Rahmen einer gemeinsamen Zubereitung oder Formulierung verwendet wird, so erfolgt die Zumischung von Abscisinsäure dabei vorzugsweise in einer Dosierung zwischen 0.0001 und 3 kg/ha, besonders bevorzugt zwischen 0.001 und 2 kg/ha, insbesondere bevorzugt zwischen 0.005 und 1 kg/ha, im Speziellen bevorzugt zwischen 0.006 und 0.25 kg/ha.

Unter der Bezeichnung Resistenz bzw. Widerstandsfähigkeit gegenüber abiotischem Stress werden im Rahmen der vorliegenden Erfindung verschiedenartige Vorteile für Pflanzen verstanden. Solche vorteilhaften Eigenschaften äußern sich beispielsweise in den nachfolgend genannten verbesserten Pflanzencharakteristika: verbessertes Wurzelwachstum hinsichtlich Oberfläche und Tiefe, vermehrte Ausläuferbildung oder Bestockung, stärkere und produktivere Ausläufer und Bestockungstriebe, Verbesserung des Sproßwachstums, erhöhte Standfestigkeit, vergrößerte Sprossbasisdurchmesser, vergrößerte Blattfläche, höhere Erträge an Nähr- und Inhaltsstoffen, wie z.B. Kohlenhydrate, Fette, Öle, Proteine, Vitamine, Mineralstoffe, ätherische Öle, Farbstoffe, Fasern, bessere Faserqualität, früheres Blühen, gesteigerte Blütenanzahl, reduzierter Gehalt an toxischen Produkten wie Mycotoxine, reduzierter Gehalt an Rückständen oder unvorteilhaften Bestandteilen jeglicher Art oder bessere Verdaulichkeit, verbesserte Lagerstabilität des Erntegutes, verbesserter Toleranz gegenüber unvorteilhaften Temperaturen, verbesserter Toleranz gegenüber Dürre und Trockenheit, wie auch Sauerstoffmangel durch Wasserüberschuß, verbesserte Toleranz gegenüber erhöhten Salzgehalten in Böden und Wasser, gesteigerte Toleranz gegenüber Ozonstress, verbesserte Verträglichkeit gegenüber Herbiziden und anderen Pflanzenbehandlungsmitteln, verbesserte Wasseraufnahme und Photosyntheseleistung, vorteilhafte Pflanzeneigenschaften, wie beispielsweise Beschleunigung der Reifung, gleichmäßigere Abreife, größere Anziehungskraft für Nützlinge, verbesserte Bestäubung oder andere Vorteile, die einem Fachmann durchaus bekannt sind.

Insbesondere zeigt die erfindungsgemäße Verwendung einer oder mehrerer erfindungsgemäßer Verbindungen der allgemeinen Formel (I) inklusive der Verbindung 1-[(2E)-1-Hydroxy-1,3-diphenylprop-2-en-1-yl]cyclopentan-carbonitril in der Sprühapplikation auf Pflanzen und Pflanzenteilen die beschriebenen Vorteile. Kombinationen von den erfindungsgemäßen substituierten Vinyl- und Alkinyl-cyanocycloalkanolen sowie Vinyl- und Alkinyl-cyanoheterocyclylalkanolen der allgemeinen Formel (I) inklusive der Verbindung 1-[(2E)-1-Hydroxy-1,3-diphenylprop-2-en-1-yl]cyclopentan-carbonitril unter anderem mit Insektiziden, Lockstoffen, Akariziden, Fungiziden, Nematiziden, Herbiziden, wachstumsregulierenden Stoffen, Safenern, die Pflanzenreife beeinflussenden Stoffen und Bakteriziden können bei der Bekämpfung von Pflanzenkrankheiten und/oder zur Steigerung des Pflanzenertrags im Rahmen der vorliegenden Erfindung ebenfalls Anwendung finden. Die kombinierte Verwendung von erfindungsgemäßen substituierten Vinyl- und Alkinyl-cyanocycloalkanolen sowie Vinyl- und Alkinyl-cyanoheterocyclylalkanolen der allgemeinen Formel (I) inklusive der Verbindung 1-[(2E)-1-Hydroxy-1,3-diphenylprop-2-en-1-yl]cyclopentan-carbonitril mit gentechnisch veränderten Sorten in Bezug auf erhöhte abiotische Stresstoleranz ist darüber hinaus ebenfalls möglich.

Die weiter oben genannten verschiedenartigen Vorteile für Pflanzen lassen sich bekannterweise partiell zusammenfassen und mit allgemein gültigen Begriffen belegen. Soche Begriffe sind beispielsweise die nachfolgend aufgeführten Bezeichnungen: phytotonischer Effekt, Widerstandsfähigkeit gegenüber Stressfaktoren, weniger Pflanzenstress, Pflanzengesundheit, gesunde Pflanzen, Pflanzenfitness, ("Plant Fitness"), "Plant Wellness", "Plant Concept", "Vigor Effect", "Stress Shield", Schutzschild, "Crop Health", "Crop Health Properties", "Crop Health Products", "Crop Health Management", "Crop Health Therapy", "Plant Health", Plant Health Properties", Plant Health Products", "Plant Health Management", "Plant Health Therapy", Grünungseffekt ("Greening Effect" oder "Re-greening Effect"), "Freshness" oder andere Begriffe, die einem Fachmann durchaus bekannt sind.

Im Rahmen der vorliegenden Erfindung wird unter einem guten Effekt auf die Widerstandsfähigkeit gegenüber abiotischem Stress nicht beschränkend
- mindestens ein um im Allgemeinen 3 %, insbesondere größer als 5 % besonders bevorzugt größer als 10 % verbessertes Auflaufen,
- mindestens einen um im Allgemeinen 3 %, insbesondere größer als 5 % besonders bevorzugt größer als 10 % gesteigerten Ertrag,
- mindestens eine um im Allgemeinen 3 %, insbesondere größer als 5 % besonders bevorzugt größer als 10 % verbesserte Wurzelentwicklung,
- mindestens eine um im Allgemeinen 3 %, insbesondere größer als 5 % besonders bevorzugt größer als 10 % ansteigende Sproßgröße,
- mindestens eine um im Allgemeinen 3 %, insbesondere größer als 5 % besonders bevorzugt größer als 10 % vergrößerte Blattfläche,
- mindestens eine um im Allgemeinen 3 %, insbesondere größer als 5 % besonders bevorzugt größer als 10 % verbesserte Photosyntheseleistung und/oder
- mindestens eine um im Allgemeinen 3 %, insbesondere größer als 5 % besonders bevorzugt größer als 10 % verbesserte Blütenausbildung
verstanden, wobei die Effekte einzeln oder aber in beliebiger Kombination von zwei oder mehreren Effekten auftreten können.

Weiterer Gegenstand der vorliegenden Erfindung ist eine Sprühlösung zur Behandlung von Pflanzen, enthaltend eine zur Steigerung der Widerstandsfähigkeit von Pflanzen gegenüber abiotischen Stressfaktoren wirksame Menge von mindestens einer Verbindung aus der Gruppe der erfindungsgemäß substituierten Vinyl- und Alkinyl-cyanocycloalkanole sowie Vinyl- und Alkinyl-cyanoheterocyclylalkanole der allgemeinen Formel (I) inklusive der Verbindung 1-[(2E)-1-Hydroxy-1,3-diphenylprop-2-en-1-yl]cyclopentan-carbonitril. Die Sprühlösung kann andere übliche Bestandteile aufweisen, wie Lösungsmittel, Formulierhilfsstoffe, insbesondere Wasser, enthalten. Weitere Bestandteile können unter anderem agrochemische Wirkstoffe sein, welche unten noch weiter beschrieben werden.

Weiterer Gegenstand der vorliegenden Erfindung ist die Verwendung von entsprechenden Sprühlösungen zur Steigerung der Widerstandsfähigkeit von Pflanzen gegenüber abiotischen Stressfaktoren. Die nachfolgenden Ausführungen gelten sowohl für die erfindungsgemäße Verwendung einer oder mehrerer erfindungsgemäßer Verbindungen der allgemeinen Formel (I) inklusive der Verbindung 1-[(2E)-1-Hydroxy-1,3-diphenylprop-2-en-1-yl]cyclopentan-carbonitril an sich als auch für die entsprechenden Sprühlösungen.

Erfindunsgemäß wurde darüber hinaus gefunden, dass die Anwendung einer oder mehrerer Verbindungen der allgemeinen Formel (I) inklusive der Verbindung 1-[(2E)-1-Hydroxy-1,3-diphenylprop-2-en-1-yl]cyclopentan-carbonitril in Kombination mit mindestens einem Düngemittel wie weiter unten stehend definiert auf Pflanzen oder in deren Umgebung möglich ist.

Düngemittel, die erfindungsgemäß zusammen mit den oben näher erläuterten erfindungsgemäßen Verbindungen der allgemeinen Formel (I) verwendet werden können, sind im Allgemeinen organische und anorganische Stickstoff-haltige Verbindungen wie beispielsweise Harnstoffe, Harnstoff-Formaldehyd-Kondensationsprodukte, Aminosäuren, Ammoniumsalze und -nitrate, Kaliumsalze (bevorzugt Chloride, Sulfate, Nitrate), Phosphorsäuresalze und/oder Salze von Phosphoriger Säure (bevorzugt Kaliumsalze und Ammoniumsalze). Insbesondere zu nennen sind in diesem Zusammenhang die NPK-Dünger, d.h. Düngemittel, die Stickstoff, Phosphor und Kalium enthalten, Kalkammonsalpeter, d.h. Düngemittel, die noch Calcium enthalten, Ammonsulfatsalpeter (Allgemeine Formel (NH₄)₂SO₄ NH₄NO₃), Ammonphosphat und Ammonsulfat. Diese Düngemittel sind dem Fachmann allgemein bekannt, siehe auch beispielsweise Ullmann's Encyclopedia of Industrial Chemistry, 5. Edition, Vol. A 10, Seiten 323 bis 431, Verlagsgesellschaft, Weinheim, 1987.

Die Düngemittel können auch Salze aus Mikronährstoffen (bevorzugt Calcium, Schwefel, Bor, Mangan, Magnesium, Eisen, Bor, Kupfer, Zink, Molybdän und Kobalt) und Phytohormonen (z. B. Vitamin B1 und Indol-3-essigsäure) oder Gemische davon enthalten. Erfindungsgemäß eingesetzte Düngemittel können auch weitere Salze wie Monoammoniumphosphat (MAP), Diammoniumphosphat (DAP), Kaliumsulfat, Kaliumchlorid, Magnesiumsulfat enthalten. Geeignete Mengen für die sekundären Nährstoffe oder Spurenelemente sind Mengen von 0,5 bis 5 Gew.-%, bezogen auf das gesamte Düngemittel. Weitere mögliche Inhaltsstoffe sind Pflanzenschutzmittel, Insektizide oder Fungizide, Wachstumsregulatoren oder Gemische davon. Hierzu folgen weiter unten weitergehende Ausführungen.

Die Düngemittel können beispielsweise in Form von Pulvern, Granulaten, Prills oder Kompaktaten eingesetzt werden. Die Düngemittel können jedoch auch in flüssiger Form, gelöst in einem wässrigen Medium, eingesetzt werden. In diesem Fall kann auch verdünnter wässriger Ammoniak als Stickstoffdüngemittel eingesetzt werden. Weitere mögliche Inhaltsstoffe für Düngemittel sind beispielsweise in Ullmann's Encyclopedia of Industrial Chemistry, 5. Auflage, 1987, Band A 10, Seiten 363 bis 401, DE-A 41 28 828, DE-A 19 05 834 und DE-A 196 31 764 beschrieben. Die allgemeine Zusammensetzung der Düngemittel, bei welchen es sich im Rahmen der vorliegenden Erfindung um Einzelnährstoff- und/oder Mehrnährstoffdünger handeln kann, beispielsweise aus Stickstoff, Kalium oder Phosphor, kann innerhalb eines breiten Bereichs variieren. Im Allgemeinen ist ein Gehalt von 1 bis 30 Gew.-% Stickstoff (bevorzugt 5 bis 20 Gew.-%), von 1 bis 20 Gew.-% Kalium (bevorzugt 3 bis 15 Gew.-%) und ein Gehalt von 1 bis 20 Gew.-% Phosphor (bevorzugt 3 bis 10 Gew.-%) vorteilhaft. Der Gehalt von Mikroelementen ist üblicherweise im ppm-Bereich, bevorzugt im Bereich von von 1 bis 1000 ppm.

Im Rahmen der vorliegenden Erfindung können das Düngemittel sowie eine oder mehrere erfindungsgemäße Verbindungen der allgemeinen Formel (I) inklusive der Verbindung 1-[(2E)-1-Hydroxy-1,3-diphenylprop-2-en-1-yl]cyclopentan-carbonitril zeitgleich verabreicht werden. Es ist jedoch auch möglich, zunächst das Düngemittel und dann eine oder mehrere erfindungsgemäße Verbindungen der allgemeinen Formel (I) oder zunächst eine oder mehrere Verbindungen der allgemeinen Formel (I) inklusive der Verbindung 1-[(2E)-1-Hydroxy-1,3-diphenylprop-2-en-1-yl]cyclopentan-carbonitril und dann das Düngemittel anzuwenden. Bei nicht zeitgleicher Anwendung einer oder mehrerer Verbindungen der allgemeinen Formel (I) inklusive der Verbindung 1-[(2E)-1-Hydroxy-1,3-diphenylprop-2-en-1-yl]cyclopentan-carbonitril und des Düngemittels erfolgt im Rahmen der vorliegenden Erfindung jedoch die Anwendung in funktionellem Zusammenhang, insbesondere innerhalb eines Zeitraums von im Allgemeinen 24 Stunden, bevorzugt 18 Stunden, besonders bevorzugt 12 Stunden, speziell 6 Stunden, noch spezieller 4 Stunden, noch weiter spezieller innerhalb 2 Stunden. In ganz besonderen Ausführungsformen der vorliegenden Erfindung erfolgt die Anwendung einer oder mehrerer erfindungsgemäßer Verbindungen der Formel (I) inklusive der Verbindung 1-[(2E)-1-Hydroxy-1,3-diphenylprop-2-en-1-yl]cyclopentan-carbonitril und des Düngemittels in einem zeitlichen Rahmen von weniger als 1 Stunden, vorzugsweise weniger als 30 Minuten, besonders bevorzugt weniger als 15 Minuten.

Bevorzugt ist die Verwendung von Verbindungen der allgemeinen Formel (I) inklusive der Verbindung 1-[(2E)-1-Hydroxy-1,3-diphenylprop-2-en-1-yl]cyclopentan-carbonitril auf Pflanzen aus der Gruppe der Nutzpflanzen, Zierpflanzen, Rasenarten, allgemein genutzte Bäume, die in öffentlichen und privaten Bereichen als Zierpflanzen Verwendungen finden, und Forstbestand. Der Forstbestand umfasst Bäume für die Herstellung von Holz, Zellstoff, Papier und Produkten die aus Teilen der Bäume hergestellt werden. Der Begriff Nutzpflanzen, wie hier verwendet, bezeichnet Kulturpflanzen, die als Pflanzen für die Gewinnung von Nahrungsmitteln, Futtermitteln, Treibstoffe oder für technische Zwecke eingesetzt werden.

Zu den Nutzpflanzen zählen z. B. folgende Pflanzenarten: Triticale, Durum (Hartweizen), Turf, Reben, Getreide, beispielsweise Weizen, Gerste, Roggen, Hafer, Reis, Mais und Hirse; Rüben, beispielsweise Zuckerrüben und Futterrüben; Früchte, beispielsweise Kernobst, Steinobst und Beerenobst, beispielsweise Äpfel, Birnen, Pflaumen, Pfirsiche, Mandeln, Kirschen und Beeren, z. B. Erdbeeren, Himbeeren, Brombeeren; Hülsenfrüchte, beispielsweise Bohnen, Linsen, Erbsen und Sojabohnen; Ölkulturen, beispielsweise Raps, Senf, Mohn, Oliven, Sonnenblumen, Kokos, Castorölpflanzen, Kakaobohnen und Erdnüsse; Gurkengewächse, beispielsweise Kürbis, Gurken und Melonen; Fasergewächse, beispielsweise Baumwolle, Flachs, Hanf und Jute; Citrusfrüchte, beispielsweise Orangen, Zitronen, Pampelmusen und Mandarinen; Gemüsesorten, beispielsweise Spinat, (Kopf)-Salat, Spargel, Kohlarten, Möhren, Zwiebeln, Tomaten, Kartoffeln und Paprika; Lorbeergewächse, beispielsweise Avocado, Cinnamomum, Kampfer, oder ebenso Pflanzen wie Tabak, Nüsse, Kaffee, Aubergine, Zuckerrohr, Tee, Pfeffer, Weinreben, Hopfen, Bananen, Naturkautschukgewächse sowie Zierpflanzen, beispielsweise Blumen, Sträucher, Laubbäume und Nadelbäume wie Koniferen. Diese Aufzählung stellt keine Limitierung dar.

Als besonders geeignete Zielkulturen für die Anwendung des erfindungsgemäßen Verfahrens sind folgende Pflanzen anzusehen: Hafer, Roggen, Triticale, Durum, Baumwolle, Aubergine, Turf, Kernobst, Steinobst, Beerenobst, Mais, Weizen, Gerste, Gurke, Tabak, Reben, Reis, Getreide, Birne, Pfeffer, Bohnen, Sojabohnen, Raps, Tomate, Paprika, Melonen, Kohl, Kartoffel und Apfel.

Als Bäume, die entsprechend dem erfindungsgemäßen Verfahren verbessert werden können, seien beispielhaft genannt: Abies sp., Eucalyptus sp., Picea sp., Pinus sp., Aesculus sp., Platanus sp., Tilia sp., Acer sp., Tsuga sp., Fraxinus sp., Sorbus sp., Betula sp., Crataegus sp., Ulmus sp., Quercus sp., Fagus sp., Salix sp., Populus sp..

Als bevorzugte Bäume, die entsprechend dem erfindungsgemäßen Verfahren verbessert werden können, können genannt werden: Aus der Baumart Aesculus: A. hippocastanum, A. pariflora, A. carnea; aus der Baumart Platanus: P. aceriflora, P. occidentalis, P. racemosa; aus der Baumart Picea: P. abies; aus der Baumart Pinus: P. radiate, P. ponderosa, P. contorta, P. sylvestre, P. elliottii, P. montecola, P. albicaulis, P. resinosa, P. palustris, P. taeda, P. flexilis, P. jeffregi, P. baksiana, P. strobes; aus der Baumart Eucalyptus: E. grandis, E. globulus, E. camadentis, E. nitens, E. obliqua, E. regnans, E. pilularus.

Als besonders bevorzugte Bäume, die entsprechend dem erfindungsgemäßen Verfahren verbessert werden können, können genannt werden: Aus der Baumart Pinus: P. radiate, P. ponderosa, P. contorta, P. sylvestre, P. strobes; aus der Baumart Eucalyptus: E. grandis, E. globulus und E. camadentis.

Als besonders bevorzugte Bäume, die entsprechend dem erfindungsgemäßen Verfahren verbessert werden können, können genannt werden: Rosskastanie, Platanengewächs, Linde und Ahornbaum.

Die vorliegende Erfindung kann auch an beliebigen Rasenarten ("turfgrasses") durchgeführt werden, einschließlich "cool season turfgrasses" und "warm season turfgrasses". Beispiele für Rasenarten für die kalte Jahreszeit sind Blaugräser ("blue grasses"; Poa spp.), wie "Kentucky bluegrass" (Poa pratensis L.), "rough bluegrass" (Poa trivialis L.), "Canada bluegrass" (Poa compressa L.), "annual bluegrass" (Poa annua L.), "upland bluegrass" (Poa glaucantha Gaudin), "wood bluegrass" (Poa nemoralis L.) und "bulbous bluegrass" (Poa bulbosa L.); Straussgräser ("Bentgrass", Agrostis spp.), wie "creeping bentgrass" (Agrostis palustris Huds.), "colonial bentgrass" (Agrostis tenuis Sibth.), "velvet bentgrass" (Agrostis canina L.), "South German Mixed Bentgrass" (Agrostis spp. einschließlich Agrostis tenius Sibth., Agrostis canina L., und Agrostis palustris Huds.), und "redtop" (Agrostis alba L.);

Schwingel ("Fescues", Festucu spp.), wie "red fescue" (Festuca rubra L. spp. rubra), "creeping fescue" (Festuca rubra L.), "chewings fescue" (Festuca rubra commutata Gaud.), "sheep fescue" (Festuca ovina L.), "hard fescue" (Festuca longifolia Thuill.), "hair fescue" (Festucu capillata Lam.), "tall fescue" (Festuca arundinacea Schreb.) und "meadow fescue" (Festuca elanor L.);

Lolch ("ryegrasses", Lolium spp.), wie "annual ryegrass" (Lolium multiflorum Lam.), "perennial ryegrass" (Lolium perenne L.) und "italian ryegrass" (Lolium multiflorum Lam.);

und Weizengräser ("wheatgrasses", Agropyron spp..), wie "fairway wheatgrass" (Agropyron cristatum (L.) Gaertn.), "crested wheatgrass" (Agropyron desertorum (Fisch.) Schult.) und "western wheatgrass" (Agropyron smithii Rydb.).

Beispiele für weitere "cool season turfgrasses" sind "beachgrass" (Ammophila breviligulata Fern.), "smooth bromegrass" (Bromus inermis Leyss.), Schilf ("cattails") wie "Timothy" (Phleum pratense L.), "sand cattail" (Phleum subulatum L.), "orchardgrass" (Dactylis glomerata L.), "weeping alkaligrass" (Puccinellia distans (L.) Parl.) und "crested dog's-tail" (Cynosurus cristatus L.).

Beispiele für "warm season turfgrasses" sind "Bermudagrass" (Cynodon spp. L. C. Rich), "zoysiagrass" (Zoysia spp. Willd.), "St. Augustine grass" (Stenotaphrum secundatum Walt Kuntze), "centipedegrass" (Eremochloa ophiuroides Munro Hack.), "carpetgrass" (Axonopus affinis Chase), "Bahia grass" (Paspalum notatum Flugge), "Kikuyugrass" (Pennisetum clandestinum Hochst. ex Chiov.), "buffalo grass" (Buchloe dactyloids (Nutt.) Engelm.), "Blue gramma" (Bouteloua gracilis (H.B.K.) Lag. ex Griffiths), "seashore paspalum" (Paspalum vaginatum Swartz) und "sideoats grama" (Bouteloua curtipendula (Michx. Torr.). "Cool season turfgrasses" sind für die erfindungsgemäße Verwendung im Allgemeinen bevorzugt. Besonders bevorzugt sind Blaugras, Straussgras und "redtop", Schwingel und Lolch. Straussgras ist insbesondere bevorzugt.

Besonders bevorzugt werden mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder mit Hilfe rekombinanter DNA-Techniken, gezüchtet worden sind. Kulturpflanzen können demnach Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten.

Das erfindungsgemäße Behandlungsverfahren kann somit auch für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, dass es ein interessierendes Protein oder Polypeptid exprimiert oder dasses ein anderes Gen, das in der Pflanze vorliegt bzw. andere Gene, die in der Pflanze vorliegen, herunterreguliert oder abschaltet (zum Beispiel mittels Antisense-Technologie, Co-suppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

Zu Pflanzen und Pflanzensorten, die vorzugsweise mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril behandelt werden können, sind solche Pflanzen, die gegen einen oder mehrere abiotische Streßfaktoren resistent sind. Zu den abiotischen Streßbedingungen können zum Beispiel Hitze, Dürre, Kälte- und Trockenstress,, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die ebenfalls mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben (WO 92/005251, WO 95/009910, WO 98/27806, WO 2005/002324, WO 2006/021972 und US 6,229,072). Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden (z. B. WO 91/002069).

Pflanzen oder Pflanzensorten (die mit Methoden der Pflanzenbiotechnologie, wie der Gentechnik, erhalten werden), die ebenfalls mit den erfindungsgemäßen Verbindungen der allgemeinen Formel (I) behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d.h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakteriums Salmonella typhimurium (Comai et al., Science (1983), 221, 370-371), das CP4-Gen des Bakteriums Agrobacterium sp. (Barry et al., Curr. Topics Plant Physiol. (1992), 7, 139-145), die Gene, die für eine EPSPS aus der Petunie (Shah et al., Science (1986), 233, 478-481), für eine EPSPS aus der Tomate (Gasser et al., J. Biol. Chem. (1988), 263, 4280-4289) oder für eine EPSPS aus Eleusine (WO 2001/66704) kodieren. Es kann sich auch um eine mutierte EPSPS handeln, wie sie zum Beispiel in EP-A 0837944, WO 2000/066746, WO 2000/066747 oder WO 2002/026995 beschrieben ist. Glyphosatetolerante Pflanzen können auch dadurch erhalten werden, dass man ein Gen exprimiert, das für ein Glyphosate-Oxidoreduktase-Enzym, wie es in US 5,776,760 und US 5,463,175 beschrieben ist, kodiert. Glyphosatetolerante Pflanzen können auch dadurch erhalten werden, dass man ein Gen exprimiert, das für ein Glyphosate-acetyltransferase-Enzym, wie es in z. B. WO 2002/036782, WO 2003/092360, WO 2005/012515 und WO 2007/024782 beschrieben ist, kodiert. Glyphosatetolerante Pflanzen können auch dadurch erhalten werden, dass man Pflanzen, die natürlich vorkommende Mutationen der oben erwähnten Gene, wie sie zum Beispiel in WO 2001/024615 oder WO 2003/013226 beschrieben sind, enthalten, selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind zum Beispiel in US 5,561,236; US 5,648,477; US 5,646,024; US 5,273,894; US 5,637,489; US 5,276,268; US 5,739,082; US 5,908,810 und US 7,112,665 beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym gemäß WO 96/038567, WO 99/024585 und WO 99/024586 kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen und Gene sind in WO 99/034008 und WO 2002/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie dies in WO 2004/024928 beschrieben ist.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen, wie dies zum Beispiel bei Tranel und Wright, Weed Science (2002), 50, 700-712, jedoch auch in US 5,605,011, US 5,378,824, US 5,141,870 und US 5,013,659, beschrieben ist. Die Herstellung von sulfonylharnstofftoleranten Pflanzen und imidazolinontoleranten Pflanzen ist in US 5,605,011; US 5,013,659; US 5,141,870; US 5,767,361; US 5,731,180; US 5,304,732; US 4,761,373; US 5,331,107; US 5,928,937; und US 5,378,824; sowie in der internationalen Veröffentlichung WO 96/033270 beschrieben. Weitere imidazolinontolerante Pflanzen sind auch in z. B. WO 2004/040012, WO 2004/106529, WO 2005/020673, WO 2005/093093, WO 2006/007373, WO 2006/015376, WO 2006/024351 und WO 2006/060634 beschrieben. Weitere sulfonylharnstoff- und imidazolinontolerante Pflanzen sind auch in z.B. WO 2007/024782 beschrieben.

Weitere Pflanzen, die gegenüber ALS-Inhibitoren, insbesondere gegenüber Imidazolinonen, Sulfonylharnstoffen und/oder Sulfamoylcarbonyltriazolinonen tolerant sind, können durch induzierte Mutagenese, Selektion in Zellkulturen in Gegenwart des Herbizids oder durch Mutationszüchtung erhalten werden, wie dies zum Beispiel für die Sojabohne in US 5,084,082, für Reis in WO 97/41218, für die Zuckerrübe in US 5,773,702 und WO 99/057965, für Salat in US 5,198,599 oder für die Sonnenblume in WO 2001/065922 beschrieben ist.

Pflanzen oder Pflanzensorten (die nach Methoden der pflanzlichen Biotechnologie, wie der Gentechnik, erhalten wurden), die ebenfalls mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus Bacillus thuringiensis oder einen insektiziden Teil davon, wie die insektiziden Kristallproteine, die von Crickmore et al., Microbiology and Molecular Biology Reviews (1998), 62, 807-813, zusammengestellt wurden, von Crickmore et al. (2005) in der Bacillus thuringiensis-Toxinnomenklatur aktualisiert (online bei:
   http://www.lifesci.sussex.ac.uk/Home/Neil_Crickmore/Bt/), oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus Bacillus thuringiensis oder einen Teil davon, der in Gegenwart eines zweiten, anderen Kristallproteins als Bacillus thuringiensis oder eines Teils davon insektizid wirkt, wie das binäre Toxin, das aus den Kristallproteinen Cy34 und Cy35 besteht (Moellenbeck et al., Nat. Biotechnol. (2001), 19, 668-72; Schnepf et al., Applied Environm. Microb. (2006), 71, 1765-1774); oder
3) ein insektizides Hybridprotein, das Teile von zwei unterschiedlichen insektiziden Kristallproteinen aus Bacillus thuringiensis umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604; oder
5) ein insektizides sezerniertes Protein aus Bacillus thuringiensis oder Bacillus cereus oder einen insektiziden Teil davon, wie die vegetativ wirkenden insektentoxischen Proteine (vegetative insecticidal proteins, VIP), die unter folgendem Link angeführt sind, z. B. Proteine der Proteinklasse VIP3Aa:
   http://www.lifesci.sussex.ac.uk/Home/Neil_Crickmore/Bt/vip.html oder
6) ein sezerniertes Protein aus Bacillus thuringiensis oder Bacillus cereus, das in Gegenwart eines zweiten sezernierten Proteins aus Bacillus thuringiensis oder B. cereus insektizid wirkt, wie das binäre Toxin, das aus den Proteinen VIP1A und VIP2A besteht (WO 94/21795); oder
7) ein insektizides Hybridprotein, das Teile von verschiedenen sezernierten Proteinen von Bacillus thuringiensis oder Bacillus cereus umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten Klassen 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag, wie dies in WO 2000/004173 oder WO2006/045633 oder WO2007/131699 beschrieben ist.
b. Pflanzen, die ein streßtoleranzförderndes Transgen enthalten, das die Expression und/oder Aktivität der für PARG kodierenden Gene der Pflanzen oder Pflanzenzellen zu reduzieren vermag, wie dies z.B. in WO 2004/090140 beschrieben ist;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase,
   Nicotinatphosphoribosyltransferase, Nicotinsäuremononukleotid-adenyltransferase, Nicotinamidadenindinukleotidsynthetase oder Nicotinamidphosphoribosyl-transferase, wie dies z. B. in WO2006/032469 oder WO 2006/133827 oder WO2007/107326 beschrieben ist.

Pflanzen oder Pflanzensorten (die nach Methoden der pflanzlichen Biotechnologie, wie der Gentechnik, erhalten wurden), die ebenfalls mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet. Diese transgenen Pflanzen, die eine modifizierte Stärke synthetisieren, sind zum Beispiel in EP 0571427, WO 95/004826, EP 0719338, WO 96/15248, WO 96/19581, WO 96/27674, WO 97/11188, WO 97/26362, WO 97/32985, WO 97/42328, WO 97/44472, WO 97/45545, WO 98/27212, WO 98/40503, WO 99/58688, WO 99/58690, WO 99/58654, WO 2000/008184, WO 2000/008185, WO 2000/28052, WO 2000/77229, WO 2001/12782, WO 2001/12826, WO 2002/101059, WO 2003/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 2000/22140, WO 2006/063862, WO 2006/072603, WO 2002/034923, WO2008/017518, WO2008/080630, WO2008/080631, WO2008/090008, WO 2001/14569, WO 2002/79410, WO 2003/33540, WO 2004/078983, WO 2001/19975, WO 95/26407, WO 96/34968, WO 98/20145, WO 99/12950, WO 99/66050, WO 99/53072, US 6,734,341, WO 2000/11192, WO 98/22604, WO 98/32326, WO 2001/98509, WO 2001/98509, WO 2005/002359, US 5,824,790, US 6,013,861, WO 94/004693, WO 94/009144, WO 94/11520, WO 95/35026 bzw. WO 97/20936 beschrieben.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, wie dies in EP 0663956, WO 96/001904, Wo 96/021023, WO 98/039460 und WO 99/024593 beschrieben ist, Pflanzen, die alpha-1,4-Glucane produzieren, wie dies in WO 95/031553, US 2002/031826, US 6,284,479, US 5,712,107, WO 97/047806, WO 97/047807, WO 97/047808 und WO 2000/14249 beschrieben ist, Pflanzen, die alpha-1,6-verzweigte alpha-1,4-Glucane produzieren, wie dies in WO 2000/73422 beschrieben ist, und Pflanzen, die Alternan produzieren, wie dies in WO 2000/047727, EP 06077301.7, US 5,908,975 und EP 0728213 beschrieben ist.
3) Transgene Pflanzen, die Hyaluronan produzieren, wie dies zum Beispiel in WO 2006/032538, WO 2007/039314, WO 2007/039315, WO 2007/039316, JP 2006/304779 und WO 2005/012529 beschrieben ist.

Pflanzen oder Pflanzensorten (die nach Methoden der pflanzlichen Biotechnologie, wie der Gentechnik, erhalten wurden), die ebenfalls mit den erfindungsgemäßen Verbindungen der allgemeinen Formel (I) behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten, wie dies in WO 98/000549 beschrieben ist,
b) Pflanzen wie Baumwollpflanzen, die eine veränderte Form von rsw2- oder rsw3-homologen Nukleinsäuren enthalten, wie dies in WO 2004/053219 beschrieben ist;
c) Pflanzen wie Baumwollpflanzen mit einer erhöhten Expression der Saccharosephosphatsynthase, wie dies in WO 2001/017333 beschrieben ist;
d) Pflanzen wie Baumwollpflanzen mit einer erhöhten Expression der Saccharosesynthase, wie dies in WO 2002/45485 beschrieben ist;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase, wie dies in WO 2005/017157 beschrieben ist;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen, wie dies in WO 2006/136351 beschrieben ist.

Pflanzen oder Pflanzensorten (die nach Methoden der pflanzlichen Biotechnologie, wie der Gentechnik, erhalten wurden), die ebenfalls mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren, wie dies zum Beispiel in US 5,969,169, US 5,840,946 oder US 6,323,392 oder US 6,063, 947 beschrieben ist;
b) Pflanzen wie Rapspflanzen, die Öl mit einem niedrigen Linolensäuregehalt produzieren, wie dies in US 6,270828, US 6,169,190 oder US 5,965,755 beschrieben ist.
c) Pflanzen wie Rapspflanzen, die Öl mit einem niedrigen gesättigten Fettsäuregehalt produzieren, wie dies z. B. in US 5,434,283 beschrieben ist.

Besonders nützliche transgene Pflanzen, die mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril behandelt werden können, sind Pflanzen, die Transformations-Events, oder eine Kombination von Transformations-Events, enthalten und die zum Beispiel in den Dateien von verschiedenen nationalen oder regionalen Behörden angeführt sind.

Besonders nützliche transgene Pflanzen, die mit den erfindungsgemäßen Verbindungen der allgemeinen Formel (I) inklusive der Verbindung 1-[(2E)-1-Hydroxy-1,3-diphenylprop-2-en-1-yl]cyclopentan-carbonitril behandelt werden können, sind beispielhaft Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), NuCOTN® (Baumwolle), NuCOTN 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Die erfindungsgemäß zu verwendenden Verbindungen der Formel (I) inklusive der Verbindung 1-[(2E)-1-Hydroxy-1,3-diphenylprop-2-en-1-yl]cyclopentan-carbonitril können in übliche Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen. Im Rahmen der vorliegenden Erfindung ist es insbesondere bevorzugt, wenn die Verbindungen der allgemeinen Formel (I) inklusive der Verbindung 1-[(2E)-1-Hydroxy-1,3-diphenylprop-2-en-1-yl]cyclopentan-carbonitril in der Form einer Sprühformulieruing verwendet werden.

Die vorliegende Erfindung betrifft daher darüber hinaus auch eine Sprühformulierung zur Steigerung der Widerstandsfähigkeit von Pflanzen gegenüber abiotischem Stress. Im Folgenden wird eine Sprühformulierung näher beschrieben:
Die Formulierungen zur Sprühapplikation werden in bekannter Weise hergestellt, z.B. durch Vermischen der erfindungsgemäß zu verwendenden Verbindungen der allgmeinen Formel (I) inklusive der Verbindung 1-[(2E)-1-Hydroxy-1,3-diphenylprop-2-en-1-yl]cyclopentan-carbonitril mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Weitere übliche Zusatzstoffe, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser, können gegebenenfalls auch verwendet werden. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Als Netzmittel, die in den erfindungsgemäß verwendbaren Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutylnaphthalin-Sulfonate.

Als Dispergiermittel und/oder Emulgatoren, die in den erfindungsgemäß verwendbaren Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose. Als Gibberelline, die in den erfindungsgemäß verwendbaren Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Weitere Additive können Duftstoffe, mineralische oder vegetabilische gegebenenfalls modifizierte Öle, Wachse und Nährstoffe (auch Spurennährstoffe), wie Salze von Eisen, Mangan, Bor, Kupfer, Kobalt, Molybdän und Zink sein. Weiterhin enthalten sein können Stabilisatoren wie Kältestabilisatoren, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-%, vorzugsweise zwischen 0,5 und 90 %, der Verbindung der allgemeinen Formel (I).

Die erfindungsgemäßen Verbindungen der allgemeinen Formel (I) können in handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Ferner lässt sich die beschriebene positive Wirkung der Verbindungen der Formel (I) inklusive der Verbindung 1-[(2E)-1-Hydroxy-1,3-diphenylprop-2-en-1-yl]cyclopentan-carbonitril auf die pflanzeneigenen Abwehrkräfte durch eine zusätzliche Behandlung mit insektziden, fungiziden oder bakteriziden Wirkstoffen unterstützen.

Bevorzugte Zeitpunkte für die Applikation der erfindungsgemäß zu verwendenden Verbindungen der allgemeinen Formel (I) inklusive der Verbindung 1-[(2E)-1-Hydroxy-1,3-diphenylprop-2-en-1-yl]cyclopentan-carbonitril oder deren Salze zur Steigerung der Resistenz gegenüber abiotischem Stress sind Boden-, Stamm- und/oder Blattbehandlungen mit den zugelassenen Aufwandmengen.

Die erfindungsgemäßen Wirkstoffe der allgemeinen Formel (I) inklusive der Verbindung 1-[(2E)-1-Hydroxy-1,3-diphenylprop-2-en-1-yl]cyclopentan-carbonitril oder deren jewielige Salze können im Allgemeinen darüber hinaus in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, Bakteriziden, wachstumsregulierenden Stoffen, die Pflanzenreife beeinflussenden Stoffen, Safenern oder Herbiziden vorliegen.

Die Erfindung soll durch die nachfolgenden biologischen Beispiele veranschaulicht werden, ohne sie jedoch darauf einzuschränken.

### Biologische Beispiele:

### In vivo-Analysen

Samen von mono- bzw. dikotylen Kulturpflanzen wurden in Plastiktöpfen in sandigem Lehmboden ausgesät, mit Erde oder Sand abgedeckt und im Gewächshaus unter guten Wachstumsbedingungen angezogen. Die Behandlung der Versuchspflanzen erfolgte im frühen Laubblattstadium (BBCH10 - BBCH13). Zur Gewährleistung einer uniformen Wasserversorgung vor Stressbeginn wurden die bepflanzten Töpfe vor Substanzapplikation durch Anstaubewässerung mit Wasser versorgt.

Die in Form von benetzbaren Pulvern (WP) formulierten erfindungsgemässen Verbindungen wurden als wässrige Suspension mit einer Wasseraufwandmenge von umgerechnet 600 l/ha unter Zusatz von 0,2% Netzmittel (z.B. Agrotin) auf die grünen Pflanzenteile gesprüht. Unmittelbar nach Substanzapplikation erfolgte die Stressbehandlung der Pflanzen.

Der Trockenstress wurde durch langsames Abtrocknen unter folgenden Bedingungen induziert:
"Tag": 14 Stunden beleuchtet bei ∼ 26-30°C
"Nacht": 10 Stunden ohne Beleuchtung bei ∼ 18-20°C.

Die Dauer der jeweiligen Stressphasen richtete sich hauptsächlich nach dem Zustand der gestressten Kontrollpflanzen. Sie wurde (durch Wiederbewässerung und Transfer in ein Gewächshaus mit guten Wachstumsbedingungen) beendet, sobald irreversible Schäden an den gestressten Kontrollpflanzen zu beobachten waren.

Nach Beendigung der Stressphase folgte eine ca. 4-7 tägige Erholungsphase, während der die Pflanzen abermals unter guten Wachstumsbedingungen im Gewächshaus gehalten wurden. Die Dauer der Erholungsphase richtete sich hauptsächlich danach, wann die Versuchspflanzen einen Zustand erreicht hatten, der eine visuelle Bonitur potenzieller Effekte ermöglichte, und war daher variabel.

Wenn dieser Zeitpunkt erreicht war, wurde das Erscheinungsbild der mit Testsubstanzen behandelten Pflanzen im Vergleich zu den gestressten Kontrollpflanzen nach folgenden Kategorien erfasst:

| | |
|---|---|
| 0 | kein positiver Effekt |
| 10 | leicht positiver Effekt |
| 20 | deutlich positiver Effekt |
| 30 | stark positiver Effekt |

Um auszuschliessen, dass die beobachteten Effekte von der ggf. fungiziden oder insektiziden Wirkung der Testverbindungen beeinflusst wurden, wurde zudem darauf geachtet, dass die Versuche ohne Pilzinfektion oder Insektenbefall abliefen.

In jedem Versuch wurden pro Kultur und Dosierung Pflanzen in 3 Töpfen behandelt und separat ausgewertet. Die in den unten stehenden Tabellen A-1 bis A-3 angegebenen Werte für die erhaltenen Effekte stellen Mittelwerte aus den erhaltenen Bonituren dar.

Wirkungen ausgewählter Verbindungen der allgemeinen Formel (I) unter Trockenstress:

**Tabelle A-1**

| No. | Substanz | Dosierung | Einheit | Effekt (ZEAMX) |
|---|---|---|---|---|
| 1 | I.1-130 | 250 | g/ha | 20 |

**Tabelle A-2**

| No. | Substanz | Dosierung | Einheit | Effekt (TRZAS) |
|---|---|---|---|---|
| 1 | I.1-130 | 250 | g/ha | 20 |
| 2 | I.1-244 | 25 | g/ha | 10-20 |
| 3 | I.1-621 | 250 | g/ha | 10 |
| 4 | I.1-622 | 25 | g/ha | 10-20 |
| 5 | I.1-623 | 250 | g/ha | 10-20 |
| 6 | I.1-624 | 25 | g/ha | 10-20 |

**Tabelle A-3**

| No. | Substanz | Dosierung | Einheit | Effekt (BRSNS) |
|---|---|---|---|---|
| 1 | I.1-244 | 25 | g/ha | 20 |
| 2 | I.1-623 | 25 | g/ha | 20 |
| 3 | I.1-624 | 25 | g/ha | 20 |

In den zuvor genannten Tabellen bedeuten:
- TRZAS: = Triticum aestivum
- ZEAMX: = Zea mays
- BRSNS: = Brassica napus

### In vitro-Analysen

Effekte des Phytohormons Abscisinsäure (ABA) auf das Verhalten von Pflanzen unter abiotischem Stress und der Wirkmechanismus von ABA sind in der Literatur beschrieben (vgl. Abrams et al, WO97/23441, Cutler, Park et al. Science, 2009, 324, 1068; Grill et al. Science, 2009, 324, 1064; Tanokura et al. Biophysics, 2011, 7, 123; Schroeder et al. Plant J. 2010, 61, 290). Daher kann man mit Hilfe eines geeigneten in vitro-Testsystems eine Korrelation zwischen der Wirkung von ABA und der Stressantwort einer Pflanze unter abiotischem Stress ableiten. Unter Wassermangel (Trockenstress) bilden Pflanzen das Phytohormon Abscisinsäure (ABA). Dieses bindet mit einem Co-Regulator (Regulatory Component of ABA-Receptor = RCAR nach Grill et al. Science, 2009, 324, 1064 oder PYR/PYL nach Cutler et al. Science, 2009, 324, 1068) an eine Phosphatase (z.B. ABI1, eine Typ 2C Proteinphosphatase, auch als PP2C abgekürzt) und hemmt diese in ihrer Aktivität. In der Folge wird eine "downstream" Kinase (z.B. SnRK2) nicht mehr dephosphoryliert. Diese somit aktive Kinase schaltet über Phosphorylierung von Transkriptionsfaktoren (z.B. AREB/ABF, vgl. Yoshida et al Plant J. 2010, 61, 672) ein genetisches Schutzprogramm zur Erhöhung der Trockenstresstoleranz an.
In dem im Folgenden beschriebenen Assay wird die Hemmung der Phosphatase ABI1 über den Co-Regulator RCAR11/PYR1 aus *Arabidopsis thaliana* genutzt. Für die Aktivitätsbestimmung wurde die Dephosphorylierung von 4-Methylumbelliferylphosphat (MUP) bei 460nm gemessen. Der in vitro Assay wurde in Greiner 384-well PS-Mikroplatten F-well durchgeführt unter Verwendung von zwei Kontrollen, a) Dimethylsulfoxid (DMSO) 0.5% (f.c.) sowie b) 5 µM (f.c.) Abscisinsäure (ABA). Der hier beschriebene Assay wurde im Allgemeinen mit Substratkonzentrationen der entsprechenden chemischen Testsubstanzen in einem Konzentrationsbereich von 0.1 µM bis 100 µM in einer Lösung aus DMSO und Wasser durchgeführt. Die so erhaltene Substanzlösung wurde gegebenenfalls mit Esterase aus Schweineleber (EC 3.1.1.1) 3 h lang bei Raumtemperatur gerührt und 30 Min lang bei 4000rpm zentrifugiert. In jede Kavität der Mikroplatte wurde ein Gesamtvolumen von 45 µL gegeben, das sich wie folgt zusammensetzte:
1) 5µL Substanzlösung, d.h. a) DMSO 5% oder b) Abscisinsäurelösung oder c) die entsprechende Beispielverbindung der allgemeinen Formel (I) gelöst in 5% DMSO.
2) 20µL Enzympuffermix, der sich aus a) 40 Vol% Enzympuffer (10 mL enthalten zu gleichen Volumenanteilen 500 mM Tris-HCl pH8, 500 mM NaCl, 3.33 mM MnCl₂, 40 mM Dithiothreitol (DTT)), b) 4 Vol% ABI1-Verdünnung (Proteinstammlösung wurde so verdünnt, daß nach Zugabe eine Endkonzentration im Assay von 0.15 µg ABI1/well entsteht), c) 4 Vol% RCAR11-Verdünnung (Enzymstock wurde so verdünnt, daß bei Zugabe der Verdünnung in den Enzympuffermix eine Endkonzentration im Assay von 0.30 µg Enzym/well entsteht), d) 5 Vol% Tween20 (1%), e) 47 Vol% H₂O bi-dest zusammensetzt.
3) 20µL Substratmix, der sich aus a) 10 Vol% 500 mM Tris-HCl pH8, b) 10 Vol% 500 mM NaCl, c) 10 Vol% 3.33 mM MnCl₂, d) 5 Vol% 25 mM MUP, 5 Vol% Tween20 (1%), 60 Vol% H₂O bi-dest zusammensetzt

Enzympuffermix und Substratmix wurden 5 Minuten vor der Zugabe angesetzt und auf eine Temperatur von 35 °C erwärmt. Nach vollständigem Pipettieren aller Lösungen und vollständiger Durchmischung wurde die Platte 20 Minuten lang bei 35 °C inkubiert. Abschließend erfolgte eine relative Fluoreszenzmessung bei 35 °C mit einem Mikroplatten-Lesegerät "POLARstar Optima" der Firma BMG Labtech unter Verwendung eines Anregungsfilters 340/10 nm und einem Emmissionsfilter von 460 nm. Die Wirkstärke der Verbindungen der allgemeinen Formel (I) wird in der nachfolgenden Tabelle unter Verwendung von Abscisinsäure (No. 5) als Vergleichssubstanz nach folgender Einteilung angegeben: ++++ (Inhibition ≥ 90 %), +++ (90 % > Inhibition ≥ 70%), ++ (70 % > Inhibition ≥ 50%), + (50 % > Inhibition ≥ 30%).
Effekte ausgewählter Verbindungen der allgemeinen Formel (I) im oben beschriebenen in vitro-Assay bei einer Konzentration von 5mM der betreffenden Substanz der allgemeinen Formel (I) in einer Lösung aus DMSO und Wasser:

**Tabelle B-1**

| No. | Substanz | ABI1 Inhibition |
|---|---|---|
| 1 | I.1-127 | +++ |
| 2 | I.1-244 | ++ |
| 3 | I.1-558 | ++ |
| 4 | I.1-624 | ++ |
| 5 | Abscisinsäure | ++++ |

Ähnliche Ergebnisse konnten auch noch mit weiteren Verbindungen der allgemeinen Formel (I) auch bei Applikation auf andere Pflanzenarten erzielt werden.

## Patentansprüche

1. Substituierte Vinyl- und Alkinyl-cyanocycloalkanole sowie Vinyl- und Alkinyl-cyanoheterocyclylalkanole der allgemeinen Formel (I) oder deren Salze, 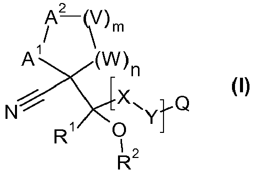 wobei
[X-Y] für die Gruppierungen 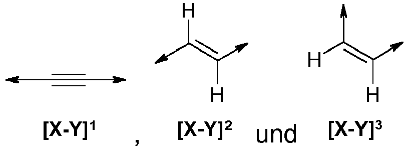 steht,
Q für die carbocyclischen und heterocyclischen Gruppierungen 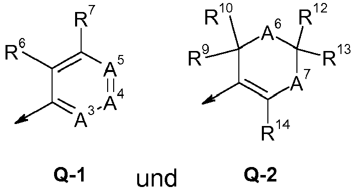 steht, wobei die Gruppierungen R⁶, R⁷, R⁹, R¹⁰, R¹², R¹³, R¹⁴, A³, A⁴, A⁵, A⁶ und A⁷ jeweils die Bedeutung gemäß der nachstehenden Definitionen haben und wobei der Pfeil für eine Bindung zur jeweiligen Gruppierung [X-Y] steht,
R¹ für Alkyl, Aryl, Heteroaryl, Heterocyclyl, Cycloalkyl, Alkenyl, Alkinyl, Alkoxyalkyl, Hydroxyalkyl, Haloalkyl, Haloalkenyl, Haloalkoxyalkyl, Alkylthioalkyl, Arylalkyl, Heterocyclylalkyl, Halocycloalkyl, Cycloalkenyl, Alkoxyalkoxyalkyl, Cycloalkylalkyl, Cycloalkenylalkyl, Haloalkinyl, Alkylsulfinylalkyl, Alkylsulfonylalkyl, Halocycloalkylalkyl Cycloalkylsulfinylalkyl, Cycloalkylsulfonylalkyl, Arylsulfinylalkyl, Arylsulfonylalkyl, Arylthioalkyl, Cycloalkylthioalkyl, Alkoxyhaloalkyl, Haloalkoxyhaloalkyl steht,
R² für Wasserstoff, Alkyl, Alkoxyalkyl, Alkoxyalkoxyalkyl, Alkenyl, Alkinyl, Alkylcarbonyl, Arylcarbonyl, Heteroarylcarbonyl, Cycloalkylcarbonyl, Alkenylcarbonyl, Heterocyclylcarbonyl, Alkoxycarbonyl, Alkenyloxycarbonyl, Aryloxyalkyl, Arylalkoxycarbonyl, Cycloalkoxycarbonyl, Cycloalkylalkoxycarbonyl, Arylalkoxyalkyl, Arylalkyl, Alkoxyalkoxyalkyl, Alkylthioalkyl, Trialkylsilyl, Alkyl(Bis-alkyl)silyl, Alkyl(Bis-aryl)silyl, Aryl(Bis-alkyl)silyl, Cycloalkyl(Bis-alkyl)silyl, Halo(Bis-alkyl)silyl, Trialkylsilylalkoxyalkyl, Trialkylsilylalkyl, Alkinyloxycarbonyl, Cycloalkyl, Cycloalkylalkyl, Aminocarbonyl, Alkylaminocarbonyl, BisAlkylaminocarbonyl, Cycloalkylaminocarbonyl, Alkylsulfonyl, Haloalkylsulfonyl, Arylsulfonyl, Heteroarylsulfonyl, Cycloalkylsulfonyl steht,
A¹, A², V, W unabhängig voneinander für eine Gruppe CR³R⁴, eine Gruppe N-R⁵, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht,
R³ und R⁴ unabhängig voneinander für Wasserstoff, Alkyl, Halogen, Cycloalkyl, Alkoxy, Aryl, Heterocyclyl, Heteroaryl, Arylalkyl, Alkylthio, Haloalkyl, Haloalkyloxy, Haloalkylthio, Alkoxyalkyl, Alkylthioalkyl, Heteroarylalkyl, Heterocyclylalkyl, Cycloalkylalkyl, Cycloalkenyl, Alkinyl, Alkenyl, Haloalkenyl, Haloalkinyl, Alkylsulfinyl, Alkylsulfonyl, Cycloalkylsulfinyl, Cycloalkylsulfonyl, Arylsulfinyl, Arylsulfonyl, Alkoxyhaloalkyl, Haloalkoxyhaloalkyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden,
R⁵ für Wasserstoff, Alkyl, Alkenyl, Alkinyl, Alkoxyalkyl, Alkylcarbonyl, Arylcarbonyl, Heteroarylcarbonyl, Cycloalkylcarbonyl, Alkoxycarbonyl, Alkenyloxycarbonyl, Alkoxycarbonylalkyl, Alkylaminocarbonylalkyl, Arylaminocarbonylalkyl, Aryloxyalkyl, Arylalkyl, Heteroarylalkyl, Heterocyclyl, Heterocyclylalkyl, Haloalkyl stehen,
R⁶ und R⁷ unabhängig voneinander für Wasserstoff, Nitro, Amino, Cyano, Thiocyanato, Isothiocyanato, Halogen, Alkyl, Cycloalkyl, Alkenyl, Alkinyl, Aryl, Arylalkyl, Arylalkoxy, Heteroaryl, Alkoxyalkyl, Hydroxyalkyl, Haloalkyl, Halocycloalkyl, Alkoxy, Haloalkoxy, Aryloxy, Heteroaryloxy, Cycloalkyloxy, Hydroxy, Cycloalkylalkoxy, Alkoxycarbonyl, Hydroxycarbonyl, Aminocarbonyl, Alkylaminocarbonyl, Cycloalkylaminocarbonyl, Cyanoalkylaminocarbonyl, Alkenylaminocarbonyl, Alkinylaminocarbonyl, Alkylamino, Alkylthio, Haloalkylthio, Hydrothio, Bisalkylamino, Cycloalkylamino, Alkylcarbonylamino, Cycloalkylcarbonylamino, Formylamino, Haloalkylcarbonylamino, Alkoxycarbonylamino, Alkylaminocarbonylamino, Alkyl(Alkyl-)aminocarbonylamino, Alkylsulfonylamino, Cycloalkylsulfonylamino, Arylsulfonylamino, Hetarylsulfonylamino, Sulfonylhaloalkylamino, Aminosulfonyl, Aminoalkylsulfonyl, Aminohaloalkylsulfonyl, Alkylaminosulfonyl, Bisalkylaminosulfonyl, Cycloalkylaminosulfonyl, Haloalkylaminosulfonyl, Arylaminosulfonyl, Arylalkylaminosulfonyl, Alkylsulfonyl, Cycloalkylsulfonyl, Arylsulfonyl, Alkylsulfinyl, Cycloalkylsulfinyl, Arylsulfinyl, N,S-Dialkylsulfonimidoyl, S-Alkylsulfonimidoyl, Alkylsulfonylaminocarbonyl, Cycloalkylsulfonylaminocarbonyl, Cycloalkylaminosulfonyl, Arylalkylcarbonylamino, Cycloalkylalkylcarbonylamino, Heteroarylcarbonylamino, Alkoxyalkylcarbonylamino, Hydroxyalkylcarbonylamino, Trialkylsilyl stehen,
A³, A⁴, A⁵ gleich oder verschieden sind und unabhängig voneinander für N (Stickstoff) oder die Gruppierung C-R⁸ stehen, wobei jedoch in keinem Fall mehr als zwei N-Atome benachbart sind, und wobei R⁸ in der Gruppierung C-R⁸ jeweils gleiche oder verschiedene Bedeutungen gemäß der nachstehenden Definition hat und
A³ und A⁴, wenn beide für eine Gruppe C-R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
A⁴ und A⁵, wenn beide für eine Gruppe C-R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
R⁸ und R¹⁴, unabhängig voneinander jeweils für Wasserstoff, Nitro, Amino, Hydroxy, Hydrothio, Thiocyanato, Isothiocyanato, Halogen, Alkyl, Cycloalkyl, Alkenyl, Alkinyl, Aryl, Arylalkyl, Arylalkoxy, Heteroaryl, Haloalkyl, Halocycloalkyl, Alkoxy, Haloalkoxy, Aryloxy, Heteroaryloxy, Cycloalkyloxy, Cycloalkylalkoxy, Hydroxyalkyl, Alkoxyalkyl, Aryloxyalkyl, Heteroaryloxyalkyl, Alkylamino, Alkylthio, Haloalkylthio, Bisalkylamino, Cycloalkylamino, Alkylcarbonylamino, Cycloalkylcarbonylamino, Formylamino, Haloalkylcarbonylamino, Alkoxycarbonylamino, Alkylaminocarbonylamino, Alkyl(Alkyl-)aminocarbonylamino, Alkylsulfonylamino, Cycloalkylsulfonylamino, Arylsulfonylamino, Hetarylsulfonylamino, Sulfonylhaloalkylamino, Aminoalkylsulfonyl, Aminohaloalkylsulfonyl, Alkylaminosulfonyl, Bisalkylaminosulfonyl, Cycloalkylaminosulfonyl, Haloalkylaminosulfonyl, Arylaminosulfonyl, Arylalkylaminosulfonyl, Alkylsulfonyl, Cycloalkylsulfonyl, Arylsulfonyl, Alkylsulfinyl, Cycloalkylsulfinyl, Arylsulfinyl, N,S-Dialkylsulfonimidoyl, S-Alkylsulfonimidoyl, Alkylsulfonylaminocarbonyl, Cycloalkylsulfonylaminocarbonyl, Cycloalkylaminosulfonyl, Arylalkylcarbonylamino, Cycloalkylalkylcarbonylamino, Heteroarylcarbonylamino, Alkoxyalkylcarbonylamino, Hydroxyalkylcarbonylamino, Cyano, Cyanoalkyl, Hydroxycarbonyl, Alkoxycarbonyl, Cycloalkoxycarbonyl, Cycloalkylalkoxycarbonyl, Aryloxycarbonyl, Arylalkoxycarbonyl, Aminocarbonyl, Alkylaminocarbonyl, Bisalkylaminocarbonyl, Alkyl(Alkoxy)aminocarbonyl, Cycloalkylaminocarbonyl, Arylalkylaminocarbonyl, Heteroarylalkylaminocarbonyl, Cyanoalkylaminocarbonyl, Haloalkylaminocarbonyl, Alkinylaminocarbonyl, Alkoxycarbonylaminocarbonyl, Arylalkoxycarbonylaminocarbonyl, Hydroxycarbonylalkyl, Alkoxycarbonylalkyl, Cycloalkoxycarbonylalkyl, Cycloalkylalkoxycarbonylalkyl, Alkylaminocarbonylalkyl, Aminocarbonylalkyl, Bisalkylaminocarbonylalkyl, Cycloalkylaminocarbonylalkyl, Arylalkylaminocarbonylalkyl, Heteroarylalkylaminocarbonylalkyl, Cyanoalkylaminocarbonylalkyl, Haloalkylaminocarbonylalkyl, Alkinylaminocarbonylalkyl, Cycloalkylalkylaminocarbonylalkyl, Alkoxycarbonylaminocarbonylalkyl, Arylalkoxycarbonylaminocarbonylalkyl, Alkoxycarbonylalkylaminocarbonyl, Hydroxycarbonylalkylaminocarbonyl, Aminocarbonylalkylaminocarbonyl, Alkylaminocarbonylalkylaminocarbonyl, Cycloalkylaminocarbonylalkylaminocarbonyl, Cycloalkylalkylaminocarbonyl, Cycloalkylalkylaminocarbonylalkyl, Alkenyloxycarbonyl, Alkenyloxycarbonylalkyl, Alkenylaminocarbonyl, Alkenylaminocarbonylalkyl, Alkylcarbonyl, Cycloalkylcarbonyl, Formyl, Hydroxyiminomethyl, Aminoiminomethyl, Alkoxyiminomethyl, Alkylaminoiminomethyl, Dialkylaminoiminomethyl, Cycloalkoxyiminomethyl, Cycloalkylalkoximinomethyl, Aryloximinomethyl, Arylalkoxyiminomethyl, Arylalkylaminoiminomethyl, Alkenyloxyiminomethyl, Arylaminoiminomethyl, Arylsulfonylaminoiminomethyl, Heteroarylalkyl, Heterocyclylalkyl, Hydroxycarbonylheterocyclyl, Alkoxycarbonylheterocyclyl, Alkenyloxycarbonylheterocyclyl, Alkenylalkoxycarbonylheterocyclyl, Arylalkoxycarbonylheterocyclyl, Cycloalkoxycarbonylheterocyclyl, Cycloalkylalkoxycarbonylheterocyclyl, Aminocarbonylheterocyclyl, Alkylaminocarbonylheterocyclyl, Bis-Alkylaminocarbonylheterocyclyl, Cycloalkylaminocarbonylheterocyclyl, Arylalkylaminocarbonylheterocyclyl, Alkenylaminocarbonylheterocyclyl, Hydroxycarbonylheterocyclylalkyl, Alkoxycarbonylheterocyclylalkyl, Hydroxycarbonylcycloalkylalkyl, Alkoxycarbonylcycloalkylalkyl, Heterocyclyl, Heterocyclyloxy, Heterocyclylamino, Heterocyclylcarbonyl, Heterocyclylcarbonylalkyl, Hydroxycarbonylheterocyclylcarbonyl, Alkoxycarbonylheterocyclylcarbonyl, Alkenyloxycarbonylheterocyclylcarbonyl, Alkenylalkoxycarbonylheterocyclylcarbonyl, Arylalkoxycarbonylheterocyclylcarbonyl, Cycloalkoxycarbonylheterocyclylcarbonyl, Cycloalkylalkoxycarbonylheterocyclylcarbonyl, Aminocarbonylheterocyclylcarbonyl, Alkylaminocarbonylheterocyclylcarbonyl, Bis-Alkylaminocarbonylheterocyclylcarbonyl, Cycloalkylaminocarbonylheterocyclylcarbonyl, Arylalkylaminocarbonylheterocyclylcarbonyl, Alkenylaminocarbonylheterocyclylcarbonyl stehen,
R⁹ und R¹⁰ unabhängig voneinander für Wasserstoff, Halogen, Alkyl, Alkoxy, Haloalkyl, Haloalkoxy, Aryl, Heteroaryl, Arylalkyl stehen oder gemeinsam mit dem Atom, an das sie gebunden sind, eine Carbonylgruppe bilden,
A⁶, A⁷ gleich oder verschieden sind und unabhängig voneinander für N-R¹¹, Sauerstoff, Schwefel oder die Gruppierung CR¹⁵R¹⁶ stehen, wobei jedoch in keinem Fall mehr als ein Sauerstoffatom im Heterocyclus enthalten ist, und wobei R¹¹, R¹⁵, R¹⁶ in den Gruppierungen N-R¹¹ und CR¹⁵R¹⁶ jeweils gleiche oder verschiedene Bedeutungen gemäß der nachstehenden Definition haben,
R¹¹ für Wasserstoff, Alkyl, Alkenyl, Alkinyl, Alkoxyalkyl, Alkylcarbonyl, Arylcarbonyl, Heteroarylcarbonyl, Cycloalkylcarbonyl, Alkoxycarbonyl, Alkenyloxycarbonyl, Alkoxycarbonylalkyl, Alkylaminocarbonylalkyl, Arylaminocarbonylalkyl, Aryloxyalkyl, Arylalkyl, Heteroarylalkyl, Heterocyclyl, Heterocyclylalkyl steht,
R¹² und R¹³ unabhängig voneinander für Wasserstoff, Halogen, Alkyl, Alkoxy, Haloalkyl, Haloalkoxy, Aryl, Heteroaryl, Arylalkyl, Heterocyclyl, Heteroarylalkyl, Heterocyclylalkyl stehen oder gemeinsam mit dem Atom, an das sie gebunden sind, eine Carbonylgruppe bilden und
R¹⁵ und R¹⁶ unabhängig voneinander für Wasserstoff, Alkyl, Alkenyl, Alkinyl, Alkoxy, Alkoxyalkyl, Alkylcarbonyl, Arylcarbonyl, Heteroarylcarbonyl, Hydroxycarbonyl, Alkoxycarbonyl, Heterocyclyl, Haloalkyl stehen,
mit Ausnahme der Verbindung 1-[(2E)-1-Hydroxy-1,3-diphenylprop-2-en-1-yl]cyclopentan-carbonitril.

2. Substituierte Vinyl- und Alkinyl-cyanocycloalkanole sowie Vinyl- und Alkinyl-cyanoheterocyclylalkanole gemäß Anspruch 1, wobei
[X-Y] für die Gruppierungen 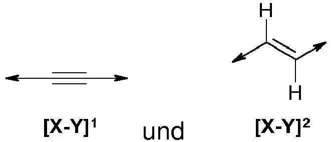 steht,
Q für die carbocyclischen und heterocyclischen Gruppierungen 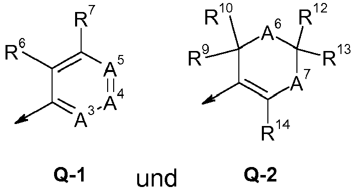 steht, wobei die Gruppierungen R⁶, R⁷, R⁹, R¹⁰, R¹², R¹³, R¹⁴, A³, A⁴, A⁵, A⁶ und A⁷ jeweils die Bedeutung gemäß der nachstehenden Definitionen haben und wobei der Pfeil für eine Bindung zur jeweiligen Gruppierung [X-Y] steht,
R¹ für (C₁-C₈)-Alkyl, Aryl, Heteroaryl, Heterocyclyl, (C₃-C₁₀)-Cycloalkyl, (C₂-C₈)-Alkenyl, (C₂-C₈)-Alkinyl, (C₁-C₈)-Alkoxy-(C₁-C₈)-alkyl, Hydroxy-(C₁-C₈)-alkyl, (C₁-C₈)-Haloalkyl, (C₂-C₈)-Haloalkenyl, (C₁-C₈)-Haloalkoxy-(C₁-C₈)-alkyl, (C₁-C₈)-Alkylthio-(C₁-C₈)-alkyl, Aryl-(C₁-C₈)-alkyl, Heterocyclyl-(C₁-C₈)-alkyl, (C₃-C₈)-Halocycloalkyl, (C₄-C₈)-Cycloalkenyl, (C₁-C₈)-Alkoxy-(C₁-C₈)-alkoxy-(C₁-C₈)-alkyl, (C₃-C₈)-Cycloalkyl-(C₁-C₈)-alkyl, (C₄-C₈)-Cycloalkenyl-(C₁-C₈)-alkyl, (C₂-C₈)-Haloalkinyl, (C₃-C₈)-Alkylsulfinyl-(C₃-C₈)-alkyl, (C₁-C₈)-Alkylsulfonyl-(C₁-C₈)-alkyl, (C₃-C₈)-Halocycloalkyl-(C₁-C₈)-alkyl, (C₃-C₈)-Cycloalkylsulfinyl-(C₁-C₈)-alkyl, (C₃-C₈)-Cycloalkylsulfonyl-(C₁-C₈)-alkyl, Arylsulfinyl-(C₁-C₈)-alkyl, Arylsulfonyl-(C₁-C₈)-alkyl, Arylthio-(C₁-C₈)-alkyl, (C₃-C₈)-Cycloalkylthio-(C₁-C₈)-alkyl, (C₁-C₈)-Alkoxy-(C₁-C₈)-haloalkyl, (C₁-C₈)-Haloalkoxy-(C₁-C₈)-haloalkyl steht,
R² für Wasserstoff, (C₁-C₈)-Alkyl, (C₁-C₈)-Alkoxy-(C₁-C₈)-alkyl, (C₁-C₈)-Alkoxy-(C₁-C₈)-alkoxy-(C₁-C₈)-alkyl, (C₂-C₈)-Alkenyl, (C₂-C₈)-Alkinyl, (C₁-C₈)-Alkylcarbonyl, Arylcarbonyl, Heteroarylcarbonyl, (C₃-C₈)-Cycloalkylcarbonyl, (C₂-C₈)-Alkenylcarbonyl, Heterocyclylcarbonyl, (C₁-C₈)-Alkoxycarbonyl, (C₂-C₈)-Alkenyloxycarbonyl, Aryloxy-(C₁-C₈)-alkyl, Aryl-(C₁-C₈)-alkoxycarbonyl, (C₃-C₈)-Cycloalkoxycarbonyl, (C₃-C₈)-Cycloalkyl-(C₁-C₈)-alkoxycarbonyl, Aryl-(C₁-C₈)-alkoxyalkyl, Aryl-(C₁-C₈)-alkyl, (C₁-C₈)-Alkoxy-(C₁-C₈)-alkoxy-(C₁-C₈)-alkyl, (C₁-C₈)-Alkylthio-(C₁-C₈)-alkyl, Tris[(C₁-C₈)-alkyl]silyl, (C₁-C₈)-Alkyl{Bis-[(C₁-C₈)-alkyl]}silyl, (C₁-C₈)-Alkyl(Bis-aryl)silyl, Aryl{Bis-[(C₁-C₈)-alkyl]}silyl, Cycloalkyl{Bis-[(C₁-C₈)-alkyl]}silyl, Halo{Bis-[(C₁-C₈)-alkyl]}silyl, Tris-[(C₁-C₈)-alkyl]silyl-(C₁-C₈)-alkoxy-(C₁-C₈)-alkyl, Tris[(C₁-C₈)-alkyl]silyl-(C₁-C₈)-alkyl, (C₂-C₈)-Alkinyloxycarbonyl, (C₃-C₈)-Cycloalkyl, (C₃-C₈)-Cycloalkyl-(C₁-C₈)-alkyl, Aminocarbonyl, (C₁-C₈)-Alkylaminocarbonyl, Bis-[(C₁-C₈)-Alkyl]aminocarbonyl, (C₃-C₈)-Cycloalkylaminocarbonyl, (C₁-C₈)-Alkylsulfonyl, (C₁-C₈)-Haloalkylsulfonyl, Arylsulfonyl, Heteroarylsulfonyl, (C₃-C₈)-Cycloalkylsulfonyl steht,
A¹, A², V, W unabhängig voneinander für eine Gruppe CR³R⁴, eine Gruppe N-R⁵, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht,
R³ und R⁴ unabhängig voneinander für Wasserstoff, (C₁-C₈)-Alkyl, Halogen, (C₃-C₈)-Cycloalkyl, (C₁-C₈)-Alkoxy, Aryl, Heterocyclyl, Heteroaryl, Aryl-(C₁-C₈)-alkyl, (C₁-C₈)-Alkylthio, (C₁-C₈)-Haloalkyl, (C₁-C₈)-Haloalkyloxy, (C₁-C₈)-Haloalkylthio, (C₁-C₈)-Alkoxy-(C₁-C₈)-alkyl, (C₁-C₈)-Alkylthio-(C₁-C₈)-alkyl, Heteroaryl-(C₁-C₈)-alkyl, Heterocyclyl-(C₁-C₈)-alkyl, (C₃-C₈)-Cycloalkyl-(C₁-C₈)-alkyl, (C₄-C₃)-Cycloalkenyl, (C₂-C₈)-Alkinyl, (C₂-C₈)-Alkenyl, (C₂-C₈)-Haloalkenyl, (C₂-C₈)-Haloalkinyl, (C₁-C₈)-Alkylsulfinyl, (C₁-C₈)-Alkylsulfonyl, (C₃-C₈)-Cycloalkylsulfinyl, (C₃-C₈)-Cycloalkylsulfonyl, Arylsulfinyl, Arylsulfonyl, (C₁-C₈)-Alkoxy-(C₁-C₈)-haloalkyl, (C₁-C₈)-Haloalkoxy-(C₁-C₈)-haloalkyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden,
R⁵ für Wasserstoff, (C₁-C₈)-Alkyl, (C₂-C₈)-Alkenyl, (C₂-C₈)-Alkinyl, (C₁-C₈)-Alkoxy-(C₁-C₈)-alkyl, (C₁-C₈)-Alkylcarbonyl, Arylcarbonyl, Heteroarylcarbonyl, (C₃-C₈)-Cycloalkylcarbonyl, (C₁-C₈)-Alkoxycarbonyl, (C₂-C₈)-Alkenyloxycarbonyl, (C₁-C₈)-Alkoxycarbonyl-(C₁-C₈)-alkyl, (C₁-C₈)-Alkylaminocarbonyl-(C₁-C₈)-alkyl, Arylaminocarbonyl-(C₁-C₈)-alkyl, Aryloxy-(C₁-C₈)-alkyl, Aryl-(C₁-C₈)-alkyl, Heteroaryl-(C₁-C₈)-alkyl, Heterocyclyl, Heterocyclyl-(C₁-C₈)-alkyl, (C₁-C₈)-Haloalkyl stehen,
R⁶ und R⁷ unabhängig voneinander für Wasserstoff, Nitro, Amino, Cyano, Thiocyanato, Isothiocyanato, Halogen, (C₁-C₈)-Alkyl, (C₃-C₈)-Cycloalkyl, (C₂-C₈)-Alkenyl, (C₂-C₈)-Alkinyl, Aryl, Aryl-(C₁-C₈)-alkyl, Aryl-(C₁-C₈)-alkoxy, Heteroaryl, (C₁-C₈)-Alkoxy-(C₁-C₈)-alkyl, Hydroxy-(C₁-C₈)-alkyl, (C₁-C₈)-Haloalkyl, (C₃-C₈)-Halocycloalkyl, (C₁-C₈)-Alkoxy, (C₁-C₈)-Haloalkoxy, Aryloxy, Heteroaryloxy, (C₃-C₈)-Cycloalkyloxy, Hydroxy, (C₃-C₈)-Cycloalkyl-(C₁-C₈)-alkoxy, (C₁-C₈)-Alkoxycarbonyl, Hydroxycarbonyl, Aminocarbonyl, (C₁-C₈)-Alkylaminocarbonyl, (C₃-C₈)-Cycloalkylaminocarbonyl, Cyano-(C₁-C₈)-alkylaminocarbonyl, (C₂-C₈)-Alkenylaminocarbonyl, (C₂-C₈)-Alkinylaminocarbonyl, (C₁-C₈)-Alkylamino, (C₁-C₈)-Alkylthio, (C₁-C₈)-Haloalkylthio, Hydrothio, Bis-[(C₁-C₈)-alkyl]amino, (C₃-C₃)-Cycloalkylamino, (C₁-C₈)-Alkylcarbonylamino, (C₃-C₈)-Cycloalkylcarbonylamino, Formylamino, (C₁-C₈)-Haloalkylcarbonylamino, (C₁-C₈)-Alkoxycarbonylamino, (C₁-C₈)-Alkylaminocarbonylamino, (C₁-C₈)-Alkyl-[(C₁-C₈)-Alkyl]aminocarbonylamino, (C₁-C₈)-Alkylsulfonylamino, (C₃-C₈)-Cycloalkylsulfonylamino, Arylsulfonylamino, Hetarylsulfonylamino, Sulfonyl-(C₁-C₈)-haloalkylamino, Aminosulfonyl, Amino-(C₁-C₈)-alkylsulfonyl, Amino-(C₁-C₈)-haloalkylsulfonyl, (C₁-C₈)-Alkylaminosulfonyl, Bis[(C₁-C₈)-alkyl]aminosulfonyl, (C₃-C₈)-Cycloalkylaminosulfonyl, (C₁-C₈)-Haloalkylaminosulfonyl, Arylaminosulfonyl, Aryl-(C₁-C₈)-alkylaminosulfonyl, (C₁-C₈)-Alkylsulfonyl, (C₃-C₈)-Cycloalkylsulfonyl, Arylsulfonyl, (C₁-C₈)-Alkylsulfinyl, (C₃-C₈)-Cycloalkylsulfinyl, Arylsulfinyl, N,S-Bis[(C₁-C₈)-alkyl]sulfonimidoyl, S-(C₁-C₈)-Alkylsulfonimidoyl, (C₁-C₈)-Alkylsulfonylaminocarbonyl, (C₃-C₈)-Cycloalkylsulfonylaminocarbonyl, (C₃-C₈)-Cycloalkylaminosulfonyl, Aryl-(C₁-C₈)-alkylcarbonylamino, (C₃-C₈)-Cycloalkyl-(C₁-C₈)-alkylcarbonylamino, Heteroarylcarbonylamino, (C₁-C₈)-Alkoxy-(C₁-C₈)-alkylcarbonylamino, Hydroxy-(C₁-C₈)-alkylcarbonylamino, Tris[(C₁-C₈)-alkyl]silyl stehen,
A³, A⁴, A⁵ gleich oder verschieden sind und unabhängig voneinander für N (Stickstoff) oder die Gruppierung C-R⁸ stehen, wobei jedoch in keinem Fall mehr als zwei N-Atome benachbart sind, und wobei R⁸ in der Gruppierung C-R⁸ jeweils gleiche oder verschiedene Bedeutungen gemäß der nachstehenden Definition hat und
A³ und A⁴, wenn beide für eine Gruppe C-R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
A⁴ und A⁵, wenn beide für eine Gruppe C-R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
R⁸ und R¹⁴ unabhängig voneinander jeweils für Wasserstoff, Nitro, Amino, Hydroxy, Hydrothio, Thiocyanato, Isothiocyanato, Halogen, (C₁-C₈)-Alkyl, (C₃-C₈)-Cycloalkyl, (C₂-C₈)-Alkenyl, (C₂-C₈)-Alkinyl, Aryl, Aryl-(C₁-C₈)-alkyl, Aryl-(C₁-C₈)-alkoxy, Heteroaryl, (C₁-C₈)-Haloalkyl, (C₃-C₈)-Halocycloalkyl, (C₁-C₈)-Alkoxy, (C₁-C₈)-Haloalkoxy, Aryloxy, Heteroaryloxy, (C₃-C₈)-Cycloalkyloxy, (C₃-C₈)-Cycloalkyl-(C₁-C₈)-alkoxy, Hydroxy-(C₁-C₈)-alkyl, (C₁-C₈)-Alkoxy-(C₁-C₈)-alkyl, Aryloxy-(C₁-C₈)-alkyl, Heteroaryloxy-(C₁-C₈)-alkyl, (C₁-C₈)-Alkylamino, (C₁-C₈)-Alkylthio, (C₁-C₈)-Haloalkylthio, Bis[(C₁-C₈)-alkyl]amino, (C₃-C₈)-Cycloalkylamino, (C₁-C₈)-Alkylcarbonylamino, (C₃-C₈)-Cycloalkylcarbonylamino, Formylamino, (C₁-C₈)-Haloalkylcarbonylamino, (C₁-C₈)-Alkoxycarbonylamino, (C₁-C₈)-Alkylaminocarbonylamino, (C₁-C₈)-Alkyl[(C₁-C₈)-Alkyl]aminocarbonylamino, (C₁-C₈)-Alkylsulfonylamino, (C₃-C₈)-Cycloalkylsulfonylamino, Arylsulfonylamino, Hetarylsulfonylamino, Sulfonyl-(C₁-C₈)-haloalkylamino, Amino-(C₁-C₈)-alkylsulfonyl, Amino-(C₁-C₈)-haloalkylsulfonyl, (C₁-C₈)-Alkylaminosulfonyl, Bis[(C₁-C₈)-alkyl]aminosulfonyl, (C₃-C₈)-Cycloalkylaminosulfonyl, (C₁-C₈)-Haloalkylaminosulfonyl, Arylaminosulfonyl, Aryl-(C₁-C₈)-alkylaminosulfonyl, (C₁-C₈)-Alkylsulfonyl, (C₃-C₈)-Cycloalkylsulfonyl, Arylsulfonyl, (C₁-C₈)-Alkylsulfinyl, (C₃-C₈)-Cycloalkylsulfinyl, Arylsulfinyl, N,S-Bis[(C₁-C₈)-alkyl]sulfonimidoyl, S-(C₁-C₈)-Alkylsulfonimidoyl, (C₁-C₈)-Alkylsulfonylaminocarbonyl, (C₃-C₈)-Cycloalkylsulfonylaminocarbonyl, (C₃-C₈)-Cycloalkylaminosulfonyl, Aryl-(C₁-C₈)-alkylcarbonylamino, (C₃-C₈)-Cycloalkyl-(C₁-C₈)-alkylcarbonylamino, Heteroarylcarbonylamino, (C₁-C₈)-Alkoxy-(C₁-C₈)-alkylcarbonylamino, Hydroxy-(C₁-C₈)-alkylcarbonylamino, Cyano, Cyano-(C₁-C₈)-alkyl, Hydroxycarbonyl, (C₁-C₈)-Alkoxycarbonyl, (C₃-C₈)-Cycloalkoxycarbonyl, (C₃-C₈)-Cycloalkyl-(C₁-C₈)-alkoxycarbonyl, Aryloxycarbonyl, Aryl-(C₁-C₈)-alkoxycarbonyl, Aminocarbonyl, (C₁-C₈)-Alkylaminocarbonyl, Bis[(C₁-C₈)-alkyl]aminocarbonyl, (C₁-C₈)-Alkyl[(C₁-C₈)-Alkoxy]aminocarbonyl, (C₃-C₈)-Cycloalkylaminocarbonyl, Aryl-(C₁-C₈)-alkylaminocarbonyl, Heteroaryl-(C₁-C₈)-alkylaminocarbonyl, Cyano-(C₁-C₈)-alkylaminocarbonyl, (C₁-C₈)-Haloalkylaminocarbonyl, (C₂-C₈)-Alkinylaminocarbonyl, (C₁-C₈)-Alkoxycarbonylaminocarbonyl, Aryl-(C₁-C₈)-alkoxycarbonylaminocarbonyl, Hydroxycarbonyl-(C₁-C₈)-alkyl, (C₁-C₈)-Alkoxycarbonyl-(C₁-C₈)-alkyl, (C₃-C₈)-Cycloalkoxycarbonyl-(C₁-C₈)-alkyl, (C₃-C₈)-Cycloalkyl-(C₁-C₈)-alkoxycarbonyl-(C₁-C₈)-alkyl, (C₁-C₈)-Alkylaminocarbonyl-(C₁-C₈)-alkyl, Aminocarbonyl-(C₁-C₈)-alkyl, Bis[(C₁-C₈)-alkyl]aminocarbonyl-(C₁-C₈)-alkyl, (C₃-C₈)-Cycloalkylaminocarbonyl-(C₁-C₈)-alkyl, Aryl-(C₁-C₈)-alkylaminocarbonyl-(C₁-C₈)-alkyl, Heteroaryl-(C₁-C₈)-alkylaminocarbonyl-(C₁-C₈)-alkyl, Cyano-(C₁-C₈)-alkylaminocarbonyl-(C₁-C₈)-alkyl, (C₁-C₈)-Haloalkylaminocarbonyl-(C₁-C₈)-alkyl, (C₂-C₈)-Alkinylaminocarbonyl-(C₁-C₈)-alkyl, (C₃-C₈)-Cycloalkyl-(C₁-C₈)-alkylaminocarbonyl-(C₁-C₈)-alkyl, (C₁-C₈)-Alkoxycarbonylaminocarbonyl-(C₁-C₈)-alkyl, Aryl-(C₁-C₈)-alkoxycarbonylaminocarbonyl-(C₁-C₈)-alkyl, (C₁-C₈)-Alkoxycarbonyl-(C₁-C₈)-alkylaminocarbonyl, Hydroxycarbonyl-(C₁-C₈)-alkylaminocarbonyl, Aminocarbonyl-(C₁-C₈)-alkylaminocarbonyl, (C₁-C₈)-Alkylaminocarbonyl-(C₁-C₈)-alkylaminocarbonyl, (C₃-C₈)-Cycloalkylaminocarbonyl-(C₁-C₈)-alkylanninocarbonyl, (C₃-C₈)-Cycloalkyl-(C₁-C₈)-alkylaminocarbonyl, (C₃-C₈)-Cycloalkyl-(C₁-C₈)-alkylaminocarbonyl-(C₁-C₈)-alkyl, (C₂-C₈)-Alkenyloxycarbonyl, (C₂-C₈)-Alkenyloxycarbonyl-(C₁-C₈)-alkyl, (C₂-C₈)-Alkenylaminocarbonyl, (C₂-C₈)-Alkenylaminocarbonyl-(C₁-C₈)-alkyl, (C₁-C₈)-Alkylcarbonyl, (C₃-C₈)-Cycloalkylcarbonyl, Formyl, Hydroxyiminomethyl, Aminoiminomethyl, (C₁-C₈)-Alkoxyiminomethyl, (C₁-C₈)-Alkylaminoiminomethyl, Bis[(C₁-C₈)-alkyl]aminoiminomethyl, (C₃-C₈)-Cycloalkoxyiminomethyl, (C₃-C₈)-Cycloalkyl-(C₁-C₈)-alkoximinomethyl, Aryloximinomethyl, Aryl-(C₁-C₈)-alkoxyiminomethyl, Aryl-(C₁-C₈)-alkylaminoiminomethyl, (C₂-C₈)-Alkenyloxyiminomethyl, Arylaminoiminomethyl, Arylsulfonylaminoiminomethyl, Heteroaryl-(C₁-C₈)-alkyl, Heterocyclyl-(C₁-C₈)-alkyl, Hydroxycarbonylheterocyclyl, (C₁-C₈)-Alkoxycarbonylheterocyclyl, (C₂-C₈)-Alkenyloxycarbonylheterocyclyl, (C₂-C₈)-Alkenyl-(C₁-C₈)-alkoxycarbonylheterocyclyl, Aryl-(C₁-C₈)-alkoxycarbonylheterocyclyl, (C₃-C₈)-Cycloalkoxycarbonylheterocyclyl, (C₃-C₈)-Cycloalkyl-(C₁-C₈)-alkoxycarbonylheterocyclyl, Aminocarbonylheterocyclyl, (C₁-C₈)-Alkylaminocarbonylheterocyclyl, Bis-[(C₁-C₈)-Alkyl]aminocarbonylheterocyclyl, (C₃-C₈)-Cycloalkylaminocarbonylheterocyclyl, Aryl-(C₁-C₈)-alkylaminocarbonylheterocyclyl, (C₂-C₈)-Alkenylaminocarbonylheterocyclyl, Hydroxycarbonylheterocyclyl-(C₁-C₈)-alkyl, (C₁-C₈)-Alkoxycarbonylheterocyclyl-(C₁-C₈)-alkyl, Hydroxycarbonyl-(C₃-C₈)-cycloalkyl-(C₁-C₈)-alkyl, (C₁-C₈)-Alkoxycarbonyl-(C₃-C₈)-cycloalkyl-(C₁-C₈)-alkyl, Heterocyclyl, Heterocyclyloxy, Heterocyclylamino, Heterocyclylcarbonyl, Heterocyclylcarbonyl-(C₁-C₈)-alkyl, Hydroxycarbonylheterocyclylcarbonyl, (C₁-C₈)-Alkoxycarbonylheterocyclylcarbonyl, (C₂-C₈)-Alkenyloxycarbonylheterocyclylcarbonyl, (C₂-C₈)-Alkenyl-(C₁-C₈)-alkoxycarbonylheterocyclylcarbonyl, Aryl-(C₁-C₈)-alkoxycarbonylheterocyclylcarbonyl, (C₃-C₈)-Cycloalkoxycarbonylheterocyclylcarbonyl, (C₃-C₈)-Cycloalkyl-(C₁-C₈)-alkoxycarbonylheterocyclylcarbonyl, Aminocarbonylheterocyclylcarbonyl, (C₁-C₈)-Alkylaminocarbonylheterocyclylcarbonyl, Bis-[(C₁-C₈)-Alkyl]aminocarbonylheterocyclylcarbonyl, (C₃-C₈)-Cycloalkylaminocarbonylheterocyclylcarbonyl, Aryl-(C₁-C₈)-alkylaminocarbonylheterocyclylcarbonyl, (C₂-C₈)-Alkenylaminocarbonylheterocyclylcarbonyl stehen,
R⁹ und R¹⁰ unabhängig voneinander für Wasserstoff, Halogen, (C₁-C₈)-Alkyl, (C₁-C₈)-Alkoxy, (C₁-C₈)-Haloalkyl, (C₁-C₈)-Haloalkoxy, Aryl, Heteroaryl, Aryl-(C₁-C₈)-alkyl stehen oder gemeinsam mit dem Atom, an das sie gebunden sind, eine Carbonylgruppe bilden,
A⁶, A⁷ gleich oder verschieden sind und unabhängig voneinander für N-R¹¹, Sauerstoff, Schwefel oder die Gruppierung CR¹⁵R¹⁶ stehen, wobei jedoch in keinem Fall mehr als ein Sauerstoffatom im Heterocyclus enthalten ist, und wobei R¹¹, R¹⁵, R¹⁶ in den Gruppierungen N-R¹¹ und CR¹⁵R¹⁶ jeweils gleiche oder verschiedene Bedeutungen gemäß der nachstehenden Definition haben,
R¹¹ für Wasserstoff, (C₁-C₈)-Alkyl, (C₂-C₈)-Alkenyl, (C₂-C₈)-Alkinyl, (C₁-C₈)-Alkoxy-(C₁-C₈)-alkyl, (C₁-C₈)-Alkylcarbonyl, Arylcarbonyl, Heteroarylcarbonyl, (C₃-C₈)-Cycloalkylcarbonyl, (C₁-C₈)-Alkoxycarbonyl, (C₂-C₈)-Alkenyloxycarbonyl, (C₁-C₈)-Alkoxycarbonyl-(C₁-C₈)-alkyl, (C₁-C₈)-Alkylaminocarbonyl-(C₁-C₈)-alkyl, Arylaminocarbonyl-(C₁-C₈)-alkyl, Aryloxy-(C₁-C₈)-alkyl, Aryl-(C₁-C₈)-alkyl, Heteroaryl-(C₁-C₈)-alkyl, Heterocyclyl, Heterocyclyl-(C₁-C₈)-alkyl steht,
R¹² und R¹³ unabhängig voneinander für Wasserstoff, Halogen, (C₁-C₈)-Alkyl, (C₁-C₈)-Alkoxy, (C₁-C₈)-Haloalkyl, (C₁-C₈)-Haloalkoxy, Aryl, Heteroaryl, Aryl-(C₁-C₈)-alkyl, Heterocyclyl, Heteroaryl-(C₁-C₈)-alkyl, Heterocyclyl-(C₁-C₈)-alkyl stehen oder gemeinsam mit dem Atom, an das sie gebunden sind, eine Carbonylgruppe bilden und
R¹⁵ und R¹⁶ unabhängig voneinander für Wasserstoff, (C₁-C₈)-Alkyl, (C₂-C₈)-Alkenyl, (C₂-C₈)-Alkinyl, (C₁-C₈)-Alkoxy, (C₁-C₈)-Alkoxy-(C₁-C₈)-alkyl, (C₁-C₈)-Alkylcarbonyl, Arylcarbonyl, Heteroarylcarbonyl, Hydroxycarbonyl, (C₁-C₈)-Alkoxycarbonyl, Heterocyclyl, (C₁-C₈)-Haloalkyl stehen,
mit Ausnahme der Verbindung 1-[(2E)-1-Hydroxy-1,3-diphenylprop-2-en-1-yl]cyclopentan-carbonitril.

3. Substituierte Vinyl- und Alkinyl-cyanocycloalkanole sowie Vinyl- und Alkinylcyanoheterocyclylalkanole gemäß Anspruch 1, wobei
[X-Y] für die Gruppierungen 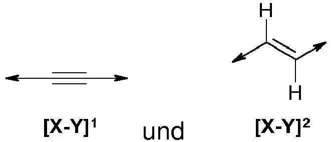 steht,
Q für die carbocyclischen und heterocyclischen Gruppierungen 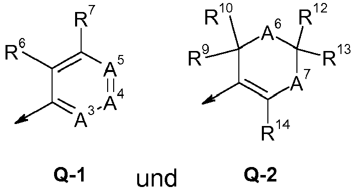 steht, wobei die Gruppierungen R⁶, R⁷, R⁹, R¹⁰, R¹², R¹³, R¹⁴, A³, A⁴, A⁵, A⁶ und A⁷ jeweils die Bedeutung gemäß der nachstehenden Definitionen haben und wobei der Pfeil für eine Bindung zur jeweiligen Gruppierung [X-Y] steht,
R¹ für (C₁-C₇)-Alkyl, Aryl, Heteroaryl, Heterocyclyl, (C₃-C₁₀)-Cycloalkyl, (C₂-C₇)-Alkenyl, (C₂-C₇)-Alkinyl, (C₁-C₇)-Alkoxy-(C₁-C₇)-alkyl, Hydroxy-(C₁-C₇)-alkyl, (C₁-C₇)-Haloalkyl, (C₂-C₇)-Haloalkenyl, (C₁-C₇)-Haloalkoxy-(C₁-C₇)-alkyl, (C₁-C₇)-Alkylthio-(C₁-C₇)-alkyl, Aryl-(C₁-C₇)-alkyl, Heterocyclyl-(C₁-C₇)-alkyl, (C₃-C₇)-Halocycloalkyl, (C₄-C₇)-Cycloalkenyl, (C₁-C₇)-Alkoxy-(C₁-C₇)-alkoxy-(C₁-C₇)-alkyl, (C₁-C₇)-Alkoxy-(C₁-C₇)-haloalkyl, (C₁-C₇)-Haloalkoxy-(C₁-C₇)-haloalkyl, (C₃-C₈)-Cycloalkyl-(C₁-C₇)-alkyl steht,
R² für Wasserstoff, (C₁-C₇)-Alkyl, (C₁-C₇)-Alkoxy-(C₁-C₇)-alkyl, (C₁-C₇)-Alkoxy-(C₁-C₇)-alkoxy-(C₁-C₇)-alkyl, (C₂-C₇)-Alkenyl, (C₂-C₇)-Alkinyl, (C₁-C₇)-Alkylcarbonyl, Arylcarbonyl, Heteroarylcarbonyl, (C₃-C₇)-Cycloalkylcarbonyl, (C₂-C₇)-Alkenylcarbonyl, Heterocyclylcarbonyl, (C₁-C₇)-Alkoxycarbonyl, (C₂-C₇)-Alkenyloxycarbonyl, Aryloxy-(C₁-C₇)-alkyl, Aryl-(C₁-C₇)-alkoxycarbonyl, (C₃-C₇)-Cycloalkoxycarbonyl, (C₃-C₇)-Cycloalkyl-(C₁-C₇)-alkoxycarbonyl, Aryl-(C₁-C₇)-alkoxy-(C₁-C₇)-alkyl, Aryl-(C₁-C₇)-alkyl, (C₁-C₇)-Alkoxy-(C₁-C₇)-alkoxy-(C₁-C₇)-alkyl, (C₁-C₇)-Alkylthio-(C₁-C₇)-alkyl, Tris[(C₁-C₇)-alkyl]silyl, (C₁-C₇)-Alkyl-Bis-[(C₁-C₇)-alkyl]silyl, (C₁-C₇)-Alkyl-Bis(aryl)silyl, Aryl-Bis[(C₁-C₇)-alkyl]silyl, (C₃-C₇)-Cycloalkyl-Bis[(C₁-C₇)-alkyl]silyl, Halo-Bis[(C₁-C₇)-alkyl]silyl, Tris[(C₁-C₇)-alkyl]silyl-(C₁-C₇)-alkoxy-(C₁-C₇)-alkyl, Tris[(C₁-C₇)-alkyl]silyl-(C₁-C₇)-alkyl steht,
A¹, A², V, W unabhängig voneinander für eine Gruppe CR³R⁴, eine Gruppe N-R⁵, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht,
R³ und R⁴ unabhängig voneinander für Wasserstoff, (C₁-C₇)-Alkyl, Halogen, (C₃-C₇)-Cycloalkyl, (C₁-C₇)-Alkoxy, Aryl, Heterocyclyl, Heteroaryl, Aryl-(C₁-C₇)-alkyl, (C₁-C₇)-Alkylthio, (C₁-C₇)-Haloalkyl, (C₁-C₇)-Haloalkyloxy, (C₁-C₇)-Haloalkylthio, (C₁-C₇)-Alkoxy-(C₁-C₇)-alkyl, (C₁-C₇)-Alkylthio-(C₁-C₇)-alkyl, Heteroaryl-(C₁-C₇)-alkyl, Heterocyclyl-(C₁-C₇)-alkyl, (C₃-C₇)-Cycloalkyl-(C₁-C₇)-alkyl, (C₄-C₇)-Cycloalkenyl, (C₂-C₇)-Alkinyl, (C₂-C₇)-Alkenyl, (C₂-C₇)-Haloalkenyl, (C₂-C₇)-Haloalkinyl, (C₁-C₇)-Alkylsulfinyl, (C₁-C₇)-Alkylsulfonyl, (C₃-C₇)-Cycloalkylsulfinyl, (C₃-C₇)-Cycloalkylsulfonyl, Arylsulfinyl, Arylsulfonyl, (C₁-C₇)-Alkoxy-(C₁-C₇)-haloalkyl, (C₁-C₇)-Haloalkoxy-(C₁-C₇)-haloalkyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden,
R⁵ für Wasserstoff, (C₁-C₇)-Alkyl, (C₂-C₇)-Alkenyl, (C₂-C₇)-Alkinyl, (C₁-C₇)-Alkoxy-(C₁-C₇)-alkyl, (C₁-C₇)-Alkylcarbonyl, Arylcarbonyl, Heteroarylcarbonyl, (C₃-C₇)-Cycloalkylcarbonyl, (C₁-C₇)-Alkoxycarbonyl, (C₂-C₇)-Alkenyloxycarbonyl, (C₁-C₇)-Alkoxycarbonyl-(C₁-C₇)-alkyl, (C₁-C₇)-Alkylaminocarbonyl-(C₁-C₇)-alkyl, Arylaminocarbonyl-(C₁-C₇)-alkyl, Aryloxy-(C₁-C₇)-alkyl, Aryl-(C₁-C₇)-alkyl, Heteroaryl-(C₁-C₇)-alkyl, Heterocyclyl, Heterocyclyl-(C₁-C₇)-alkyl, stehen,
R⁶ und R⁷ unabhängig voneinander für Wasserstoff, Nitro, Amino, Cyano, Thiocyanato, Isothiocyanato, Halogen, (C₁-C₇)-Alkyl, (C₃-C₇)-Cycloalkyl, (C₂-C₇)-Alkenyl, (C₂-C₇)-Alkinyl, Aryl, Aryl-(C₁-C₇)-alkyl, Aryl-(C₁-C₇)-alkoxy, Heteroaryl, (C₁-C₇)-Alkoxy-(C₁-C₇)-alkyl, Hydroxy-(C₁-C₇)-alkyl, (C₁-C₇)-Haloalkyl, (C₃-C₇)-Halocycloalkyl, (C₁-C₇)-Alkoxy, (C₁-C₇)-Haloalkoxy, Aryloxy, Heteroaryloxy, (C₃-C₇)-Cycloalkyloxy, Hydroxy, (C₃-C₇)-Cycloalkyl-(C₁-C₇)-alkoxy, (C₁-C₇)-Alkoxycarbonyl, Hydroxycarbonyl, Aminocarbonyl, (C₁-C₇)-Alkylaminocarbonyl, (C₃-C₇)-Cycloalkylaminocarbonyl, Cyano-(C₁-C₇)-alkylaminocarbonyl, (C₂-C₇)-Alkenylaminocarbonyl, (C₂-C₇)-Alkinylaminocarbonyl, (C₁-C₇)-Alkylamino, (C₁-C₇)-Alkylthio, (C₁-C₇)-Haloalkylthio, Hydrothio, Bis-[(C₁-C₇)-alkyl]amino, (C₃-C₇)-Cycloalkylamino, (C₁-C₇)-Alkylcarbonylamino, (C₃-C₇)-Cycloalkylcarbonylamino, Formylamino, (C₁-C₇)-Haloalkylcarbonylamino, (C₁-C₇)-Alkoxycarbonylamino, (C₁-C₇)-Alkylaminocarbonylamino, (C₁-C₇)-Alkyl-[(C₁-C₇)-Alkyl]aminocarbonylamino, (C₁-C₇)-Alkylsulfonylamino, (C₃-C₇)-Cycloalkylsulfonylamino, Arylsulfonylamino, Hetarylsulfonylamino, Sulfonyl-(C₁-C₇)-haloalkylamino, Aminosulfonyl, Amino-(C₁-C₇)-alkylsulfonyl, Amino-(C₁-C₇)-haloalkylsulfonyl, (C₁-C₇)-Alkylaminosulfonyl, Bis[(C₁-C₇)-alkyl]aminosulfonyl, (C₃-C₇)-Cycloalkylaminosulfonyl, (C₁-C₇)-Haloalkylaminosulfonyl, Arylaminosulfonyl, Aryl-(C₁-C₇)-alkylaminosulfonyl, (C₁-C₇)-Alkylsulfonyl, (C₃-C₇)-Cycloalkylsulfonyl, Arylsulfonyl, (C₁-C₇)-Alkylsulfinyl, (C₃-C₇)-Cycloalkylsulfinyl, Arylsulfinyl, N,S-Bis[(C₁-C₇)-alkyl]sulfonimidoyl, S-(C₁-C₇)-Alkylsulfonimidoyl, (C₁-C₇)-Alkylsulfonylaminocarbonyl, (C₃-C₇)-Cycloalkylsulfonylaminocarbonyl, (C₃-C₇)-Cycloalkylaminosulfonyl, Aryl-(C₁-C₇)-alkylcarbonylamino, (C₃-C₇)-Cycloalkyl-(C₁-C₇)-alkylcarbonylamino, Heteroarylcarbonylamino, (C₁-C₇)-Alkoxy-(C₁-C₇)-alkylcarbonylamino, Hydroxy-(C₁-C₇)-alkylcarbonylamino, Tris[(C₁-C₇)-alkyl]silyl stehen,
A³, A⁴, A⁵ gleich oder verschieden sind und unabhängig voneinander für N (Stickstoff) oder die Gruppierung C-R⁸ stehen, wobei jedoch in keinem Fall mehr als zwei N-Atome benachbart sind, und wobei R⁸ in der Gruppierung C-R⁸ jeweils gleiche oder verschiedene Bedeutungen gemäß der nachstehenden Definition hat und
A³ und A⁴, wenn beide für eine Gruppe C-R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
A⁴ und A⁵, wenn beide für eine Gruppe C-R⁸ stehen, mit den Atomen, an die sie gebunden sind, einen vollständig gesättigten, teilgesättigten oder vollständig ungesättigten, gegebenenfalls durch Heteroatome unterbrochenen und gegebenenfalls weiter substituierten 5 bis 6-gliedrigen Ring bilden,
R⁸ und R¹⁴ unabhängig voneinander jeweils für Wasserstoff, Nitro, Amino, Hydroxy, Hydrothio, Thiocyanato, Isothiocyanato, Halogen, (C₁-C₇)-Alkyl, (C₃-C₇)-Cycloalkyl, (C₂-C₇)-Alkenyl, (C₂-C₇)-Alkinyl, Aryl, Aryl-(C₁-C₇)-alkyl, Aryl-(C₁-C₇)-alkoxy, Heteroaryl, (C₁-C₇)-Haloalkyl, (C₃-C₇)-Halocycloalkyl, (C₁-C₇)-Alkoxy, (C₁-C₇)-Haloalkoxy, Aryloxy, Heteroaryloxy, (C₃-C₇)-Cycloalkyloxy, (C₃-C₇)-Cycloalkyl-(C₁-C₇)-alkoxy, Hydroxy-(C₁-C₇)-alkyl, (C₁-C₇)-Alkoxy-(C₁-C₇)-alkyl, Aryloxy-(C₁-C₇)-alkyl, Heteroaryloxy-(C₁-C₇)-alkyl, (C₁-C₇)-Alkylamino, (C₁-C₇)-Alkylthio, (C₁-C₇)-Haloalkylthio, Bis[(C₁-C₇)-alkyl]amino, (C₃-C₇)-Cycloalkylamino, (C₁-C₇)-Alkylcarbonylamino, (C₃-C₇)-Cycloalkylcarbonylamino, Formylamino, (C₁-C₇)-Haloalkylcarbonylamino, (C₁-C₇)-Alkoxycarbonylamino, (C₁-C₇)-Alkylaminocarbonylamino, (C₁-C₇)-Alkyl[(C₁-C₇)-Alkyl]aminocarbonylamino, (C₁-C₇)-Alkylsulfonylamino, (C₃-C₇)-Cycloalkylsulfonylamino, Arylsulfonylamino, Hetarylsulfonylamino, Sulfonyl-(C₁-C₇)-haloalkylamino, Amino-(C₁-C₇)-alkylsulfonyl, Amino-(C₁-C₇)-haloalkylsulfonyl, (C₁-C₇)-Alkylaminosulfonyl, Bis[(C₁-C₇)-alkyl]aminosulfonyl, (C₃-C₇)-Cycloalkylaminosulfonyl, (C₁-C₇)-Haloalkylaminosulfonyl, Arylaminosulfonyl, Aryl-(C₁-C₇)-alkylaminosulfonyl, (C₁-C₇)-Alkylsulfonyl, (C₃-C₇)-Cycloalkylsulfonyl, Arylsulfonyl, (C₁-C₇)-Alkylsulfinyl, (C₃-C₇)-Cycloalkylsulfinyl, Arylsulfinyl, N,S-Bis[(C₁-C₇)-alkyl]sulfonimidoyl, S-(C₁-C₇)-Alkylsulfonimidoyl, (C₁-C₇)-Alkylsulfonylaminocarbonyl, (C₃-C₇)-Cycloalkylsulfonylaminocarbonyl, (C₃-C₇)-Cycloalkylaminosulfonyl, Aryl-(C₁-C₇)-alkylcarbonylamino, (C₃-C₇)-Cycloalkyl-(C₁-C₇)-alkylcarbonylamino, Heteroarylcarbonylamino, (C₁-C₇)-Alkoxy-(C₁-C₇)-alkylcarbonylamino, Hydroxy-(C₁-C₇)-alkylcarbonylamino, Cyano, Cyano-(C₁-C₇)-alkyl, Hydroxycarbonyl, (C₁-C₇)-Alkoxycarbonyl, (C₃-C₇)-Cycloalkoxycarbonyl, (C₃-C₇)-Cycloalkyl-(C₁-C₇)-alkoxycarbonyl, Aryloxycarbonyl, Aryl-(C₁-C₇)-alkoxycarbonyl, Aminocarbonyl, (C₁-C₇)-Alkylaminocarbonyl, Bis[(C₁-C₇)-alkyl]aminocarbonyl, (C₁-C₇)-Alkyl[(C₁-C₇)-Alkoxy]aminocarbonyl, (C₃-C₇)-Cycloalkylaminocarbonyl, Aryl-(C₁-C₇)-alkylaminocarbonyl, Heteroaryl-(C₁-C₇)-alkylaminocarbonyl, Cyano-(C₁-C₇)-alkylaminocarbonyl, (C₁-C₇)-Haloalkylaminocarbonyl, (C₂-C₇)-Alkinylaminocarbonyl, (C₁-C₇)-Alkoxycarbonylaminocarbonyl, Aryl-(C₁-C₇)-alkoxycarbonylaminocarbonyl, Hydroxycarbonyl-(C₁-C₇)-alkyl, (C₁-C₇)-Alkoxycarbonyl-(C₁-C₇)-alkyl, (C₃-C₇)-Cycloalkoxycarbonyl-(C₁-C₇)-alkyl, (C₃-C₇)-Cycloalkyl-(C₁-C₇)-alkoxycarbonyl-(C₁-C₇)-alkyl, (C₁-C₇)-Alkylaminocarbonyl-(C₁-C₇)-alkyl, Aminocarbonyl-(C₁-C₇)-alkyl, Bis[(C₁-C₇)-alkyl]aminocarbonyl-(C₁-C₇)-alkyl, (C₃-C₇)-Cycloalkylaminocarbonyl-(C₁-C₇)-alkyl, Aryl-(C₁-C₇)-alkylaminocarbonyl-(C₁-C₇)-alkyl, Heteroaryl-(C₁-C₇)-alkylaminocarbonyl-(C₁-C₇)-alkyl, Cyano-(C₁-C₇)-alkylaminocarbonyl-(C₁-C₇)-alkyl, (C₁-C₇)-Haloalkylaminocarbonyl-(C₁-C₇)-alkyl, (C₂-C₇)-Alkinylaminocarbonyl-(C₁-C₇)-alkyl, (C₃-C₇)-Cycloalkyl-(C₁-C₇)-alkylaminocarbonyl-(C₁-C₇)-alkyl, (C₁-C₇)-Alkoxycarbonylaminocarbonyl-(C₁-C₇)-alkyl, Aryl-(C₁-C₇)-alkoxycarbonylaminocarbonyl-(C₁-C₇)-alkyl, (C₁-C₇)-Alkoxycarbonyl-(C₁-C₇)-alkylaminocarbonyl, Hydroxycarbonyl-(C₁-C₇)-alkylaminocarbonyl, Aminocarbonyl-(C₁-C₇)-alkylaminocarbonyl, (C₁-C₇)-Alkylaminocarbonyl-(C₁-C₇)-alkylaminocarbonyl, (C₃-C₇)-Cycloalkylaminocarbonyl-(C₁-C₇)-alkylaminocarbonyl, (C₃-C₇)-Cycloalkyl-(C₁-C₇)-alkylaminocarbonyl, (C₃-C₇)-Cycloalkyl-(C₁-C₇)-alkylaminocarbonyl-(C₁-C₇)-alkyl, (C₂-C₇)-Alkenyloxycarbonyl, (C₂-C₇)-Alkenyloxycarbonyl-(C₁-C₇)-alkyl, (C₂-C₇)-Alkenylaminocarbonyl, (C₂-C₇)-Alkenylaminocarbonyl-(C₁-C₇)-alkyl, (C₁-C₇)-Alkylcarbonyl, (C₃-C₇)-Cycloalkylcarbonyl, Formyl, Hydroxyiminomethyl, Aminoiminomethyl, (C₁-C₇)-Alkoxyiminomethyl, (C₁-C₇)-Alkylaminoiminomethyl, Bis[(C₁-C₇)-alkyl]aminoiminomethyl, (C₃-C₇)-Cycloalkoxyiminomethyl, (C₃-C₇)-Cycloalkyl-(C₁-C₇)-alkoximinomethyl, Aryloximinomethyl, Aryl-(C₁-C₇)-alkoxyiminomethyl, Aryl-(C₁-C₇)-alkylaminoiminomethyl, (C₂-C₇)-Alkenyloxyiminomethyl, Arylaminoiminomethyl, Arylsulfonylaminoiminomethyl, Heteroaryl-(C₁-C₇)-alkyl, Heterocyclyl-(C₁-C₇)-alkyl, Hydroxycarbonylheterocyclyl, (C₁-C₇)-Alkoxycarbonylheterocyclyl, (C₂-C₇)-Alkenyloxycarbonylheterocyclyl, (C₂-C₇)-Alkenyl-(C₁-C₇)-alkoxycarbonylheterocyclyl, Aryl-(C₁-C₇)-alkoxycarbonylheterocyclyl, (C₃-C₇)-Cycloalkoxycarbonylheterocyclyl, (C₃-C₇)-Cycloalkyl-(C₁-C₇)-alkoxycarbonylheterocyclyl, Aminocarbonylheterocyclyl, (C₁-C₇)-Alkylaminocarbonylheterocyclyl, Bis-[(C₁-C₇)-Alkyl]aminocarbonylheterocyclyl, (C₃-C₇)-Cycloalkylaminocarbonylheterocyclyl, Aryl-(C₁-C₇)-alkylaminocarbonylheterocyclyl, (C₂-C₇)-Alkenylaminocarbonylheterocyclyl, Hydroxycarbonylheterocyclyl-(C₁-C₇)-alkyl, (C₁-C₇)-Alkoxycarbonylheterocyclyl-(C₁-C₇)-alkyl, Hydroxycarbonyl-(C₃-C₇)-cycloalkyl-(C₁-C₇)-alkyl, (C₁-C₇)-Alkoxycarbonyl-(C₃-C₇)-cycloalkyl-(C₁-C₇)-alkyl, Heterocyclyl, Heterocyclyloxy, Heterocyclylamino, Heterocyclylcarbonyl, Heterocyclylcarbonyl-(C₁-C₇)-alkyl, Hydroxycarbonylheterocyclylcarbonyl, (C₁-C₇)-Alkoxycarbonylheterocyclylcarbonyl, (C₂-C₇)-Alkenyloxycarbonylheterocyclylcarbonyl, (C₂-C₇)-Alkenyl-(C₁-C₇)-alkoxycarbonylheterocyclylcarbonyl, Aryl-(C₁-C₇)-alkoxycarbonylheterocyclylcarbonyl, (C₃-C₇)-Cycloalkoxycarbonylheterocyclylcarbonyl, (C₃-C₇)-Cycloalkyl-(C₁-C₇)-alkoxycarbonylheterocyclylcarbonyl, Aminocarbonylheterocyclylcarbonyl, (C₁-C₇)-Alkylaminocarbonylheterocyclylcarbonyl, Bis-[(C₁-C₇)-Alkyl]aminocarbonylheterocyclylcarbonyl, (C₃-C₇)-Cycloalkylaminocarbonylheterocyclylcarbonyl, Aryl-(C₁-C₇)-alkylaminocarbonylheterocyclylcarbonyl, (C₂-C₇)-Alkenylaminocarbonylheterocyclylcarbonyl stehen,
R⁹ und R¹⁰ unabhängig voneinander für Wasserstoff, Halogen, (C₁-C₇)-Alkyl, (C₁-C₇)-Alkoxy, (C₁-C₇)-Haloalkyl, (C₁-C₇)-Haloalkoxy, Aryl, Heteroaryl, Aryl-(C₁-C₇)-alkyl stehen oder gemeinsam mit dem Atom, an das sie gebunden sind, eine Carbonylgruppe bilden,
A⁶, A⁷ gleich oder verschieden sind und unabhängig voneinander für N-R¹¹, Sauerstoff, Schwefel oder die Gruppierung CR¹⁵R¹⁶ stehen, wobei jedoch in keinem Fall mehr als ein Sauerstoffatom im Heterocyclus enthalten ist, und wobei R¹¹, R¹⁵, R¹⁶ in den Gruppierungen N-R¹¹ und CR¹⁵R¹⁶ jeweils gleiche oder verschiedene Bedeutungen gemäß der nachstehenden Definition haben,
R¹¹ für Wasserstoff, (C₁-C₇)-Alkyl, (C₂-C₇)-Alkenyl, (C₂-C₇)-Alkinyl, (C₁-C₇)-Alkoxy-(C₁-C₇)-alkyl, (C₁-C₇)-Alkylcarbonyl, Arylcarbonyl, Heteroarylcarbonyl, (C₃-C₇)-Cycloalkylcarbonyl, (C₁-C₇)-Alkoxycarbonyl, (C₂-C₇)-Alkenyloxycarbonyl, (C₁-C₇)-Alkoxycarbonyl-(C₁-C₇)-alkyl, (C₁-C₇)-Alkylaminocarbonyl-(C₁-C₇)-alkyl, Arylaminocarbonyl-(C₁-C₇)-alkyl, Aryloxy-(C₁-C₇)-alkyl, Aryl-(C₁-C₇)-alkyl, Heteroaryl-(C₁-C₇)-alkyl, Heterocyclyl, Heterocyclyl-(C₁-C₇)-alkyl steht,
R¹² und R¹³ unabhängig voneinander für Wasserstoff, Halogen, (C₁-C₇)-Alkyl, (C₁-C₇)-Alkoxy, (C₁-C₇)-Haloalkyl, (C₁-C₇)-Haloalkoxy, Aryl, Heteroaryl, Aryl-(C₁-C₇)-alkyl, Heterocyclyl, Heteroaryl-(C₁-C₇)-alkyl, Heterocyclyl-(C₁-C₇)-alkyl stehen oder gemeinsam mit dem Atom, an das sie gebunden sind, eine Carbonylgruppe bilden und
R¹⁵ und R¹⁶ unabhängig voneinander für Wasserstoff, (C₁-C₇)-Alkyl, (C₂-C₇)-Alkenyl, (C₂-C₇)-Alkinyl, (C₁-C₇)-Alkoxy, (C₁-C₇)-Alkoxy-(C₁-C₇)-alkyl, (C₁-C₇)-Alkylcarbonyl, Arylcarbonyl, Heteroarylcarbonyl, Hydroxycarbonyl, (C₁-C₇)-Alkoxycarbonyl, Heterocyclyl, (C₁-C₇)-Haloalkyl stehen,
mit Ausnahme der Verbindung 1-[(2E)-1-Hydroxy-1,3-diphenylprop-2-en-1-yl]cyclopentan-carbonitril.

4. Verwendung einer oder mehrerer Verbindungen der Formel (I) oder deren Salze gemäß einem der Ansprüche 1 bis 3 sowie der Verbindung 1-[(2E)-1-Hydroxy-1,3-diphenylprop-2-en-1-yl]cyclopentan-carbonitril zur Toleranzerhöhung gegenüber abiotischem Stress in Pflanzen.

5. Behandlung von Pflanzen, umfassend die Applikation einer zur Steigerung der Widerstandsfähigkeit von Pflanzen gegenüber abiotischen Stressfaktoren wirksamen, nicht-toxischen Menge einer oder mehrerer Verbindungen der allgemeinen Formel (I) oder deren Salze gemäß einem der Ansprüche 1 bis 3 sowie der Verbindung 1-[(2E)-1-Hydroxy-1,3-diphenylprop-2-en-1-yl]cyclopentan-carbonitril.

6. Behandlung gemäß Anspruch 5, wobei die abiotischen Streßbedingungen einer oder mehrer Bedingungen ausgewählt aus der Gruppe von Hitze, Dürre, Kälte- und Trockenstress, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen entsprechen.

7. Verwendung einer oder mehrerer Verbindungen der allgemeinen Formel (I) oder deren Salze gemäß einem der Ansprüche 1 bis 3 sowie der Verbindung 1-[(2E)-1-Hydroxy-1,3-diphenylprop-2-en-1-yl]cyclopentan-carbonitril in der Sprühapplikation auf Pflanzen und Pflanzenteilen in Kombinationen mit einem oder mehrer Wirkstoffen ausgewählt aud der Gruppe der Insektizide, Lockstoffe, Akarizide, Fungizide, Nematizide, Herbizide, wachstumsregulatorische Stoffe, Safener, die Pflanzenreife beeinflussende Stoffe und Bakterizide.

8. Verwendung einer oder mehrerer der Verbindungen der allgemeinen Formel (I) oder deren Salze gemäß einem der Ansprüche 1 bis 3 sowie der Verbindung 1-[(2E)-1-Hydroxy-1,3-diphenylprop-2-en-1-yl]cyclopentan-carbonitril in der Sprühapplikation auf Pflanzen und Pflanzenteilen in Kombinationen mit Düngemitteln.

9. Verwendung einer oder meherer der Verbindungen der allgemeinen Formel (I) oder deren Salze gemäß einem der Ansprüche 1 bis 3 sowie der Verbindung 1-[(2E)-1-Hydroxy-1,3-diphenylprop-2-en-1-yl]cyclopentan-carbonitril zur Applikation auf gentechnisch veränderten Sorten, deren Saatgut, oder auf Anbauflächen auf denen diese Sorten wachsen.

10. Sprühlösung zur Behandlung von Pflanzen, enthaltend eine zur Steigerung der Widerstandsfähigkeit von Pflanzen gegenüber abiotischen Stressfaktoren wirksame Menge einer oder meherer Verbindungen der allgemeinen Formel (I) gemäß einem der Ansprüche 1 bis 3 sowie der Verbindung 1-[(2E)-1-Hydroxy-1,3-diphenylprop-2-en-1-yl]cyclopentan-carbonitril oder deren Salze.

11. Verwendung von Sprühlösungen, die eine oder mehrere der Verbindungen der allgemeinen Formel (I) gemäß einem der Ansprüche 1 bis 3 sowie der Verbindung 1-[(2E)-1-Hydroxy-1,3-diphenylprop-2-en-1-yl]cyclopentan-carbonitril oder deren Salze enthalten, zur Steigerung der Widerstandsfähigkeit von Pflanzen gegenüber abiotischen Stressfaktoren.

12. Verfahren zur Erhöhung der Stresstoleranz bei Pflanzen ausgewählt aus der Gruppe der Nutzpflanzen, Zierpflanzen, Rasenarten, oder Bäumen, welches die Applikation einer ausreichenden, nicht-toxischen Menge einer oder mehrere Verbindungen der allgemeinen Formel (I) gemäß einem der Ansprüche 1 bis 3 sowie der Verbindung 1-[(2E)-1-Hydroxy-1,3-diphenylprop-2-en-1-yl]cyclopentan-carbonitril oder deren jeweilige Salze auf die Fläche, wo die entsprechende Wirkung gewünscht wird, umfasst, wobei die Applikation auf die Pflanzen, deren Saatgut oder auf die Fläche, auf der die Pflanzen wachsen, erfolgt.

13. Verfahren gemäß Anspruch 12, wobei die Widerstandsfähigkeit der so behandelten Pflanzen gegenüber abiotischem Stress gegenüber nicht behandelten Pflanzen unter ansonsten gleichen physiologischen Bedingungen um mindestens 3% erhöht ist.

14. Verbindungen der allgemeinen Formel (II), oder deren Salze 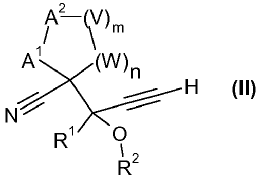 wobei
R¹ für Wasserstoff, (C₁-C₈)-Alkyl, Aryl, Heteroaryl, Heterocyclyl, (C₃-C₈)-Cycloalkyl, (C₂-C₈)-Alkenyl, (C₂-C₈)-Alkinyl, (C₂-C₈)-Alkinyl-(C₁-C₈)-alkyl, (C₁-C₈)-Alkoxy-(C₁-C₈)-alkyl, Hydroxy-(C₁-C₈)-alkyl, (C₁-C₈)-Haloalkyl, (C₂-C₈)-Haloalkenyl, (C₁-C₈)-Haloalkoxy-(C₁-C₈)-alkyl, (C₁-C₈)-Alkylthio-(C₁-C₈)-alkyl, Aryl-(C₁-C₈)-alkyl, Heterocyclyl-(C₁-C₈)-alkyl, (C₃-C₈)-Halocycloalkyl, (C₄-C₈)-Cycloalkenyl, (C₁-C₈)-Alkoxy-(C₁-C₈)-alkoxy-(C₁-C₈)-alkyl, (C₁-C₈)-Alkoxy-(C₁-C₈)-haloalkyl, (C₁-C₈)-Haloalkoxy-(C₁-C₈)-haloalkyl, (C₃-C₈)-Cycloalkyl-(C₁-C₈)-alkyl steht,
R² für Wasserstoff, (C₁-C₈)-Alkyl, (C₁-C₈)-Alkoxy-(C₁-C₈)-alkyl, (C₁-C₈)-Alkoxy-(C₁-C₈)-alkoxy-(C₁-C₈)-alkyl, (C₂-C₈)-Alkenyl, (C₂-C₈)-Alkinyl, (C₁-C₈)-Alkylcarbonyl, Arylcarbonyl, Heteroarylcarbonyl, (C₃-C₈)-Cycloalkylcarbonyl, (C₂-C₈)-Alkenylcarbonyl, Heterocyclylcarbonyl, (C₁-C₈)-Alkoxycarbonyl, (C₂-C₈)-Alkenyloxycarbonyl, Aryloxy-(C₁-C₈)-alkyl, Aryl-(C₁-C₈)-alkoxycarbonyl, (C₃-C₈)-Cycloalkoxycarbonyl, (C₃-C₈)-Cycloalkyl-(C₁-C₈)-alkoxycarbonyl, Aryl-(C₁-C₈)-alkoxy-(C₁-C₈)-alkyl, Aryl-(C₁-C₈)-alkyl, (C₁-C₈)-Alkoxy-(C₁-C₈)-alkoxy-(C₁-C₈)-alkyl, (C₁-C₈)-Alkylthio-(C₁-C₈)-alkyl, Tris[(C₁-C₈)-alkyl]silyl, (C₁-C₈)-Alkyl-Bis-[(C₁-C₈)-alkyl]silyl, (C₁-C₈)-Alkyl-Bis(aryl)silyl, Aryl-Bis[(C₁-C₈)-alkyl]silyl, (C₃-C₈)-Cycloalkyl-Bis[(C₁-C₈)-alkyl]silyl, Halo-Bis[(C₁-C₈)-alkyl]silyl, Tris[(C₁-C₈)-alkyl]silyl-(C₁-C₈)-alkoxy-(C₁-C₈)-alkyl, Tris[(C₁-C₈)-alkyl]silyl-(C₁-C₈)-alkyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden seind und wobei die Sauerstoff- oder Schwefelatome jeweils nicht benachbart sind,
m für 0, 1, 2 steht,
n für 0, 1, 2 steht und R³ und R⁴ unabhängig voneinander für Wasserstoff, (C₁-C₈)-Alkyl, Halogen, (C₃-C₈)-Cycloalkyl, (C₁-C₈)-Alkoxy, Aryl, Heterocyclyl, Heteroaryl, Aryl-(C₁-C₈)-alkyl, (C₁-C₈)-Alkylthio, (C₁-C₈)-Haloalkyl, (C₁-C₈)-Haloalkyloxy, (C₁-C₈)-Haloalkylthio, (C₁-C₈)-Alkoxy-(C₁-C₈)-alkyl, (C₁-C₈)-Alkylthio-(C₁-C₈)-alkyl, Heteroaryl-(C₁-C₈)-alkyl, Heterocyclyl-(C₁-C₈)-alkyl, (C₁-C₈)-Alkoxy-(C₁-C₈)-haloalkyl, (C₁-C₈)-Haloalkoxy-(C₁-C₈)-haloalkyl, (C₂-C₈)-Alkenyl, (C₂-C₈)-Alkinyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden.

15. Verbindungen der Formel (II) gemäß Anspruch 14, wobei
R¹ für Wasserstoff, (C₁-C₆)-Alkyl, Aryl, Heteroaryl, Heterocyclyl, (C₃-C₆)-Cycloalkyl, (C₂-C₆)-Alkenyl, (C₂-C₆)-Alkinyl, (C₂-C₆)-Alkinyl-(C₁-C₆)-alkyl, (C₁-C₆)-Alkoxy-(C₁-C₆)-alkyl, Hydroxy-(C₁-C₆)-alkyl, (C₁-C₆)-Haloalkyl, (C₂-C₆)-Haloalkenyl, (C₁-C₆)-Haloalkoxy-(C₁-C₆)-alkyl, (C₁-C₆)-Alkylthio-(C₁-C₆)-alkyl, Aryl-(C₁-C₆)-alkyl, Heterocyclyl-(C₁-C₆)-alkyl, (C₃-C₆)-Halocycloalkyl, (C₄-C₆)-Cycloalkenyl, (C₁-C₆)-Alkoxy-(C₁-C₆)-alkoxy-(C₁-C₆)-alkyl, (C₁-C₆)-Alkoxy-(C₁-C₆)-haloalkyl, (C₁-C₆)-Haloalkoxy-(C₁-C₆)-haloalkyl, (C₃-C₆)-Cycloalkyl-(C₁-C₆)-alkyl steht,
R² für Wasserstoff, (C₁-C₆)-Alkyl, (C₁-C₆)-Alkoxy-(C₁-C₆)-alkyl, (C₁-C₆)-Alkoxy-(C₁-C₆)-alkoxy-(C₁-C₆)-alkyl, (C₂-C₆)-Alkenyl, (C₂-C₆)-Alkinyl, (C₁-C₆)-Alkylcarbonyl, Arylcarbonyl, Heteroarylcarbonyl, (C₃-C₆)-Cycloalkylcarbonyl, (C₂-C₆)-Alkenylcarbonyl, Heterocyclylcarbonyl, (C₁-C₆)-Alkoxycarbonyl, (C₂-C₆)-Alkenyloxycarbonyl, Aryloxy-(C₁-C₆)-alkyl, Aryl-(C₁-C₆)-alkoxycarbonyl, (C₃-C₆)-Cycloalkoxycarbonyl, (C₃-C₆)-Cycloalkyl-(C₁-C₆)-alkoxycarbonyl, Aryl-(C₁-C₆)-alkoxy-(C₁-C₆)-alkyl, Aryl-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, Tris[(C₁-C₆)-alkyl]silyl, (C₁-C₆)-Alkyl-Bis-[(C₁-C₆)-alkyl]silyl, (C₁-C₆)-Alkyl-Bis(aryl)silyl, Aryl-Bis[(C₁-C₆)-alkyl]silyl, (C₃-C₆)-Cycloalkyl-Bis[(C₁-C₆)-alkyl]silyl, Halo-Bis[(C₁-C₆)-alkyl]silyl, Tris[(C₁-C₆)-alkyl]silyl-(C₁-C₆)-alkoxy-(C₁-C₆)-alkyl, Tris[(C₁-C₆)-alkyl]silyl-(C₁-C₆)-alkyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffato, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht und R³ und R⁴ unabhängig voneinander für Wasserstoff, (C₁-C₆)-Alkyl, Halogen, (C₃-C₆)-Cycloalkyl, (C₁-C₆)-Alkoxy, Aryl, Heterocyclyl, Heteroaryl, Aryl-(C₁-C₆)-alkyl, (C₁-C₆)-Alkylthio, (C₁-C₆)-Haloalkyl, (C₁-C₆)-Haloalkyloxy, (C₁-C₆)-Haloalkylthio, (C₁-C₆)-Alkoxy-(C₁-C₆)-alkyl, (C₁-C₆)-Alkylthio-(C₁-C₆)-alkyl, Heteroaryl-(C₁-C₆)-alkyl, Heterocyclyl-(C₁-C₆)-alkyl, (C₁-C₆)-Alkoxy-(C₁-C₆)-haloalkyl, (C₁-C₆)-Haloalkoxy-(C₁-C₆)-haloalkyl, (C₂-C₆)-Alkenyl, (C₂-C₆)-Alkinyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden.

16. Verbindungen der allgemeinen Formel (III), oder deren Salze 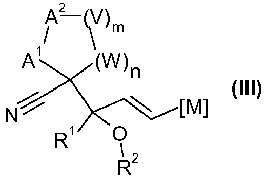 wobei
R¹ für Wasserstoff, (C₁-C₈)-Alkyl, Aryl, Heteroaryl, Heterocyclyl, (C₃-C₈)-Cycloalkyl, (C₂-C₈)-Alkenyl, (C₂-C₈)-Alkinyl, (C₂-C₈)-Alkinyl-(C₁-C₈)-alkyl, (C₁-C₈)-Alkoxy-(C₁-C₈)-alkyl, Hydroxy-(C₁-C₈)-alkyl, (C₁-C₈)-Haloalkyl, (C₂-C₈)-Haloalkenyl, (C₁-C₈)-Haloalkoxy-(C₁-C₈)-alkyl, (C₁-C₈)-Alkylthio-(C₁-C₈)-alkyl, Aryl-(C₁-C₈)-alkyl, Heterocyclyl-(C₁-C₈)-alkyl, (C₃-C₈)-Halocycloalkyl, (C₄-C₈)-Cycloalkenyl, (C₁-C₈)-Alkoxy-(C₁-C₈)-alkoxy-(C₁-C₈)-alkyl, (C₁-C₈)-Alkoxy-(C₁-C₈)-haloalkyl, (C₁-C₈)-Haloalkoxy-(C₁-C₈)-haloalkyl steht,
R² für Wasserstoff, (C₁-C₈)-Alkyl, (C₁-C₈)-Alkoxy-(C₁-C₈)-alkyl, (C₁-C₈)-Alkoxy-(C₁-C₈)-alkoxy-(C₁-C₈)-alkyl, (C₂-C₈)-Alkenyl, (C₂-C₈)-Alkinyl, (C₁-C₈)-Alkylcarbonyl, Arylcarbonyl, Heteroarylcarbonyl, (C₃-C₈)-Cycloalkylcarbonyl, (C₂-C₈)-Alkenylcarbonyl, Heterocyclylcarbonyl, (C₁-C₈)-Alkoxycarbonyl, (C₂-C₈)-Alkenyloxycarbonyl, Aryloxy-(C₁-C₈)-alkyl, Aryl-(C₁-C₈)-alkoxycarbonyl, (C₃-C₈)-Cycloalkoxycarbonyl, (C₃-C₈)-Cycloalkyl-(C₁-C₈)-alkoxycarbonyl, Aryl-(C₁-C₈)-alkoxy-(C₁-C₈)-alkyl, Aryl-(C₁-C₈)-alkyl, (C₁-C₈)-Alkoxy-(C₁-C₈)-alkoxy-(C₁-C₈)-alkyl, (C₁-C₈)-Alkylthio-(C₁-C₈)-alkyl, Tris[(C₁-C₈)-alkyl]silyl, (C₁-C₈)-Alkyl-Bis-[(C₁-C₈)-alkyl]silyl, (C₁-C₈)-Alkyl-Bis(aryl)silyl, Aryl-Bis[(C₁-C₈)-alkyl]silyl, (C₃-C₈)-Cycloalkyl-Bis[(C₁-C₈)-alkyl]silyl, Halo-Bis[(C₁-C₈)-alkyl]silyl, Tris[(C₁-C₈)-alkyl]silyl-(C₁-C₈)-alkoxy-(C₁-C₈)-alkyl, Tris[(C₁-C₈)-alkyl]silyl-(C₁-C₈)-alkyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht,
R³ und R⁴ unabhängig voneinander für Wasserstoff, (C₁-C₈)-Alkyl, Halogen, (C₃-C₈)-Cycloalkyl, (C₁-C₈)-Alkoxy, Aryl, Heterocyclyl, Heteroaryl, Aryl-(C₁-C₈)-alkyl, (C₁-C₈)-Alkylthio, (C₁-C₈)-Haloalkyl, (C₁-C₈)-Haloalkyloxy, (C₁-C₈)-Haloalkylthio, (C₁-C₈)-Alkoxy-(C₁-C₈)-alkyl, (C₁-C₈)-Alkylthio-(C₁-C₈)-alkyl, Heteroaryl-(C₁-C₈)-alkyl, Heterocyclyl-(C₁-C₈)-alkyl, (C₁-C₈)-Alkoxy-(C₁-C₈)-haloalkyl, (C₁-C₈)-Haloalkoxy-(C₁-C₈)-haloalkyl, (C₂-C₈)-Alkenyl, (C₂-C₈)-Alkinyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden und
[M] für Tris-[(C₁-C₆)-Alkyl]stannyl, Tris-[(C₃-C₈)-Cycloalkyl]stannyl, Tris-[(C₁-C₆)-Alkyl]germanyl, Tris-[(C₃-C₈)-Cycloalkyl]germanyl, Bis-(Cyclopentadienyl)zirconyl, Bis-(1,2,3,4,5-Pentamethylcyclopentadienyl)zirconyl, Bis-(Cyclopentadienyl)hafnyl, Bis-(1,2,3,4,5-Pentamethylcyclopentadienyl)hafnyl, Bis-(Hydroxy)boryl, Bis-[(C₁-C₆)-Alkoxy]-boryl, (C₁-C₆)-Alkyl-1,3,2-dioxaborolan-2-yl, Bis-[(C₁-C₆)-Alkyl]-1,3,2-dioxaborolan-2-yl, Tetrakis-[(C₁-C₆)-Alkyl]-1,3,2-dioxaborolan-2-yl, 1,3,2-Dioxaborinan-2-yl, Bis-[(C₁-C₆)-Alkyl]-1,3,2-Dioxaborinan-2-yl, (C₁-C₆)-Alkyl-1,3,2-Dioxaborinan-2-yl, Tris-[(C₁-C₆)-Alkyl]-1,3,2-Dioxaborinan-2-yl, 2,6,7-Trioxa-1-Boranuidabicyclo[2.2.2]octanyl, (C₁-C₆)-Alkyl-2,6,7-Trioxa-1-Boranuidabicyclo[2.2.2]octanyl, Tris-[(C₁-C₆)-Alkyl]plumbanyl, Tris-[(C₃-C₈)-Cycloalkyl]plumbanyl, Tris-[(C₁-C₆)-Alkylcarbonyloxy]plumbanyl, Tris-Aryl-plumbanyl, Bis-[(C₁-C₆)-Alkylcarbonyloxy]-arylplumbanyl, Bis-[(C₁-C₆)-Alkyl]-alanyl, Bis-[(C₁-C₆)-Cycloalkyl]-alanyl, Dichloralanyl, Chlormagnesyl, Brommagnesyl, Chlorzinkyl, Chlorhydrargyl, Bromhydrargyl, (C₁-C₆)-Alkylhydrargyl, (C₃-C₆)-Cycloalkylhydrargyl, Tris-[(C₁-C₆)-alkyl]silyl, (C₁-C₆)-Alkyl-[bis-(C₁-C₆)-alkyl]silyl, (C₁-C₆)-Alkyl-bis-(aryl)silyl, Aryl-bis-[(C₁-C₆)-alkyl)]silyl, (C₃-C₇)-Cycloalkyl-bis-[(C₁-C₆)-alkyl]silyl steht.

17. Verbindungen der Formel (III) gemäß Anspruch 16, wobei
R¹ für Wasserstoff, (C₁-C₆)-Alkyl, Aryl, Heteroaryl, Heterocyclyl, (C₃-C₆)-Cycloalkyl, (C₂-C₆)-Alkenyl, (C₂-C₆)-Alkinyl, (C₂-C₆)-Alkinyl-(C₁-C₆)-alkyl, (C₁-C₆)-Alkoxy-(C₁-C₆)-alkyl, Hydroxy-(C₁-C₆)-alkyl, (C₁-C₆)-Haloalkyl, (C₂-C₆)-Haloalkenyl, (C₁-C₆)-Haloalkoxy-(C₁-C₆)-alkyl, (C₁-C₆)-Alkylthio-(C₁-C₆)-alkyl, Aryl-(C₁-C₆)-alkyl, Heterocyclyl-(C₁-C₆)-alkyl, (C₃-C₆)-Halocycloalkyl, (C₄-C₆)-Cycloalkenyl, (C₁-C₆)-Alkoxy-(C₁-C₆)-alkoxy-(C₁-C₆)-alkyl, (C₁-C₈)-Alkoxy-(C₁-C₈)-haloalkyl, (C₁-C₈)-Haloalkoxy-(C₁-C₈)-haloalkyl steht,
R² für Wasserstoff, (C₁-C₆)-Alkyl, (C₁-C₆)-Alkoxy-(C₁-C₆)-alkyl, (C₁-C₆)-Alkoxy-(C₁-C₆)-alkoxy-(C₁-C₆)-alkyl, (C₂-C₆)-Alkenyl, (C₂-C₆)-Alkinyl, (C₁-C₆)-Alkylcarbonyl, Arylcarbonyl, Heteroarylcarbonyl, (C₃-C₆)-Cycloalkylcarbonyl, (C₂-C₆)-Alkenylcarbonyl, Heterocyclylcarbonyl, (C₁-C₆)-Alkoxycarbonyl, (C₂-C₆)-Alkenyloxycarbonyl, Aryloxy-(C₁-C₆)-alkyl, Aryl-(C₁-C₆)-alkoxycarbonyl, (C₃-C₆)-Cycloalkoxycarbonyl, (C₃-C₆)-Cycloalkyl-(C₁-C₆)-alkoxycarbonyl, Aryl-(C₁-C₆)-alkoxy-(C₁-C₆)-alkyl, Aryl-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, Tris[(C₁-C₆)-alkyl]silyl, (C₁-C₆)-Alkyl-Bis-[(C₁-C₆)-alkyl]silyl, (C₁-C₆)-Alkyl-Bis(aryl)silyl, Aryl-Bis[(C₁-C₆)-alkyl]silyl, (C₃-C₆)-Cycloalkyl-Bis[(C₁-C₆)-alkyl]silyl, Halo-Bis[(C₁-C₆)-alkyl]silyl, Tris[(C₁-C₆)-alkyl]silyl-(C₁-C₆)-alkoxy-(C₁-C₆)-alkyl, Tris[(C₁-C₆)-alkyl]silyl-(C₁-C₆)-alkyl steht,
A¹, A², V, W unabhängig voneinander für eine Gruppe CR³R⁴, Sauerstoff oder Schwefel stehen, wobei jeweils maximal 2 Sauerstoff- oder 2 Schwefelatome in dem durch die Gruppen A¹, A², V, W und dem Kohlenstoffatom, an das sie gebunden sind, gebildeten Ring vorhanden sind und wobei die Sauerstoff- oder Schwefelatome jeweils nicht benachbart sind,
m für 0, 1, 2 steht,
n für 0, 1, 2 steht,
R³ und R⁴ unabhängig voneinander für Wasserstoff, (C₁-C₆)-Alkyl, Halogen, (C₃-C₆)-Cycloalkyl, (C₁-C₆)-Alkoxy, Aryl, Heterocyclyl, Heteroaryl, Aryl-(C₁-C₆)-alkyl, (C₁-C₆)-Alkylthio, (C₁-C₆)-Haloalkyl, (C₁-C₆)-Haloalkyloxy, (C₁-C₆)-Haloalkylthio, (C₁-C₆)-Alkoxy-(C₁-C₆)-alkyl, (C₁-C₆)-Alkylthio-(C₁-C₆)-alkyl, Heteroaryl-(C₁-C₆)-alkyl, Heterocyclyl-(C₁-C₆)-alkyl, (C₁-C₆)-Alkoxy-(C₁-C₆)-haloalkyl, (C₁-C₆)-Haloalkoxy-(C₁-C₆)-haloalkyl, (C₂-C₆)-Alkenyl, (C₂-C₆)-Alkinyl stehen oder R³ und R⁴ mit dem Atom, an das sie gebunden sind, einen vollständig gesättigten, gegebenenfalls durch Heteroatome unterbrochenen und gegebenenfalls weiter substituierten 3 bis 6-gliedrigen Ring bilden und
[M] für Tris-[(C₁-C₆)-Alkyl]stannyl, Tris-[(C₃-C₆)-Cycloalkyl]stannyl, Tris-[(C₁-C₆)-Alkyl]germanyl, Tris-[(C₃-C₆)-Cycloalkyl]germanyl, Bis-(Cyclopentadienyl)zirconyl, Bis-(1,2,3,4,5-Pentamethylcyclopentadienyl)zirconyl, Bis-(Cyclopentadienyl)hafnyl, Bis-(1,2,3,4,5-Pentamethylcyclopentadienyl)hafnyl, Bis-(Hydroxy)boryl, Bis-[(C₁-C₆)-Alkoxy]-boryl, (C₁-C₆)-Alkyl-1,3,2-dioxaborolan-2-yl, Bis-[(C₁-C₆)-Alkyl]-1,3,2-dioxaborolan-2-yl, Tetrakis-[(C₁-C₆)-Alkyl]-1,3,2-dioxaborolan-2-yl, 1,3,2-Dioxaborinan-2-yl, Bis-[(C₁-C₆)-Alkyl]-1,3,2-Dioxaborinan-2-yl, (C₁-C₆)-Alkyl-1,3,2-Dioxaborinan-2-yl, Tris-[(C₁-C₆)-Alkyl]-1,3,2-Dioxaborinan-2-yl, 2,6,7-Trioxa-1-Boranuidabicyclo[2.2.2]octanyl, (C₁-C₆)-Alkyl-2,6,7-Trioxa-1-Boranuidabicyclo[2.2.2]octanyl, Tris-[(C₁-C₆)-Alkyl]plumbanyl, Tris-[(C₃-C₆)-Cycloalkyl]plumbanyl, Tris-[(C₁-C₆)-Alkylcarbonyloxy]plumbanyl, Tris-Aryl-plumbanyl, Bis-[(C₁-C₆)-Alkylcarbonyloxy]-arylplumbanyl, Bis-[(C₁-C₆)-Alkyl]-alanyl, Bis-[(C₁-C₆)-Cycloalkyl]-alanyl, Dichloralanyl, Chlormagnesyl, Brommagnesyl, Chlorzinkyl, Chlorhydrargyl, Bromhydrargyl, (C₁-C₆)-Alkylhydrargyl, (C₃-C₆)-Cycloalkylhydrargyl, Tris-[(C₁-C₆)-alkyl]silyl, (C₁-C₆)-Alkyl-[bis-(C₁-C₆)-alkyl]silyl, (C₁-C₆)-Alkyl-bis-(aryl)silyl, Aryl-bis-[(C₁-C₆)-alkyl)]silyl, (C₃-C₇)-Cycloalkyl-bis-[(C₁-C₆)-alkyl]silyl steht.

## Claims

1. Substituted vinyl- and alkynylcyanocycloalkanols and vinyl- and alkynylcyanoheterocyclylalkanols of the general formula (I) or salts thereof 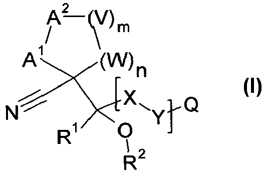 where
[X-Y] represents the moieties 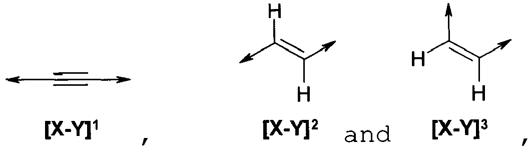
Q represents the carbocyclic and heterocyclic moieties 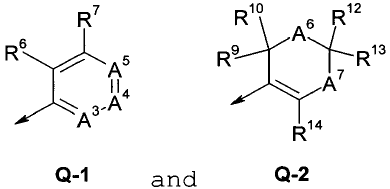 where the R⁶, R⁷, R⁹, R¹⁰, R¹², R¹³, R¹⁴, A³, A⁴, A⁵, A⁶ and A⁷ moieties are each as defined below and where the arrow represents a bond to the respective [X-Y] moiety,
R¹ is alkyl, aryl, heteroaryl, heterocyclyl, cycloalkyl, alkenyl, alkynyl, alkoxyalkyl, hydroxyalkyl, haloalkyl, haloalkenyl, haloalkoxyalkyl, alkylthioalkyl, arylalkyl, heterocyclylalkyl, halocycloalkyl, cycloalkenyl, alkoxyalkoxyalkyl, cycloalkylalkyl, cycloalkenylalkyl, haloalkynyl, alkylsulphinylalkyl, alkylsulphonylalkyl, halocycloalkylalkyl cycloalkylsulphinylalkyl, cycloalkylsulphonylalkyl, arylsulphinylalkyl, arylsulphonylalkyl, arylthioalkyl, cycloalkylthioalkyl, alkoxyhaloalkyl, haloalkoxyhaloalkyl,
R² is hydrogen, alkyl, alkoxyalkyl, alkoxyalkoxyalkyl, alkenyl, alkynyl, alkylcarbonyl, arylcarbonyl, heteroarylcarbonyl, cycloalkylcarbonyl, alkenylcarbonyl, heterocyclylcarbonyl, alkoxycarbonyl, alkenyloxycarbonyl, aryloxyalkyl, arylalkoxycarbonyl, cycloalkoxycarbonyl, cycloalkylalkoxycarbonyl, arylalkoxyalkyl, arylalkyl, alkoxyalkoxyalkyl, alkylthioalkyl, trialkylsilyl, alkyl(bisalkyl)silyl, alkyl(bisaryl)silyl, aryl(bisalkyl)silyl, cycloalkyl(bisalkyl)silyl, halo(bisalkyl)silyl, trialkylsilylalkoxyalkyl, trialkylsilylalkyl, alkynyloxycarbonyl, cycloalkyl, cycloalkylalkyl, aminocarbonyl, alkylaminocarbonyl, bisalkylaminocarbonyl, cycloalkylaminocarbonyl, alkylsulphonyl, haloalkylsulphonyl, arylsulphonyl, heteroarylsulphonyl, cycloalkylsulphonyl,
A¹, A², V, W are each independently a CR³R⁴ group, an N-R⁵ group, oxygen or sulphur, where not more than 2 oxygen or 2 sulphur atoms are present in each ring formed by the A¹, A², V, W groups and the carbon atom to which they are bonded, and where the oxygen and sulphur atoms are not adjacent to one another,
m is 0, 1, 2,
n is 0, 1, 2,
R³ and R⁴ are each independently hydrogen, alkyl, halogen, cycloalkyl, alkoxy, aryl, heterocyclyl, heteroaryl, arylalkyl, alkylthio, haloalkyl, haloalkyloxy, haloalkylthio, alkoxyalkyl, alkylthioalkyl, heteroarylalkyl, heterocyclylalkyl, cycloalkylalkyl, cycloalkenyl, alkynyl, alkenyl, haloalkenyl, haloalkynyl, alkylsulphinyl, alkylsulphonyl, cycloalkylsulphinyl, cycloalkylsulphonyl, arylsulphinyl, arylsulphonyl, alkoxyhaloalkyl, haloalkoxyhaloalkyl, or
R³ and R⁴ together with the atom to which they are bonded form a fully saturated 3- to 6-membered ring optionally interrupted by heteroatoms and optionally having further substitution,
R⁵ is hydrogen, alkyl, alkenyl, alkynyl, alkoxyalkyl, alkylcarbonyl, arylcarbonyl, heteroarylcarbonyl, cycloalkylcarbonyl, alkoxycarbonyl, alkenyloxycarbonyl, alkoxycarbonylalkyl, alkylaminocarbonylalkyl, arylaminocarbonylalkyl, aryloxyalkyl, arylalkyl, heteroarylalkyl, heterocyclyl, heterocyclylalkyl, haloalkyl,
R⁶ and R⁷ are each independently hydrogen, nitro, amino, cyano, thiocyanato, isothiocyanato, halogen, alkyl, cycloalkyl, alkenyl, alkynyl, aryl, arylalkyl, arylalkoxy, heteroaryl, alkoxyalkyl, hydroxyalkyl, haloalkyl, halocycloalkyl, alkoxy, haloalkoxy, aryloxy, heteroaryloxy, cycloalkyloxy, hydroxyl, cycloalkylalkoxy, alkoxycarbonyl, hydroxycarbonyl, aminocarbonyl, alkylaminocarbonyl, cycloalkylaminocarbonyl, cyanoalkylaminocarbonyl, alkenylaminocarbonyl, alkynylaminocarbonyl, alkylamino, alkylthio, haloalkylthio, hydrothio, bisalkylamino, cycloalkylamino, alkylcarbonylamino, cycloalkylcarbonylamino, formylamino, haloalkylcarbonylamino, alkoxycarbonylamino, alkylaminocarbonylamino, alkyl(alkyl)aminocarbonylamino, alkylsulphonylamino, cycloalkylsulphonylamino, arylsulphonylamino, hetarylsulphonylamino, sulphonylhaloalkylamino, aminosulphonyl, aminoalkylsulphonyl, aminohaloalkylsulphonyl, alkylaminosulphonyl, bisalkylaminosulphonyl, cycloalkylaminosulphonyl, haloalkylaminosulphonyl, arylaminosulphonyl, arylalkylaminosulphonyl, alkylsulphonyl, cycloalkylsulphonyl, arylsulphonyl, alkylsulphinyl, cycloalkylsulphinyl, arylsulphinyl, N,S-dialkylsulphonimidoyl, S-alkylsulphonimidoyl, alkylsulphonylaminocarbonyl, cycloalkylsulphonylaminocarbonyl, cycloalkylaminosulphonyl, arylalkylcarbonylamino, cycloalkylalkylcarbonylamino, heteroarylcarbonylamino, alkoxyalkylcarbonylamino, hydroxyalkylcarbonylamino, trialkylsilyl,
A³, A⁴, A⁵ are the same or different and are each independently N (nitrogen) or the C-R⁸ moiety, but there are never more than two adjacent nitrogen atoms, and where each R⁸ in the C-R⁸ moiety has identical or different meanings according to the definition below, and
A³ and A⁴, when each is a C-R⁸ group, together with the atoms to which they are bonded form a fully saturated, partly saturated or fully unsaturated 5- to 6-membered ring optionally interrupted by heteroatoms and optionally having further substitution,
A⁴ and A⁵, when each is a C-R⁸ group, together with the atoms to which they are bonded form a fully saturated, partly saturated or fully unsaturated 5- to 6-membered ring optionally interrupted by heteroatoms and optionally having further substitution,
R⁸ and R¹⁴ are each independently hydrogen, nitro, amino, hydroxyl, hydrothio, thiocyanato, isothiocyanato, halogen, alkyl, cycloalkyl, alkenyl, alkynyl, aryl, arylalkyl, arylalkoxy, heteroaryl, haloalkyl, halocycloalkyl, alkoxy, haloalkoxy, aryloxy, heteroaryloxy, cycloalkyloxy, cycloalkylalkoxy, hydroxyalkyl, alkoxyalkyl, aryloxyalkyl, heteroaryloxyalkyl, alkylamino, alkylthio, haloalkylthio, bisalkylamino, cycloalkylamino, alkylcarbonylamino, cycloalkylcarbonylamino, formylamino, haloalkylcarbonylamino, alkoxycarbonylamino, alkylaminocarbonylamino, alkyl(alkyl)aminocarbonylamino, alkylsulphonylamino, cycloalkylsulphonylamino, arylsulphonylamino, hetarylsulphonylamino, sulphonylhaloalkylamino, aminoalkylsulphonyl, aminohaloalkylsulphonyl, alkylaminosulphonyl, bisalkylaminosulphonyl, cycloalkylaminosulphonyl, haloalkylaminosulphonyl, arylaminosulphonyl, arylalkylaminosulphonyl, alkylsulphonyl, cycloalkylsulphonyl, arylsulphonyl, alkylsulphinyl, cycloalkylsulphinyl, arylsulphinyl, N,S-dialkylsulphonimidoyl, S-alkylsulphonimidoyl, alkylsulphonylaminocarbonyl, cycloalkylsulphonylaminocarbonyl, cycloalkylaminosulphonyl, arylalkylcarbonylamino, cycloalkylalkylcarbonylamino, heteroarylcarbonylamino, alkoxyalkylcarbonylamino, hydroxyalkylcarbonylamino, cyano, cyanoalkyl, hydroxycarbonyl, alkoxycarbonyl, cycloalkoxycarbonyl, cycloalkylalkoxycarbonyl, aryloxycarbonyl, arylalkoxycarbonyl, aminocarbonyl, alkylaminocarbonyl, bisalkylaminocarbonyl, alkyl(alkoxy)aminocarbonyl, cycloalkylaminocarbonyl, arylalkylaminocarbonyl, heteroarylalkylaminocarbonyl, cyanoalkylaminocarbonyl, haloalkylaminocarbonyl, alkynylaminocarbonyl, alkoxycarbonylaminocarbonyl, arylalkoxycarbonylaminocarbonyl, hydroxycarbonylalkyl, alkoxycarbonylalkyl, cycloalkoxycarbonylalkyl, cycloalkylalkoxycarbonylalkyl, alkylaminocarbonylalkyl, aminocarbonylalkyl, bisalkylaminocarbonylalkyl, cycloalkylaminocarbonylalkyl, arylalkylaminocarbonylalkyl, heteroarylalkylaminocarbonylalkyl, cyanoalkylaminocarbonylalkyl, haloalkylaminocarbonylalkyl, alkynylaminocarbonylalkyl, cycloalkylalkylaminocarbonylalkyl, alkoxycarbonylaminocarbonylalkyl, arylalkoxycarbonylaminocarbonylalkyl, alkoxycarbonylalkylaminocarbonyl, hydroxycarbonylalkylaminocarbonyl, aminocarbonylalkylaminocarbonyl, alkylaminocarbonylalkylaminocarbonyl, cycloalkylaminocarbonylalkylaminocarbonyl, cycloalkylalkylaminocarbonyl, cycloalkylalkylaminocarbonylalkyl, alkenyloxycarbonyl, alkenyloxycarbonylalkyl, alkenylaminocarbonyl, alkenylaminocarbonylalkyl, alkylcarbonyl, cycloalkylcarbonyl, formyl, hydroxyiminomethyl, aminoiminomethyl, alkoxyiminomethyl, alkylaminoiminomethyl, dialkylaminoiminomethyl, cycloalkoxyiminomethyl, cycloalkylalkoximinomethyl, aryloximinomethyl, arylalkoxyiminomethyl, arylalkylaminoiminomethyl, alkenyloxyiminomethyl, arylaminoiminomethyl, arylsulphonylaminoiminomethyl, heteroarylalkyl, heterocyclylalkyl, hydroxycarbonylheterocyclyl, alkoxycarbonylheterocyclyl, alkenyloxycarbonylheterocyclyl, alkenylalkoxycarbonylheterocyclyl, arylalkoxycarbonylheterocyclyl, cycloalkoxycarbonylheterocyclyl, cycloalkylalkoxycarbonylheterocyclyl, aminocarbonylheterocyclyl, alkylaminocarbonylheterocyclyl, bisalkylaminocarbonylheterocyclyl, cycloalkylaminocarbonylheterocyclyl, arylalkylaminocarbonylheterocyclyl, alkenylaminocarbonylheterocyclyl, hydroxycarbonylheterocyclylalkyl, alkoxycarbonylheterocyclylalkyl, hydroxycarbonylcycloalkylalkyl, alkoxycarbonylcycloalkylalkyl, heterocyclyl, heterocyclyloxy, heterocyclylamino, heterocyclylcarbonyl, heterocyclylcarbonylalkyl, hydroxycarbonylheterocyclylcarbonyl, alkoxycarbonylheterocyclylcarbonyl, alkenyloxycarbonylheterocyclylcarbonyl, alkenylalkoxycarbonylheterocyclylcarbonyl, arylalkoxycarbonylheterocyclylcarbonyl, cycloalkoxycarbonylheterocyclylcarbonyl, cycloalkylalkoxycarbonylheterocyclylcarbonyl, aminocarbonylheterocyclylcarbonyl, alkylaminocarbonylheterocyclylcarbonyl, bisalkylaminocarbonylheterocyclylcarbonyl, cycloalkylaminocarbonylheterocyclylcarbonyl, arylalkylaminocarbonylheterocyclylcarbonyl, alkenylaminocarbonylheterocyclylcarbonyl,
R⁹ and R¹⁰ are each independently hydrogen, halogen, alkyl, alkoxy, haloalkyl, haloalkoxy, aryl, heteroaryl, arylalkyl, or together with the atom to which they are bonded form a carbonyl group,
A⁶, A⁷ are the same or different and one each independently N-R¹¹, oxygen, sulphur or the CR¹⁵R¹⁶ moiety, but there is never more than one oxygen atom present in the heterocycle, and where each R¹¹, R¹⁵, R¹⁶ in the N-R¹¹ and CR¹⁵R¹⁶ moieties has identical or different meanings according to the definition below,
R¹¹ is hydrogen, alkyl, alkenyl, alkynyl, alkoxyalkyl, alkylcarbonyl, arylcarbonyl, heteroarylcarbonyl, cycloalkylcarbonyl, alkoxycarbonyl, alkenyloxycarbonyl, alkoxycarbonylalkyl, alkylaminocarbonylalkyl, arylaminocarbonylalkyl, aryloxyalkyl, arylalkyl, heteroarylalkyl, heterocyclyl, heterocyclylalkyl,
R¹² and R¹³ are each independently hydrogen, halogen, alkyl, alkoxy, haloalkyl, haloalkoxy, aryl, heteroaryl, arylalkyl, heterocyclyl, heteroarylalkyl, heterocyclylalkyl, or together with the atom to which they are bonded form a carbonyl group, and
R¹⁵ and R¹⁶ are each independently hydrogen, alkyl, alkenyl, alkynyl, alkoxy, alkoxyalkyl, alkylcarbonyl, arylcarbonyl, heteroarylcarbonyl, hydroxycarbonyl, alkoxycarbonyl, heterocyclyl, haloalkyl,
excluding the compound 1-[(2E)-1-hydroxy-1,3-diphenylprop-2-en-1-yl]cyclopentanecarbonitrile.

2. Substituted vinyl- and alkynylcyanocycloalkanols and vinyl- and alkynylcyanoheterocyclylalkanols according to Claim 1, wherein
[X-Y] represents the moieties 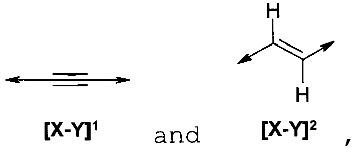
Q represents the carbocyclic and heterocyclic moieties 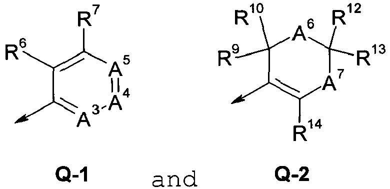 where the R⁶, R⁷, R⁹, R¹⁰, R¹², R¹³, R¹⁴, A³, A⁴, A⁵, A⁶ and A⁷ moieties are each as defined below and where the arrow represents a bond to the respective [X-Y] moiety,
R¹ is (C₁-C₈)-alkyl, aryl, heteroaryl, heterocyclyl, (C₃-C₁₀)-cycloalkyl, (C₂-C₈)-alkenyl, (C₂-C₈)-alkynyl, (C₁-C₈)-alkoxy-(C₁-C₈)-alkyl, hydroxy-(C₁-C₈)-alkyl, (C₁-C₈)-haloalkyl, (C₂-C₈)-haloalkenyl, (C₁-C₈)-haloalkoxy-(C₁-C₈)-alkyl, (C₁-C₈)-alkylthio-(C₁-C₈)-alkyl, aryl-(C₁-C₈)-alkyl, heterocyclyl-(C₁-C₈)-alkyl, (C₃-C₈)-halocycloalkyl, (C₄-C₈)-cycloalkenyl, (C₁-C₈) alkoxy-(C₁-C₈)-alkoxy-(C₁-C₈)-alkyl, (C₃-C₈)-cycloalkyl-(C₁-C₈)-alkyl, (C₄-C₈)-cycloalkenyl-(C₁-C₈)-alkyl, (C₂-C₈)-haloalkynyl, (C₃-C₈)-alkylsulphinyl-(C₃-C₈)-alkyl, (C₁-C₈)-alkylsulphonyl-(C₁-C₈)-alkyl, (C₃-C₈)-halocycloalkyl-(C₁-C₈)-alkyl, (C₃-C₈)-cycloalkylsulphinyl-(C₁-C₈)-alkyl, (C₃-C₈)-cycloalkylsulphonyl-(C₁-C₈)-alkyl, arylsulphinyl-(C₁-C₈)-alkyl, arylsulphonyl-(C₁-C₈)-alkyl, arylthio-(C₁-C₈)-alkyl, (C₃-C₈)-cycloalkylthio-(C₁-C₈)-alkyl, (C₁-C₈)-alkoxy-(C₁-C₈)-haloalkyl, (C₁-C₈)-haloalkoxy-(C₁-C₈)-haloalkyl,
R² is hydrogen, (C₁-C₈)-alkyl, (C₁-C₈)-alkoxy-(C₁-C₈)-alkyl, (C₁-C₈)-alkoxy-(C₁-C₈)-alkoxy-(C₁-C₈)-alkyl, (C₂-C₈)-alkenyl, (C₂-C₈)-alkynyl, (C₁-C₈)-alkylcarbonyl, arylcarbonyl, heteroarylcarbonyl, (C₃-C₈)-cycloalkylcarbonyl, (C₂-C₈)-alkenylcarbonyl, heterocyclylcarbonyl, (C₁-C₈)-alkoxycarbonyl, (C₂-C₈)-alkenyloxycarbonyl, aryloxy-(C₁-C₈)-alkyl, aryl-(C₁-C₈)-alkoxycarbonyl, (C₃-C₈)-cycloalkoxycarbonyl, (C₃-C₈)-cycloalkyl-(C₁-C₈)-alkoxycarbonyl, aryl-(C₁-C₈)-alkoxyalkyl, aryl-(C₁-C₈)-alkyl, (C₁-C₈)-alkoxy-(C₁-C₈)-alkoxy-(C₁-C₈)-alkyl, (C₁-C₈)-alkylthio-(C₁-C₈)-alkyl, tris[(C₁-C₈)-alkyl]silyl, (C₁-C₈)-alkyl{bis[(C₁-C₈)-alkyl]}silyl, (C₁-C₈)-alkyl(bisaryl)silyl, aryl{bis[(C₁-C₈)-alkyl]}silyl, cycloalkyl{bis[(C₁-C₈)-alkyl]}silyl, halo{bis[(C₁-C₈)-alkyl]}silyl, tris[(C₁-C₈)-alkyl]silyl-(C₁-C₈)-alkoxy-(C₁-C₈)-alkyl, tris[(C₁-C₈)-alkyl]silyl-(C₁-C₈)-alkyl, (C₂-C₈)-alkynyloxycarbonyl, (C₃-C₈)-cycloalkyl, (C₃-C₈)-cycloalkyl-(C₁-C₈)-alkyl, aminocarbonyl, (C₁-C₈)-alkylaminocarbonyl, bis[(C₁-C₈)-alkyl]aminocarbonyl, (C₃-C₈)-cycloalkylaminocarbonyl, (C₁-C₈)-alkylsulphonyl, (C₁-C₈)-haloalkylsulphonyl, arylsulphonyl, heteroarylsulphonyl, (C₃-C₈)-cycloalkylsulphonyl,
A¹, A², V, W are each independently a CR³R⁴ group, an N-R⁵ group, oxygen or sulphur, where not more than 2 oxygen or 2 sulphur atoms are present in each ring formed by the A¹, A², V, W groups and the carbon atom to which they are bonded, and where the oxygen and sulphur atoms are not adjacent to one another,
m is 0, 1, 2,
n is 0, 1, 2, and
R³ and R⁴ are each independently hydrogen, (C₁-C₈)-alkyl, halogen, (C₃-C₈)-cycloalkyl, (C₁-C₈)-alkoxy, aryl, heterocyclyl, heteroaryl, aryl-(C₁-C₈)-alkyl, (C₁-C₈)-alkylthio, (C₁-C₈)-haloalkyl, (C₁-C₈)-haloalkyloxy, (C₁-C₈)-haloalkylthio, (C₁-C₈)-alkoxy-(C₁-C₈)-alkyl, (C₁-C₈)-alkylthio-(C₁-C₈)-alkyl, heteroaryl-(C₁-C₈)-alkyl, heterocyclyl-(C₁-C₈)-alkyl, (C₃-C₈)-cycloalkyl-(C₁-C₈)-alkyl, (C₄-C₈)-cycloalkenyl, (C₂-C₈)-alkinyl, (C₂-C₈)-alkenyl, (C₂-C₈)-haloalkenyl, (C₂-C₈)-haloalkinyl, (C₁-C₈)-alkylsulphinyl, (C₁-C₈)-alkylsulphonyl, (C₃-C₈)-cycloalkylsulphinyl, (C₃-C₈)-cycloalkylsulphonyl, arylsulphinyl, arylsulphonyl, (C₁-C₈)-alkoxy-(C₁-C₈)-haloalkyl, (C₁-C₈)-haloalkoxy-(C₁-C₈)-haloalkyl, or
R³ and R⁴ together with the atom to which they are bonded form a fully saturated 3- to 6-membered ring optionally interrupted by heteroatoms and optionally having further substitution,
R⁵ is hydrogen, (C₁-C₈)-alkyl, (C₂-C₈)-alkenyl, (C₂-C₈)-alkynyl, (C₁-C₈)-alkoxy-(C₁-C₈)-alkyl, (C₁-C₈)-alkylcarbonyl, arylcarbonyl, heteroarylcarbonyl, (C₃-C₈)-cycloalkylcarbonyl, (C₁-C₈)-alkoxycarbonyl, (C₂-C₈)-alkenyloxycarbonyl, (C₁-C₈)-alkoxycarbonyl-(C₁-C₈)-alkyl, (C₁-C₈)-alkylaminocarbonyl-(C₁-C₈)-alkyl, arylaminocarbonyl-(C₁-C₈)-alkyl, aryloxy-(C₁-C₈)-alkyl, aryl-(C₁-C₈)-alkyl, heteroaryl-(C₁-C₈)-alkyl, heterocyclyl, heterocyclyl-(C₁-C₈)-alkyl, (C₁-C₈)-haloalkyl,
R⁶ and R⁷ are each independently hydrogen, nitro, amino, cyano, thiocyanato, isothiocyanato, halogen, (C₁-C₈)-alkyl, (C₃-C₈)-cycloalkyl, (C₂-C₈)-alkenyl, (C₂-C₈)-alkynyl, aryl, aryl-(C₁-C₈)-alkyl, aryl-(C₁-C₈)-alkoxy, heteroaryl, (C₁-C₈)-alkoxy-(C₁-C₈)-alkyl, hydroxy-(C₁-C₈)-alkyl, (C₁-C₈)-haloalkyl, (C₃-C₈)-halocycloalkyl, (C₁-C₈)-alkoxy, (C₁-C₈)-haloalkoxy, aryloxy, heteroaryloxy, (C₃-C₈)-cycloalkyloxy, hydroxyl, (C₃-C₈)-cycloalkyl-(C₁-C₈)-alkoxy, (C₁-C₈)-alkoxycarbonyl, hydroxycarbonyl, aminocarbonyl, (C₁-C₈)-alkylaminocarbonyl, (C₃-C₈)-cycloalkylaminocarbonyl, cyano-(C₁-C₈)-alkylaminocarbonyl, (C₂-C₈)-alkenylaminocarbonyl, (C₂-C₈)-alkynylaminocarbonyl, (C₁-C₈)-alkylamino, (C₁-C₈)-alkylthio, (C₁-C₈)-haloalkylthio, hydrothio, bis[(C₁-C₈)-alkyl]amino, (C₃-C₈)-cycloalkylamino, (C₁-C₈)-alkylcarbonylamino, (C₃-C₈)-cycloalkylcarbonylamino, formylamino, (C₁-C₈)-haloalkylcarbonylamino, (C₁-C₈)-alkoxycarbonylamino, (C₁-C₈)-alkylaminocarbonylamino, (C₁-C₈)-alkyl-[(C₁-C₈)-alkyl]aminocarbonylamino, (C₁-C₈)-alkylsulphonylamino, (C₃-C₈)-cycloalkylsulphonylamino, arylsulphonylamino, hetarylsulphonylamino, sulphonyl-(C₁-C₈)-haloalkylamino, aminosulphonyl, amino-(C₁-C₈)-alkylsulphonyl, amino-(C₁-C₈)-haloalkylsulphonyl, (C₁-C₈)-alkylaminosulphonyl, bis[(C₁-C₈)-alkyl]aminosulphonyl, (C₃-C₈)-cycloalkylaminosulphonyl, (C₁-C₈)-haloalkylaminosulphonyl, arylaminosulphonyl, aryl-(C₁-C₈)-alkylaminosulphonyl, (C₁-C₈)-alkylsulphonyl, (C₃-C₈)-cycloalkylsulphonyl, arylsulphonyl, (C₁-C₈)-alkylsulphinyl, (C₃-C₈) cycloalkylsulphinyl, arylsulphinyl, N,S-bis [(C₁-C₈)-alkyl]sulphonimidoyl, S-(C₁-C₈)-alkylsulphonimidoyl, (C₁-C₈)-alkylsulphonylaminocarbonyl, (C₃-C₈)-cycloalkylsulphonylaminocarbonyl, (C₃-C₈)-cycloalkylaminosulphonyl, aryl-(C₁-C₈)-alkylcarbonylamino, (C₃-C₈)-cycloalkyl-(C₁-C₈)-alkylcarbonylamino, heteroarylcarbonylamino, (C₁-C₈)-alkoxy-(C₁-C₈)-alkylcarbonylamino, hydroxy-(C₁-C₈)-alkylcarbonylamino, tris-[(C₁-C₈)-alkyl]silyl,
A³, A⁴, A⁵ are the same or different and are each independently N (nitrogen) or the C-R⁸ moiety, but there are never more than two adjacent nitrogen atoms, and where each R⁸ in the C-R⁸ moiety has identical or different meanings according to the definition below, and
A³ and A⁴, when each is a C-R⁸ group, together with the atoms to which they are bonded form a fully saturated, partly saturated or fully unsaturated 5- to 6-membered ring optionally interrupted by heteroatoms and optionally having further substitution,
A⁴ and A⁵, when each is a C-R⁸ group, together with the atoms to which they are bonded form a fully saturated, partly saturated or fully unsaturated 5- to 6-membered ring optionally interrupted by heteroatoms and optionally having further substitution,
R⁸ and R¹⁴ are each independently hydrogen, nitro, amino, hydroxyl, hydrothio, thiocyanato, isothiocyanato, halogen, (C₁-C₈)-alkyl, (C₃-C₈)-cycloalkyl, (C₂-C₈)-alkenyl, (C₂-C₈)-alkynyl, aryl, aryl-(C₁-C₈)-alkyl, aryl-(C₁-C₈)-alkoxy, heteroaryl, (C₁-C₈)-haloalkyl, (C₃-C₈)-halocycloalkyl, (C₁-C₈)-alkoxy, (C₁-C₈)-haloalkoxy, aryloxy, heteroaryloxy, (C₃-C₈)-cycloalkyloxy, (C₃-C₈)-cycloalkyl-(C₁-C₈)-alkoxy, hydroxy-(C₁-C₈)-alkyl, (C₁-C₈)-alkoxy-(C₁-C₈)-alkyl, aryloxy- (C₁-C₈)-alkyl, heteroaryloxy- (C₁-C₈)-alkyl, (C₁-C₈)-alkylamino, (C₁-C₈)-alkylthio, (C₁-C₈)-haloalkylthio, bis [(C₁-C₈)-alkyl]amino, (C₃-C₈)-cycloalkylamino, (C₁-C₈) alkylcarbonylamino, (C₃-C₈)-cycloalkylcarbonylamino, formylamino, (C₁-C₈)-haloalkylcarbonylamino, (C₁-C₈) alkoxycarbonylamino, (C₁-C₈)-alkylaminocarbonylamino, (C₁-C₈)-alkyl[(C₁-C₈)-alkyl] aminocarbonylamino, (C₁-C₈)-alkylsulphonylamino, (C₃-C₈)-cycloalkylsulphonylamino, arylsulphonylamino, hetarylsulphonylamino, sulphonyl-(C₁-C₈)-haloalkylamino, amino-(C₁-C₈)-alkylsulphonyl, amino-(C₁-C₈)-haloalkylsulphonyl, (C₁-C₈)-alkylaminosulphonyl, bis[(C₁-C₈)-alkyl] aminosulphonyl, (C₃-C₈)-cycloalkylaminosulphonyl, (C₁-C₈)-haloalkylaminosulphonyl, arylaminosulphonyl, aryl-(C₁-C₈)-alkylaminosulphonyl, (C₁-C₈)-alkylsulphonyl, (C₃-C₈)-cycloalkylsulphonyl, arylsulphonyl, (C₁-C₈)-alkylsulphinyl, (C₃-C₈) cycloalkylsulphinyl, arylsulphinyl, N,S-bis [(C₁-C₈)-alkyl]sulphonimidoyl, S-(C₁-C₈)-alkylsulphonimidoyl, (C₁-C₈)-alkylsulphonylaminocarbonyl, (C₃-C₈)-cycloalkylsulphonylaminocarbonyl, (C₃-C₈)-cycloalkylaminosulphonyl, aryl-(C₁-C₈)-alkylcarbonylamino, (C₃-C₈)-cycloalkyl-(C₁-C₈)-alkylcarbonylamino, heteroarylcarbonylamino, (C₁-C₈)-alkoxy-(C₁-C₈)-alkylcarbonylamino, hydroxy-(C₁-C₈)-alkylcarbonylamino, cyano, cyano- (C₁-C₈)-alkyl, hydroxycarbonyl, (C₁-C₈)-alkoxycarbonyl, (C₃-C₈)-cycloalkoxycarbonyl, (C₃-C₈)-cycloalkyl-(C₁-C₈)-alkoxycarbonyl, aryloxycarbonyl, aryl-(C₁-C₈)-alkoxycarbonyl, aminocarbonyl, (C₁-C₈)-alkylaminocarbonyl, bis[(C₁-C₈)-alkyl]aminocarbonyl, (C₁-C₈)-alkyl[(C₁-C₈)-alkoxy]aminocarbonyl, (C₃-C₈)-cycloalkylaminocarbonyl, aryl-(C₁-C₈)-alkylaminocarbonyl, heteroaryl-(C₁-C₈)-alkylaminocarbonyl, cyano-(C₁-C₈)-alkylaminocarbonyl, (C₁-C₈)-haloalkylaminocarbonyl, (C₂-C₈) alkynylaminocarbonyl, (C₁-C₈)-alkoxycarbonylaminocarbonyl, aryl-(C₁-C₈)-alkoxycarbonylaminocarbonyl, hydroxycarbonyl-(C₁-C₈)-alkyl, (C₁-C₈)-alkoxycarbonyl-(C₁-C₈)-alkyl, (C₃-C₈)-cycloalkoxycarbonyl-(C₁-C₈)-alkyl, (C₃-C₈)-cycloalkyl-(C₁-C₈)-alkoxycarbonyl-(C₁-C₈)-alkyl, (C₁-C₈)-alkylaminocarbonyl-(C₁-C₈)-alkyl, aminocarbonyl-(C₁-C₈)-alkyl, bis [(C₁-C₈)-alkyl] aminocarbonyl-(C₁-C₈)-alkyl, (C₃-C₈)-cycloalkylaminocarbonyl-(C₁-C₈)-alkyl, aryl-(C₁-C₈)-alkylaminocarbonyl-(C₁-C₈)-alkyl, heteroaryl-(C₁-C₈)-alkylaminocarbonyl-(C₁-C₈)-alkyl, cyano-(C₁-C₈)-alkylaminocarbonyl-(C₁-C₈)-alkyl, (C₁-C₈)-haloalkylaminocarbonyl-(C₁-C₈)-alkyl, (C₂-C₈)-alkynylaminocarbonyl-(C₁-C₈)-alkyl, (C₃-C₈)-cycloalkyl-(C₁-C₈)-alkylaminocarbonyl-(C₁-C₈)-alkyl, (C₁-C₈)-alkoxycarbonylaminocarbonyl-(C₁-C₈)-alkyl, aryl-(C₁-C₈)-alkoxycarbonylaminocarbonyl-(C₁-C₈)-alkyl, (C₁-C₈)-alkoxycarbonyl-(C₁-C₈)-alkylaminocarbonyl, hydroxycarbonyl-(C₁-C₈)-alkylaminocarbonyl, aminocarbonyl-(C₁-C₈)-alkylaminocarbonyl, (C₁-C₈)-alkylaminocarbonyl-(C₁-C₈)-alkylaminocarbonyl, (C₃-C₈)-cycloalkylaminocarbonyl-(C₁-C₈)-alkylaminocarbonyl, (C₃-C₈)-cycloalkyl-(C₁-C₈)-alkylaminocarbonyl, (C₃-C₈)-cycloalkyl-(C₁-C₈)-alkylaminocarbonyl-(C₁-C₈)-alkyl, (C₂-C₈)-alkenyloxycarbonyl, (C₂-C₈)-alkenyloxycarbonyl-(C₁-C₈)-alkyl, (C₂-C₈)-alkenylaminocarbonyl, (C₂-C₈)-alkenylaminocarbonyl-(C₁-C₈)-alkyl, (C₁-C₈)-alkylcarbonyl, (C₃-C₈)-cycloalkylcarbonyl, formyl, hydroxyiminomethyl, aminoiminomethyl, (C₁-C₈)-alkoxyiminomethyl, (C₁-C₈)-alkylaminoiminomethyl, bis[(C₁-C₈)-alkyl]aminoiminomethyl, (C₃-C₈)-cycloalkoxyiminomethyl, (C₃-C₈)-cycloalkyl-(C₁-C₈)-alkoximinomethyl, aryloximinomethyl, aryl-(C₁-C₈)-alkoxyiminomethyl, aryl- (C₁-C₈)-alkylaminoiminomethyl, (C₂-C₈)-alkenyloxyiminomethyl, arylaminoiminomethyl, arylsulphonylaminoiminomethyl, heteroaryl-(C₁-C₈)-alkyl, heterocyclyl-(C₁-C₈)-alkyl, hydroxycarbonylheterocyclyl, (C₁-C₈)-alkoxycarbonylheterocyclyl, (C₂-C₈)-alkenyloxycarbonylheterocyclyl, (C₂-C₈) alkenyl-(C₁-C₈)-alkoxycarbonylheterocyclyl, aryl-(C₁-C₈)-alkoxycarbonylheterocyclyl, (C₃-C₈)-cycloalkoxycarbonylheterocyclyl, (C₃-C₈)-cycloalkyl-(C₁-C₈)-alkoxycarbonylheterocyclyl, aminocarbonylheterocyclyl, (C₁-C₈)-alkylaminocarbonylheterocyclyl, bis [(C₁-C₈)-alkyl]aminocarbonylheterocyclyl, (C₃-C₈)-cycloalkylaminocarbonylheterocyclyl, aryl-(C₁-C₈)-alkylaminocarbonylheterocyclyl, (C₂-C₈)-alkenylaminocarbonylheterocyclyl, hydroxycarbonylheterocyclyl-(C₁-C₈)-alkyl, (C₁-C₈)-alkoxycarbonylheterocyclyl-(C₁-C₈)-alkyl, hydroxycarbonyl-(C₃-C₈)-cycloalkyl-(C₁-C₈)-alkyl, (C₁-C₈)-alkoxycarbonyl-(C₃-C₈)-cycloalkyl-(C₁-C₈)-alkyl, heterocyclyl, heterocyclyloxy, heterocyclylamino, heterocyclylcarbonyl, heterocyclylcarbonyl-(C₁-C₈)-alkyl, hydroxycarbonylheterocyclylcarbonyl, (C₁-C₈)-alkoxycarbonylheterocyclylcarbonyl, (C₂-C₈)-alkenyloxycarbonylheterocyclylcarbonyl, (C₂-C₈)-alkenyl-(C₁-C₈)-alkoxycarbonylheterocyclylcarbonyl, aryl-(C₁-C₈)-alkoxycarbonylheterocyclylcarbonyl, (C₃-C₈)-cycloalkoxycarbonylheterocyclylcarbonyl, (C₃-C₈)-cycloalkyl-(C₁-C₈)-alkoxycarbonylheterocyclylcarbonyl, aminocarbonylheterocyclylcarbonyl, (C₁-C₈)-alkylaminocarbonylheterocyclylcarbonyl, bis[(C₁-C₈)-alkyl]aminocarbonylheterocyclylcarbonyl, (C₃-C₈)-cycloalkylaminocarbonylheterocyclylcarbonyl, aryl-(C₁-C₈)-alkylaminocarbonylheterocyclylcarbonyl, (C₂-C₈)-alkenylaminocarbonylheterocyclylcarbonyl,
R⁹ and R¹⁰ are each independently hydrogen, halogen, (C₁-C₈)-alkyl, (C₁-C₈)-alkoxy, (C₁-C₈)-haloalkyl, (C₁-C₈)-haloalkoxy, aryl, heteroaryl, aryl-(C₁-C₈)-alkyl, or together with the atom to which they are bonded form a carbonyl group,
A⁶, A⁷ are the same or different and one each independently N-R¹¹, oxygen, sulphur or the CR¹⁵R¹⁶ moiety, but there is never more than one oxygen atom present in the heterocycle, and where each R¹¹, R¹⁵, R¹⁶ in the N-R¹¹ and CR¹⁵R¹⁶ moieties has identical or different meanings according to the definition below,
R¹¹ is hydrogen, (C₁-C₈)-alkyl, (C₂-C₈)-alkenyl, (C₂-C₈)-alkynyl, (C₁-C₈)-alkoxy-(C₁-C₈)-alkyl, (C₁-C₈)-alkylcarbonyl, arylcarbonyl, heteroarylcarbonyl, (C₃-C₈)-cycloalkylcarbonyl, (C₁-C₈)-alkoxycarbonyl, (C₂-C₈)-alkenyloxycarbonyl, (C₁-C₈)-alkoxycarbonyl-(C₁-C₈)-alkyl, (C₁-C₈)-alkylaminocarbonyl-(C₁-C₈)-alkyl, arylaminocarbonyl-(C₁-C₈)-alkyl, aryloxy-(C₁-C₈)-alkyl, aryl-(C₁-C₈)-alkyl, heteroaryl-(C₁-C₈)-alkyl, heterocyclyl, heterocyclyl-(C₁-C₈)-alkyl,
R¹² and R¹³ are each independently hydrogen, halogen, (C₁-C₈)-alkyl, (C₁-C₈)-alkoxy, (C₁-C₈)-haloalkyl, (C₁-C₈)-haloalkoxy, aryl, heteroaryl, aryl-(C₁-C₈)-alkyl, heterocyclyl, heteroaryl-(C₁-C₈)-alkyl, heterocyclyl-(C₁-C₈)-alkyl, or together with the atom to which they are bonded form a carbonyl group, and
R¹⁵ and R¹⁶ are each independently hydrogen, (C₁-C₈)-alkyl, (C₂-C₈)-alkenyl, (C₂-C₈)-alkynyl, (C₁-C₈)-alkoxy, (C₁-C₈)-alkoxy-(C₁-C₈)-alkyl, (C₁-C₈)-alkylcarbonyl, arylcarbonyl, heteroarylcarbonyl, hydroxycarbonyl, (C₁-C₈) alkoxycarbonyl, heterocyclyl, (C₁-C₈)-haloalkyl,
excluding the compound 1-[(2E)-1-hydroxy-1,3-diphenylprop-2-en-1-yl]cyclopentanecarbonitrile.

3. Substituted vinyl- and alkynylcyanocycloalkanols and vinyl- and alkynylcyanoheterocyclylalkanols according to Claim 1, wherein
[X-Y] represents the moieties 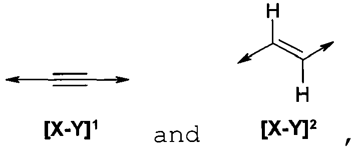
Q represents the carbocyclic and heterocyclic moieties 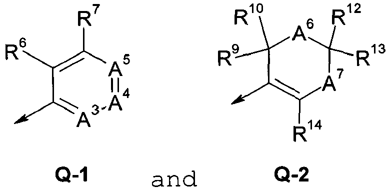 where the R⁶, R⁷, R⁹, R¹⁰, R¹², R¹³, R¹⁴, A³, A⁴, A⁵, A⁶ and A⁷ moieties are each as defined below and where the arrow represents a bond to the respective [X-Y] moiety,
R¹ is (C₁-C₇)-alkyl, aryl, heteroaryl, heterocyclyl, (C₃-C₁₀)-cycloalkyl, (C₂-C₇)-alkenyl, (C₂-C₇)-alkynyl, (C₁-C₇)-alkoxy-(C₁-C₇)-alkyl, hydroxy-(C₁-C₇)-alkyl, (C₁-C₇)-haloalkyl, (C₂-C₇)-haloalkenyl, (C₁-C₇)-haloalkoxy-(C₁-C₇)-alkyl, (C₁-C₇)-alkylthio-(C₁-C₇)-alkyl, aryl-(C₁-C₇)-alkyl, heterocyclyl-(C₁-C₇)-alkyl, (C₃-C₇)-halocycloalkyl, (C₄-C₇)-cycloalkenyl, (C₁-C₇) alkoxy-(C₁-C₇)-alkoxy-(C₁-C₇)-alkyl, (C₁-C₇)-alkoxy-(C₁-C₇)-haloalkyl, (C₁-C₇)-haloalkoxy-(C₁-C₇)-haloalkyl, (C₃-C₈)-cycloalkyl-(C₁-C₇)-alkyl,
R² is hydrogen, (C₁-C₇)-alkyl, (C₁-C₇)-alkoxy-(C₁-C₇)-alkyl, (C₁-C₇)-alkoxy-(C₁-C₇)-alkoxy-(C₁-C₇)-alkyl, (C₂-C₇)-alkenyl, (C₂-C₇)-alkynyl, (C₁-C₇)-alkylcarbonyl, arylcarbonyl, heteroarylcarbonyl, (C₃-C₇)-cycloalkylcarbonyl, (C₂-C₇)-alkenylcarbonyl, heterocyclylcarbonyl, (C₁-C₇)-alkoxycarbonyl, (C₂-C₇)-alkenyloxycarbonyl, aryloxy-(C₁-C₇)-alkyl, aryl-(C₁-C₇)-alkoxycarbonyl, (C₃-C₇)-cycloalkoxycarbonyl, (C₃-C₇)-cycloalkyl-(C₁-C₇)-alkoxycarbonyl, aryl-(C₁-C₇)-alkoxy-(C₁-C₇)-alkyl, aryl-(C₁-C₇)-alkyl, (C₁-C₇)-alkoxy-(C₁-C₇)-alkoxy-(C₁-C₇)-alkyl, (C₁-C₇)-alkylthio-(C₁-C₇)-alkyl, tris[(C₁-C₇)-alkyl]silyl, (C₁-C₇)-alkylbis[(C₁-C₇)-alkyl]silyl, (C₁-C₇)-alkylbis(aryl)silyl, arylbis[(C₁-C₇)-alkyl]silyl, (C₃-C₇)-cycloalkylbis[(C₁-C₇)-alkyl]silyl, halobis[(C₁-C₇)-alkyl]silyl, tris[(C₁-C₇)-alkyl]silyl-(C₁-C₇)-alkoxy-(C₁-C₇)-alkyl, tris[(C₁-C₇)-alkyl]silyl-(C₁-C₇)-alkyl,
A¹, A², V, W are each independently a CR³R⁴ group, an N-R⁵ group, oxygen or sulphur, where not more than 2 oxygen or 2 sulphur atoms are present in each ring formed by the A¹, A², V, W groups and the carbon atom to which they are bonded, and where the oxygen and sulphur atoms are not adjacent to one another,
m is 0, 1, 2,
n is 0, 1, 2,
R³ and R⁴ are each independently hydrogen, (C₁-C₇)-alkyl, halogen, (C₃-C₇)-cycloalkyl, (C₁-C₇)-alkoxy, aryl, heterocyclyl, heteroaryl, aryl-(C₁-C₇)-alkyl, (C₁-C₇)-alkylthio, (C₁-C₇) haloalkyl, (C₁-C₇)-haloalkyloxy, (C₁-C₇)-haloalkylthio, (C₁-C₇)-alkoxy-(C₁-C₇)-alkyl, (C₁-C₇)-alkylthio-(C₁-C₇)-alkyl, heteroaryl-(C₁-C₇)-alkyl, heterocyclyl-(C₁-C₇)-alkyl, (C₃-C₇)-cycloalkyl-(C₁-C₇)-alkyl, (C₄-C₇)-cycloalkenyl, (C₂-C₇)-alkinyl, (C₂-C₇)-alkenyl, (C₂-C₇)-haloalkenyl, (C₂-C₇)-haloalkinyl, (C₁-C₇)-alkylsulphinyl, (C₁-C₇)-alkylsulphonyl, (C₃-C₇)-cycloalkylsulphinyl, (C₃-C₇) cycloalkylsulphonyl, arylsulphinyl, arylsulphonyl, (C₁-C₇)-alkoxy-(C₁-C₇)-haloalkyl, (C₁-C₇)-haloalkoxy-(C₁-C₇)-haloalkyl, or
R³ and R⁴ together with the atom to which they are bonded form a fully saturated 3- to 6-membered ring optionally interrupted by heteroatoms and optionally having further substitution,
R⁵ is hydrogen, (C₁-C₇)-alkyl, (C₂-C₇)-alkenyl, (C₂-C₇)-alkynyl, (C₁-C₇)-alkoxy-(C₁-C₇)-alkyl, (C₁-C₇)-alkylcarbonyl, arylcarbonyl, heteroarylcarbonyl, (C₃-C₇)-cycloalkylcarbonyl, (C₁-C₇)-alkoxycarbonyl, (C₂-C₇)-alkenyloxycarbonyl, (C₁-C₇)-alkoxycarbonyl-(C₁-C₇)-alkyl, (C₁-C₇)-alkylaminocarbonyl-(C₁-C₇)-alkyl, arylaminocarbonyl-(C₁-C₇)-alkyl, aryloxy-(C₁-C₇)-alkyl, aryl-(C₁-C₇)-alkyl, heteroaryl-(C₁-C₇)-alkyl, heterocyclyl, heterocyclyl-(C₁-C₇)-alkyl,
R⁶ and R⁷ are each independently hydrogen, nitro, amino, cyano, thiocyanato, isothiocyanato, halogen, (C₁-C₇)-alkyl, (C₃-C₇)-cycloalkyl, (C₂-C₇)-alkenyl, (C₂-C₇)-alkynyl, aryl, aryl-(C₁-C₇)-alkyl, aryl-(C₁-C₇)-alkoxy, heteroaryl, (C₁-C₇)-alkoxy-(C₁-C₇)-alkyl, hydroxy-(C₁-C₇)-alkyl, (C₁-C₇)-haloalkyl, (C₃-C₇)-halocycloalkyl, (C₁-C₇)-alkoxy, (C₁-C₇)-haloalkoxy, aryloxy, heteroaryloxy, (C₃-C₇)-cycloalkyloxy, hydroxyl, (C₃-C₇)-cycloalkyl-(C₁-C₇)-alkoxy, (C₁-C₇)-alkoxycarbonyl, hydroxycarbonyl, aminocarbonyl, (C₁-C₇)-alkylaminocarbonyl, (C₃-C₇)-cycloalkylaminocarbonyl, cyano-(C₁-C₇)-alkylaminocarbonyl, (C₂-C₇)-alkenylaminocarbonyl, (C₂-C₇)-alkynylaminocarbonyl, (C₁-C₇)-alkylamino, (C₁-C₇)-alkylthio, (C₁-C₇)-haloalkylthio, hydrothio, bis [(C₁-C₇)-alkyl]amino, (C₃-C₇)-cycloalkylamino, (C₁-C₇)-alkylcarbonylamino, (C₃-C₇)-cycloalkylcarbonylamino, formylamino, (C₁-C₇)-haloalkylcarbonylamino, (C₁-C₇)-alkoxycarbonylamino, (C₁-C₇)-alkylaminocarbonylamino, (C₁-C₇)-alkyl-[(C₁-C₇)-alkyl]aminocarbonylamino, (C₁-C₇)-alkylsulphonylamino, (C₃-C₇)-cycloalkylsulphonylamino, arylsulphonylamino, hetarylsulphonylamino, sulphonyl-(C₁-C₇)-haloalkylamino, aminosulphonyl, amino-(C₁-C₇)-alkylsulphonyl, amino-(C₁-C₇)-haloalkylsulphonyl, (C₁-C₇)-alkylaminosulphonyl, bis[(C₁-C₇)-alkyl]aminosulphonyl, (C₃-C₇)-cycloalkylaminosulphonyl, (C₁-C₇)-haloalkylaminosulphonyl, arylaminosulphonyl, aryl-(C₁-C₇)-alkylaminosulphonyl, (C₁-C₇)-alkylsulphonyl, (C₃-C₇)-cycloalkylsulphonyl, arylsulphonyl, (C₁-C₇)-alkylsulphinyl, (C₃-C₇)-cycloalkylsulphinyl, arylsulphinyl, N,S-bis[(C₁-C₇)-alkyl]sulphonimidoyl, S-(C₁-C₇)-alkylsulphonimidoyl, (C₁-C₇)-alkylsulphonylaminocarbonyl, (C₃-C₇)-cycloalkylsulphonylaminocarbonyl, (C₃-C₇) cycloalkylaminosulphonyl, aryl-(C₁-C₇)-alkylcarbonylamino, (C₃-C₇)-cycloalkyl-(C₁-C₇)-alkylcarbonylamino, heteroarylcarbonylamino, (C₁-C₇)-alkoxy-(C₁-C₇)-alkylcarbonylamino, hydroxy-(C₁-C₇)-alkylcarbonylamino, tris-[(C₁-C₇)-alkyl]silyl,
A3, A⁴, A⁵ are the same or different and are each independently N (nitrogen) or the C-R⁸ moiety, but there are never more than two adjacent nitrogen atoms, and where each R⁸ in the C-R⁸ moiety has identical or different meanings according to the definition below, and
A³ and A⁴, when each is a C-R⁸ group, together with the atoms to which they are bonded form a fully saturated, partly saturated or fully unsaturated 5- to 6-membered ring optionally interrupted by heteroatoms and optionally having further substitution,
A⁴ and A⁵, when each is a C-R⁸ group, together with the atoms to which they are bonded form a fully saturated, partly saturated or fully unsaturated 5- to 6-membered ring optionally interrupted by heteroatoms and optionally having further substitution,
R⁸ and R¹⁴ are each independently hydrogen, nitro, amino, hydroxyl, hydrothio, thiocyanato, isothiocyanato, halogen, (C₁-C₇)-alkyl, (C₃-C₇)-cycloalkyl, (C₂-C₇)-alkenyl, (C₂-C₇)-alkynyl, aryl, aryl-(C₁-C₇)-alkyl, aryl-(C₁-C₇)-alkoxy, heteroaryl, (C₁-C₇)-haloalkyl, (C₃-C₇)-halocycloalkyl, (C₁-C₇)-alkoxy, (C₁-C₇)-haloalkoxy, aryloxy, heteroaryloxy, (C₃-C₇)-cycloalkyloxy, (C₃-C₇)-cycloalkyl-(C₁-C₇)-alkoxy, hydroxy-(C₁-C₇)-alkyl, (C₁-C₇)-alkoxy-(C₁-C₇)-alkyl, aryloxy-(C₁-C₇)-alkyl, heteroaryloxy-(C₁-C₇)-alkyl, (C₁-C₇)-alkylamino, (C₁-C₇)-alkylthio, (C₁-C₇)-haloalkylthio, bis[(C₁-C₇)-alkyl]amino, (C₃-C₇)-cycloalkylamino, (C₁-C₇)-alkylcarbonylamino, (C₃-C₇)-cycloalkylcarbonylamino, formylamino, (C₁-C₇)-haloalkylcarbonylamino, (C₁-C₇)-alkoxycarbonylamino, (C₁-C₇)-alkylaminocarbonylamino, (C₁-C₇)-alkyl[(C₁-C₇)-alkyl]aminocarbonylamino, (C₁-C₇)-alkylsulphonylamino, (C₃-C₇)-cycloalkylsulphonylamino, arylsulphonylamino, hetarylsulphonylamino, sulphonyl-(C₁-C₇)-haloalkylamino, amino-(C₁-C₇)-alkylsulphonyl, amino-(C₁-C₇)-haloalkylsulphonyl, (C₁-C₇)-alkylaminosulphonyl, bis[(C₁-C₇)-alkyl] aminosulphonyl, (C₃-C₇)-cycloalkylaminosulphonyl, (C₁-C₇)-haloalkylaminosulphonyl, arylaminosulphonyl, aryl-(C₁-C₇)-alkylaminosulphonyl, (C₁-C₇)-alkylsulphonyl, (C₃-C₇)-cycloalkylsulphonyl, arylsulphonyl, (C₁-C₇)-alkylsulphinyl, (C₃-C₇)-cycloalkylsulphinyl, arylsulphinyl, N,S-bis[(C₁-C₇)-alkyl] sulphonimidoyl, S-(C₁-C₇)-alkylsulphonimidoyl, (C₁-C₇)-alkylsulphonylaminocarbonyl, (C₃-C₇) cycloalkylsulphonylaminocarbonyl, (C₃-C₇)-cycloalkylaminosulphonyl, aryl-(C₁-C₇)-alkylcarbonylamino, (C₃-C₇)-cycloalkyl-(C₁-C₇)-alkylcarbonylamino, heteroarylcarbonylamino, (C₁-C₇)-alkoxy-(C₁-C₇)-alkylcarbonylamino, hydroxy-(C₁-C₇)-alkylcarbonylamino, cyano, cyano-(C₁-C₇)-alkyl, hydroxycarbonyl, (C₁-C₇)-alkoxycarbonyl, (C₃-C₇)-cycloalkoxycarbonyl, (C₃-C₇)-cycloalkyl-(C₁-C₇)-alkoxycarbonyl, aryloxycarbonyl, aryl-(C₁-C₇)-alkoxycarbonyl, aminocarbonyl, (C₁-C₇)-alkylaminocarbonyl, bis[(C₁-C₇)-alkyl] aminocarbonyl, (C₁-C₇)-alkyl[(C₁-C₇)-alkoxy]aminocarbonyl, (C₃-C₇)-cycloalkylaminocarbonyl, aryl-(C₁-C₇)-alkylaminocarbonyl, heteroaryl-(C₁-C₇)-alkylaminocarbonyl, cyano-(C₁-C₇)-alkylaminocarbonyl, (C₁-C₇)-haloalkylaminocarbonyl, (C₂-C₇)-alkynylaminocarbonyl, (C₁-C₇)-alkoxycarbonylaminocarbonyl, aryl-(C₁-C₇)-alkoxycarbonylaminocarbonyl, hydroxycarbonyl-(C₁-C₇)-alkyl, (C₁-C₇)-alkoxycarbonyl-(C₁-C₇)-alkyl, (C₃-C₇)-cycloalkoxycarbonyl-(C₁-C₇)-alkyl, (C₃-C₇)-cycloalkyl-(C₁-C₇)-alkoxycarbonyl-(C₁-C₇)-alkyl, (C₁-C₇)-alkylaminocarbonyl-(C₁-C₇)-alkyl, aminocarbonyl-(C₁-C₇)-alkyl, bis[(C₁-C₇)-alkyl]aminocarbonyl-(C₁-C₇)-alkyl, (C₃-C₇)-cycloalkylaminocarbonyl-(C₁-C₇)-alkyl, aryl-(C₁-C₇)-alkylaminocarbonyl-(C₁-C₇)-alkyl, heteroaryl-(C₁-C₇)-alkylaminocarbonyl-(C₁-C₇)-alkyl, cyano-(C₁-C₇)-alkylaminocarbonyl-(C₁-C₇)-alkyl, (C₁-C₇)-haloalkylaminocarbonyl-(C₁-C₇)-alkyl, (C₂-C₇)-alkynylaminocarbonyl-(C₁-C₇)-alkyl, (C₃-C₇)-cycloalkyl-(C₁-C₇)-alkylaminocarbonyl-(C₁-C₇)-alkyl, (C₁-C₇)-alkoxycarbonylaminocarbonyl-(C₁-C₇)-alkyl, aryl-(C₁-C₇)-alkoxycarbonylaminocarbonyl-(C₁-C₇)-alkyl, (C₁-C₇)-alkoxycarbonyl-(C₁-C₇)-alkylaminocarbonyl, hydroxycarbonyl-(C₁-C₇)-alkylaminocarbonyl, aminocarbonyl-(C₁-C₇)-alkylaminocarbonyl, (C₁-C₇)-alkylaminocarbonyl-(C₁-C₇)-alkylaminocarbonyl, (C₃-C₇)-cycloalkylaminocarbonyl-(C₁-C₇)-alkylaminocarbonyl, (C₃-C₇)-cycloalkyl-(C₁-C₇)-alkylaminocarbonyl, (C₃-C₇)-cycloalkyl-(C₁-C₇)-alkylaminocarbonyl-(C₁-C₇)-alkyl, (C₂-C₇)-alkenyloxycarbonyl, (C₂-C₇)-alkenyloxycarbonyl-(C₁-C₇)-alkyl, (C₂-C₇)-alkenylaminocarbonyl, (C₂-C₇)-alkenylaminocarbonyl-(C₁-C₇)-alkyl, (C₁-C₇)-alkylcarbonyl, (C₃-C₇)-cycloalkylcarbonyl, formyl, hydroxyiminomethyl, aminoiminomethyl, (C₁-C₇)-alkoxyiminomethyl, (C₁-C₇)-alkylaminoiminomethyl, bis[(C₁-C₇)-alkyl]aminoiminomethyl, (C₃-C₇)-cycloalkoxyiminomethyl, (C₃-C₇)-cycloalkyl-(C₁-C₇)-alkoximinomethyl, aryloximinomethyl, aryl-(C₁-C₇)-alkoxyiminomethyl, aryl-(C₁-C₇)-alkylaminoiminomethyl, (C₂-C₇)-alkenyloxyiminomethyl, arylaminoiminomethyl, arylsulphonylaminoiminomethyl, heteroaryl-(C₁-C₇)-alkyl, heterocyclyl-(C₁-C₇)-alkyl, hydroxycarbonylheterocyclyl, (C₁-C₇)-alkoxycarbonylheterocyclyl, (C₂-C₇)-alkenyloxycarbonylheterocyclyl, (C₂-C₇) alkenyl-(C₁-C₇)-alkoxycarbonylheterocyclyl, aryl-(C₁-C₇)-alkoxycarbonylheterocyclyl, (C₃-C₇)-cycloalkoxycarbonylheterocyclyl, (C₃-C₇)-cycloalkyl-(C₁-C₇)-alkoxycarbonylheterocyclyl, aminocarbonylheterocyclyl, (C₁-C₇)-alkylaminocarbonylheterocyclyl, bis[(C₁-C₇)-alkyl]aminocarbonylheterocyclyl, (C₃-C₇)-cycloalkylaminocarbonylheterocyclyl, aryl-(C₁-C₇)-alkylaminocarbonylheterocyclyl, (C₂-C₇)-alkenylaminocarbonylheterocyclyl, hydroxycarbonylheterocyclyl-(C₁-C₇)-alkyl, (C₁-C₇)-alkoxycarbonylheterocyclyl-(C₁-C₇)-alkyl, hydroxycarbonyl-(C₃-C₇)-cycloalkyl- (C₁-C₇)-alkyl, (C₁-C₇)-alkoxycarbonyl-(C₃-C₇)-cycloalkyl-(C₁-C₇)-alkyl, heterocyclyl, heterocyclyloxy, heterocyclylamino, heterocyclylcarbonyl, heterocyclylcarbonyl-(C₁-C₇)-alkyl, hydroxycarbonylheterocyclylcarbonyl, (C₁-C₇)-alkoxycarbonylheterocyclylcarbonyl, (C₂-C₇)-alkenyloxycarbonylheterocyclylcarbonyl, (C₂-C₇)-alkenyl-(C₁-C₇)-alkoxycarbonylheterocyclylcarbonyl, aryl-(C₁-C₇)-alkoxycarbonylheterocyclylcarbonyl, (C₃-C₇)-cycloalkoxycarbonylheterocyclylcarbonyl, (C₃-C₇)-cycloalkyl-(C₁-C₇)-alkoxycarbonylheterocyclylcarbonyl, aminocarbonylheterocyclylcarbonyl, (C₁-C₇)-alkylaminocarbonylheterocyclylcarbonyl, bis[(C₁-C₇)-alkyl]aminocarbonylheterocyclylcarbonyl, (C₃-C₇)-cycloalkylaminocarbonylheterocyclylcarbonyl, aryl-(C₁-C₇)-alkylaminocarbonylheterocyclylcarbonyl, (C₂-C₇)-alkenylaminocarbonylheterocyclylcarbonyl,
R⁹ and R¹⁰ are each independently hydrogen, halogen, (C₁-C₇)-alkyl, (C₁-C₇)-alkoxy, (C₁-C₇)-haloalkyl, (C₁-C₇)-haloalkoxy, aryl, heteroaryl, aryl-(C₁-C₇)-alkyl, or together with the atom to which they are bonded form a carbonyl group,
A⁶, A⁷ are the same or different and one each independently N-R¹¹, oxygen, sulphur or the CR¹⁵R¹⁶ moiety, but there is never more than one oxygen atom present in the heterocycle, and where each R¹¹, R¹⁵, R¹⁶ in the N-R¹¹ and CR¹⁵R¹⁶ moieties has identical or different meanings according to the definition below,
R¹¹ is hydrogen, (C₁-C₇)-alkyl, (C₂-C₇)-alkenyl, (C₂-C₇)-alkynyl, (C₁-C₇)-alkoxy-(C₁-C₇)-alkyl, (C₁-C-₇)-alkylcarbonyl, arylcarbonyl, heteroarylcarbonyl, (C₃-C₇)-cycloalkylcarbonyl, (C₁-C₇)-alkoxycarbonyl, (C₂-C₇)-alkenyloxycarbonyl, (C₁-C₇)-alkoxycarbonyl-(C₁-C₇)-alkyl, (C₁-C₇)-alkylaminocarbonyl-(C₁-C₇)-alkyl, arylaminocarbonyl-(C₁-C₇)-alkyl, aryloxy-(C₁-C₇)-alkyl, aryl-(C₁-C₇)-alkyl, heteroaryl- (C₁-C₇)-alkyl, heterocyclyl, heterocyclyl-(C₁-C₇)-alkyl,
R¹² and R¹³ are each independently hydrogen, halogen, (C₁-C₇)-alkyl, (C₁-C₇)-alkoxy, (C₁-C₇)-haloalkyl, (C₁-C₇)-haloalkoxy, aryl, heteroaryl, aryl-(C₁-C₇)-alkyl, heterocyclyl, heteroaryl-(C₁-C₇)-alkyl, heterocyclyl-(C₁-C₇)-alkyl, or together with the atom to which they are bonded form a carbonyl group, and
R¹⁵ and R¹⁶ are each independently hydrogen, (C₁-C₇)-alkyl, (C₂-C₇)-alkenyl, (C₂-C₇)-alkynyl, (C₁-C₇)-alkoxy, (C₁-C₇)-alkoxy-(C₁-C₇)-alkyl, (C₁-C₇)-alkylcarbonyl, arylcarbonyl, heteroarylcarbonyl, hydroxycarbonyl, (C₁-C₇)-alkoxycarbonyl, heterocyclyl, (C₁-C₇)-haloalkyl,
excluding the compound 1-[(2E)-1-hydroxy-1,3-diphenylprop-2-en-1-yl]cyclopentanecarbonitrile.

4. Use of one or more compounds of the formula (I) or salts thereof according to any of Claims 1 to 3 and of the compound 1-[(2E)-1-hydroxy-1,3-diphenylprop-2-en-1-yl]cyclopentanecarbonitrile for increasing tolerance to abiotic stress in plants.

5. Treatment of plants comprising the application of a nontoxic amount, effective for enhancing the resistance of plants to abiotic stress factors, of one or more of the compounds of the general formula (I) or salts thereof according to any of Claims 1 to 3 and of the compound 1-[(2E)-1-hydroxy-1,3-diphenylprop-2-en-1-yl]cyclopentanecarbonitrile.

6. Treatment according to Claim 5, wherein the abiotic stress conditions correspond to one or more conditions selected from the group of heat, drought, cold and drought stress, osmotic stress, waterlogging, elevated soil salinity, elevated exposure to minerals, ozone conditions, strong light conditions, limited availability of nitrogen nutrients, limited availability of phosphorus nutrients.

7. Use of one or more compounds of the general formula (I) or salts thereof according to any of Claims 1 to 3 and of the compound 1-[(2E)-1-hydroxy-1,3-diphenylprop-2-en-1-yl]cyclopentanecarbonitrile in spray application to plants and plant parts in combinations with one or more active ingredients selected from the group of insecticides, attractants, acaricides, fungicides, nematicides, herbicides, growth regulators, safeners, substances which influence plant maturity and bactericides.

8. Use of one or more of the compounds of the general formula (I) or salts thereof according to any of Claims 1 to 3 and of the compound 1-[(2E)-1-hydroxy-1,3-diphenylprop-2-en-1-yl]cyclopentanecarbonitrile in spray application to plants and plant parts in combinations with fertilizers.

9. Use of one or more of the compounds of the general formula (I) or salts thereof according to any of Claims 1 to 3 and of the compound 1-[(2E)-1-hydroxy-1,3-diphenylprop-2-en-1-yl]cyclopentanecarbonitrile for application to genetically modified cultivars, the seed thereof, or to cultivated areas in which these cultivars grow.

10. Spray solution for treatment of plants, comprising an amount, effective for enhancing the resistance of plants to abiotic stress factors, of one or more compounds of the general formula (I) according to any of Claims 1 to 3 and of the compound 1-[(2E)-1-hydroxy-1,3-diphenylprop-2-en-1-yl]cyclopentanecarbonitrile or salts thereof.

11. Use of spray solutions comprising one or more of the compounds of the general formula (I) according to any of Claims 1 to 3 and of the compound 1-[(2E)-1-hydroxy-1,3-diphenylprop-2-en-1-yl]cyclopentanecarbonitrile or salts thereof for enhancing the resistance of plants to abiotic stress factors.

12. Method for increasing stress tolerance in plants selected from the group of useful plants, ornamental plants, turfgrass types and trees, which comprises the application of a sufficient, nontoxic amount of one or more compounds of the general formula (I) according to any of Claims 1 to 3 and of the compound 1-[(2E)-1-hydroxy-1,3-diphenylprop-2-en-1-yl]cyclopentanecarbonitrile or salts thereof to the area where the corresponding effect is desired, involving application to the plants, the seed thereof or to the area in which the plants grow.

13. Method according to Claim 12, wherein the resistance of the plants thus treated to abiotic stress is increased by at least 3% compared to untreated plants under otherwise identical physiological conditions.

14. Compounds of the general formula (II) or salts thereof 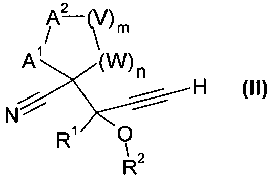 where
R¹ is hydrogen, (C₁-C₈)-alkyl, aryl, heteroaryl, heterocyclyl, (C₃-C₈)-cycloalkyl, (C₂-C₈)-alkenyl, (C₂-C₈)-alkynyl, (C₂-C₈)-alkynyl- (C₁-C₈)-alkyl, (C₁-C₈)-alkoxy-(C₁-C₈)-alkyl, hydroxy-(C₁-C₈)-alkyl, (C₁-C₈)-haloalkyl, (C₂-C₈)-haloalkenyl, (C₁-C₈)-haloalkoxy-(C₁-C₈)-alkyl, (C₁-C₈)-alkylthio-(C₁-C₈)-alkyl, aryl-(C₁-C₈)-alkyl, heterocyclyl-(C₁-C₈)-alkyl, (C₃-C₈)-halocycloalkyl, (C₄-C₈)-cycloalkenyl, (C₁-C₈)-alkoxy-(C₁-C₈)-alkoxy-(C₁-C₈)-alkyl, (C₁-C₈)-alkoxy-(C₁-C₈)-haloalkyl, (C₁-C₈)-haloalkoxy-(C₁-C₈)-haloalkyl, (C₃-C₈)-cycloalkyl-(C₁-C₈)-alkyl,
R² is hydrogen, (C₁-C₈)-alkyl, (C₁-C₈)-alkoxy-(C₁-C₈)-alkyl, (C₁-C₈)-alkoxy-(C₁-C₈)-alkoxy-(C₁-C₈)-alkyl, (C₂-C₈)-alkenyl, (C₂-C₈)-alkynyl, (C₁-C₈)-alkylcarbonyl, arylcarbonyl, heteroarylcarbonyl, (C₃-C₈)-cycloalkylcarbonyl, (C₂-C₈)-alkenylcarbonyl, heterocyclylcarbonyl, (C₁-C₈)-alkoxycarbonyl, (C₂-C₈)-alkenyloxycarbonyl, aryloxy-(C₁-C₈)-alkyl, aryl-(C₁-C₈)-alkoxycarbonyl, (C₃-C₈)-cycloalkoxycarbonyl, (C₃-C₈)-cycloalkyl-(C₁-C₈)-alkoxycarbonyl, aryl-(C₁-C₈)-alkoxy-(C₁-C₈)-alkyl, aryl-(C₁-C₈)-alkyl, (C₁-C₈)-alkoxy-(C₁-C₈)-alkoxy-(C₁-C₈)-alkyl, (C₁-C₈)-alkylthio-(C₁-C₈)-alkyl, tris[(C₁-C₈)-alkyl]silyl, (C₁-C₈)-alkylbis[(C₁-C₈)-alkyl]silyl, (C₁-C₈)-alkylbis(aryl)silyl, arylbis[(C₁-C₈)-alkyl]silyl, (C₃-C₈)-cycloalkylbis[(C₁-C₈)-alkyl] silyl, halobis[(C₁-C₈)-alkyl]silyl, tris[(C₁-C₈)-alkyl]silyl-(C₁-C₈)-alkoxy-(C₁-C₈)-alkyl, tris[(C₁-C₈)-alkyl]silyl-(C₁-C₈)-alkyl,
A¹, A², V, W are each independently a CR³R⁴ group, oxygen or sulphur, where not more than 2 oxygen or 2 sulphur atoms are present in each ring formed by the A¹, A², V, W groups and the carbon atom to which they are bonded, and where the oxygen and sulphur atoms are not adjacent to one another,
m is 0, 1, 2,
n is 0, 1, 2, and
R³ and R⁴ are each independently hydrogen, (C₁-C₈)-alkyl, halogen, (C₃-C₈)-cycloalkyl, (C₁-C₈)-alkoxy, aryl, heterocyclyl, heteroaryl, aryl-(C₁-C₈)-alkyl, (C₁-C₈)-alkylthio, (C₁-C₈)-haloalkyl, (C₁-C₈)-haloalkyloxy, (C₁-C₈)-haloalkylthio, (C₁-C₈)-alkoxy-(C₁-C₈)-alkyl, (C₁-C₈)-alkylthio-(C₁-C₈)-alkyl, heteroaryl-(C₁-C₈)-alkyl, heterocyclyl-(C₁-C₈)-alkyl, (C₁-C₈)-alkoxy-(C₁-C₈)-haloalkyl, (C₁-C₈)-haloalkoxy-(C₁-C₈)-haloalkyl, (C₂-C₈)-alkenyl, (C₂-C₈)-alkynyl or
R³ and R⁴ together with the atom to which they are bonded form a fully saturated 3- to 6-membered ring optionally interrupted by heteroatoms and optionally having further substitution.

15. Compounds of the formula (II) according to Claim 14, where
R¹ is hydrogen, (C₁-C₆)-alkyl, aryl, heteroaryl, heterocyclyl, (C₃-C₆)-cycloalkyl, (C₂-C₆)-alkenyl, (C₂-C₆)-alkynyl, (C₂-C₆)-alkynyl-(C₁-C₆)-alkyl, (C₁-C₆)-alkoxy-(C₁-C₆)-alkyl, hydroxy-(C₁-C₆)-alkyl, (C₁-C₆)-haloalkyl, (C₂-C₆)-haloalkenyl, (C₁-C₆)-haloalkoxy-(C₁-C₆)-alkyl, (C₁-C₆)-alkylthio-(C₁-C₆)-alkyl, aryl-(C₁-C₆)-alkyl, heterocyclyl-(C₁-C₆)-alkyl, (C₃-C₆)-halocycloalkyl, (C₄-C₆)-cycloalkenyl, (C₁-C₆)-alkoxy-(C₁-C₆)-alkoxy-(C₁-C₆)-alkyl, (C₁-C₆)-alkoxy-(C₁-C₆)-haloalkyl, (C₁-C₆)-haloalkoxy-(C₁-C₆)-haloalkyl, (C₃-C₆)-cycloalkyl-(C₁-C₆)-alkyl,
R² is hydrogen, (C₁-C₆)-alkyl, (C₁-C₆)-alkoxy-(C₁-C₆)-alkyl, (C₁-C₆)-alkoxy-(C₁-C₆)-alkoxy-(C₁-C₆)-alkyl, (C₂-C₆)-alkenyl, (C₂-C₆)-alkynyl, (C₁-C₆)-alkylcarbonyl, arylcarbonyl, heteroarylcarbonyl, (C₃-C₆)-cycloalkylcarbonyl, (C₂-C₆)-alkenylcarbonyl, heterocyclylcarbonyl, (C₁-C₆)-alkoxycarbonyl, (C₂-C₆)-alkenyloxycarbonyl, aryloxy-(C₁-C₆)-alkyl, aryl-(C₁-C₆)-alkoxycarbonyl, (C₃-C₆)-cycloalkoxycarbonyl, (C₃-C₆)-cycloalkyl-(C₁-C₆)-alkoxycarbonyl, aryl-(C₁-C₆)-alkoxy-(C₁-C₆)-alkyl, aryl-(C₁-C₆)-alkyl, (C₁-C₆)-alkoxy-(C₁-C₆)-alkoxy-(C₁-C₆)-alkyl, (C₁-C₆)-alkylthio-(C₁-C₆)-alkyl, tris[(C₁-C₆)-alkyl] silyl, (C₁-C₆)-alkylbis [(C₁-C₆)-alkyl]silyl, (C₁-C₆)-alkylbis(aryl)silyl, arylbis[(C₁-C₆)-alkyl]silyl, (C₃-C₆)-cycloalkylbis[(C₁-C₆)-alkyl] silyl, halobis[(C₁-C₆)-alkyl]silyl, tris[(C₁-C₆)-alkyl]silyl-(C₁-C₆)-alkoxy-(C₁-C₆)-alkyl, tris[(C₁-C₆)-alkyl]silyl-(C₁-C₆)-alkyl,
A¹, A², V, W are each independently a CR³R⁴ group, oxygen or
sulphur, where not more than 2 oxygen or 2 sulphur atoms are present in each ring formed by the A¹, A², V, W groups and the carbon atom to which they are bonded, and where the oxygen and sulphur atoms are not adjacent to one another,
m is 0, 1, 2,
n is 0, 1, 2, and
R³ and R⁴ are each independently hydrogen, (C₁-C₆)-alkyl, halogen, (C₃-C₆)-cycloalkyl, (C₁-C₆)-alkoxy, aryl, heterocyclyl, heteroaryl, aryl-(C₁-C₆)-alkyl, (C₁-C₆)-alkylthio, (C₁-C₆)-haloalkyl, (C₁-C₆)-haloalkyloxy, (C₁-C₆)-haloalkylthio, (C₁-C₆)-alkoxy-(C₁-C₆)-alkyl, (C₁-C₆)-alkylthio-(C₁-C₆)-alkyl, heteroaryl-(C₁-C₆)-alkyl, heterocyclyl-(C₁-C₆)-alkyl, (C₁-C₆)-alkoxy-(C₁-C₆)-haloalkyl, (C₁-C₆)-haloalkoxy-(C₁-C₆)-haloalkyl, (C₂-C₆)-alkenyl, (C₂-C₆)-alkynyl or
R³ and R⁴ together with the atom to which they are bonded form a fully saturated 3- to 6-membered ring optionally interrupted by heteroatoms and optionally having further substitution.

16. Compounds of the general formula (III) or salts thereof 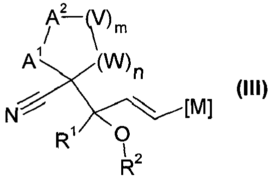 where
R¹ is hydrogen, (C₁-C₈)-alkyl, aryl, heteroaryl, heterocyclyl, (C₃-C₈)-cycloalkyl, (C₂-C₈)-alkenyl, (C₂-C₈)-alkynyl, (C₂-C₈)-alkynyl- (C₁-C₈)-alkyl, (C₁-C₈)-alkoxy-(C₁-C₈)-alkyl, hydroxy-(C₁-C₈)-alkyl, (C₁-C₈)-haloalkyl, (C₂-C₈)-haloalkenyl, (C₁-C₈)-haloalkoxy-(C₁-C₈)-alkyl, (C₁-C₈)-alkylthio-(C₁-C₈)-alkyl, aryl-(C₁-C₈)-alkyl, heterocyclyl-(C₁-C₈)-alkyl, (C₃-C₈)-halocycloalkyl, (C₄-C₈)-cycloalkenyl, (C₁-C₈)-alkoxy-(C₁-C₈)-alkoxy-(C₁-C₈)-alkyl, (C₁-C₈)-alkoxy-(C₁-C₈)-haloalkyl, (C₁-C₈)-haloalkoxy-(C₁-C₈)-haloalkyl,
R² is hydrogen, (C₁-C₈)-alkyl, (C₁-C₈)-alkoxy-(C₁-C₈)-alkyl, (C₁-C₈)-alkoxy-(C₁-C₈)-alkoxy-(C₁-C₈)-alkyl, (C₂-C₈)-alkenyl, (C₂-C₈)-alkynyl, (C₁-C₈)-alkylcarbonyl, arylcarbonyl, heteroarylcarbonyl, (C₃-C₈)-cycloalkylcarbonyl, (C₂-C₈)-alkenylcarbonyl, heterocyclylcarbonyl, (C₁-C₈)-alkoxycarbonyl, (C₂-C₈)-alkenyloxycarbonyl, aryloxy-(C₁-C₈)-alkyl, aryl-(C₁-C₈)-alkoxycarbonyl, (C₃-C₈)-cycloalkoxycarbonyl, (C₃-C₈)-cycloalkyl-(C₁-C₈)-alkoxycarbonyl, aryl-(C₁-C₈)-alkoxy-(C₁-C₈)-alkyl, aryl-(C₁-C₈)-alkyl, (C₁-C₈)-alkoxy-(C₁-C₈)-alkoxy-(C₁-C₈)-alkyl, (C₁-C₈)-alkylthio-(C₁-C₈)-alkyl, tris[(C₁-C₈)-alkyl]silyl, (C₁-C₈)-alkylbis[(C₁-C₈)-alkyl]silyl, (C₁-C₈)-alkylbis(aryl)silyl, arylbis[(C₁-C₈)-alkyl]silyl, (C₃-C₈)-cycloalkylbis[(C₁-C₈)-alkyl]silyl, halobis[(C₁-C₈)-alkyl]silyl, tris[(C₁-C₈)-alkyl]silyl-(C₁-C₈)-alkoxy-(C₁-C₈)-alkyl, tris[(C₁-C₈)-alkyl]silyl-(C₁-C₈)-alkyl,
A¹, A², V, W are each independently a CR³R⁴ group, oxygen or sulphur, where not more than 2 oxygen or 2 sulphur atoms are present in each ring formed by the A¹, A², V, W groups and the carbon atom to which they are bonded, and where the oxygen and sulphur atoms are not adjacent to one another,
m is 0, 1, 2,
n is 0, 1, 2,
R³ and R⁴ are each independently hydrogen, (C₁-C₈)-alkyl, halogen, (C₃-C₈)-cycloalkyl, (C₁-C₈)-alkoxy, aryl, heterocyclyl, heteroaryl, aryl-(C₁-C₈)-alkyl, (C₁-C₈)-alkylthio, (C₁-C₈)-haloalkyl, (C₁-C₈)-haloalkyloxy, (C₁-C₈)-haloalkylthio, (C₁-C₈)-alkoxy-(C₁-C₈)-alkyl, (C₁-C₈)-alkylthio-(C₁-C₈)-alkyl, heteroaryl-(C₁-C₈)-alkyl, heterocyclyl-(C₁-C₈)-alkyl, (C₁-C₈)-alkoxy-(C₁-C₈)-haloalkyl, (C₁-C₈)-haloalkoxy-(C₁-C₈)-haloalkyl, (C₂-C₈)-alkenyl, (C₂-C₈)-alkynyl, or
R³ and R⁴ together with the atom to which they are bonded form a fully saturated 3- to 6-membered ring optionally interrupted by heteroatoms and optionally having further substitution and
[M] is tris[(C₁-C₆)-alkyl] stannyl, tris[(C₃-C₈)-cycloalkyl]stannyl, tris-[(C₁-C₆)-alkyl]germanyl, tris-[(C₃-C₈)-cycloalkyl]germanyl, bis(cyclopentadienyl)zirconyl, bis(1,2,3,4,5-pentamethylcyclopentadienyl)zirconyl, bis(cyclopentadienyl)hafnyl, bis(1,2,3,4,5-pentamethylcyclopentadienyl)hafnyl, bis(hydroxy)boryl, bis[(C₁-C₆)-alkoxy]boryl, (C₁-C₆)-alkyl-1,3,2-dioxaborolan-2-yl, bis[(C₁-C₆)-alkyl]-1,3,2-dioxaborolan-2-yl, tetrakis[(C₁-C₆)-alkyl]-1,3,2-dioxaborolan-2-yl, 1,3,2-dioxaborinan-2-yl, bis[(C₁-C₆)-alkyl]-1,3,2-dioxaborinan-2-yl, (C₁-C₆)-alkyl-1,3,2-dioxaborinan-2-yl, tris[(C₁-C₆)-alkyl]-1,3,2-dioxaborinan-2-yl, 2,6,7-trioxa-1-boranuidabicyclo[2.2.2]octanyl, (C₁-C₆)-alkyl-2,6,7-trioxa-1-boranuidabicyclo[2.2.2]octanyl, tris[(C₁-C₆)-alkyl]plumbanyl, tris[(C₃-C₈)-cycloalkyl]plumbanyl, tris[(C₁-C₆)-alkylcarbonyloxy]plumbanyl, trisarylplumbanyl, bis[(C₁-C₆)-alkylcarbonyloxy]arylplumbanyl, bis[(C₁-C₆)-alkyl]alanyl, bis[(C₁-C₆)-cycloalkyl]alanyl, dichloroalanyl, chloromagnesyl, bromomagnesyl, chlorozincyl, chlorohydrargyl, bromohydrargyl, (C₁-C₆)-alkylhydrargyl, (C₃-C₆)-cycloalkylhydrargyl, tris[(C₁-C₆)-alkyl]silyl, (C₁-C₆)-alkyl[bis-(C₁-C₆)-alkyl]silyl, (C₁-C₆)-alkylbis(aryl)silyl, arylbis[(C₁-C₆)-alkyl)]silyl, (C₃-C₇)-cycloalkylbis[(C₁-C₆)-alkyl]silyl.

17. Compounds of the formula (III) according to Claim 16, where
R¹ is hydrogen, (C₁-C₆)-alkyl, aryl, heteroaryl, heterocyclyl, (C₃-C₆)-cycloalkyl, (C₂-C₆)-alkenyl, (C₂-C₆)-alkynyl, (C₂-C₆)-alkynyl- (C₁-C₆)-alkyl, (C₁-C₆)-alkoxy-(C₁-C₆)-alkyl, hydroxy-(C₁-C₆)-alkyl, (C₁-C₆)-haloalkyl, (C₂-C₆)-haloalkenyl, (C₁-C₆)-haloalkoxy-(C₁-C₆)-alkyl, (C₁-C₆)-alkylthio-(C₁-C₆)-alkyl, aryl-(C₁-C₆)-alkyl, heterocyclyl-(C₁-C₆)-alkyl, (C₃-C₆)-halocycloalkyl, (C₄-C₆) -cycloalkenyl, (C₁-C₆)-alkoxy-(C₁-C₆)-alkoxy-(C₁-C₆)-alkyl, (C₁-C₈)-alkoxy-(C₁-C₈)-haloalkyl, (C₁-C₈)-haloalkoxy-(C₁-C₈)-haloalkyl,
R² is hydrogen, (C₁-C₆)-alkyl, (C₁-C₆)-alkoxy-(C₁-C₆)-alkyl, (C₁-C₆)-alkoxy-(C₁-C₆)-alkoxy-(C₁-C₆)-alkyl, (C₂-C₆)-alkenyl, (C₂-C₆)-alkynyl, (C₁-C₆)-alkylcarbonyl, arylcarbonyl, heteroarylcarbonyl, (C₃-C₆)-cycloalkylcarbonyl, (C₂-C₆)-alkenylcarbonyl, heterocyclylcarbonyl, (C₁-C₆)-alkoxycarbonyl, (C₂-C₆)-alkenyloxycarbonyl, aryloxy-(C₁-C₆)-alkyl, aryl-(C₁-C₆)-alkoxycarbonyl, (C₃-C₆)-cycloalkoxycarbonyl, (C₃-C₆)-cycloalkyl-(C₁-C₆)-alkoxycarbonyl, aryl-(C₁-C₆)-alkoxy-(C₁-C₆)-alkyl, aryl-(C₁-C₆)-alkyl, (C₁-C₆)-alkoxy-(C₁-C₆)-alkoxy-(C₁-C₆)-alkyl, (C₁-C₆)-alkylthio-(C₁-C₆)-alkyl, tris[(C₁-C₆)-alkyl]silyl, (C₁-C₆)-alkylbis[(C₁-C₆)-alkyl]silyl, (C₁-C₆)-alkylbis(aryl)silyl, arylbis[(C₁-C₆)-alkyl]silyl, (C₃-C₆)-cycloalkylbis[(C₁-C₆)-alkyl] silyl, halobis[(C₁-C₆)-alkyl]silyl, tris[(C₁-C₆)-alkyl]silyl-(C₁-C₆)-alkoxy-(C₁-C₆)-alkyl, tris[(C₁-C₆)-alkyl]silyl-(C₁-C₆)-alkyl,
A¹, A², V, W are each independently a CR³R⁴ group, oxygen or sulphur, where not more than 2 oxygen or 2 sulphur atoms are present in each ring formed by the A¹, A², V, W groups and the carbon atom to which they are bonded, and where the oxygen and sulphur atoms are not adjacent to one another,
m is 0, 1, 2,
n is 0, 1, 2,
R³ and R⁴ are each independently hydrogen, (C₁-C₆)-alkyl, halogen, (C₃-C₆)-cycloalkyl, (C₁-C₆)-alkoxy, aryl, heterocyclyl, heteroaryl, aryl-(C₁-C₆)-alkyl, (C₁-C₆)-alkylthio, (C₁-C₆)-haloalkyl, (C₁-C₆)-haloalkyloxy, (C₁-C₆)-haloalkylthio, (C₁-C₆)-alkoxy-(C₁-C₆)-alkyl, (C₁-C₆)-alkylthio-(C₁-C₆)-alkyl, heteroaryl-(C₁-C₆)-alkyl, heterocyclyl-(C₁-C₆)-alkyl, (C₁-C₆)-alkoxy-(C₁-C₆)-haloalkyl, (C₁-C₆)-haloalkoxy-(C₁-C₆)-haloalkyl, (C₂-C₆)-alkenyl, (C₂-C₆)-alkynyl, or
R³ and R⁴ together with the atom to which they are bonded form a fully saturated 3- to 6-membered ring optionally interrupted by heteroatoms and optionally having further substitution and
[M] is tris[(C₁-C₆)-alkyl]stannyl, tris[(C₃-C₆)-cycloalkyl]stannyl, tris-[(C₁-C₆)-alkyl]germanyl, tris-[(C₃-C₆)-cycloalkyl]germanyl, bis(cyclopentadienyl)zirconyl, bis(1,2,3,4,5-pentamethylcyclopentadienyl)zirconyl, bis(cyclopentadienyl)hafnyl, bis(1,2,3,4,5-pentamethylcyclopentadienyl)hafnyl, bis(hydroxy)boryl, bis[(C₁-C₆)-alkoxy]boryl, (C₁-C₆)-alkyl-1,3,2-dioxaborolan-2-yl, bis[(C₁-C₆)-alkyl]-1,3,2-dioxaborolan-2-yl, tetrakis[(C₁-C₆)-alkyl]-1,3,2-dioxaborolan-2-yl, 1,3,2-dioxaborinan-2-yl, bis[(C₁-C₆)-alkyl]-1,3,2-dioxaborinan-2-yl, (C₁-C₆)-alkyl-1,3,2-dioxaborinan-2-yl, tris[(C₁-C₆)-alkyl]-1,3,2-dioxaborinan-2-yl, 2,6,7-trioxa-1-boranuidabicyclo[2.2.2]octanyl, (C₁-C₆)-alkyl-2,6,7-trioxa-1-boranuidabicyclo[2.2.2]octanyl, tris[(C₁-C₆)-alkyl]plumbanyl, tris[(C₃-C₆)-cycloalkyl]plumbanyl, tris[(C₁-C₆)-alkylcarbonyloxy]plumbanyl, trisarylplumbanyl, bis[(C₁-C₆)-alkylcarbonyloxy]arylplumbanyl, bis[(C₁-C₆)-alkyl]alanyl, bis[(C₁-C₆)-cycloalkyl]alanyl, dichloroalanyl, chloromagnesyl, bromomagnesyl, chlorozincyl, chlorohydrargyl, bromohydrargyl, (C₁-C₆)-alkylhydrargyl, (C₃-C₆)-cycloalkylhydrargyl, tris[(C₁-C₆)-alkyl]silyl, (C₁-C₆)-alkyl[bis(C₁-C₆)-alkyl]silyl, (C₁-C₆)-alkylbis(aryl)silyl, arylbis[(C₁-C₆)-alkyl)]silyl, (C₃-C₇)-cycloalkylbis[(C₁-C₆)-alkyl]silyl.

## Revendications

1. Vinylcyanocycloalcanols et alcynylcyanocycloalcanols substitués ainsi que vinylcyanohétérocyclylalcanols et alcynylcyanohétérocyclylalcanols substitués de formule générale (I) ou leurs sels, 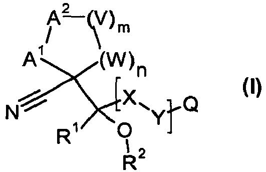 dans laquelle
[X-Y] représente les groupements 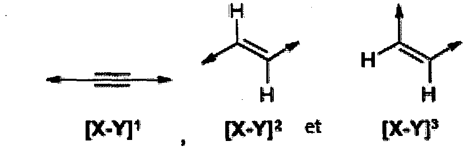
Q représente les groupements carbocycliques et hétérocycliques 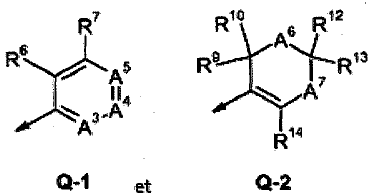 les groupements R⁶, R⁷, R⁹, R¹⁰, R¹², R¹³, R¹⁴, A³, A⁴, A⁵, A⁶ et A⁷ présentant respectivement la signification selon les définitions ci-dessous et la flèche représentant une liaison avec un groupement respectif [X-Y],
R¹ représente alkyle, aryle, hétéroaryle, hétérocyclyle, cycloalkyle, alcényle, alcynyle, alcoxyalkyle, hydroxyalkyle, halogénoalkyle, halogénoalcényle, halogénoalcoxyalkyle, alkylthioalkyle, arylalkyle, hétérocyclylalkyle, halogénocycloalkyle, cycloalcényle, alcoxyalcoxyalkyle, cycloalkylalkyle, cycloalcénylalkyle, halogénoalcynyle, alkylsulfinylalkyle, alkylsulfonylalkyle, halogénocycloalkylalkyle, cycloalkylsulfinylalkyle, cycloalkylsulfonylalkyle, arylsulfinylalkyle, arylsulfonylalkyle, arylthioalkyle, cycloalkylthioalkyle, alcoxyhalogénoalkyle, halogénoalcoxyhalogénoalkyle,
R² représente hydrogène, alkyle, alcoxyalkyle, alcoxyalcoxyalkyle, alcényle, alcynyle, alkylcarbonyle, arylcarbonyle, hétéroarylcarbonyle, cycloalkylcarbonyle, alcénylcarbonyle, hétérocyclylcarbonyle, alcoxycarbonyle, alcényloxycarbonyle, aryloxyalkyle, arylalcoxycarbonyle, cycloalcoxycarbonyle, cycloalkylalcoxycarbonyle, arylalcoxyalkyle, arylalkyle, alcoxyalcoxyalkyle, alkylthioalkyle, trialkylsilyle, alkyl(bis-alkyl)silyle, alkyl(bis-aryl)silyle, aryl(bis-alkyl)silyle, cycloalkyl(bis-alkyl)silyle, halogéno(bis-alkyl)silyle, trialkylsilylalcoxyalkyle, trialkylsilylalkyle, alcynyloxycarbonyle, cycloalkyle, cycloalkylalkyle, aminocarbonyle, alkylaminocarbonyle, bis-alkylaminocarbonyle, cycloalkylaminocarbonyle, alkylsulfonyle, halogénoalkylsulfonyle, arylsulfonyle, hétéroarylsulfonyle, cycloalkylsulfonyle,
A¹, A², V, W représentent, indépendamment les uns des autres, un groupe CR³R⁴, un groupe N-R⁵, oxygène ou soufre, au maximum 2 atomes d'oxygène ou 2 atomes de soufre étant à chaque fois présents dans le cycle formé par les groupes A¹, A², V, W et l'atome de carbone auquel ils sont liés et les différents atomes d'oxygène et de soufre n'étant pas adjacents,
m représente 0, 1, 2,
n représente 0, 1, 2,
R³ et R⁴ représentent, indépendamment l'un de l'autre, hydrogène, alkyle, halogène, cycloalkyle, alcoxy, aryle, hétérocyclyle, hétéroaryle, arylalkyle, alkylthio, halogénoalkyle, halogénoalkyloxy, halogénoalkylthio, alcoxyalkyle, alkylthioalkyle, hétéroarylalkyle, hétérocyclylalkyle, cycloalkylalkyle, cycloalcényle, alcynyle, alcényle, halogénoalcényle, halogénoalcynyle, alkylsulfinyle, alkylsulfonyle, cycloalkylsulfinyle, cycloalkylsulfonyle, arylsulfinyle, arylsulfonyle, alcoxyhalogénoalkyle, halogénoalcoxyhalogénoalkyle ou
R³ et R⁴ forment, ensemble avec l'atome auquel ils sont liés, un cycle de 3 à 6 chaînons complètement saturé, le cas échéant interrompu par des hétéroatomes et le cas échéant substitué davantage,
R⁵ représente hydrogène, alkyle, alcényle, alcynyle, alcoxyalkyle, alkylcarbonyle, arylcarbonyle, hétéroarylcarbonyle, cycloalkylcarbonyle, alcoxycarbonyle, alcényloxycarbonyle, alcoxycarbonylalkyle, alkylaminocarbonylalkyle, arylaminocarbonylalkyle, aryloxyalkyle, arylalkyle, hétéroarylalkyle, hétérocyclyle, hétérocyclylalkyle, halogénoalkyle,
R⁶ et R⁷ représentent, indépendamment l'un de l'autre, hydrogène, nitro, amino, cyano, thiocyanato, isothiocyanato, halogène, alkyle, cycloalkyle, alcényle, alcynyle, aryle, arylalkyle, arylalcoxy, hétéroaryle, alcoxyalkyle, hydroxyalkyle, halogénoalkyle, halogénocycloalkyle, alcoxy, halogénoalcoxy, aryloxy, hétéroaryloxy, cycloalkyloxy, hydroxy, cycloalkylalcoxy, alcoxycarbonyle, hydroxycarbonyle, aminocarbonyle, alkylaminocarbonyle, cycloalkylaminocarbonyle, cyanoalkylaminocarbonyle, alcénylaminocarbonyle, alcynylaminocarbonyle, alkylamino, alkylthio, halogénoalkylthio, hydrothio, bis-alkylamino, cycloalkylamino, alkylcarbonylamino, cycloalkylcarbonylamino, formylamino, halogénoalkylcarbonylamino, alcoxycarbonylamino, alkylaminocarbonylamino, alkyl(alkyl-)aminocarbonylamino, alkylsulfonylamino, cycloalkylsulfonylamino, arylsulfonylamino, hétarylsulfonylamino, sulfonylhalogénoalkylamino, aminosulfonyle, aminoalkylsulfonyle, aminohalogénoalkylsulfonyle, alkylaminosulfonyle, bisalkylaminosulfonyle, cycloalkylaminosulfonyle, halogénoalkylaminosulfonyle, arylaminosulfonyle, arylalkylaminosulfonyle, alkylsulfonyle, cycloalkylsulfonyle, arylsulfonyle, alkylsulfinyle, cycloalkylsulfinyle, arylsulfinyle, N,S-dialkylsulfonimidoyle, S-alkylsulfonimidoyle, alkylsulfonylaminocarbonyle, cycloalkylsulfonylaminocarbonyle, cycloalkylaminosulfonyle, arylalkylcarbonylamino, cycloalkylalkylcarbonylamino, hétéroarylcarbonylamino, alcoxyalkylcarbonylamino, hydroxyalkylcarbonylamino, trialkylsilyle,
A³, A⁴, A⁵ sont identiques ou différents et représentent, indépendamment les uns des autres, N (azote) ou le groupement C-R⁸, où, dans aucun cas, plus de deux atomes de N ne sont adjacents et R⁸ dans le groupement C-R⁸ présente à chaque fois des significations identiques ou différentes selon la définition ci-dessous et
A³ et A⁴ forment, lorsque les deux représentent un groupe C-R⁸, ensemble avec les atomes auxquels ils sont liés, un cycle de 5 à 6 chaînons complètement saturé, partiellement saturé ou complètement insaturé, le cas échéant interrompu par des hétéroatomes et le cas échéant substitué davantage,
A⁴ et A⁵ forment, lorsque les deux représentent un groupe C-R⁸, ensemble avec les atomes auxquels ils sont liés, un cycle de 5 à 6 chaînons complètement saturé, partiellement saturé ou complètement insaturé, le cas échéant interrompu par des hétéroatomes et le cas échéant substitué davantage,
R⁸ et R¹⁴, représentent, indépendamment l'un de l'autre, respectivement, hydrogène, nitro, amino, hydroxy, hydrothio, thiocyanato, isothiocyanato, halogène, alkyle, cycloalkyle, alcényle, alcynyle, aryle, arylalkyle, arylalcoxy, hétéroaryle, halogénoalkyle, halogénocycloalkyle, alcoxy, halogénoalcoxy, aryloxy, hétéroaryloxy, cycloalkyloxy, cycloalkylalcoxy, hydroxyalkyle, alcoxyalkyle, aryloxyalkyle, hétéroaryloxyalkyle, alkylamino, alkylthio, halogénoalkylthio, bisalkylamino, cycloalkylamino, alkylcarbonylamino, cycloalkylcarbonylamino, formylamino, halogénoalkylcarbonylamino, alcoxycarbonylamino, alkylaminocarbonylamino, alkyl(alkyl-)aminocarbonylamino, alkylsulfonylamino, cycloalkylsulfonylamino, arylsulfonylamino, hétarylsulfonylamino, sulfonylhalogénoalkylamino, aminoalkylsulfonyle, aminohalogénoalkylsulfonyle, alkylaminosulfonyle, bisalkylaminosulfonyle, cycloalkylaminosulfonyle, halogénoalkylaminosulfonyle, arylaminosulfonyle, arylalkylaminosulfonyle, alkylsulfonyle, cycloalkylsulfonyle, arylsulfonyle, alkylsulfinyle, cycloalkylsulfinyle, arylsulfinyle, N,S-dialkylsulfonimidoyle, S-alkylsulfonimidoyle, alkylsulfonylaminocarbonyle, cycloalkylsulfonylaminocarbonyle, cycloalkylaminosulfonyle, arylalkylcarbonylamino, cycloalkylalkylcarbonylamino, hétéroarylcarbonylamino, alcoxyalkylcarbonylamino, hydroxyalkylcarbonylamino, cyano, cyanoalkyle, hydroxycarbonyle, alcoxycarbonyle, cycloalcoxycarbonyle, cycloalkylalcoxycarbonyle, aryloxycarbonyle, arylalcoxycarbonyle, aminocarbonyle, alkylaminocarbonyle, bisalkylaminocarbonyle, alkyl(alcoxy)aminocarbonyle, cycloalkylaminocarbonyle, arylalkylaminocarbonyle, hétéroarylalkylaminocarbonyle, cyanoalkylaminocarbonyle, halogénoalkylaminocarbonyle, alcynylaminocarbonyle, alcoxycarbonylaminocarbonyle, arylalcoxycarbonylaminocarbonyle, hydroxycarbonylalkyle, alcoxycarbonylalkyle, cycloalcoxycarbonylalkyle, cycloalkylalcoxycarbonylalkyle, alkylaminocarbonylalkyle, aminocarbonylalkyle, bisalkylaminocarbonylalkyle, cycloalkylaminocarbonylalkyle, arylalkylaminocarbonylalkyle, hétéroarylalkylaminocarbonylalkyle, cyanoalkylaminocarbonylalkyle, halogénoalkylaminocarbonylalkyle, alcynylaminocarbonylalkyle, cycloalkylalkylaminocarbonylalkyle, alcoxycarbonylaminocarbonylalkyle, arylalcoxycarbonylaminocarbonylalkyle, alcoxycarbonylalkylaminocarbonyle, hydroxycarbonylalkylaminocarbonyle, aminocarbonylalkylaminocarbonyle, alkylaminocarbonylalkylaminocarbonyle, cycloalkylaminocarbonylalkylaminocarbonyle, cycloalkylalkylaminocarbonyle, cycloalkylalkylaminocarbonylalkyle, alcényloxycarbonyle, alcényloxycarbonylalkyle, alcénylaminocarbonyle, alcénylaminocarbonylalkyle, alkylcarbonyle, cycloalkylcarbonyle, formyle, hydroxyiminométhyle, amino-iminométhyle, alcoxyiminométhyle, alkylamino-iminométhyle, dialkylamino-iminométhyle, cycloalcoxyiminométhyle, cycloalkylalcoxyiminométhyle, aryloxyiminométhyle, arylalcoxyiminométhyle, arylalkylaminoiminométhyle, alcényloxyiminométhyle, arylaminoiminométhyle, arylsulfonylamino-iminométhyle, hétéroarylalkyle, hétérocyclylalkyle, hydroxycarbonylhétérocyclyle, alcoxycarbonylhétérocyclyle, alcényloxycarbonylhétérocyclyle, alcénylalcoxycarbonylhétérocyclyle, arylalcoxycarbonylhétérocyclyle, cycloalcoxycarbonylhétérocyclyle, cycloalkylalcoxycarbonylhétérocyclyle, aminocarbonylhétérocyclyle, alkylaminocarbonylhétérocyclyle, bis-alkylaminocarbonylhétérocyclyle, cycloalkylaminocarbonylhétérocyclyle, arylalkylaminocarbonylhétérocyclyle, alcénylaminocarbonylhétérocyclyle, hydroxycarbonylhétérocyclylalkyle, alcoxycarbonylhétérocyclylalkyle, hydroxycarbonylcycloalkylalkyle, alcoxycarbonylcycloalkylalkyle, hétérocyclyle, hétérocyclyloxy, hétérocyclylamino, hétérocyclylcarbonyle, hétérocyclylcarbonylalkyle, hydroxycarbonylhétérocyclylcarbonyle, alcoxycarbonylhétérocyclylcarbonyle, alcényloxycarbonylhétérocyclylcarbonyle, alcénylalcoxycarbonylhétérocyclylcarbonyle, arylalcoxycarbonylhétérocyclylcarbonyle, cycloalcoxycarbonylhétérocyclylcarbonyle, cycloalkylalcoxycarbonylhétérocyclylcarbonyle, aminocarbonylhétérocyclylcarbonyle, alkylaminocarbonylhétérocyclylcarbonyle, bis-alkylaminocarbonylhétérocyclylcarbonyle, cycloalkylaminocarbonylhétérocyclylcarbonyle, arylalkylaminocarbonylhétérocyclylcarbonyle, alcénylaminocarbonylhétérocyclylcarbonyle,
R⁹ et R¹⁰ représentent, indépendamment l'un de l'autre, hydrogène, halogène, alkyle, alcoxy, halogénoalkyle, halogénoalcoxy, aryle, hétéroaryle, arylalkyle ou forment, ensemble avec l'atome auquel ils sont liés, un groupe carbonyle,
A⁶, A⁷ sont identiques ou différents et représentent, indépendamment l'un de l'autre, N-R¹¹, oxygène, soufre ou le groupement CR¹⁵R¹⁶, où, dans aucun cas, plus d'un atome d'oxygène n'est contenu dans l'hétérocycle et R¹¹, R¹⁵, R¹⁶ dans les groupements N-R¹¹ et CR¹⁵R¹⁶ présentent respectivement des significations identiques ou différentes selon la définition ci-dessous,
R¹¹ représente hydrogène, alkyle, alcényle, alcynyle, alcoxyalkyle, alkylcarbonyle, arylcarbonyle, hétéroarylcarbonyle, cycloalkylcarbonyle, alcoxycarbonyle, alcényloxycarbonyle, alcoxycarbonylalkyle, alkylaminocarbonylalkyle, arylaminocarbonylalkyle, aryloxyalkyle, arylalkyle, hétéroarylalkyle, hétérocyclyle, hétérocyclylalkyle,
R¹² et R¹³ représentent, indépendamment l'un de l'autre, hydrogène, halogène, alkyle, alcoxy, halogénoalkyle, halogénoalcoxy, aryle, hétéroaryle, arylalkyle, hétérocyclyle, hétéroarylalkyle, hétérocyclylalkyle ou forment, ensemble avec l'atome auquel ils sont liés, un groupe carbonyle et
R¹⁵ et R¹⁶ représentent, indépendamment l'un de l'autre, hydrogène, alkyle, alcényle, alcynyle, alcoxy, alcoxyalkyle, alkylcarbonyle, arylcarbonyle, hétéroarylcarbonyle, hydroxycarbonyle, alcoxycarbonyle, hétérocyclyle, halogénoalkyle,
à l'exception du composé 1-[(2E)-1-hydroxy-1,3-diphénylprop-2-én-1-yl]cyclopentane-carbonitrile.

2. Vinylcyanocycloalcanols et alcynylcyanocycloalcanols substitués ainsi que vinylcyanohétérocyclylalcanols et alcynylcyanohétérocyclylalcanols substitués selon la revendication 1, dans lesquels
[X-Y] représente les groupements 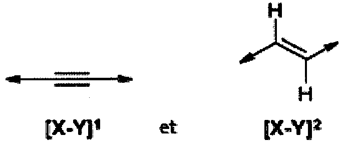
Q représente les groupements carbocycliques et hétérocycliques 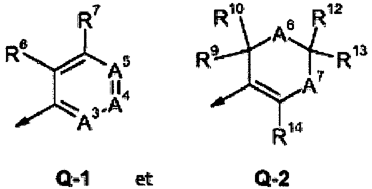 les groupements R⁶, R⁷, R⁹, R¹⁰, R¹², R¹³, R¹⁴, A³, A⁴, A⁵, A⁶ et A⁷ présentant respectivement la signification selon les définitions ci-dessous et la flèche représentant une liaison avec un groupement respectif [X-Y],
R¹ représente (C₁-C₈)-alkyle, aryle, hétéroaryle, hétérocyclyle, (C₃-C₁₀)-cycloalkyle, (C₂-C₈)-alcényle, (C₂-C₈)-alcynyle, (C₁-C₈)-alcoxy-(C₁-C₈)-alkyle, hydroxy- (C₁-C₈)-alkyle, (C₁-C₈)-halogénoalkyle, (C₂-C₈)-halogénoalcényle, (C₁-C₈)-halogénoalcoxy-(C₁-C₈)-alkyle, (C₁-C₈)-alkylthio-(C₁-C₈)-alkyle, aryl-(C₁-C₈)-alkyle, hétérocyclyl-(C₁-C₈)-alkyle, (C₃-C₈)-halogénocycloalkyle, (C₄-C₈)-cycloalcényle, (C₁-C₈)-alcoxy- (C₁-C₈)-alcoxy-(C₁-C₈)-alkyle, (C₃-C₈)-cycloalkyl- (C₁-C₈)-alkyle, (C₄-C₈)-cycloalcényl-(C₁-C₈)-alkyle, (C₂-C₈)-halogénoalcynyle, (C₃-C₈)-alkylsulfinyl-(C₃-C₈)-alkyle, (C₁-C₈)-alkylsulfonyl-(C₁-C₈)-alkyle, (C₃-C₈)-halogénocycloalkyl-(C₁-C₈)-alkyle, (C₃-C₈)-cycloalkylsulfinyl-(C₁-C₈)-alkyle, (C₃-C₈)-cycloalkylsulfonyl-(C₁-C₈)-alkyle, arylsulfinyl-(C₁-C₈)-alkyle, arylsulfonyl-(C₁-C₈)-alkyle, arylthio-(C₁-C₈)-alkyle, (C₃-C₈)-cycloalkylthio-(C₁-C₈)-alkyle, (C₁-C₈)-alcoxy-(C₁-C₈)-halogénoalkyle, (C₁-C₈)-halogénoalcoxy-(C₁-C₈)-halogénoalkyle,
R² représente hydrogène, (C₁-C₈)-alkyle, (C₁-C₈)-alcoxy-(C₁-C₈)-alkyle, (C₁-C₈)-alcoxy-(C₁-C₈)-alcoxy-(C₁-C₈)-alkyle, (C₂-C₈)-alcényle, (C₂-C₈)-alcynyle, (C₁-C₈)-alkylcarbonyle, arylcarbonyle, hétéroarylcarbonyle, (C₃-C₈)-cycloalkylcarbonyle, (C₂-C₈)-alcénylcarbonyle, hétérocyclylcarbonyle, (C₁-C₈)-alcoxycarbonyle, (C₂-C₈)-alcényloxycarbonyle, aryloxy-(C₁-C₈)-alkyle, aryl-(C₁-C₈)-alcoxycarbonyle, (C₃-C₈)-cycloalcoxycarbonyle, (C₃-C₈)-cycloalkyl- (C₁-C₈)-alcoxycarbonyle, aryl-(C₁-C₈)-alcoxyalkyle, aryl-(C₁-C₈)-alkyle, (C₁-C₈)-alcoxy- (C₁-C₈)-alcoxy-(C₁-C₈)-alkyle, (C₁-C₈)-alkylthio- (C₁-C₈)-alkyle, tris[(C₁-C₈)-alkyl]silyle, (C₁-C₈)-alkyl{bis-[(C₁-C₈)-alkyl]}silyle, (C₁-C₈)-alkyl(bis-aryl)silyle, aryl{bis-[(C₁-C₈)-alkyl]}silyle, cycloalkyl{bis-[(C₁-C₈)-alkyl]}silyle, halogéno{bis-[(C₁-C₈)-alkyl]}silyle, tris-[(C₁-C₈)-alkyl]silyl-(C₁-C₈)-alcoxy-(C₁-C₈)-alkyle, tris[(C₁-C₈)-alkyl]silyl-(C₁-C₈)-alkyle, (C₂-C₈)-alcynyloxycarbonyle, (C₃-C₈)-cycloalkyle, (C₃-C₈)-cycloalkyl- (C₁-C₈)-alkyle, aminocarbonyle, (C₁-C₈)-alkylaminocarbonyle, bis-[(C₁-C₈)-alkyl]aminocarbonyle, (C₃-C₈)-cycloalkylaminocarbonyle, (C₁-C₈)-alkylsulfonyle, (C₁-C₈)-halogénoalkylsulfonyle, arylsulfonyle, hétéroarylsulfonyle, (C₃-C₈)-cycloalkylsulfonyle,
A¹, A², V, W représentent, indépendamment les uns des autres, un groupe CR³R⁴, un groupe N-R⁵, oxygène ou soufre, au maximum 2 atomes d'oxygène ou 2 atomes de soufre étant à chaque fois présents dans le cycle formé par les groupes A¹, A², V, W et l'atome de carbone auquel ils sont liés et les différents atomes d'oxygène et de soufre n'étant pas adjacents,
m représente 0, 1, 2,
n représente 0, 1, 2,
R³ et R⁴ représentent, indépendamment l'un de l'autre, hydrogène, (C₁-C₈)-alkyle, halogène, (C₃-C₈)-cycloalkyle, (C₁-C₈)-alcoxy, aryle, hétérocyclyle, hétéroaryle, aryl-(C₁-C₈)-alkyle, (C₁-C₈)-alkylthio, (C₁-C₈)-halogénoalkyle, (C₁-C₈)-halogénoalkyloxy, (C₁-C₈)-halogénoalkylthio, (C₁-C₈)-alcoxy-(C₁-C₈)-alkyle, (C₁-C₈)-alkylthio-(C₁-C₈)-alkyle, hétéroaryl-(C₁-C₈)-alkyle, hétérocyclyl-(C₁-C₈)-alkyle, (C₃-C₈)-cycloalkyl- (C₁-C₈)-alkyle, (C₄-C₈)-cycloalcényle, (C₂-C₈)-alcynyle, (C₂-C₈)-alcényle, (C₂-C₈)-halogénoalcényle, (C₂-C₈)-halogénoalcynyle, (C₁-C₈)-alkylsulfinyle, (C₁-C₈)-alkylsulfonyle, (C₃-C₈)-cycloalkylsulfinyle, (C₃-C₈)-cycloalkylsulfonyle, arylsulfinyle, arylsulfonyle, (C₁-C₈)-alcoxy-(C₁-C₈)-halogénoalkyle, (C₁-C₈)-halogénoalcoxy-(C₁-C₈)-halogénoalkyle ou
R³ et R⁴ forment, ensemble avec l'atome auquel ils sont liés, un cycle de 3 à 6 chaînons complètement saturé, le cas échéant interrompu par des hétéroatomes et le cas échéant substitué davantage,
R⁵ représente hydrogène, (C₁-C₈)-alkyle, (C₂-C₈)-alcényle, (C₂-C₈)-alcynyle, (C₁-C₈)-alcoxy- (C₁-C₈)-alkyle, (C₁-C₈)-alkylcarbonyle, arylcarbonyle, hétéroarylcarbonyle, (C₃-C₈)-cycloalkylcarbonyle, (C₁-C₈)-alcoxycarbonyle, (C₂-C₈)-alcényloxycarbonyle, (C₁-C₈)-alcoxycarbonyl-(C₁-C₈)-alkyle, (C₁-C₈)-alkylaminocarbonyl-(C₁-C₈)-alkyle, arylaminocarbonyl-(C₁-C₈)-alkyle, aryloxy-(C₁-C₈)-alkyle, aryl- (C₁-C₈)-alkyle, hétéroaryl- (C₁-C₈)-alkyle, hétérocyclyle, hétérocyclyl-(C₁-C₈)-alkyle, (C₁-C₈)-halogénoalkyle,
R⁶ et R⁷ représentent, indépendamment l'un de l'autre, hydrogène, nitro, amino, cyano, thiocyanato, isothiocyanato, halogène, (C₁-C₈)-alkyle, (C₃-C₈)-cycloalkyle, (C₂-C₈)-alcényle, (C₂-C₈)-alcynyle, aryle, aryl- (C₁-C₈)-alkyle, aryl- (C₁-C₈)-alcoxy, hétéroaryle, (C₁-C₈)-alcoxy- (C₁-C₈)-alkyle, hydroxy-(C₁-C₈)-alkyle, (C₁-C₈)-halogénoalkyle, (C₃-C₈)-halogénocycloalkyle, (C₁-C₈)-alcoxy, (C₁-C₈)-halogénoalcoxy, aryloxy, hétéroaryloxy, (C₃-C₈)-cycloalkyloxy, hydroxy, (C₃-C₈)-cycloalkyl-(C₁-C₈)-alcoxy, (C₁-C₈)-alcoxycarbonyle, hydroxycarbonyle, aminocarbonyle, (C₁-C₈)-alkylaminocarbonyle, (C₃-C₈)-cycloalkylaminocarbonyle, cyano-(C₁-C₈)-alkylaminocarbonyle, (C₂-C₈)-alcénylaminocarbonyle, (C₂-C₈)-alcynylaminocarbonyle, (C₁-C₈)-alkylamino, (C₁-C₈)-alkylthio, (C₁-C₈)-halogénoalkylthio, hydrothio, bis-[(C₁-C₈)-alkyl] amino, (C₃-C₈)-cycloalkylamino, (C₁-C₈)-alkylcarbonylamino, (C₃-C₈)-cycloalkylcarbonylamino, formylamino, (C₁-C₈)-halogénoalkylcarbonylamino, (C₁-C₈)-alcoxycarbonylamino, (C₁-C₈)-alkylaminocarbonylamino, (C₁-C₈)-alkyl-[(C₁-C₈)-alkyl]aminocarbonylamino, (C₁-C₈)-alkylsulfonylamino, (C₃-C₈)-cycloalkylsulfonylamino, arylsulfonylamino, hétarylsulfonylamino, sulfonyl-(C₁-C₈)-halogénoalkylamino, aminosulfonyle, amino-(C₁-C₈)-alkylsulfonyle, amino-(C₁-C₈)-halogénoalkylsulfonyle, (C₁-C₈)-alkylaminosulfonyle, bis[(C₁-C₈)-alkyl]aminosulfonyle, (C₃-C₈)-cycloalkylaminosulfonyle, (C₁-C₈)-halogénoalkylaminosulfonyle, arylaminosulfonyle, aryl- (C₁-C₈)-alkylaminosulfonyle, (C₁-C₈)-alkylsulfonyle, (C₃-C₈)-cycloalkylsulfonyle, arylsulfonyle, (C₁-C₈)-alkylsulfinyle, (C₃-C₈)-cycloalkylsulfinyle, arylsulfinyle, N,S-bis[(C₁-C₈)-alkyl]sulfonimidoyle, S-(C₁-C₈)-alkylsulfonimidoyle, (C₁-C₈)-alkylsulfonylaminocarbonyle, (C₃-C₈)-cycloalkylsulfonylaminocarbonyle, (C₃-C₈)-cycloalkylaminosulfonyle, aryl-(C₁-C₈)-alkylcarbonylamino, (C₃-C₈)-cycloalkyl-(C₁-C₈)-alkylcarbonylamino, hétéroarylcarbonylamino, (C₁-C₈)-alcoxy-(C₁-C₈)-alkylcarbonylamino, hydroxy-(C₁-C₈)-alkylcarbonylamino, tris[(C₁-C₈)-alkyl]silyle,
A³, A⁴, A⁵ sont identiques ou différents et représentent, indépendamment les uns des autres, N (azote) ou le groupement C-R⁸, où, dans aucun cas, plus de deux atomes de N ne sont adjacents et R⁸ dans le groupement C-R⁸ présente à chaque fois des significations identiques ou différentes selon la définition ci-dessous et
A³ et A⁴ forment, lorsque les deux représentent un groupe C-R⁸, ensemble avec les atomes auxquels ils sont liés, un cycle de 5 à 6 chaînons complètement saturé, partiellement saturé ou complètement insaturé, le cas échéant interrompu par des hétéroatomes et le cas échéant substitué davantage,
A⁴ et A⁵ forment, lorsque les deux représentent un groupe C-R⁸, ensemble avec les atomes auxquels ils sont liés, un cycle de 5 à 6 chaînons complètement saturé, partiellement saturé ou complètement insaturé, le cas échéant interrompu par des hétéroatomes et le cas échéant substitué davantage,
R⁸ et R¹⁴ représentent, indépendamment l'un de l'autre, respectivement hydrogène, nitro, amino, hydroxy, hydrothio, thiocyanato, isothiocyanato, halogène, (C₁-C₈)-alkyle, (C₃-C₈)-cycloalkyle, (C₂-C₈)-alcényle, (C₂-C₈)-alcynyle, aryle, aryl-(C₁-C₈)-alkyle, aryl- (C₁-C₈)-alcoxy, hétéroaryle, (C₁-C₈)-halogénoalkyle, (C₃-C₈)-halogénocycloalkyle, (C₁-C₈)-alcoxy, (C₁-C₈)-halogénoalcoxy, aryloxy, hétéroaryloxy, (C₃-C₈)-cycloalkyloxy, (C₃-C₈)-cycloalkyl-(C₁-C₈)-alcoxy, hydroxy-(C₁-C₈)-alkyle, (C₁-C₈)-alcoxy-(C₁-C₈)-alkyle, aryloxy-(C₁-C₈)-alkyle, hétéroaryloxy-(C₁-C₈)-alkyle, (C₁-C₈)-alkylamino, (C₁-C₈)-alkylthio, (C₁-C₈)-halogénoalkylthio, bis[(C₁-C₈)-alkyl]amino, (C₃-C₈)-cycloalkylamino, (C₁-C₈)-alkylcarbonylamino, (C₃-C₈)-cycloalkylcarbonylamino, formylamino, (C₁-C₈)-halogénoalkylcarbonylamino, (C₁-C₈)-alcoxycarbonylamino, (C₁-C₈)-alkylaminocarbonylamino, (C₁-C₈)-alkyl[(C₁-C₈)-alkyl]aminocarbonylamino, (C₁-C₈)-alkylsulfonylamino, (C₃-C₈)-cycloalkylsulfonylamino, arylsulfonylamino, hétarylsulfonylamino, sulfonyl-(C₁-C₈)-halogénoalkylamino, amino-(C₁-C₈)-alkylsulfonyle, amino-(C₁-C₈)-halogénoalkylsulfonyle, (C₁-C₈)-alkylaminosulfonyle, bis[(C₁-C₈)-alkyl]aminosulfonyle, (C₃-C₈)-cycloalkylaminosulfonyle, (C₁-C₈)-halogénoalkylaminosulfonyle, arylaminosulfonyle, aryl- (C₁-C₈)-alkylaminosulfonyle, (C₁-C₈)-alkylsulfonyle, (C₃-C₈)-cycloalkylsulfonyle, arylsulfonyle, (C₁-C₈)-alkylsulfinyle, (C₃-C₈)-cycloalkylsulfinyle, arylsulfinyle, N,S-bis[(C₁-C₈)-alkyl]sulfonimidoyle, S-(C₁-C₈)-alkylsulfonimidoyle, (C₁-C₈)-alkylsulfonylaminocarbonyle, (C₃-C₈)-cycloalkylsulfonylaminocarbonyle, (C₃-C₈)-cycloalkylaminosulfonyle, aryl-(C₁-C₈)-alkylcarbonylamino, (C₃-C₈)-cycloalkyl- (C₁-C₈)-alkylcarbonylamino, hétéroarylcarbonylamino, (C₁-C₈)-alcoxy-(C₁-C₈)-alkylcarbonylamino, hydroxy-(C₁-C₈)-alkylcarbonylamino, cyano, cyano-(C₁-C₈)-alkyle, hydroxycarbonyle, (C₁-C₈)-alcoxycarbonyle, (C₃-C₈)-cycloalcoxycarbonyle, (C₃-C₈)-cycloalkyl-(C₁-C₈)-alcoxycarbonyle, aryloxycarbonyle, aryl-(C₁-C₈)-alcoxycarbonyle, aminocarbonyle, (C₁-C₈)-alkylaminocarbonyle, bis[(C₁-C₈)-alkyl]aminocarbonyle, (C₁-C₈)-alkyl[(C₁-C₈)-alcoxy]aminocarbonyle, (C₃-C₈)-cycloalkylaminocarbonyle, aryl-(C₁-C₈)-alkylaminocarbonyle, hétéroaryl-(C₁-C₈)-alkylaminocarbonyle, cyano-(C₁-C₈)-alkylaminocarbonyle, (C₁-C₈)-halogénoalkylaminocarbonyle, (C₂-C₈)-alcynylaminocarbonyle, (Ci-Cs)-alcoxycarbonylaminocarbonyle, aryl-(C₁-C₈)-alcoxycarbonylaminocarbonyle, hydroxycarbonyl-(C₁-C₈)-alkyle, (C₁-C₈)-alcoxycarbonyl-(C₁-C₈)-alkyle, (C₃-C₈)-cycloalcoxycarbonyl-(C₁-C₈)-alkyle, (C₃-C₈)-cycloalkyl-(C₁-C₈)-alcoxycarbonyl-(C₁-C₈)-alkyle, (C₁-C₈)-alkylaminocarbonyl-(C₁-C₈)-alkyle, aminocarbonyl- (C₁-C₈)-alkyle, bis[(C₁-C₈)-alkyl]aminocarbonyl-(C₁-C₈)-alkyle, (C₃-C₈)-cycloalkylaminocarbonyl-(C₁-C₈)-alkyle, aryl-(C₁-C₈)-alkylaminocarbonyl-(C₁-C₈)-alkyle, hétéroaryl-(C₁-C₈)-alkylaminocarbonyl-(C₁-C₈)-alkyle, cyano-(C₁-C₈)-alkylaminocarbonyl-(C₁-C₈)-alkyle, (C₁-C₈)-halogénoalkylaminocarbonyl-(C₁-C₈)-alkyle, (C₂-C₈)-alcynylaminocarbonyl-(C₁-C₈)-alkyle, (C₃-C₈)-cycloalkyl-(C₁-C₈)-alkylaminocarbonyl-(C₁-C₈)-alkyle, (C₁-C₈)-alcoxycarbonylaminocarbonyl-(C₁-C₈)-alkyle, aryl-(C₁-C₈)-alcoxycarbonylaminocarbonyl-(C₁-C₈)-alkyle, (C₁-C₈)-alcoxycarbonyl-(C₁-C₈)-alkylaminocarbonyle, hydroxycarbonyl-(C₁-C₈)-alkylaminocarbonyle, aminocarbonyl-(C₁-C₈)-alkylaminocarbonyle, (C₁-C₈)-alkylaminocarbonyl-(C₁-C₈)-alkylaminocarbonyle, (C₃-C₈)-cycloalkylaminocarbonyl-(C₁-C₈)-alkylaminocarbonyle, (C₃-C₈)-cycloalkyl-(C₁-C₈)-alkylaminocarbonyle, (C₃-C₈)-cycloalkyl- (C₁-C₈)-alkylaminocarbonyl-(C₁-C₈)-alkyle, (C₂-C₈)-alcényloxycarbonyle, (C₂-C₈)-alcényloxycarbonyl-(C₁-C₈)-alkyle, (C₂-C₈)-alcénylaminocarbonyle, (C₂-C₈)-alcénylaminocarbonyl-(C₁-C₈)-alkyle, (C₁-C₈)-alkylcarbonyle, (C₃-C₈)-cycloalkylcarbonyle, formyle, hydroxyiminométhyle, amino-iminométhyle, (C₁-C₈)-alcoxyiminométhyle, (C₁-C₈)-alkylamino-iminométhyle, bis[(C₁-C₈)-alkyl]amino-iminométhyle, (C₃-C₈)-cycloalcoxyiminométhyle, (C₃-C₈)-cycloalkyl-(C₁-C₈)-alcoxyiminométhyle, aryloxyiminométhyle, aryl-(C₁-C₈)-alcoxyiminométhyle, aryl-(C₁-C₈)-alkylamino-iminométhyle, (C₂-C₈)-alcényloxyiminométhyle, arylamino-iminométhyle, arylsulfonylamino-iminométhyle, hétéroaryl-(C₁-C₈)-alkyle, hétérocyclyl-(C₁-C₈)-alkyle, hydroxycarbonylhétérocyclyle, (C₁-C₈)-alcoxycarbonylhétérocyclyle, (C₂-C₈)-alcényloxycarbonylhétérocyclyle, (C₂-C₈)-alcényl-(C₁-C₈)-alcoxycarbonylhétérocyclyle, aryl-(C₁-C₈)-alcoxycarbonylhétérocyclyle, (C₃-C₈)-cycloalcoxycarbonylhétérocyclyle, (C₃-C₈)-cycloalkyl-(C₁-C₈)-alcoxycarbonylhétérocyclyle, aminocarbonylhétérocyclyle, (C₁-C₈)-alkylaminocarbonylhétérocyclyle, bis-[(C₁-C₈)-alkyl]aminocarbonylhétérocyclyle, (C₃-C₈)-cycloalkylaminocarbonylhétérocyclyle, aryl-(C₁-C₈)-alkylaminocarbonylhétérocyclyle, (C₂-C₈)-alcénylaminocarbonylhétérocyclyle, hydroxycarbonylhétérocyclyl-(C₁-C₈)-alkyle, (C₁-C₈)-alcoxycarbonylhétérocyclyl-(C₁-C₈)-alkyle, hydroxycarbonyl-(C₃-C₈)-cycloalkyl-(C₁-C₈)-alkyle, (C₁-C₈)-alcoxycarbonyl-(C₃-C₈)-cycloalkyl-(C₁-C₈)-alkyle, hétérocyclyle, hétérocyclyloxy, hétérocyclylamino, hétérocyclylcarbonyle, hétérocyclylcarbonyl-(C₁-C₈)-alkyle, hydroxycarbonylhétérocyclylcarbonyle, (C₁-C₈)-alcoxycarbonylhétérocyclylcarbonyle, (C₂-C₈)-alcényloxycarbonylhétérocyclylcarbonyle, (C₂-C₈)-alcényl-(C₁-C₈)-alcoxycarbonylhétérocyclylcarbonyle, aryl-(C₁-C₈)-alcoxycarbonylhétérocyclylcarbonyle, (C₃-C₈)-cycloalcoxycarbonylhétérocyclylcarbonyle, (C₃-C₈)-cycloalkyl-(C₁-C₈)-alcoxycarbonylhétérocyclylcarbonyle, aminocarbonylhétérocyclylcarbonyle, (C₁-C₈)-alkylaminocarbonylhétérocyclylcarbonyle, bis-[(C₁-C₈)-alkyl]aminocarbonylhétérocyclylcarbonyle, (C₃-C₈)-cycloalkylaminocarbonylhétérocyclylcarbonyle, aryl- (C₁-C₈)-alkylaminocarbonylhétérocyclylcarbonyle, (C₂-C₈)-alcénylaminocarbonylhétérocyclylcarbonyle,
R⁹ et R¹⁰ représentent, indépendamment l'un de l'autre, hydrogène, halogène, (C₁-C₈)-alkyle, (C₁-C₈)-alcoxy, (C₁-C₈)-halogénoalkyle, (C₁-C₈)-halogénoalcoxy, aryle, hétéroaryle, aryl-(C₁-C₈)-alkyle ou forment, ensemble avec l'atome auquel ils sont liés, un groupe carbonyle,
A⁶, A⁷ sont identiques ou différents et représentent, indépendamment l'un de l'autre, N-R¹¹, oxygène, soufre ou le groupement CR¹⁵R¹⁶, où, dans aucun cas, plus d'un atome d'oxygène n'est contenu dans l'hétérocycle et R¹¹, R¹⁵, R¹⁶ dans les groupements N-R¹¹ et CR¹⁵R¹⁶ présentent respectivement des significations identiques ou différentes selon la définition ci-dessous,
R¹¹ représente hydrogène, (C₁-C₈)-alkyle, (C₂-C₈)-alcényle, (C₂-C₈)-alcynyle, (C₁-C₈)-alcoxy-(C₁-C₈)-alkyle, (C₁-C₈)-alkylcarbonyle, arylcarbonyle, hétéroarylcarbonyle, (C₃-C₈)-cycloalkylcarbonyle, (C₁-C₈)-alcoxycarbonyle, (C₂-C₈)-alcényloxycarbonyle, (C₁-C₈)-alcoxycarbonyl-(C₁-C₈)-alkyle, (C₁-C₈)-alkylaminocarbonyl-(C₁-C₈)-alkyle, arylaminocarbonyl-(C₁-C₈)-alkyle, aryloxy-(C₁-C₈)-alkyle, aryl-(C₁-C₈)-alkyle, hétéroaryl-(C₁-C₈)-alkyle, hétérocyclyle, hétérocyclyl-(C₁-C₈)-alkyle,
R¹² et R¹³ représentent, indépendamment l'un de l'autre, hydrogène, halogène, (C₁-C₈)-alkyle, (C₁-C₈)-alcoxy, (C₁-C₈)-halogénoalkyle, (C₁-C₈)-halogénoalcoxy, aryle, hétéroaryle, aryl-(C₁-C₈)-alkyle, hétérocyclyle, hétéroaryl-(C₁-C₈)-alkyle, hétérocyclyl-(C₁-C₈)-alkyle ou forment, ensemble avec l'atome auquel ils sont liés, un groupe carbonyle et
R¹⁵ et R¹⁶ représentent, indépendamment l'un de l'autre, hydrogène, (C₁-C₈)-alkyle, (C₂-C₈)-alcényle, (C₂-C₈)-alcynyle, (C₁-C₈)-alcoxy, (C₁-C₈)-alcoxy-(C₁-C₈)-alkyle, (C₁-C₈)-alkylcarbonyle, arylcarbonyle, hétéroarylcarbonyle, hydroxycarbonyle, (C₁-C₈)-alcoxycarbonyle, hétérocyclyle, (C₁-C₈)-halogénoalkyle,
à l'exception du composé 1-[(2E)-1-hydroxy-1,3-diphénylprop-2-én-1-yl]cyclopentane-carbonitrile.

3. Vinylcyanocycloalcanols et alcynylcyanocycloalcanols substitués ainsi que vinylcyanohétérocyclylalcanols et alcynylcyanohétérocyclylalcanols substitués selon la revendication 1, dans lesquels
[X-Y] représente les groupements 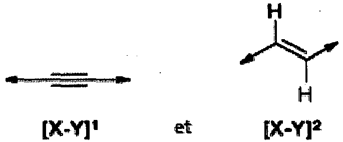
Q représente les groupements carbocycliques et hétérocycliques 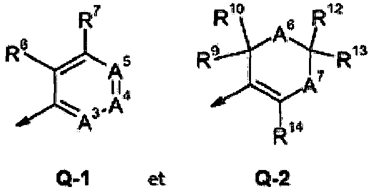 les groupements R⁶, R⁷, R⁹, R¹⁰, R¹², R¹³, R¹⁴, A³, A⁴, A⁵, A⁶ et A⁷ présentant respectivement la signification selon les définitions ci-dessous et la flèche représentant une liaison avec un groupement respectif [X-Y],
R¹ représente (C₁-C₇)-alkyle, aryle, hétéroaryle, hétérocyclyle, (C₃-C₁₀)-cycloalkyle, (C₂-C₇)-alcényle, (C₂-C₇)-alcynyle, (C₁-C₇)-alcoxy-(C₁-C₇)-alkyle, hydroxy-(C₁-C₇)-alkyle, (C₁-C₇)-halogénoalkyle, (C₂-C₇)-halogénoalcényle, (C₁-C₇)-halogénoalcoxy-(C₁-C₇)-alkyle, (C₁-C₇)-alkylthio-(C₁-C₇)-alkyle, aryl-(C₁-C₇)-alkyle, hétérocyclyl-(C₁-C₇)-alkyle, (C₃-C₇)-halogénocycloalkyle, (C₄-C₇)-cycloalcényle, (C₁-C₇)-alcoxy-(C₁-C₇)-alcoxy-(C₁-C₇)-alkyle, (C₁-C₇)-alcoxy-(C₁-C₇)-halogénoalkyle, (C₁-C₇)-halogénoalcoxy-(C₁-C₇)-halogénoalkyle, (C₃-C₈)-cycloalkyl-(C₁-C₇)-alkyle,
R² représente hydrogène, (C₁-C₇)-alkyle, (C₁-C₇)-alcoxy-(C₁-C₇)-alkyle, (C₁-C₇)-alcoxy-(C₁-C₇)-alcoxy-(C₁-C₇)-alkyle, (C₂-C₇)-alcényle, (C₂-C₇)-alcynyle, (C₁-C₇)-alkylcarbonyle, arylcarbonyle, hétéroarylcarbonyle, (C₃-C₇)-cycloalkylcarbonyle, (C₂-C₇)-alcénylcarbonyle, hétérocyclylcarbonyle, (C₁-C₇)-alcoxycarbonyle, (C₂-C₇)-alcényloxycarbonyle, aryloxy-(C₁-C₇)-alkyle, aryl-(C₁-C₇)-alcoxycarbonyle, (C₃-C₇)-cycloalcoxycarbonyle, (C₃-C₇)-cycloalkyl-(C₁-C₇)-alcoxycarbonyle, aryl- (C₁-C₇)-alcoxy-(C₁-C₇)-alkyle, aryl-(C₁-C₇)-alkyle, (C₁-C₇)-alcoxy-(C₁-C₇)-alcoxy-(C₁-C₇)-alkyle, (C₁-C₇)-alkylthio-(C₁-C₇)-alkyle, tris[(C₁-C₇)-alkyl]silyle, (C₁-C₇)-alkyl-bis-[(C₁-C₇)-alkyl]silyle, (C₁-C₇)-alkyl-bis(aryl)silyle, aryl-bis[(C₁-C₇)-alkyl]silyle, (C₃-C₇)-cycloalkyl-bis[(C₁-C₇)-alkyl]silyle, halogéno-bis[(C₁-C₇)-alkyl]silyle, tris[(C₁-C₇)-alkyl]silyl-(C₁-C₇)-alcoxy-(C₁-C₇)-alkyle, tris[(C₁-C₇)-alkyl]silyl-(C₁-C₇)-alkyle,
A¹, A², V, W représentent, indépendamment les uns des autres, un groupe CR³R⁴, un groupe N-R⁵, oxygène ou soufre, au maximum 2 atomes d'oxygène ou 2 atomes de soufre étant à chaque fois présents dans le cycle formé par les groupes A¹, A², V, W et l'atome de carbone auquel ils sont liés et les différents atomes d'oxygène et de soufre n'étant pas adjacents,
m représente 0, 1, 2,
n représente 0, 1, 2,
R³ et R⁴ représentent, indépendamment l'un de l'autre, hydrogène, (C₁-C₇)-alkyle, halogène, (C₃-C₇)-cycloalkyle, (C₁-C₇)-alcoxy, aryle, hétérocyclyle, hétéroaryle, aryl- (C₁-C₇)-alkyle, (C₁-C₇)-alkylthio, (C₁-C₇)-halogénoalkyle, (C₁-C₇)-halogénoalkyloxy, (C₁-C₇)-halogénoalkylthio, (C₁-C₇)-alcoxy-(C₁-C₇)-alkyle, (C₁-C₇)-alkylthio-(C₁-C₇)-alkyle, hétéroaryl-(C₁-C₇)-alkyle, hétérocyclyl-(C₁-C₇)-alkyle, (C₃-C₇)-cycloalkyl-(C₁-C₇)-alkyle, (C₄-C₇)-cycloalcényle, (C₂-C₇)-alcynyle, (C₂-C₇)-alcényle, (C₂-C₇)-halogénoalcényle, (C₂-C₇)-halogénoalcynyle, (C₁-C₇)-alkylsulfinyle, (C₁-C₇)-alkylsulfonyle, (C₃-C₇)-cycloalkylsulfinyle, (C₃-C₇)-cycloalkylsulfonyle, arylsulfinyle, arylsulfonyle, (C₁-C₇)-alcoxy-(C₁-C₇)-halogénoalkyle, (C₁-C₇)-halogénoalcoxy-(C₁-C₇)-halogénoalkyle ou
R³ et R⁴ forment, ensemble avec l'atome auquel ils sont liés, un cycle de 3 à 6 chaînons complètement saturé, le cas échéant interrompu par des hétéroatomes et le cas échéant substitué davantage,
R⁵ représente hydrogène, (C₁-C₇)-alkyle, (C₂-C₇)-alcényle, (C₂-C₇)-alcynyle, (C₁-C₇)-alcoxy-(C₁-C₇)-alkyle, (C₁-C₇)-alkylcarbonyle, arylcarbonyle, hétéroarylcarbonyle, (C₃-C₇)-cycloalkylcarbonyle, (C₁-C₇)-alcoxycarbonyle, (C₂-C₇)-alcényloxycarbonyle, (C₁-C₇)-alcoxycarbonyl-(C₁-C₇)-alkyle, (C₁-C₇)-alkylaminocarbonyl-(C₁-C₇)-alkyle, arylaminocarbonyl-(C₁-C₇)-alkyle, aryloxy-(C₁-C₇)-alkyle, aryl-(C₁-C₇)-alkyle, hétéroaryl- (C₁-C₇)-alkyle, hétérocyclyle, hétérocyclyl-(C₁-C₇)-alkyle,
R⁶ et R⁷ représentent, indépendamment l'un de l'autre, hydrogène, nitro, amino, cyano, thiocyanato, isothiocyanato, halogène, (C₁-C₇)-alkyle, (C₃-C₇)-cycloalkyle, (C₂-C₇)-alcényle, (C₂-C₇)-alcynyle, aryle, aryl- (C₁-C₇)-alkyle, aryl- (C₁-C₇)-alcoxy, hétéroaryle, (C₁-C₇)-alcoxy- (C₁-C₇)-alkyle, hydroxy-(C₁-C₇)-alkyle, (C₁-C₇)-halogénoalkyle, (C₃-C₇)-halogénocycloalkyle, (C₁-C₇)-alcoxy, (C₁-C₇)-halogénoalcoxy, aryloxy, hétéroaryloxy, (C₃-C₇)-cycloalkyloxy, hydroxy, (C₃-C₇)-cycloalkyl- (C₁-C₇)-alcoxy, (C₁-C₇)-alcoxycarbonyle, hydroxycarbonyle, aminocarbonyle, (C₁-C₇)-alkylaminocarbonyle, (C₃-C₇)-cycloalkylaminocarbonyle, cyano-(C₁-C₇)-alkylaminocarbonyle, (C₂-C₇)-alcénylaminocarbonyle, (C₂-C₇)-alcynylaminocarbonyle, (C₁-C₇)-alkylamino, (C₁-C₇)-alkylthio, (C₁-C₇)-halogénoalkylthio, hydrothio, bis-[(C₁-C₇)-alkyl]amino, (C₃-C₇)-cycloalkylamino, (C₁-C₇)-alkylcarbonylamino, (C₃-C₇)-cycloalkylcarbonylamino, formylamino, (C₁-C₇)-halogénoalkylcarbonylamino, (C₁-C₇)-alcoxycarbonylamino, (C₁-C₇)-alkylaminocarbonylamino, (C₁-C₇)-alkyl[(C₁-C₇)-alkyl]aminocarbonylamino, (C₁-C₇)-alkylsulfonylamino, (C₃-C₇)-cycloalkylsulfonylamino, arylsulfonylamino, hétarylsulfonylamino, sulfonyl-(C₁-C₇)-halogénoalkylamino, aminosulfonyle, amino-(C₁-C₇)-alkylsulfonyle, amino-(C₁-C₇)-halogénoalkylsulfonyle, (C₁-C₇)-alkylaminosulfonyle, bis[(C₁-C₇)-alkyl]aminosulfonyle, (C₃-C₇)-cycloalkylaminosulfonyle, (C₁-C₇)-halogénoalkylaminosulfonyle, arylaminosulfonyle, aryl- (C₁-C₇)-alkylaminosulfonyle, (C₁-C₇)-alkylsulfonyle, (C₃-C₇)-cycloalkylsulfonyle, arylsulfonyle, (C₁-C₇)-alkylsulfinyle, (C₃-C₇)-cycloalkylsulfinyle, arylsulfinyle, N,S-bis[(C₁-C₇)-alkyl]sulfonimidoyle, S-(C₁-C₇)-alkylsulfonimidoyle, (C₁-C₇)-alkylsulfonylaminocarbonyle, (C₃-C₇)-cycloalkylsulfonylaminocarbonyle, (C₃-C₇)-cycloalkylaminosulfonyle, aryl-(C₁-C₇)-alkylcarbonylamino, (C₃-C₇)-cycloalkyl- (C₁-C₇)-alkylcarbonylamino, hétéroarylcarbonylamino, (C₁-C₇)-alcoxy-(C₁-C₇)-alkylcarbonylamino, hydroxy-(C₁-C₇)-alkylcarbonylamino, tris[(C₁-C₇)-alkyl]silyle,
A³, A⁴, A⁵ sont identiques ou différents et représentent, indépendamment les uns des autres, N (azote) ou le groupement C-R⁸, où, dans aucun cas, plus de deux atomes de N ne sont adjacents et R⁸ dans le groupement C-R⁸ présente à chaque fois des significations identiques ou différentes selon la définition ci-dessous et
A³ et A⁴ forment, lorsque les deux représentent un groupe C-R⁸, ensemble avec les atomes auxquels ils sont liés, un cycle de 5 à 6 chaînons complètement saturé, partiellement saturé ou complètement insaturé, le cas échéant interrompu par des hétéroatomes et le cas échéant substitué davantage,
A⁴ et A⁵ forment, lorsque les deux représentent un groupe C-R⁸, ensemble avec les atomes auxquels ils sont liés, un cycle de 5 à 6 chaînons complètement saturé, partiellement saturé ou complètement insaturé, le cas échéant interrompu par des hétéroatomes et le cas échéant substitué davantage,
R⁸ et R¹⁴ représentent, indépendamment l'un de l'autre, respectivement hydrogène, nitro, amino, hydroxy, hydrothio, thiocyanato, isothiocyanato, halogène, (C₁-C₇)-alkyle, (C₃-C₇)-cycloalkyle, (C₂-C₇)-alcényle, (C₂-C₇)-alcynyle, aryle, aryl- (C₁-C₇)-alkyle, aryl-(C₁-C₇)-alcoxy, hétéroaryle, (C₁-C₇)-halogénoalkyle, (C₃-C₇)-halogénocycloalkyle, (C₁-C₇)-alcoxy, (C₁-C₇)-halogénoalcoxy, aryloxy, hétéroaryloxy, (C₃-C₇)-cycloalkyloxy, (C₃-C₇)-cycloalkyl-(C₁-C₇)-alcoxy, hydroxy-(C₁-C₇)-alkyle, (C₁-C₇)-alcoxy-(C₁-C₇)-alkyle, aryloxy-(C₁-C₇)-alkyle, hétéroaryloxy-(C₁-C₇)-alkyle, (C₁-C₇)-alkylamino, (C₁-C₇)-alkylthio, (C₁-C₇)-halogénoalkylthio, bis[(C₁-C₇)-alkyl]amino, (C₃-C₇)-cycloalkylamino, (C₁-C₇)-alkylcarbonylamino, (C₃-C₇)-cycloalkylcarbonylamino, formylamino, (C₁-C₇)-halogénoalkylcarbonylamino, (C₁-C₇)-alcoxycarbonylamino, (C₁-C₇)-alkylaminocarbonylamino, (C₁-C₇)-alkyl[(C₁-C₇)-alkyl]aminocarbonylamino, (C₁-C₇)-alkylsulfonylamino, (C₃-C₇)-cycloalkylsulfonylamino, arylsulfonylamino, hétarylsulfonylamino, sulfonyl-(C₁-C₇)-halogénoalkylamino, amino-(C₁-C₇)-alkylsulfonyle, amino-(C₁-C₇)-halogénoalkylsulfonyle, (C₁-C₇)-alkylaminosulfonyle, bis[(C₁-C₇)-alkyl]aminosulfonyle, (C₃-C₇)-cycloalkylaminosulfonyle, (C₁-C₇)-halogénoalkylaminosulfonyle, arylaminosulfonyle, aryl-(C₁-C₇)-alkylaminosulfonyle, (C₁-C₇)-alkylsulfonyle, (C₃-C₇)-cycloalkylsulfonyle, arylsulfonyle, (C₁-C₇)-alkylsulfinyle, (C₃-C₇)-cycloalkylsulfinyle, arylsulfinyle, N,S-bis[(C₁-C₇)-alkyl]sulfonimidoyle, S-(C₁-C₇)-alkylsulfonimidoyle, (C₁-C₇)-alkylsulfonylaminocarbonyle, (C₃-C₇)-cycloalkylsulfonylaminocarbonyle, (C₃-C₇)-cycloalkylaminosulfonyle, aryl-(C₁-C₇)-alkylcarbonylamino, (C₃-C₇)-cycloalkyl-(C₁-C₇)-alkylcarbonylamino, hétéroarylcarbonylamino, (C₁-C₇)-alcoxy-(C₁-C₇)-alkylcarbonylamino, hydroxy-(C₁-C₇)-alkylcarbonylamino, cyano, cyano-(C₁-C₇)-alkyle, hydroxycarbonyle, (C₁-C₇)-alcoxycarbonyle, (C₃-C₇)-cycloalcoxycarbonyle, (C₃-C₇)-cycloalkyl-(C₁-C₇)-alcoxycarbonyle, aryloxycarbonyle, aryl-(C₁-C₇)-alcoxycarbonyle, aminocarbonyle, (C₁-C₇)-alkylaminocarbonyle, bis[(C₁-C₇)-alkyl]aminocarbonyle, (C₁-C₇)-alkyl[(C₁-C₇)-alcoxy]aminocarbonyle, (C₃-C₇)-cycloalkylaminocarbonyle, aryl-(C₁-C₇)-alkylaminocarbonyle, hétéroaryl-(C₁-C₇)-alkylaminocarbonyle, cyano-(C₁-C₇)-alkylaminocarbonyle, (C₁-C₇)-halogénoalkylaminocarbonyle, (C₂-C₇)-alcynylaminocarbonyle, (C₁-C₇)-alcoxycarbonylaminocarbonyle, aryl-(C₁-C₇)-alcoxycarbonylaminocarbonyle, hydroxycarbonyl-(C₁-C₇)-alkyle, (C₁-C₇)-alcoxycarbonyl-(C₁-C₇)-alkyle, (C₃-C₇)-cycloalcoxycarbonyl-(C₁-C₇)-alkyle, (C₃-C₇)-cycloalkyl-(C₁-C₇)-alcoxycarbonyl-(C₁-C₇)-alkyle, (C₁-C₇)-alkylaminocarbonyl-(C₁-C₇)-alkyle, aminocarbonyl-(C₁-C₇)-alkyle, bis[(C₁-C₇)-alkyl]aminocarbonyl-(C₁-C₇)-alkyle, (C₃-C₇)-cycloalkylaminocarbonyl-(C₁-C₇)-alkyle, aryl-(C₁-C₇)-alkylaminocarbonyl-(C₁-C₇)-alkyle, hétéroaryl-(C₁-C₇)-alkylaminocarbonyl-(C₁-C₇)-alkyle, cyano-(C₁-C₇)-alkylaminocarbonyl-(C₁-C₇)-alkyle, (C₁-C₇)-halogénoalkylaminocarbonyl-(C₁-C₇)-alkyle, (C₂-C₇)-alcynylaminocarbonyl-(C₁-C₇)-alkyle, (C₃-C₇)-cycloalkyl-(C₁-C₇)-alkylaminocarbonyl-(C₁-C₇)-alkyle, (C₁-C₇)-alcoxycarbonylaminocarbonyl-(C₁-C₇)-alkyle, aryl-(C₁-C₇)-alcoxycarbonylaminocarbonyl-(C₁-C₇)-alkyle, (C₁-C₇)-alcoxycarbonyl-(C₁-C₇)-alkylaminocarbonyle, hydroxycarbonyl-(C₁-C₇)-alkylaminocarbonyle, aminocarbonyl-(C₁-C₇)-alkylaminocarbonyle, (C₁-C₇)-alkylaminocarbonyl-(C₁-C₇)-alkylaminocarbonyle, (C₃-C₇)-cycloalkylaminocarbonyl-(C₁-C₇)-alkylaminocarbonyle, (C₃-C₇)-cycloalkyl-(C₁-C₇)-alkylaminocarbonyle, (C₃-C₇)-cycloalkyl- (C₁-C₇)-alkylaminocarbonyl-(C₁-C₇)-alkyle, (C₂-C₇)-alcényloxycarbonyle, (C₂-C₇)-alcényloxycarbonyl-(C₁-C₇)-alkyle, (C₂-C₇)-alcénylaminocarbonyle, (C₂-C₇)-alcénylaminocarbonyl-(C₁-C₇)-alkyle, (C₁-C₇)-alkylcarbonyle, (C₃-C₇)-cycloalkylcarbonyle, formyle, hydroxyiminométhyle, amino-iminométhyle, (C₁-C₇)-alcoxyiminométhyle, (C₁-C₇)-alkylamino-iminométhyle, bis[(C₁-C₇)-alkyl]amino-iminométhyle, (C₃-C₇)-cycloalcoxyiminométhyle, (C₃-C₇)-cycloalkyl-(C₁-C₇)-alcoxyiminométhyle, aryloxyiminométhyle, aryl-(C₁-C₇)-alcoxyiminométhyle, aryl-(C₁-C₇)-alkylamino-iminométhyle, (C₂-C₇)-alcényloxyiminométhyle, arylamino-iminométhyle, arylsulfonylamino-iminométhyle, hétéroaryl-(C₁-C₇)-alkyle, hétérocyclyl-(C₁-C₇)-alkyle, hydroxycarbonylhétérocyclyle, (C₁-C₇)-alcoxycarbonylhétérocyclyle, (C₂-C₇)-alcényloxycarbonylhétérocyclyle, (C₂-C₇)-alcényl-(C₁-C₇)-alcoxycarbonylhétérocyclyle, aryl-(C₁-C₇)-alcoxycarbonylhétérocyclyle, (C₃-C₇)-cycloalcoxycarbonylhétérocyclyle, (C₃-C₇)-cycloalkyl-(C₁-C₇)-alcoxycarbonylhétérocyclyle, aminocarbonylhétérocyclyle, (C₁-C₇)-alkylaminocarbonylhétérocyclyle, bis-[(C₁-C₇)-alkyl]aminocarbonylhétérocyclyle, (C₃-C₇)-cycloalkylaminocarbonylhétérocyclyle, aryl-(C₁-C₇)-alkylaminocarbonylhétérocyclyle, (C₂-C₇)-alcénylaminocarbonylhétérocyclyle, hydroxycarbonylhétérocyclyl-(C₁-C₇)-alkyle, (C₁-C₇)-alcoxycarbonylhétérocyclyl-(C₁-C₇)-alkyle, hydroxycarbonyl-(C₃-C₇)-cycloalkyl-(C₁-C₇)-alkyle, (C₁-C₇)-alcoxycarbonyl-(C₃-C₇)-cycloalkyl-(C₁-C₇)-alkyle, hétérocyclyle, hétérocyclyloxy, hétérocyclylamino, hétérocyclylcarbonyle, hétérocyclylcarbonyl-(C₁-C₇)-alkyle, hydroxycarbonylhétérocyclylcarbonyle, (C₁-C₇)-alcoxycarbonylhétérocyclylcarbonyle, (C₂-C₇)-alcényloxycarbonylhétérocyclylcarbonyle, (C₂-C₇)-alcényl-(C₁-C₇)-alcoxycarbonylhétérocyclylcarbonyle, aryl-(C₁-C₇)-alcoxycarbonylhétérocyclylcarbonyle, (C₃-C₇)-cycloalcoxycarbonylhétérocyclylcarbonyle, (C₃-C₇)-cycloalkyl-(C₁-C₇)-alcoxycarbonylhétérocyclylcarbonyle, aminocarbonylhétérocyclylcarbonyle, (C₁-C₇)-alkylaminocarbonylhétérocyclylcarbonyle, bis-[(C₁-C₇)-alkyl]aminocarbonylhétérocyclylcarbonyle, (C₃-C₇)-cycloalkylaminocarbonylhétérocyclylcarbonyle, aryl- (C₁-C₇)-alkylaminocarbonylhétérocyclylcarbonyle, (C₂-C₇)-alcénylaminocarbonylhétérocyclylcarbonyle,
R⁹ et R¹⁰ représentent, indépendamment l'un de l'autre, hydrogène, halogène, (C₁-C₇)-alkyle, (C₁-C₇)-alcoxy, (C₁-C₇)-halogénoalkyle, (C₁-C₇)-halogénoalcoxy, aryle, hétéroaryle, aryl-(C₁-C₇)-alkyle ou forment, ensemble avec l'atome auquel ils sont liés, un groupe carbonyle,
A⁶, A⁷ sont identiques ou différents et représentent, indépendamment l'un de l'autre, N-R¹¹, oxygène, soufre ou le groupement CR¹⁵R¹⁶, où, dans aucun cas, plus d'un atome d'oxygène n'est contenu dans l'hétérocycle et R¹¹, R¹⁵, R¹⁶ dans les groupements N-R¹¹ et CR¹⁵R¹⁶ présentent respectivement des significations identiques ou différentes selon la définition ci-dessous,
R¹¹ représente hydrogène, (C₁-C₇)-alkyle, (C₂-C₇)-alcényle, (C₂-C₇)-alcynyle, (C₁-C₇)-alcoxy- (C₁-C₇)-alkyle, (C₁-C₇)-alkylcarbonyle, arylcarbonyle, hétéroarylcarbonyle, (C₃-C₇)-cycloalkylcarbonyle, (C₁-C₇)-alcoxycarbonyle, (C₂-C₇)-alcényloxycarbonyle, (C₁-C₇)-alcoxycarbonyl-(C₁-C₇)-alkyle, (C₁-C₇)-alkylaminocarbonyl-(C₁-C₇)-alkyle, arylaminocarbonyl-(C₁-C₇)-alkyle, aryloxy-(C₁-C₇)-alkyle, aryl- (C₁-C₇)-alkyle, hétéroaryl- (C₁-C₇)-alkyle, hétérocyclyle, hétérocyclyl-(C₁-C₇)-alkyle,
R¹² et R¹³ représentent, indépendamment l'un de l'autre, hydrogène, halogène, (C₁-C₇)-alkyle, (C₁-C₇)-alcoxy, (C₁-C₇)-halogénoalkyle, (C₁-C₇)-halogénoalcoxy, aryle, hétéroaryle, aryl-(C₁-C₇)-alkyle, hétérocyclyle, hétéroaryl-(C₁-C₇)-alkyle, hétérocyclyl-(C₁-C₇)-alkyle ou forment, ensemble avec l'atome auquel ils sont liés, un groupe carbonyle et
R¹⁵ et R¹⁶ représentent, indépendamment l'un de l'autre, hydrogène, (C₁-C₇)-alkyle, (C₂-C₇)-alcényle, (C₂-C₇)-alcynyle, (C₁-C₇)-alcoxy, (C₁-C₇)-alcoxy-(C₁-C₇)-alkyle, (C₁-C₇)-alkylcarbonyle, arylcarbonyle, hétéroarylcarbonyle, hydroxycarbonyle, (C₁-C₇)-alcoxycarbonyle, hétérocyclyle, (C₁-C₇)-halogénoalkyle,
à l'exception du composé 1-[(2E)-1-hydroxy-1,3-diphénylprop-2-én-1-yl]cyclopentane-carbonitrile.

4. Utilisation d'un ou de plusieurs composés de formule (I) ou de ses/leurs sels selon l'une quelconque des revendications 1 à 3 ainsi que du composé 1-[(2E)-1-hydroxy-1,3-diphénylprop-2-én-1-yl]cyclopentane-carbonitrile pour l'augmentation de la tolérance au stress abiotique dans les plantes.

5. Traitement de plantes, comprenant l'application d'une quantité non toxique, active pour l'augmentation de la résistance de plantes à des facteurs de stress abiotique, d'un ou de plusieurs composés de formule (I) ou de ses/leurs sels selon l'une quelconque des revendications 1 à 3 ainsi que du composé 1-[(2E)-1-hydroxy-1,3-diphénylprop-2-én-1-yl]cyclopentane-carbonitrile.

6. Traitement selon la revendication 5, les conditions de stress abiotique correspondant à une ou à plusieurs conditions choisies dans le groupe formé par la chaleur, l'aridité, le stress par le froid et la sécheresse, le stress osmotique, l'engorgement hydrique, une teneur augmentée en sels dans le sol, l'exposition augmentée aux minéraux, les conditions d'ozone, les conditions de lumière forte, la disponibilité limitée de substances nutritives azotées, la disponibilité limitée de substances nutritives phosphorées.

7. Utilisation d'un ou de plusieurs composés de formule générale (I) ou de ses/leurs sels selon l'une quelconque des revendications 1 à 3 ainsi que du composé 1-[(2E)-1-hydroxy-1,3-diphénylprop-2-én-1-yl]cyclopentane-carbonitrile dans l'application par pulvérisation sur des plantes et des parties de plante en combinaison avec une ou plusieurs substances actives du groupe formé par les insecticides, les appâts, les acaricides, les fongicides, les nématicides, les herbicides, les substances de régulation de la croissance, les phytoprotecteurs, les substances influençant la maturité des plantes et les bactéricides.

8. Utilisation d'un ou de plusieurs composés de formule générale (I) ou de ses/leurs sels selon l'une quelconque des revendications 1 à 3 ainsi que du composé 1-[(2E)-1-hydroxy-1,3-diphénylprop-2-én-1-yl]cyclopentane-carbonitrile dans l'application par pulvérisation sur des plantes et des parties de plante en combinaison avec des engrais.

9. Utilisation d'un ou de plusieurs composés de formule générale (I) ou de ses/leurs sels selon l'une quelconque des revendications 1 à 3 ainsi que du composé 1-[(2E)-1-hydroxy-1,3-diphénylprop-2-én-1-yl]cyclopentane-carbonitrile pour l'application sur des variétés génétiquement modifiées, leurs semences ou sur des surfaces de culture sur lesquelles ces variétés croissent.

10. Solution de pulvérisation pour le traitement de plantes, contenant une quantité active pour l'augmentation de la résistance de plantes à des facteurs de stress abiotique, d'un ou de plusieurs composés de formule générale (I) selon l'une quelconque des revendications 1 à 3 ainsi que du composé 1-[(2E)-1-hydroxy-1,3-diphénylprop-2-én-1-yl]cyclopentane-carbonitrile ou leurs sels.

11. Utilisation de solutions de pulvérisation, qui contiennent un ou plusieurs des composés de formule générale (I) selon l'une quelconque des revendications 1 à 3 ainsi que le composé 1-[(2E)-1-hydroxy-1,3-diphénylprop-2-én-1-yl]cyclopentane-carbonitrile ou leurs sels, pour augmenter la résistance de plantes à des facteurs de stress abiotique.

12. Procédé pour augmenter la tolérance au stress sur des plantes choisies dans le groupe des plantes utiles, des plantes décoratives, des types de gazon ou des arbres, qui comprend l'application d'une quantité suffisante, non toxique d'un ou de plusieurs composés de formule générale (I) selon l'une quelconque des revendications 1 à 3 ainsi que du composé 1-[(2E)-1-hydroxy-1,3-diphénylprop-2-én-1-yl]cyclopentane-carbonitrile ou de leurs sels respectifs sur la surface où l'action correspondante est souhaitée, l'application ayant lieu sur les plantes, leurs semences ou sur la surface sur laquelle les plantes croissent.

13. Procédé selon la revendication 12, l'aptitude à la résistance des plantes ainsi traitées au stress abiotique étant augmentée d'au moins 3% par rapport aux plantes non traitées dans des conditions physiologiques pour le reste identiques.

14. Composés de formule générale (II) ou leurs sels 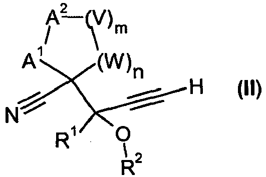 dans laquelle
R¹ représente hydrogène, (C₁-C₈)-alkyle, aryle, hétéroaryle, hétérocyclyle, (C₃-C₈)-cycloalkyle, (C₂-C₈)-alcényle, (C₂-C₈)-alcynyle, (C₂-C₈)-alcynyl-(C₁-C₈)-alkyle, (C₁-C₈)-alcoxy-(C₁-C₈)-alkyle, hydroxy-(C₁-C₈)-alkyle, (C₁-C₈)-halogénoalkyle, (C₂-C₈)-halogénoalcényle, (C₁-C₈)-halogénoalcoxy-(C₁-C₈)-alkyle, (C₁-C₈)-alkylthio-(C₁-C₈)-alkyle, aryl-(C₁-C₈)-alkyle, hétérocyclyl-(C₁-C₈)-alkyle, (C₃-C₈)-halogénocycloalkyle, (C₄-C₈)-cycloalcényle, (C₁-C₈)-alcoxy-(C₁-C₈)-alcoxy-(C₁-C₈)-alkyle, (C₁-C₈)-alcoxy-(C₁-C₈)-halogénoalkyle, (C₁-C₈)-halogénoalcoxy-(C₁-C₈)-halogénoalkyle, (C₃-C₈)-cycloalkyl-(C₁-C₈)-alkyle,
R² représente hydrogène, (C₁-C₈)-alkyle, (C₁-C₈)-alcoxy-(C₁-C₈)-alkyle, (C₁-C₈)-alcoxy-(C₁-C₈)-alcoxy-(C₁-C₈)-alkyle, (C₂-C₈)-alcényle, (C₂-C₈)-alcynyle, (C₁-C₈)-alkylcarbonyle, arylcarbonyle, hétéroarylcarbonyle, (C₃-C₈)-cycloalkylcarbonyle, (C₂-C₈)-alcénylcarbonyle, hétérocyclylcarbonyle, (C₁-C₈)-alcoxycarbonyle, (C₂-C₈)-alcényloxycarbonyle, aryloxy-(C₁-C₈)-alkyle, aryl-(C₁-C₈)-alcoxycarbonyle, (C₃-C₈)-cycloalcoxycarbonyle, (C₃-C₈)-cycloalkyl-(C₁-C₈)-alcoxycarbonyle, aryl-(C₁-C₈)-alcoxy-(C₁-C₈)-alkyle, aryl-(C₁-C₈)-alkyle, (C₁-C₈)-alcoxy-(C₁-C₈)-alcoxy-(C₁-C₈)-alkyle, (C₁-C₈)-alkylthio-(C₁-C₈)-alkyle, tris[(C₁-C₈)-alkyl]silyle, (C₁-C₈)-alkyl-bis-[(C₁-C₈)-alkyl]silyle, (C₁-C₈)-alkyl-bis(aryl)silyle, aryl-bis[(C₁-C₈)-alkyl]silyle, (C₃-C₈)-cycloalkyl-bis[(C₁-C₈)-alkyl]silyle, halogéno-bis[(C₁-C₈)-alkyl]silyle, tris[(C₁-C₈)-alkyl]silyl-(C₁-C₈)-alcoxy-(C₁-C₈)-alkyle, tris[(C₁-C₈)-alkyl]silyl-(C₁-C₃)-alkyle,
A¹, A², V, W représentent, indépendamment les uns des autres, un groupe CR³R⁴, un groupe N-R⁵, oxygène ou soufre, au maximum 2 atomes d'oxygène ou 2 atomes de soufre étant à chaque fois présents dans le cycle formé par les groupes A¹, A², V, W et l'atome de carbone auquel ils sont liés et les différents atomes d'oxygène et de soufre n'étant pas adjacents,
m représente 0, 1, 2,
n représente 0, 1, 2 et
R³ et R⁴ représentent, indépendamment l'un de l'autre, hydrogène, (C₁-C₈)-alkyle, halogène, (C₃-C₈)-cycloalkyle, (C₁-C₈)-alcoxy, aryle, hétérocyclyle, hétéroaryle, aryl-(C₁-C₈)-alkyle, (C₁-C₈)-alkylthio, (C₁-C₈)-halogénoalkyle, (C₁-C₈)-halogénoalkyloxy, (C₁-C₈)-halogénoalkylthio, (C₁-C₈)-alcoxy-(C₁-C₈)-alkyle, (C₁-C₈)-alkylthio-(C₁-C₈)-alkyle, hétéroaryl-(C₁-C₈)-alkyle, hétérocyclyl-(C₁-C₈)-alkyle, (C₁-C₈)-alcoxy-(C₁-C₈)-halogénoalkyle, (C₁-C₈)-halogénoalcoxy-(C₁-C₈)-halogénoalkyle, (C₂-C₈)-alcényle, (C₂-C₈)-alcynyle ou
R³ et R⁴ forment, ensemble avec l'atome auquel ils sont liés, un cycle de 3 à 6 chaînons complètement saturé, le cas échéant interrompu par des hétéroatomes et le cas échéant substitué davantage.

15. Composés de formule (II) selon la revendication 14, dans laquelle
R¹ représente hydrogène, (C₁-C₆)-alkyle, aryle, hétéroaryle, hétérocyclyle, (C₃-C₆)-cycloalkyle, (C₂-C₆)-alcényle, (C₂-C₆)-alcynyle, (C₂-C₆)-alcynyl-(C₁-C₆)-alkyle, (C₁-C₆)-alcoxy-(C₁-C₆)-alkyle, hydroxy-(C₁-C₆)-alkyle, (C₁-C₆)-halogénoalkyle, (C₂-C₆)-halogénoalcényle, (C₁-C₆)-halogénoalcoxy-(C₁-C₆)-alkyle, (C₁-C₆)-alkylthio-(C₁-C₆)-alkyle, aryl-(C₁-C₆)-alkyle, hétérocyclyl-(C₁-C₆)-alkyle, (C₃-C₆) -halogénocycloalkyle, (C₄-C₆)-cycloalcényle, (C₁-C₆)-alcoxy-(C₁-C₆)-alcoxy-(C₁-C₆)-alkyle, (C₁-C₆)-alcoxy-(C₁-C₆)-halogénoalkyle, (C₁-C₆)-halogénoalcoxy-(C₁-C₆)-halogénoalkyle, (C₃-C₆)-cycloalkyl-(C₁-C₆)-alkyle,
R² représente hydrogène, (C₁-C₆)-alkyle, (C₁-C₆)-alcoxy-(C₁-C₆)-alkyle, (C₁-C₆)-alcoxy-(C₁-C₆)-alcoxy-(C₁-C₆)-alkyle, (C₂-C₆)-alcényle, (C₂-C₆)-alcynyle, (C₁-C₆)-alkylcarbonyle, arylcarbonyle, hétéroarylcarbonyle, (C₃-C₆)-cycloalkylcarbonyle, (C₂-C₆)-alcénylcarbonyle, hétérocyclylcarbonyle, (C₁-C₆)-alcoxycarbonyle, (C₂-C₆)-alcényloxycarbonyle, aryloxy-(C₁-C₆)-alkyle, aryl-(C₁-C₆)-alcoxycarbonyle, (C₃-C₆)-cycloalcoxycarbonyle, (C₃-C₆)-cycloalkyl-(C₁-C₆)-alcoxycarbonyle, aryl-(C₁-C₆)-alcoxy-(C₁-C₆)-alkyle, aryl-(C₁-C₆)-alkyle, (C₁-C₆)-alcoxy-(C₁-C₆)-alcoxy-(C₁-C₆)-alkyle, (C₁-C₆)-alkylthio-(C₁-C₆)-alkyle, tris[(C₁-C₆)-alkyl] silyle, (C₁-C₆)-alkyl-bis-[(C₁-C₆)-alkyl]silyle, (C₁-C₆)-alkyl-bis(aryl)silyle, aryl-bis[(C₁-C₆)-alkyl]silyle, (C₃-C₆)-cycloalkyl-bis[(C₁-C₆)-alkyl]silyle, halogéno-bis[(C₁-C₆)-alkyl]silyle, tris[(C₁-C₆)-alkyl]silyl-(C₁-C₆)-alcoxy-(C₁-C₆)-alkyle, tris[(C₁-C₆)-alkyl]silyl-(C₁-C₆)-alkyle,
A¹, A², V, W représentent, indépendamment les uns des autres, un groupe CR³R⁴, un groupe N-R⁵, oxygène ou soufre, au maximum 2 atomes d'oxygène ou 2 atomes de soufre étant à chaque fois présents dans le cycle formé par les groupes A¹, A², V, W et l'atome de carbone auquel ils sont liés et les différents atomes d'oxygène et de soufre n'étant pas adjacents,
m représente 0, 1, 2,
n représente 0, 1, 2 et
R³ et R⁴ représentent, indépendamment l'un de l'autre hydrogène, (C₁-C₆)-alkyle, halogène, (C₃-C₆)-cycloalkyle, (C₁-C₆)-alcoxy, aryle, hétérocyclyle, hétéroaryle, aryl- (C₁-C₆)-alkyle, (C₁-C₆)-alkylthio, (C₁-C₆)-halogénoalkyle, (C₁-C₆)-halogénoalkyloxy, (C₁-C₆)-halogénoalkylthio, (C₁-C₆)-alcoxy-(C₁-C₆)-alkyle, (C₁-C₆)-alkylthio-(C₁-C₆)-alkyle, hétéroaryl-(C₁-C₆)-alkyle, hétérocyclyl-(C₁-C₆)-alkyle, (C₁-C₆)-alcoxy-(C₁-C₆)-halogénoalkyle, (C₁-C₆)-halogénoalcoxy-(C₁-C₆)-halogénoalkyle, (C₂-C₆)-alcényle, (C₂-C₆)-alcynyle ou
R³ et R⁴ forment, ensemble avec l'atome auquel ils sont liés, un cycle de 3 à 6 chaînons complètement saturé, le cas échéant interrompu par des hétéroatomes et le cas échéant substitué davantage.

16. Composés de formule générale (III) ou leurs sels 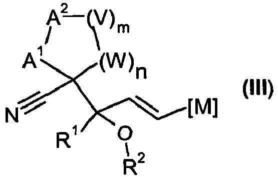 dans laquelle
R¹ représente hydrogène, (C₁-C₈)-alkyle, aryle, hétéroaryle, hétérocyclyle, (C₃-C₈)-cycloalkyle, (C₂-C₈)-alcényle, (C₂-C₈)-alcynyle, (C₂-C₈)-alcynyl-(C₁-C₈)-alkyle, (C₁-C₈)-alcoxy-(C₁-C₈)-alkyle, hydroxy-(C₁-C₈)-alkyle, (C₁-C₈)-halogénoalkyle, (C₂-C₈)-halogénoalcényle, (C₁-C₈)-halogénoalcoxy-(C₁-C₈)-alkyle, (C₁-C₈)-alkylthio-(C₁-C₈)-alkyle, aryl-(C₁-C₈)-alkyle, hétérocyclyl-(C₁-C₈)-alkyle, (C₃-C₈)-halogénocycloalkyle, (C₄-C₈)-cycloalcényle, (C₁-C₈)-alcoxy-(C₁-C₈)-alcoxy-(C₁-C₈)-alkyle, (C₁-C₈)-alcoxy-(C₁-C₈)-halogénoalkyle, (C₁-C₈)-halogénoalcoxy-(C₁-C₈)-halogénoalkyle,
R² représente hydrogène, (C₁-C₈)-alkyle, (C₁-C₈)-alcoxy-(C₁-C₈)-alkyle, (C₁-C₈)-alcoxy-(C₁-C₈)-alcoxy-(C₁-C₈)-alkyle, (C₂-C₈)-alcényle, (C₂-C₈)-alcynyle, (C₁-C₈)-alkylcarbonyle, arylcarbonyle, hétéroarylcarbonyle, (C₃-C₈)-cycloalkylcarbonyle, (C₂-C₈)-alcénylcarbonyle, hétérocyclylcarbonyle, (C₁-C₈)-alcoxycarbonyle, (C₂-C₈)-alcényloxycarbonyle, aryloxy-(C₁-C₈)-alkyle, aryl-(C₁-C₈)-alcoxycarbonyle, (C₃-C₈)-cycloalcoxycarbonyle, (C₃-C₈)-cycloalkyl-(C₁-C₈)-alcoxycarbonyle, aryl-(C₁-C₈)-alcoxy-(C₁-C₈)-alkyle, aryl-(C₁-C₈)-alkyle, (C₁-C₈)-alcoxy-(C₁-C₈)-alcoxy-(C₁-C₈)-alkyle, (C₁-C₈)-alkylthio-(C₁-C₈)-alkyle, tris[(C₁-C₈)-alkyl]silyle, (C₁-C₈)-alkyl-bis-[(C₁-C₈)-alkyl]silyle, (C₁-C₈)-alkyl-bis(aryl)silyle, aryl-bis[(C₁-C₈)-alkyl]silyle, (C₃-C₈)-cycloalkyl-bis[(C₁-C₈)-alkyl]silyle, halogéno-bis[(C₁-C₈)-alkyl]silyle, tris[(C₁-C₈)-alkyl]silyl-(C₁-C₈)-alcoxy-(C₁-C₈)-alkyle, tris[(C₁-C₈)-alkyl]silyl-(C₁-C₈)-alkyle,
A¹, A², V, W représentent, indépendamment les uns des autres, un groupe CR³R⁴, un groupe N-R⁵, oxygène ou soufre, au maximum 2 atomes d'oxygène ou 2 atomes de soufre étant à chaque fois présents dans le cycle formé par les groupes A¹, A², V, W et l'atome de carbone auquel ils sont liés et les différents atomes d'oxygène et de soufre n'étant pas adjacents,
m représente 0, 1, 2,
n représente 0, 1, 2,
R³ et R⁴ représentent, indépendamment l'un de l'autre, hydrogène, (C₁-C₈)-alkyle, halogène, (C₃-C₈)-cycloalkyle, (C₁-C₈)-alcoxy, aryle, hétérocyclyle, hétéroaryle, aryl-(C₁-C₈)-alkyle, (C₁-C₈)-alkylthio, (C₁-C₈)-halogénoalkyle, (C₁-C₈)-halogénoalkyloxy, (C₁-C₈)-halogénoalkylthio, (C₁-C₈)-alcoxy-(C₁-C₈)-alkyle, (C₁-C₈)-alkylthio-(C₁-C₈)-alkyle, hétéroaryl-(C₁-C₈)-alkyle, hétérocyclyl-(C₁-C₈)-alkyle, (C₁-C₈)-alcoxy-(C₁-C₈)-halogénoalkyle, (C₁-C₃)-halogénoalcoxy-(C₁-C₈)-halogénoalkyle, (C₂-C₈)-alcényle, (C₂-C₈)-alcynyle ou
R³ et R⁴ forment, ensemble avec l'atome auquel ils sont liés, un cycle de 3 à 6 chaînons complètement saturé, le cas échéant interrompu par des hétéroatomes et le cas échéant substitué davantage et
[M] représente tris-[(C₁-C₆)-alkyl]stannyle, tris-[(C₃-C₈)-cycloalkyl]stannyle, tris-[(C₁-C₆)-alkyl]germanyle, tris-[(C₃-C₈)-cycloalkyl]germanyle, bis-(cyclopentadiényl)zirconyle, bis-(1,2,3,4,5-pentaméthylcyclopentadiényl)zirconyle, bis-(cyclopentadiényl)hafnyle, bis-(1,2,3,4,5-pentaméthylcyclopentadiényl)hafnyle, bis-(hydroxy)boryle, bis-[(C₁-C₆)-alcoxy]-boryle, (C₁-C₆)-alkyl-1,3,2-dioxaborolan-2-yle, bis-[(C₁-C₆)-alkyl]-1,3,2-dioxaborolan-2-yle, tétrakis-[(C₁-C₆)-alkyl]-1,3,2-dioxaborolan-2-yle, 1,3,2-dioxaborinan-2-yle, bis-[(C₁-C₆)-alkyl]-1,3,2-dioxaborinan-2-yle, (C₁-C₆)-alkyl-1,3,2-dioxaborinan-2-yle, tris-[(C₁-C₆)-alkyl]-1,3,2-dioxaborinan-2-yle, 2,6,7-trioxa-1-boranuidabicyclo[2,2,2]octanyle, (C₁-C₆)-alkyl-2,6,7-trioxa-1-boranuidabicyclo[2,2,2]octanyle, tris-[(C₁-C₆)-alkyl]plombanyle, tris-[(C₃-C₈)-cycloalkyl]plombanyle, tris-[(C₁-C₆)-alkylcarbonyloxy]plombanyle, tris-arylplombanyle, bis-[(C₁-C₆)-alkylcarbonyloxy]-arylplombanyle, bis-[(C₁-C₆)-alkyl]-alanyle, bis-[(C₁-C₆)-cycloalkyl]-alanyle, dichloroalanyle, chloromagnésyle, bromomagnésyle, chlorozincyle, chlorohydrargyle, bromohydrargyle, (C₁-C₆)-alkylhydrargyle, (C₃-C₆)-cycloalkylhydrargyle, tris-[(C₁-C₆)-alkyl]silyle, (C₁-C₆)-alkyl-[bis-(C₁-C₆)-alkyl]silyle, (C₁-C₆)-alkylbis-(aryl)silyle, arylbis-[(C₁-C₆)-alkyl)]silyle, (C₃-C₇)-cycloalkylbis-[(C₁-C₆)-alkyl]silyle.

17. Composés de formule (III) selon la revendication 16, dans laquelle
R¹ représente hydrogène, (C₁-C₆)-alkyle, aryle, hétéroaryle, hétérocyclyle, (C₃-C₆)-cycloalkyle, (C₂-C₆)-alcényle, (C₂-C₆)-alcynyle, (C₂-C₆)-alcynyl-(C₁-C₆)-alkyle, (C₁-C₆)-alcoxy-(C₁-C₆)-alkyle, hydroxy-(C₁-C₆)-alkyle, (C₁-C₆)-halogénoalkyle, (C₂-C₆)-halogénoalcényle, (C₁-C₆)-halogénoalcoxy-(C₁-C₆)-alkyle, (C₁-C₆)-alkylthio-(C₁-C₆)-alkyle, aryl-(C₁-C₆)-alkyle, hétérocyclyl-(C₁-C₆)-alkyle, (C₃-C₆)-halogénocycloalkyle, (C₄-C₆)-cycloalcényle, (C₁-C₆)-alcoxy-(C₁-C₆)-alcoxy-(C₁-C₆)-alkyle, (C₁-C₈)-alcoxy-(C₁-C₈)-halogénoalkyle, (C₁-C₈)-halogénoalcoxy-(C₁-C₈)-halogénoalkyle,
R² représente hydrogène, (C₁-C₆)-alkyle, (C₁-C₆)-alcoxy-(C₁-C₆)-alkyle, (C₁-C₆)-alcoxy-(C₁-C₆)-alcoxy-(C₁-C₆)-alkyle, (C₂-C₆)-alcényle, (C₂-C₆)-alcynyle, (C₁-C₆)-alkylcarbonyle, arylcarbonyle, hétéroarylcarbonyle, (C₃-C₆)-cycloalkylcarbonyle, (C₂-C₆)-alcénylcarbonyle, hétérocyclylcarbonyle, (C₁-C₆)-alcoxycarbonyle, (C₂-C₆)-alcényloxycarbonyle, aryloxy-(C₁-C₆)-alkyle, aryl-(C₁-C₆)-alcoxycarbonyle, (C₃-C₆)-cycloalcoxycarbonyle, (C₃-C₆)-cycloalkyl-(C₁-C₆)-alcoxycarbonyle, aryl-(C₁-C₆)-alcoxy-(C₁-C₆)-alkyle, aryl-(C₁-C₆)-alkyle, (C₁-C₆)-alcoxy-(C₁-C₆)-alcoxy-(C₁-C₆)-alkyle, (C₁-C₆)-alkylthio-(C₁-C₆)-alkyle, tris[(C₁-C₆)-alkyl]silyle, (C₁-C₆)-alkyl-bis-[(C₁-C₆)-alkyl]silyle, (C₁-C₆)-alkyl-bis(aryl)silyle, aryl-bis[(C₁-C₆)-alkyl]silyle, (C₃-C₆)-cycloalkyl-bis[(C₁-C₆)-alkyl]silyle, halogéno-bis[(C₁-C₆)-alkyl]silyle, tris[(C₁-C₆)-alkyl]silyl-(C₁-C₆)-alcoxy-(C₁-C₆)-alkyle, tris[(C₁-C₆)-alkyl]silyl-(C₁-C₆)-alkyle,
A¹, A², V, W représentent, indépendamment les uns des autres, un groupe CR³R⁴, un groupe N-R⁵, oxygène ou soufre, au maximum 2 atomes d'oxygène ou 2 atomes de soufre étant à chaque fois présents dans le cycle formé par les groupes A¹, A², V, W et l'atome de carbone auquel ils sont liés et les différents atomes d'oxygène et de soufre n'étant pas adjacents,
m représente 0, 1, 2,
n représente 0, 1, 2,
R³ et R⁴ représentent, indépendamment l'un de l'autre hydrogène, (C₁-C₆)-alkyle, halogène, (C₃-C₆)-cycloalkyle, (C₁-C₆)-alcoxy, aryle, hétérocyclyle, hétéroaryle, aryl-(C₁-C₆)-alkyle, (C₁-C₆)-alkylthio, (C₁-C₆)-halogénoalkyle, (C₁-C₆)-halogénoalkyloxy, (C₁-C₆)-halogénoalkylthio, (C₁-C₆)-alcoxy-(C₁-C₆)-alkyle, (C₁-C₆)-alkylthio-(C₁-C₆)-alkyle, hétéroaryl-(C₁-C₆)-alkyle, hétérocyclyl-(C₁-C₆)-alkyle, (C₁-C₆)-alcoxy-(C₁-C₆)-halogénoalkyle, (C₁-C₆)-halogénoalcoxy-(C₁-C₆)-halogénoalkyle, (C₂-C₆)-alcényle, (C₂-C₆)-alcynyle ou
R³ et R⁴ forment, ensemble avec l'atome auquel ils sont liés, un cycle de 3 à 6 chaînons complètement saturé, le cas échéant interrompu par des hétéroatomes et le cas échéant substitué davantage et
[M] représente tris-[(C₁-C₆)-alkyl]stannyle, tris-[(C₃-C₆)-cycloalkyl]stannyle, tris-[(C₁-C₆)-alkyl]germanyle, tris-[(C₃-C₆)-cycloalkyl]germanyle, bis-(cyclopentadiényl)zirconyle, bis-(1,2,3,4,5-pentaméthylcyclopentadiényl)zirconyle, bis-(cyclopentadiényl)hafnyle, bis-(1,2,3,4,5-pentaméthylcyclopentadiényl)hafnyle, bis-(hydroxy)boryle, bis-[(C₁-C₆)-alcoxy]-boryle, (C₁-C₆)-alkyl-1,3,2-dioxaborolan-2-yle, bis-[(C₁-C₆)-alkyl]-1,3,2-dioxaborolan-2-yle, tétrakis-[(C₁-C₆)-alkyl]-1,3,2-dioxaborolan-2-yle, 1,3,2-dioxaborinan-2-yle, bis-[(C₁-C₆)-alkyl]-1,3,2-dioxaborinan-2-yle, (C₁-C₆)-alkyl-1,3,2-dioxaborinan-2-yle, tris-[(C₁-C₆)-alkyl]-1,3,2-dioxaborinan-2-yle, 2,6,7-trioxa-1-boranuidabicyclo[2,2,2]octanyle, (C₁-C₆)-alkyl-2,6,7-trioxa-1-boranuidabicyclo[2,2,2]octanyle, tris-[(C₁-C₆)-alkyl]plombanyle, tris-[(C₃-C₆)-cycloalkyl]plombanyle, tris-[(C₁-C₆)-alkylcarbonyloxy]plombanyle, tris-arylplombanyle, bis-[(C₁-C₆)-alkylcarbonyloxy]-arylplombanyle, bis-[(C₁-C₆)-alkyl]-alanyle, bis-[(C₁-C₆)-cycloalkyl]-alanyle, dichloroalanyle, chloromagnésyle, bromomagnésyle, chlorozincyle, chlorohydrargyle, bromohydrargyle, (C₁-C₆)-alkylhydrargyle, (C₃-C₆)-cycloalkylhydrargyle, tris-[(C₁-C₆)-alkyl]silyle, (C₁-C₆)-alkyl-[bis-(C₁-C₆)-alkyl]silyle, (C₁-C₆)-alkylbis-(aryl)silyle, arylbis-[(C₁-C₆)-alkyl)]silyle, (C₃-C₇)-cycloalkylbis-[(C₁-C₆)-alkyl]silyle.
